# EUROPEAN PATENT SPECIFICATION

(11) **EP 1 539 802 B1**
(45) Date of publication and mention of the grant of the patent: **05.09.2007**
(21) Application number: 03760708.2
(22) Date of filing: 20.06.2003
(51) Int. Cl.: C07K 7/00, C07K 14/705, C12N 15/12, A61K 48/00, C07K 16/28, G01N 33/566, A61K 38/17, A61P 25/00

(54) **NOVEL KCNQ POLYPEPTIDES AND THEIR USES IN THE DIAGNOSIS OF MENTAL DISORDERS**
NEUE KCNQ-POLYPEPTIDE UND DEREN VERWENDUNG BEI DER DIAGNOSE VON GEISTESKRANKHEITEN
POLYPEPTIDES KCNQ ET LEURS UTILISATIONS DANS LE DIAGNOSTIC DE TROUBLES MENTAUX

(30) Priority: 25.06.2002 US 391359 P
(43) Date of publication of application: 15.06.2005
(73) Proprietor: Serono Genetics Institute S.A., 91030 Evry Cedex (FR)
(72) Inventor: CAVAREC, Laurent, 94300 Vincennes (FR); CHUMAKOV, Ilya, F-77000 Vaux-le-Penil (FR); DESTENAVES, Benoit, F-91800 Brunoy (FR); GONTHIER, Catherine, F-91100 Corbeil-Essonnes (FR); ELIAS, Isabelle, F-78120 Sonchamp (FR)
(74) Representative: Merck Serono International S.A. Intellectual Property
(86) International application number: PCT/EP2003/050246
(87) International publication number: WO 2004/000875

(56) References cited:
- WO-A-01/09612
- WO-A-02/12279
- WO-A-99/31232
- WO-A-03/019186
- US-B1- 6 372 767
- TINEL N ET AL: "The KCNQ2 potassium channel: splice variants, functional and developmental expression. Brain localization and comparison with KCNQ3" FEBS LETTERS, ELSEVIER SCIENCE PUBLISHERS, AMSTERDAM, NL, vol. 438, no. 3, 6 November 1998 (1998-11-06), pages 171-176, XP004258585 ISSN: 0014-5793
- SMITH JEFFREY S ET AL: "Differential expression of KCNQ2 splice variants: Implications to M current function during neuronal development" JOURNAL OF NEUROSCIENCE, vol. 21, no. 4, 15 February 2001 (2001-02-15), pages 1096-1103, XP002265007 ISSN: 0270-6474

## Description

### FIELD OF THE INVENTION

This invention is in the field of mental disorders such as bipolar disorder, schizophrenia, depression and other mood disorders. More specifically, this invention relates to three novel potassium channels subunits, KCNQ2-15bx, KCNQ2-15by and KCNQ2-15bz. The invention also relates the use of potassium channels comprising KCNQ2 subunits for screening for modulators, and to the use of said modulators for treating said mental disorders. The invention further relates to the use of biallelic markers located in the KCNQ2 gene for diagnosing said mental disorders.

### BACKGROUND

### 1. KCNQ potassium channels

Malfunction in ion channels, due to mutatio ns in genes encoding channel proteins or the presence of autoantibodies, are increasingly being implicated in causing disease conditions, termed channelopathies. For instance, dysfunction of potassium channels has been associated with the pathophysiology of a number of neurological disorders both affecting the central and peripheral nervous system (e.g., episodic ataxia, epilepsy, neuromyotonia, Parkinson's disease, congenital deafness, long QT syndrome). Potassium channels, which demonstrate a high degree of diversity and ubiquity, are fundamental in the control of membrane depolarisation and cell excitability. A common feature of potassium channelopathies is a reduction or loss of membrane potential repolarisation. Marketed potassium channels openers include for example flupirtine, an analgesic drug used for treating pain.

KCNQ polypeptides belong to the potassium channel family. KCNQ polypeptides associate to form homomeric or heteromeric potassium channels, each polypeptide corresponding to a subunit of the channel. Currently, five different members of the KCNQ family are known: KCNQ1, KCNQ2, KCNQ3, KCNQ4 and KCNQ5. Heteromeric KCNQ potassium channels can be comprised either of different members of the KCNQ family, or of KCNQ polypeptides associated with other members of the potassium channel family. KCNQ potassium channels underlie the M-current, an important regulator of neuronal excitability. Both their amino-terminal and their carboxyl-terminal extremities are located on the intracellular side of the membrane. These extremities play an important role both in interactions with other proteins and in modulation of the channel's activity.

KCNQ1 is expressed in heart, cochlea, intestine and kidney. It assembles with either the product of the KCNE1 gene or with the product of the KCNE3 gene. Mutations in the KCNQ1 gene have been shown to cause one form of inherited long QT syndrome and a form of deafness.

KCNQ2 was first cloned in 1996. In 1998, geneticists discovered that an inherited form of juvenile epilepsy, the benign familial neonatal convulsions, is caused by mutations in the potassium channel KCNQ2 (Singh et al. Nat Genet, 1998, 18:25-9; Biervert et al., Science, 1998, 279:403-6). More specifically, Bievert et al. showed that a five-base pair insertion deleting more than 300 amino acids from the carboxyl-terminus of KCNQ2 leads to impairment of potassium-selective currents *in vitro.* It was thus demonstrated that loss of function mutations in KCNQ2 causes the epileptic syndrome. Wang et al. showed KCNQ2 to be expressed in brain, and to be associated with KCNQ3. In addition, they showed that the KCNQ2/3 heteromultimers underlie the M-current (Wang et al., Science, 1998, 282:1890-3). In 2000, Main et al. showed that KCNQ2 is the molecular target of retigab ine, a potent anticonvulsant compound, and that retigabine acts as a KCNQ2/3 potassium channel opener (Mol Pharmacol, 2000, 58:253-62). Biervert et al. determined that the KCNQ2 gene has at least 18 exons, occupying more than 50 kb of genomic DNA (Genet., 1999, 104:234-240). Until now six different isoforms of KCNQ2 produced by alternative splicing have been described (see, e.g., SwissProt Accession No. 043526).

KCNQ4 is expressed in inner ear, and it has been shown that mutation in the KCNQ4 gene lead to a form of inherited deafness.

KCNQ5 is expressed in brain and skeletal muscle, and can co-assemble with KCNQ3, suggesting that it may also play a role in the M-current heterogeneity. It has been suggested that KCNQ5 deficiency leads to retinal degeneration.

The activity of KCNQ channels has been shown to be modulated by Protein kinase A (PKA) and by the c-Src tyrosine kinase (Src). Schroeder et al. showed that currents generated by heteromeric KCNQ2/KCNQ3 channels can be increased by intracellular cyclic AMP, and that this effect is mediated by PKA. PKA stimulated current intensity by 66% (Schroeder et al., Epilepsia (2000) 41:1068-1069). Gamper et al. showed that coexpression of Src with KCNQ2/KCNQ3 heteromeric channels resulted in a 4.5-fold reduction of current density and a 2-fold slowing of activation kinetics at 0 mV. However, Src had no effect on currents generated by KCNQ2 homomultimeric channels (J Neurosci. (2003) 23:84-95). In view of these results, modulation of KCNQ channels by kinases and phos phatases is believed to be important for control of neuronal excitability.

Studying KCNQ channels in humans and animal models is of great importance for the understanding of how M-channels control excitability at the cellular, network, and behavioral levels. A better understanding of the physiological role of KCNQ channels is a promising way of finding of new targets for novel diseases, thus leading to the possibility of novel screenings of drug candidates.

### 2. The PP2A phosphatase

The PP2A phosphatase is an intracellular serine/threonine protein phosphatase constituted by two or three subunits. PP2A phosphatases comprise of a catalytic subunit (PP2A/C), a scaffolding subunit (PP2A/A) and eventually a regulatory subunit (PP2A/B).

Regulatory subunits are thought to confer tissue specificity, subcellular localization and developmental regulation to PP2A. More than eleven ndifferent regulatory subunits are currently known, and PP2A/Bγ is one of them. PP2A/Bγ is encoded by the PPP2R2C gene that was mapped to human chromosome 4p16 between markers D4S2925 and D4S3007 (Hu et al., Genomics., 2000, 67:83-6). The PP2A/Bγ protein can only be detected in brain and is enriched in the cytosolic fraction of the cell. Furthermore, PPP2R2C is developmentally regulated.

### 3. Mental disorders

Mental disorders encompass a wide range of CNS disorders. Mental disorders include, e.g., mood disorders, psychotic disorders, anxiety disorders, childhood disorders, eating disorders and personality disorders, all these terms being defi ned according to the DSM-IV classification (Diagnosis and Statistical Manual of Mental Disorders, Fourth Edition, American Psychiatric Association, Washington D.C., 1994). Mood Disorders encompass bipolar I disorder (mania with or without major depression), bipolar II disorder (hypomania with major depression), cyclothymic disorder (numerous brief episodes of hypomania and minor depression), dysthymic disorder (prolonged minor depression without mania/hypomania) and major depressive disorder (major depression without mania). Psychotic disorders encompass schizophrenia, schizoaffective disorder, schizophreniform disorder, brief psychotic disorder, delusional disorder and shared psychotic disorder. Bipolar disorder, schizophrenia and depression are three particularly serious and widespread mental disorders.

### 3.1. Bipolar Disorder

Bipolar disorders are relatively common disorders, occurring in about 1.3% of the population, and have been reported to constitute about half of the mood disorders seen in psychiatric clinics with severe and potentially disabling effects. Bipolar disorders have been found to vary with gender depending of the type of disorder; for example, bipolar disorder I is found equally among men and women, while bipolar disorder 11 is reportedly more common in women. The age of onset of bipolar disorders is typically in the teenage years and diagnosis is typically made in the patient's early twenties. Bipolar disorders also occur among the elderly, generally as a result of a neurological disorder or other medical conditions. In addition to the severe effects on patients' social development, suicide completion rates among bipolar patients are reported to be about 15%.

Bipolar disorders are characterized by phases of excitement and often depression; the excitement phases, referred to as mania or hypomania, and depressive phases can alternate or occur in various admixtures, and can occur to different degrees of severity and over varying duration. Since bipolar disorders can exist in different forms and display different symptoms, the classification of bipolar disorder has been the subject of extensive studies resulting in the definition of bipolar disorder subtypes and widening of the overall concept to include patients previously thought to be suffering from different disorders. Bipolar disorders often share certain clinical signs, symptoms, treatments and neurobiological features with psychotic illnesses in general and therefore present a challenge to the psychiatrist to make an accurate diagnosis. Furthermore, because the course of bipolar disorders and various mood and psychotic disorders can differ greatly, it is critical to characterize the illness as early as possible in order to offer means to manage the illness over a long term.

The mania associated with the disease impairs performance and causes psychosis, and often results in hospitalization. This disease places a heavy burden on the patient's family and relatives, both in terms of the direct and indirect costs involved and the social stigma associated with the illness, sometimes over generations. Such stigma often leads to isolation and neglect. Furthermore, the earlier the onset, the more severe are the effects of interrupted education and social development.

The DSM-IV classification of bipolar disorder distinguishes among four types of disorders based on the degree and duration of mania or hypomania as well as two types of disorders which are evident typically with medical conditions or their treatments, or to substance abuse. Mania is recognized by elevated, expansive or irritable mood as well as by distractability, impulsive behavior, increased activity, grandiosity, elation, racing thoughts, and pressured speech. Of the four types of bipolar disorder characterized by the particular degree and duration of mania, DSM-IV includes:
- bipolar disorder I, including patients displaying mania for at least one week;
- bipolar disorder II, including patients displaying hypomania for at least 4 days, characterized by milder symptoms of excitement than mania, who have not previously displayed mania, and have previously suffered from episodes of major depression;
- bipolar disorder not otherwise specified (NOS), including patients otherwise displaying features of bipolar disorder II but not meeting the 4 day duration for the excitement phase, or who display hypomania without an episode of major depression; and
- cyclothymia, including patients who show numerous manic and depressive symptoms that do not meet the criteria for hypomania or major depression, but which are displayed for over two years without a symptom-free interval of more than two months.

The remaining two types of bipolar disorder as classified in DSM-VI are disorders evident or caused by various medical disorder and their treatments, and disorders involving or related to substance abuse. Medical disorders which can cause bipolar disorders typically include endocrine disorders and cerebrovascular injuries, and medical treatments causing bipolar disorder are known to include glucocorticoids and the abuse of stimulants. The disorder associated with the use or abuse of a substance is referred to as "substance induced mood disorder with manic or mixed features".

Evidence from twin and adoption studies, and the lack of variation in incidence worldwide, indicate that bipolar disorder is primarily a genetic condition, although environmental risk factors are also involved at some level as necessary, sufficient, or interactive causes. Aggregation of bipolar disorder and schizophrenia in families suggests that these two distinct disorders share some common genetic susceptibility. Several linkage studies of bipolar disorder have been reported, and several susceptibility regions have been identified. The regions that are associated with bipolar disorder include 1q31-q32, 4p16, 7q31, 12q23-q24, 13q32, 18p11.2, 21q22 and 22q11-q13 (Detera-Wadleigh et al. (1999) Proc Natl Acad Sci USA A96(10):5604-9). Some of these regions, like 4p16, 12q24, 18p11, 21q21 and 22q11 have been repeatedly implicated by independent investigators. Furthermore, some regions that are linked to bipolar disorder such as, e.g., 13q32 and 18p11.2, are also implicated in genome scans of schizophrenia, confirming that these two distinct disorders share some common genetic susceptibility. However, the genes underlying bipolar disorder and/or schizophrenia have not yet been identified.

### 3.2. Schizophrenia

There are an estimated 45 million people with schizophrenia in the world, with more than 33 million of them in the developing countries. In developed countries schizophrenia occurs in approximately 1% of the adult population at some point during their lives. If there is one grandparent with schizophrenia, the risk of getting the illness increases to about 3%; one parent with Schizophrenia, to about 10%. When both parents have schizophrenia, the risk rises to approximately 40%. Most schizophrenia patients are never able to work. Standardized mortality ratios (SMRs) for schizophrenic patients are estimate d to be two to four times higher than the general population and their life expectancy overall is 20 % shorter than for the general population. The most common cause of death among schizophrenic patients is suicide (in 10% of patients) which represents a 2 0 times higher risk than for the general population. Deaths from heart disease and from diseases of the respiratory and digestive system are also increased among schizophrenic patients.

Schizophrenia comprises a group of psychoses with either 'positive' or 'negative' symptoms. Positive symptoms consist of hallucinations, delusions and disorders of thought; negative symptoms include emotional flattening, lack of volition and a decrease in motor activity.

A number of biochemical abnormalities have been identified and, in consequence, several neurotransmitter based hypotheses have been advanced over recent years; the most popular one has been "the dopamine hypothesis," one variant of which states that there is over-activity of the mesolimbic dopamine pathways at the level of the D₂ receptor. However, researchers have been unable to consistently find an association between various receptors of the dopaminergic system and schizophrenia.

### 3.3. Depression

Depression is a serious medical illness that affects 340 million people worldwide. In contrast to the normal emotional experiences of sadness, loss, or passing mood states, clinical depression is persistent and can interfere significantly with an individual's ability to function. As a result, depression is the leading cause of disability throughout the world.

Symptoms of depression include depressed mood, diminished interest or pleasure in activities, change in appetite or weight, insomnia or hypersomnia, psycho-motor agitation or retardation, fatigue or loss of energy, feelings of worthlessness or excessive guilt, anxiety, inability to concentrate or act decisively, and recurrent thoughts of death or suicide. A diagnosis of unipolar major depression (or major depressive disorder) is made if a person has five or more of these symptoms and impairment in usual functioning nearly every day during the same two-week period. The onset of depression generally begins in late adolescence or early adult life; however, recent evidence suggests depression may be occurring earlier in life in people born in the past thirty years.

The World Health Organization predicts that by the year 2020 depression will be the greatest burden of ill-health to people in the developing world, and that by then depression will be the second largest cause of death and disability. Beyond the almost unbearable misery it causes, the big risk in major depression is suicide. Within five years of suffering a major depression, an estimated 25% of sufferers try to kill themselves. In addition, depression is a frequent and serious complication of heart attack, stroke, diabetes, and cancer. According to one recent study that covered a 13-year period, individuals with a history of major depression were four times as likely to suffer a heart attack compared to people without such a history. Depression may also be a feature in up to 50% of patients with mental disorders such as Parkinson's disease and Alzheimer's disease.

### 3.4. Treatment

There are currently no cures for mental disorders such as bipolar disorder, schizophrenia, depression and other mood disorders, so the objective of treatment is to reduce the severity of the symptoms, if possible to the point of remission. Due to the similarities in symptoms, schizophrenia, depression and bipolar disorder are often treated with some of the same medicaments.

### 3.4.1. Treatment of bipolar disorder

Depressive episodes may be treated like depression. However, most antidepressants can cause swings from depression to hypomania or mania and sometimes cause rapid cycling between them. Therefore, these drugs are used for only short periods, and their effect on mood is closely monitored. At the first sign of a swing to hypomania or mania, the antidepressant is stopped. Most people with manic-depressive disorder are given drugs with a mood-stabilizing effect such as lithium, carbamazepine and divalproex.

Lithium has no effect on normal mood but reduces the tendency toward mood swings in about 70% of the people with manic-depressive illness. A doctor monitors the level of lithium in the blood with blood tests. Possible adverse effects of lithium include tremor, muscle twitching, nausea, vomiting, diarrhea, thirst, excessive urination, and weight gain. Lithium can make acne or psoriasis worse, can cause the blood levels of thyroid hormone to fall, and rarely can cause excessive urination. A very high level of lithium in the blood can cause a persistent headache, mental confusion, drowsiness, seizures, and abnormal heart rhythms. Adverse effects are more likely to occur in the elderly. Women who are trying to become pregnant must stop taking lithium, because lithium may cause heart defects in a developing fetus.

Newer drug treatments have evolved over the past several years. These include the carbamazepine and divalproex. However, carbamazepine can seriously reduce the number of red and white blood cells, and divalproex can cause liver damage (primarily in children). With careful monitoring by a doctor, these problems are rare, and carbamazepine and divalproex are useful alternatives to lithium, especially for people with the mixed or rapid cycling form of manic-depressive illness who haven't responded to other treatments.

### 3.4.2. Treatment of schizophrenia

For schizophrenia, antipsychotic medications are the most com mon and most valuable treatments. There are four main classes of antipsychotic drugs which are commonly prescribed for schizophrenia. The first, neuroleptics, exemplified by chlorpromazine (Thorazine), has revolutionized the treatment of schizophrenic pa tients by reducing positive (psychotic) symptoms and preventing their recurrence. Patients receiving chlorpromazine have been able to leave mental hospitals and live in community programs or their own homes. But these drugs are far from ideal. Some 20% to 30% of patients do not respond to them at all, and others eventually relapse. These drugs were named neuroleptics because they produce serious neurological side effects, including rigidity and tremors in the arms and legs, muscle spasms, abnormal body movements, and akathisia (restless pacing and fidgeting). These side effects are so troublesome that many patients simply refuse to take the drugs. Besides, neuroleptics do not improve the so-called negative symptoms of schizophrenia and the side effects may even exacerbate these symptoms. Thus, despite the clear beneficial effects of neuroleptics, even some patients who have a good short -term response will ultimately deteriorate in overall functioning.

The well known deficiencies in the standard neuroleptics have stimulated a search for new treatments and have led to a new class of drugs termed atypical neuroleptics. The first atypical neuroleptic, Clozapine, is effective for about one third of patients who do not respond to standard neuroleptics. It seems to reduce negative as well as positive symptoms, or at least exacerbates negative symptoms less than standard neuroleptics do. Moreover, it has beneficial effects on overall functioning and may reduce the chance of suicide in schizophrenic patients. It does not produce the troubling neurological symptoms of the standard neuroleptics, or raise blood levels of the hormone prolactin, excess of which may cause menstrual irregularities and infertility in women, impotence or breast enlargement in men. Many patients who cannot tolerate standard neuroleptics have been able to take clozapine. However, clozapine has serious limitations. It was originally withdrawn from the market because it can cause agranulocytosis, a potentially lethal inability to prod uce white blood cells. Agranulocytosis remains a threat that requires careful monitoring and periodic blood tests. Clozapine can also cause seizures and other disturbing side effects (e.g., drowsiness, lowered blood pressure, drooling, bed-wetting, and weight gain). Thus only patients who do not respond to other drugs usually take Clozapine.

Researchers have developed a third class of antipsychotic drugs that have the virtues of clozapine without its defects. One of these drugs is risperidone (Risperdal). Early studies suggest that it is as effective as standard neuroleptic drugs for positive symptoms and may be somewhat more effective for negative symptoms. It produces more neurological side effects than clozapine but fewer than standard neuroleptics. However, it raises prolactin levels. Risperidone is now prescribed for a broad range of psychotic patients, and many clinicians seem to use it before clozapine for patients who do not respond to standard drugs, because they regard it as safer. Another new drug is Olanzapine (Zyprexa) which is at least as effective as standard drugs for positive symptoms and more effective for negative symptoms. It has few neurological side effects at ordinary clinical doses, and it does not significantly raise prolactin levels. Although it does not produce most of clozapine's most troubling side effects, including agranulocytosis, some patients taking olanzapine may become sedated or dizzy, develop dry mouth, or gain weight. In rare cases, liver function tests become transiently abnormal.

### 3.4.3. Treatment of depression

Several types of antidepressants are available. These antidepressants belong to four main categories: tricyclic antidepressants, selective serotonin reuptake inhibitors, monoamine oxidase inhibitors and psychostimulants. Tricyclic antidepressants include, e.g., Amitriptyline, Amoxapine, Bupropion, Clomipramine, Desipramine, Doxepin, lmipramine, Maprotiline, Nefazodone, Nortriptyline, Protriptyline, Trazodone, Trimipramine and Venlafaxine. Selective serotonin reuptake inhibitors include, e.g., Fluoxetine, Fluvoxamine, Paroxetine and Sertraline, Monoamine oxidase inhibitors include, e.g., Isocarboxazid, Pargyline, Phenelzine and Tranylcypromine. Psychostimulants include, e.g., Dextroamphetamine and Methylphenidate.

All these antidepressants must be taken regularly for at least several weeks before they begin to work. The chances that any given antidepressant will work for a particular person are about 65%. However, most of these drugs have side effects varying with each type of drug. For example, the tricyclic antidepressants often cause sedation and lead to weight gain. They can also be associated with side effects such as an increased heart rate, a decrease in blood pressure when the person stands or bl urred vision.

Thus, for mental disorders such as bipolar disorder, schizophrenia, depression and other mood disorders, known molecules used for the treatment have side effects and act only against the symptoms of the disease. Consequently, there is a strong need for new molecules without associated side effects that are specifically directed against targets which are involved in the causal mechanisms of such mental disorders. Therefore, there is a need to identify proteins involved in bipolar disorder and schizophrenia. Providing new targets involved in bipolar disorder and schizophrenia will allow new screenings for drugs, resulting in new drugs that are efficient in treatment of these serious mental disorders.

Furthermore, there is also a need for diagnostic tools. There is increasing evidence that leaving schizophrenia untreated for long periods early in course of the illness may negatively affect the outcome. However, the use of drugs is often delayed for patients experiencing a first episode of the illness. The patients may not realize that they are ill, or they may be afraid to seek help; family members sometimes hope the problem will simply disappear or cannot persuade the patient to seek treatment; clinicians may hesitate to prescribe antipsychotic medications when the diagnosis is uncertain because of potential side effects. Indeed, at the first manifestation of the disease, schizophrenia or bipolar disorder is difficult to distinguish from, e.g., drug-related disorders and stress-related disorders. Accordingly, there is a need for new methods for detecting a susceptibility to mental disorders such as bipolar disorder, schizophrenia, and depression.

### SUMMARY OF THE INVENTION

The present invention is based on the identification of novel splice variants of the KCNQ2 potassium channel.

Therefore, in a first aspect, the present invention is directed to an isolated KCNQ2 - 15b polypeptide selected from the group consisting of:
a) a polypeptide comprising a span of at least twenty amino acids of amino acids 589 to 643 of SEQ ID NO: 2;
b) a polypeptide comprising amino acids 589 to 643 of SEQ ID NO: 2;
c) a polypeptide comprising amino acids 545 to 643 of SEQ ID NO: 2;
d) a polypeptide comprising SEQ ID NO: 2;
e) a polypeptide comprising SEQ ID NO: 4;
f) a polypeptide comprising SEQ ID NO: 6;
g) a polypeptide comprising a mutein of any of (d) to (f), wherein the amino acid sequence has at least 95%, 96%, 97%, 98%, or 99% identity to at least one of the sequences in (d) to (f);
h) a mutein of any of (b) to (f) wherein any changes in the amino acid sequence are conservative amino acid substitutions to the amino acid sequences in (b) to (f).

The present invention further relates to a potassium channel comprising at least one KCNQ2-15b polypeptide.

The invention further relates to a purified KCNQ2-15b polynucleotide encoding a KCNQ2-15b polypeptide or a polynucleotide complementary thereto.

An expression vector comprising a KCNQ2-15b polynucleotides, a host cell comprising an expression vector comprising a KCNQ2-15b polynucleotides and an antibody that specifically binds to a KCNQ2-15b polypeptide are also within the present invention.

Further, the present invention pertains to a method of making a polypeptide, said method comprising the steps of culturing a host cell comprising an expression vector comprising a KCNQ2-15b polynucleotides under conditions suitable for the production of a KCNQ2-15b polypeptide within said host cell.

The present invention is further based on the finding that KCNQ2 -15b is associated with the onset and the development of mental disorders.

Therefore, in a second aspect, the present invention is directed to the use of a KCNQ2-15b polypeptide as a target for screening candidate modulators.

In the frame of the present invention, biallelic markers located in the KCNQ2 -15b gene have been identified and validated.

Therefore, a third object of the invention consists of the u se of at least one CNQ2 -15b related biallelic marker for diagnosing whether an individual suffers from or is at risk of suffering from a mental disorder.

The invention further encompasses the use of at least one KCNQ2-15b-related biallelic marker for determining whether there is a significant association between said marker and a mental disorder.

The invention also relates to a method of genotyping comprising the step of determining the identity of a nucleotide at a KCNQ2-15b-related biallelic marker or the complement thereof in a biological sample.

The invention further pertains to a method of diagnosing a mental disorder in an individual comprising the step of genotyping at least one KCNQ2-15b-related biallelic marker using a method of genotyping comprising the step of determining the identity of a nucleotide at said KCNQ2-15b-related biallelic marker or the complement thereof in a biological sample.

### BRIEF DESCRIPTION OF THE FIGURES

Figure 1A and 1B show an alignment between the full-length KCNQ2 polypeptide (KCNQ2-fl, SEQ ID NO: 7), KCNQ2-15bx (SEQ ID NO: 2), KCNQ2-15by (SEQ ID NO: 4) and KCNQ2-15bz (SEQ ID NO: 6). The box highlights the amino acids that are unique to KCNQ2-15bx, KCNQ2-15by and KCNQ2-15bz compared to KCNQ2-fl.
Figure 2 shows a sheme of the structure of the KCNQ2-15bx, KCNQ2-15by and KCNQ2-15bz cDNAs.
Figure 3 shows the results of a mating test between PP2A/Bγ and different KCNQ2 polypeptides, as described in detail in Example 3.
Figure 4 shows the results of a mating test between different KCNQ2 polypeptides, as described in detail in Example 3.
Figure 5 compares the intensity of the currents generated by homotetrameric potassium channels comprised of KCNQ2-15bx, KCNQ2-15by, KCNQ2-15bz or KCNQ2-fl subunits respectively.
Figure 6A shows the voltage clamp traces of the current generated by a homotetrameric potassium channels comprised of KCNQ2-15bx subunits.
Figure 6B shows the voltage clamp traces of the current generated by a homotetrameric potassium channels comprised of KCNQ2-15by subunits.

### BRIEF DESCRIPTION OF THE SEQUENCES OF THE SEQUENCE LISTING

SEQ ID NO: 1 corresponds to a polynucleotide consisting of the CDS of KCNQ2-15bx
SEQ ID NO: 2 corresponds to the KCNQ2-15bx polypeptide.
SEQ ID NO: 3 corresponds to a polynucleotide consisting of the CDS of KCNQ2 -15by
SEQ ID NO: 4 corresponds to the KCNQ2-15by polypeptide.
SEQ ID NO: 5 corresponds to a polynucleotide consisting of the CDS of KCNQ2-15bz
SEQ ID NO: 6 corresponds to the KCNQ2-15bz polypeptide.
SEQ ID NO: 7 corresponds to the KCNQ2-fl polypeptide.
SEQ ID Nos. 8 to 36 correspond to primers and probes used in Examples 1 to 4.
SEQ ID NO: 37 corresponds to the PPP2R2C gene which encodes the PP2A/Bγ subunit, on which PP2A/Bγ-related biallelic markers are indicated.
SEQ ID NO: 38 corresponds to the PP2A/Bγ subunit.
SEQ ID Nos. 39 to 41 correspond to primers used for microsequencing some of the PP2A/Bγ-related biallelic markers.
SEQ ID Nos. 42 to 47 correspond to regions of the KCNQ2 gene, on which KCNQ2 -related biallelic markers are indicated.

### BRIEF DESCRIPTION OF THE TABLES

Table 1 presents the structure of KCNQ2-fl, KCNQ2-15bx KCNQ2-15by and KCNQ2-15bz.
Tables 2A and 2B present the location of the primers used for amplification of genomic DNA by PCR in PPP2R2C and in the KCNQ2 gene respectively
Table 3A and 3B present biallelic markers located in the PP2R2C and in the KCNQ2 gene respectively.
Tables 4A and 4B present the the primers used for microsequencing biallelic markers located in PP2R2C and in the KCNQ2 gene respectively.
Tables 5A and 5B present the p-values for biallelic markers located in PPP2R2C and in the KCNQ2 gene respectively.
Tables 6A and 6B present the genotypic odds ratios for a biallelic marker located in PPP2R2C and in the KCNQ2 gene respectively.
Tables 7A and 7B present the risk haplotypes for two sets of biallelic markers located in PPP2R2C

### DETAILED DESCRIPTION OF THE INVENTION

The present invention stems from the cloning and the sequencing of three novel splice variants of the KCNQ2 gene, KCNQ2-15bx, KCNQ2-15by and KCNQ2-15bz. These splice variants all display a novel exon (exon 15b), corresponding to amino acids 545 to 643 of SEQ ID NO: 2. Data showing that KCNQ2-15bx and KCNQ2-15by can assemble as functional homotetrameric potassium channels are provided. In the frame of the present invention, it has been demonstrated that these novel splice variants interact with the Bγ subunit of the serine/threonine protein phosphatase 2A (PP2A/Bγ) both *in vitro* and *in vivo.* Furthermore, association studies are described in example 15, and it was shown that both the KCNQ2 gene and the gene coding for PP2A/Bγ are strongly associated with bipolar disorder. Novel validated biallelic markers located in the KCNQ2 gene and associated with bipolar disorder are provided. In the frame of the present invention it was further shown that KCNQ2-15bx, KCNQ2-15by and KCNQ2-15bz are (i) dephosphorylated by PP2A; and (ii) phosphorylated by the PKA and GSK3β kinases. Moreover, the phosphorylation of KCNQ2-15bx, KCNQ2-15by and KCNQ2-15bz is inhibited in the presence of lithium, a known mood-stabilizing agent.

Accordingly, the present invention provides novel KCNQ2 polypeptides and means to identify compounds useful in the treatment of mental disorders such as bipolar disorder, schizophrenia, depression and other mood disorders. The invention further relates to the use of KCNQ2 polypeptides as targets for screening for modulators thereof. The use of said modulators for treating mental disorders, and the use of biallelic markers located in the KCNQ2 gene for diagnosing mental disorders are further aspects of the present invention.

### 1. Definitions

The term "treat" or "treating" as used herein is meant to ameliorate, alleviate symptoms, eliminate the causation of the symptoms either on a temporary or permanent basis, or to prevent or slow the appearance of symptoms of the named disorder or condition. The term "treatment" as used herein also encompasses the term "prevention of the disorder", which is, e.g., manifested by delaying the onset of the symptoms of the disorder to a medically significant extent. Treatment of the disorder is, e.g., manifested by a dec rease in the symptoms associated with the disorder or an amelioration of the reoccurrence of the symptoms of the disorder.

The term "mental disorder" refers to diseases characterized as mood disorders, psychotic disorders, anxiety disorders, childhood disorders, eating disorders, personality disorders, adjustment disorder, autistic disorder, delirium, dementia, multi -infarct dementia and Tourette's disorder in the DSM-IV classification (Diagnosis and Statistical Manual of Mental Disorders, Fourth Edition, American Psychiatric Association, Washington D.C., 1994).

The term "schizophrenia" refers to a condition characterized as schizophrenia in the DSM-IV classification (Diagnosis and Statistical Manual of Mental Disorders, Fourth Edition, American Psychiatric Association, Washington D.C., 1994).

The term "bipolar disorder" as used herein refers to a condition characterized as a Bipolar Disorder in the DSM-IV. Bipolar disorder may be bipolar and bipolar disorder II as described in the DSM-IV. The term further includes cyclothymic disorder. Cyclothymic disorder refers to an alternation of depressive symptoms and hypomanic symptoms. The skilled artisan will recognize that there are alternative nomenclatures, posologies, and classification systems for patholog ic psychological conditions and that these systems evolve with medical scientific progress.

The terms "comprising", "consisting of", or "consisting essentially of" have distinct meanings. However, each term may be substituted for another herein to change the scope of the invention.

As used interchangeably herein, the term "oligonucleotides", and "polynucleotides" include RNA, DNA, or RNA/DNA hybrid sequences of more than one nucleotide in either single chain or duplex form. The term "nucleotide" as used herein as an adjective to describe compounds comprising RNA, DNA, or RNA/DNA hybrid sequences of any length in single-stranded or duplex form. The term "nucleotide" is also used herein as a noun to refer to individual nucleotides or varieties of nucleotides, meaning a compound, or individual unit in a larger nucleic acid compound, comprising a purine or pyrimidine, a ribose or deoxyribose sugar moiety, and a phosphate group, or phosphodiester linkage in the case of nucleotides within an oligonucleotide or polynucleotide. Although the term "nucleotide" is also used herein to encompass "modified nucleotides" which comprise at least one modifications (a) an alternative linking group, (b) an analogous form of purine, (c) an analogous form of pyrimidine, or (d) an analogous sugar, for examples of analogous linking groups, purine, pyrimidines, and sugars see for example PCT publication No. WO 95/04064. However, the polynucleotides of the invention are preferably comprised of greater than 50% conventional deoxyribose nucleotides, and most preferably greater than 90% conventional deoxyribose nucleotides. The polynucleotide sequences of the invention may be prepared by any known method, including synthetic, recombinant, ex vivo generation, or a combination thereof, as well as utilizing any purification methods known in the art.

The term "isolated" requires that the material be removed from its original environment (e.g., the natural environment if it is naturally occurring). For example, a naturally-occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

The term "primer" denotes a specific oligonucleotide sequence which is complementary to a target nucleotide sequence and used to hybridize to the target nucleotide sequence. A primer serves as an initiation point for nucleotide polymerization catalyzed by either DNA polymerase, RNA polymerase or reverse transcriptase.

The term "probe" denotes a defined nucleic acid segment (or nucleotide analog segment, e.g., polynucleotide as defined herein) which can be used to identify a specific polynucleotide sequence present in samples, said nucleic acid segment comprising a nucleotide sequence complementary of the specific polynucleotide sequence to be identified.

The terms "complementary" or "complement thereof" are used herein to refer to the sequences of polynucleotides which are capable of forming Watson & Crick base pairing with another specified polynucleotide throughout the entirety of the complementary region. This term is applied to pairs of polynucleotides based solely upon their sequences and not any particular set of conditions under which the two polynucleotides would actually bind.

The term "polypeptide" refers to a polymer of amino acids without regard to the length of the polymer; thus, peptides, oligopeptides, and proteins are included within the definition of polypeptide. This term also does not specify or exclude prost-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non -naturally occurring amino acids, amino acids which only occur naturally in an unrelated biological system, modified amino acids from mammalian systems etc.), polypeptides with substituted linkages, as well as other modifications known in the art, both naturally occurring and non - naturally occurring.

As used herein, the term "exon" refers as well to the portion of a DNA that codes for portion of spliced mRNA as to the amino acids encoded by said part of a DNA.

As used herein, "splice variants" refer to different mRNAs produced by alternative splicing events and translated from the same gene. The term splice variant refers as well to the mRNA as to the corresponding polypeptide.

As used herein, the term "non-human animal" refers to any non-human vertebrate, birds and more usually mammals, preferably primates, farm animals such as swine, g oats, sheep, donkeys, and horses, rabbits or rodents, more preferably rats or mice. As used herein, the term "animal" is used to refer to any vertebrate, preferable a mammal. Both the terms "animal" and "mammal" expressly embrace human subjects unless preceded with the term "non-human".

The terms "trait" and "phenotype" are used interchangeably herein and refer to any clinically distinguishable, detectable or otherwise measurable property of an organism such as symptoms of, or susceptibility to a disease for example. Typically the terms "trait" or "phenotype" are used herein to refer to symptoms of, or susceptibility to bipolar disorder; or to refer to an individual's response to an agent acting on bipolar disorder; or to refer to symptoms of, or susceptibility to side effects to an agent acting on bipolar disorder.

As used herein, the term "allele" refers to one of the variant forms of a biallelic marker, differing from other forms in its nucleotide sequence. Typically the first identified allele is designated as the original allele whereas other alleles are designated as alternative alleles. Diploid organisms may be homozygous or heterozygous for an allelic form.

The term "polymorphism" as used herein refers to the occurrence of two or more alternative genomic sequences or alleles between or among different genomes or individuals. "Polymorphic" refers to the condition in which two or more variants of a specific genomic sequence can be found in a population. A "polymorphic site" is the locus at which the variation occurs. A polymorphism may comprise a substitution, deletion or insertion of one or more nucleotides. A single nucleotide polymorphism is a single base pair change. Typically a single nucleotide polymorphism is the replacement of one nucleotide by another nucleotide at the polymorphic site. A "single nucleotide polymorphism" (SNP) refers to a sequence polymorphism differing in a single base pair.

### 2. KCNQ2-15b polypeptides of the present invention

The term "KCNQ2-15b polypeptides" is used herein to embrace all of the polypeptides of the present invention.

The KCNQ2-15b is selected from a peptide, a polypeptide or a protein selected from the group consisting of:
a) a polypeptide comprising a span of at least twenty amino acids of amino acids 589 to 643 of SEQ ID NO: 2;
b) a polypeptide comprising amino acids 589 to 643 of SEQ ID NO: 2;
c) a polypeptide comprising amino acids 545 to 643 of SEQ ID NO: 2;
d) a polypeptide comprising SEQ ID NO: 2;
e) a polypeptide comprising SEQ ID NO: 4;
f) a polypeptide comprising SEQ ID NO: 6;
g) a polypeptide comprising a mutein of any of (d) to (f), wherein the amino acid sequence has at least 95% or 99% identity to at least one of the sequences in (d) to (f); and
h) a mutein of any of (b) to (f) wherein any changes in the amino acid sequence are conservative amino acid substitutions to the amino acid sequences in (b) to (f).

KCNQ2-15b polypeptides of the present invention all comprise an amino acid sequence of a span of at least 20 amino acids of SEQ ID NO: 2, wherein said span falls within amino acids 589 to 643 of SEQ ID NO: 2. Preferably, KCNQ2-15b polypeptides comprise amino acids 589 to 643 of SEQ ID NO: 2.

In an embodiment of the invention, KCNQ2-15b polypeptides comprise any of SEQ ID NO: 2, SEQ ID NO: 4 or SEQ ID NO: 6. Preferred KCNQ2-15b polypeptides consist of SEQ ID NO: 2, SEQ ID NO: 4 or SEQ ID NO: 6. As further used herein, "KCNQ2-15bx" refers to a polypeptide of SEQ ID NO: 2, "KCNQ2-15by" refers to a polypeptide of SEQ ID NO: 4 and "KCNQ2-15bz" refers to a polypeptide of SEQ ID NO: 6.

In a preferred embodiment, KCNQ2-15b polypeptides are capable of binding to the By subunit of the PP2A phosphatase (PP2A/Bγ). In other words, said KCNQ2-15b polypeptides bind to PP2A/Bγ when the binding is tested by any suitable assay. Such assay s encompass, e.g., the yeast mating test described in example 9 and the solid phase overlay assay described in example 6. As further used herein, the term "KCNQ2-15b binding activity" or "binding activity" refers to the capacity of the KCNQ2-15b polypeptide to bind to PP2A/By.

In another preferred embodiment, KCNQ2-15b polypeptides correspond to a subunit of a potassium channel. In a more preferred embodiment, KCNQ2-15b polypeptides correspond to isoforms of the KCNQ2 polypeptide that are produced by alter native splicing events. Such KCNQ2-15b polypeptides may associate either with other KCNQ2-15b polypeptides or with other potassium channels subunits to form a potassium channel. As further used herein, the term "KCNQ2-15b biological activity" or "biological activity" refers to the activity of a potassium channel comprising the KCNQ2-15b polypeptide.

A preferred embodiment is directed to a potassium channel comprising at least one KCNQ2-15b polypeptide. The potassium channel may be a homomeric potassium cha nnel comprised of several KCNQ2-15b polypeptides. Alternatively, the potassium channel may be a heteromeric potassium channel comprised of a KCNQ2-15b polypeptide associated with other KCNQ polypeptides and/or other potassium channel subunits. The KCNQ2 -15b biological activity can be measured by methods well known by those skilled in the art such as, e.g., measurement of the M current.

As further used herein, the terms "KCNQ2-15b biological properties", "biological properties" and "activity" encompass both the biological activity and the binding activity of the KCNQ2-15b polypeptide. KCNQ2-15b biological properties further include, but are not limited to, e.g., KCNQ2-15b-specific antibody binding, binding to KCNQ subunits and modulation of potassium channel activity.

In further preferred embodiments, KCNQ2-15b polypeptides comprise the novel exon 15b. The term "exon 15b" refers to the amino acids at position 545 to 643 of SEQ ID NO: 2. Preferably, exon 15b is the most carboxyl-terminal exon of said KCNQ2-15b polypeptide. KCNQ2-15b polypeptides may further comprise any combination of exons 1 to 14 of the KCNQ2 gene.

The present invention is also directed to fragments of at least 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 or 610 amino acids of KCNQ2-15bx, KCNQ2-15by or KCNQ2-15bz.

Further embodiments are directed to muteins. As used herein the term "muteins" refers to analogs of KCNQ2-15bx, KCNQ2-15by or KCNQ2-15bz, in which one or more of the amino acid residues of a natural KCNQ2-15bx, KCNQ2-15by or KCNQ2-15bz are replaced by different amino acid residues, or are deleted, or one or more amino acid residues are added to the natural sequence of KCNQ2-15bx, KCNQ2-15by or KCNQ2-15bz, without lowering considerably the activity of the resulting products as compared with the wild-type KCNQ2-15bx, KCNQ2-15by or KCNQ2-15bz. These muteins are prepared by known synthesis and/or by site-directed mutagenesis techniques, or any other known technique suitable therefore.

Muteins of KCNQ2-15bx, KCNQ2-15by or KCNQ2-15bz, which can be used in accordance with the present invention, or nucleic acid coding thereof, include a finite set of substantially corresponding sequences as substitution peptides or polynucleotides which can be routinely obtained by one of ordinary skill in the art, without undue experimentation, based on the teachings and guidance presented herein.

Also disclosed herein are KCNQ2-15bx, KCNQ2-15by or KCNQ2-15bz encoded by a nucleic acid, such as DNA or RNA, which hybridizes to DNA or RNA, which encodes KCNQ2-15b, in accordance with the present invention, under moderately or highly stringent conditions. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §§6.3 and 6.4 (1987, 1992), and Sambrook et al. (Sambrook, J. C., Fritsch, E. F., and Maniatis, T. (1989) Molecular Cloning: A Laboratory Manual, Cold Spring Harbor Laboratory Press, Cold Spring Harbor, NY).

Without limitation, examples of stringent conditions include washing conditions 12-20°C below the calculated Tm of the hybrid under study in, *e.g.,* 2 x SSC and 0.5% SDS for 5 minutes, 2 x SSC and 0.1% SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC.

The polypeptides of the present invention include muteins having an amino acid sequence at least 95%, 96%, 97%, 98% or 99% identical to a KCNQ2-15b polypeptide of the present invention. By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Methods for comparing the identity and homology of two or more sequences are well known in the art. Thus for instance, programs available in the Wisconsin Sequence Analysis Package, version 9.1 (Devereux J et al., 1984), for example the programs BESTFIT and GAP, may be used to determine the % identity between two polynucleotides and the % identity and the % homology between two polypeptide sequences. BESTFIT uses the "local homology" algorithm of Smith and Waterman (1981) and finds the best single region of similarity between two sequences. Other programs for determining identity and/or similarity between sequences are also known in the art, for instance the BLAST family of programs (Altschul S F et al, 1990, Altschul S F et al, 1997, accessible through the home page of the NCBI at world wide web site ncbi.nlm.nih.gov) and FASTA (Pearson W R, 1990; Pearson 1988).

Preferred changes for muteins in accordance with the present invention are what are known as "conservative" substitutions. Conservative amino acid substitutions of KCNQ2-15bx, KCNQ2-15by or KCNQ2-15bz polypeptides, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g. under thirty, and preferably under ten, and do not remove or displace amino acids which are critical to a functional conformation, e.g. cysteine residues. Proteins and muteins produced by such deletions and/or insertions come within the purview of the present invention.

Preferably, the synonymous amino acid groups are those defined in Table I. More preferably, the synonymous amino acid groups are those defined in Table II; and most preferably the synonymous amino acid groups are those defined in Table III.

**TABLE I**

| **Preferred Groups of Synonymous Amino Acids** | |
|---|---|
| Amino Acid | Synonymous Group |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE II**

| **More Preferred Groups of Synonymous Amino Acids** | |
|---|---|
| Amino Acid | Synonymous Group |
| Ser | Ser |

| | |
|---|---|
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE III**

| **Most Preferred Groups of Synonymous Amino Acids** | |
|---|---|
| Amino Acid | Synonymous Group |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining muteins of KCNQ2-15bx, KCNQ2-15by or KCNQ2-15bz, polypeptides for use in the present invention include any known method steps, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

Preferably, the muteins of the present invention exhibit substancially the same biological properties as the KCNQ2-15b polypeptide to which it corresponds.

In some embodiments, KCNQ2-15b polypeptides and muteins or fragments thereof have biological activity or binding activity as defined above. In other embodiments, KCNQ2-15b polypeptides and muteins or fragments thereof do not have activity as defined above. Other uses of the polypeptides of the present invention include, *inter alia,* as epitope tags, in epitope mapping, and as molecular weight markers on SDS-PAGE gels or on molecular sieve gel filtration columns using methods known to those of skill in the art. Such polypeptides can be used to raise polyclonal and monoclonal antibodies, which are useful in assays for detecting KCNQ2-15bx, KCNQ2-15by and KCNQ2-15bz expression, or for purifying KCNQ2-15bx, KCNQ2-15by and KCNQ2-15bz. As a matter of example, a further specific use for KCNQ2-15b polypeptides is the use of such polypeptides the yeast two-hybrid system to capture KCNQ2-15bx, KCNQ2-15by or KCNQ2-15bz binding proteins, which are candidate modulators according to the present invention, as further detailed below.

### 3. KCNQ2-15b polynucleotides of the present invention

The present invention is further directed to KCNQ2-15b polynucleotides encoding any of the KCNQ2-15b polypeptides described above, and to sequence comp lementary thereto.

In a preferred embodiment, said polynucleotide is selected from the group consisting of:
a) a polynucleotide comprising nucleotides 1776 to 1929 of SEQ ID NO: 1.
b) a polynucleotide comprising nucleotides 1632 to 1929 of SEQ ID NO: 1.
c) a polynucleotide comprising SEQ ID NO: 1,
d) a polynucleotide comprising SEQ ID NO: 3,
e) a polynucleotide comprising SEQ ID NO: 5,
f) a polynucleotide complementary to the polynucleotides of (a) to (e).

The invention encompasses a purified, isolated and/or recombinant nucleic acid comprising a nucleotide sequence selected from the group consisting of polynucleotides encoding a KCNQ2-15b polypeptides, including splice variants as well as allelic variants, and fragments of KCNQ2-15bx, KCNQ2-15by and KCNQ2-15bz polypeptides. Preferably, said fragments comprise nucleotides at position 1776 to 1929 of SEQ ID NO:1. More preferably, said fragments comprise nucleotides at position 1632 to 1929 of SEQ ID NO:1.

Preferred KCNQ2-15b polynucleotides of the invention include isolated and/or recombinant polynucleotides comprising a contiguous span of at least 60, 70, 80, 90, 100, 200, 300, 400, 500, 600, 700, 800, 900, 1000, 1100, 1200, 1300, 1400, 1500, 1600, 1700 or 1800 nucleotides of SEQ ID NO: 1, SEQ ID NO:3 or SEQ ID NO: 5.

In a further preferred embodiment, the purified KCNQ2-15b polynucleotide has at least 95, 96, 97, 98 or 99% nucleotide identity with a polynucleotide selected from the group consisting of SEQ ID NO: 1, SEQ ID NO: 3, SEQ ID NO: 5, sequences complementery thereto and fragments thereof.

Another object of the invention relates to purified polynucleotides that hybridize under moderately stringent conditions or under highly stringent conditions with a polynucleotide selected from the group consisting of sequences complementery thereto and fragments thereof.

Most preferred KCNQ2-15b polynucleotides of the invention include polynucleotides encoding a KCNQ2-15bx polypeptide, a KCNQ2-15by polypeptide or a KCNQ2-15bz polypeptide. A KCNQ2-15bx polynucleotide corresponds to a polynucleotide encoding a KCNQ2-15bx polypeptide. A KCNQ2-15by polynucleotide corresponds to a polynucleotides encoding a KCNQ2-15by polypeptide. A KCNQ2-15bz polynucleotide corresponds to a polynucleotide encoding a KCNQ2-15bz polypeptide.

In some embodiments, said KCNQ2-15b polynucleotide comprises or consists of the coding sequence (CDS) encoding the KCNQ2-15b polypeptide. In other embodiments, said KCNQ2-15b polynucleotide comprises or consists of the messenger RNA (mRNA) encoding the KCNQ2-15b polypeptide. In further embodiments, said KCNQ2-15b polynucleotide comprises or consists of the complementary DNA (cDNA) encoding the KCNQ2-15b polypeptide. Preferred KCNQ2-15b polynucleotides are polynucleotides comprising a CDS having the sequence of SEQ ID NO: 1, SEQ ID NO: 3 or SEQ ID NO: 5, mRNAs comprising these CDSs and cDNAs comprising these CDSs.

The present invention also encompasses fragments of KCNQ2-15bx, KCNQ2-15by or KCNQ2-15bz polynucleotides for use as primers and probes. Such primers are useful in order to detect the presence of at least a copy of a KCNQ2-15bx, KCNQ2-15by or KCNQ2-15bz polynucleotide, complement, or variant thereof in a test sample. The probes of the present invention are useful for a number of purposes. They can notably be used in Southern hybridization to genomic DNA. The probes can also be used to detect PCR amplification products. They may also be used to detect mismatches in the KCNQ2-15bx, KCNQ2-15by or KCNQ2-15bz mRNAs using other techniques. They may further be used for *in situ* hybridization.

- Any of the polynucleotides, primers and probes of the present invention can be conveniently immobilized on a solid substrate, such as, e.g., a microarray. A substrate comprising a plurality of oligonucleotide primers or probes of the invention may be used either for detecting or amplifying targeted sequences in the KCNQ2-15bx, KCNQ2-15by or KCNQ2-15bz gene, may be used for detecting mutations in the coding or in the non-coding sequences of the KCNQ2-15bx, KCNQ2-15by or KCNQ2-15bz mRNAs, and may also be used to determine expression of KCNQ2-15bx, KCNQ2-15by or KCNQ2-15bz mRNAs in different contexts such as in different tissues, at different stages of a process (embryo development, disease treatment), and in patients versus healthy individuals.

Methods of cloning or constructing KCNQ2-15b polynucleotides are well known by those of skill in the art. For example, the methods described in the examples may be used to clone or construct the KCNQ2-15b polynucleotides of the present invention.

### 4. Vectors, host cells and host organisms of the present invention

The present invention also relates to vectors including the KCNQ2-15b polynucleotides of the present invention. More particularly, the present invention relates to expression vectors which include a KCNQ2-15b polynucleotide. Preferably, such expression vectors comprise a polynucleotide encoding a KCNQ2-15bx, a KCNQ2-15by, a KCNQ2-15bz polypeptide, a mutein thereof or a fragment thereof.

The term "vector" is used herein to designate either a circular or a linear DNA or RNA compound, which is either double-stranded or single-stranded, and which comprise at least one polynucleotide of the present invention to be transferred in a cell host or in a unicellular or multicellular host organism. An "expression vector" comprises appropriate signals in the vectors, said signals including various regulatory elements, such as enhancers/promoters from both viral and mammalian sources that drive expression of the inserted polynucleotide in host cells. Selectable markers for establishing permanent, stable cell clones expressing the products such as, e.g., a dominant drug selection, are generally included in the expression vectors of the invention, as they are elements that link expression of the drug selection markers to expression of the polypeptide.

Additionally, the expression vector may be a fusion vector driving the expression of a fusion polypeptide between a KCNQ2-15b polypeptide and a heterologous polypeptide. For example, the heterologous polypeptide may be a selectable marker such as, e.g, a luminescent protein, or a polypeptide allowing the purification of the fusion polypeptide.

The polynucleotides of the present invention may be used to, e.g., express the encoded polypeptide in a host cell for producing the encoded polypeptide. The polynucleotides of the present invention may further be used to express the encoded polypeptide in a host cell for screening assays. Screenings assays ar e of particular interest for identifying modulators and/or binding partners of KCNQ2-15b polypeptides as further detailed below. The polynucleotides of the present invention may also be used to express the encoded polypeptide in a host organism for producing a beneficial effect. In such procedures, the encoded protein may be transiently expressed in the host organism or stably expressed in the host organism. The encoded polypeptide may have any of the properties described herein. The encoded polypeptide may be a protein which the host organism lacks or, alternatively, the encoded protein may augment the existing levels of the protein in the host organism.

In one embodiment, the expression vector is a gene therapy vector. Viral vector systems that have application in gene therapy have been derived from, e.g., herpes virus, vaccinia virus, and several RNA viruses. In particular, herpes virus vectors may provide a unique strategy for persistence of inserted gene expression in cells of the central nervous system and ocular tissue.

Another object of the invention comprises a host cell that has been transformed, transfected or transduced with a polynucleotide encoding a KCNQ2-15b polypeptide. Also included are host cells that are transformed, transfected or transduced with a recombinant vector such as one of those described above. The cell hosts of the present invention can comprise any of the polynucleotides of the present invention.

Any host cell known by one of skill in the art may be used. Preferred host c ells used as recipients for the polynucleotides and expression vectors of the invention include:
a) Prokaryotic host cells: *Escherichia coli* strains (I.E.DH5-α strain), *Bacillus subtilis, Salmonella typhimurium,* and strains from species like *Pseudomonas, Streptomyces* and *Staphylococcus.*
b) Eukaryotic host cells: CHO (ATCC No. CCL-61), HeLa cells (ATCC No.CCL2; No.CCL2.1; No.CCL2.2), Cv 1 cells (ATCC No.CCL70), COS cells (ATCC No.CRL1650; No.CRL1651), Sf-9 cells (ATCC No.CRL1711), C127 cells (ATCC No. CRL-1804), 3T3 (ATCC No. CRL-6361), human kidney 293. (ATCC No. 45504; No. CRL-1573), BHK (ECACC No. 84100501; No. 84111301), *Saccharomyces cerevisiae* strains such as AH109 and Y184, and *Aspergillus niger* strains.

Another object of the invention comprises me thods of making the above vectors and host cells by recombinant techniques. Any well-known technique for constructing an expression vector and for delivering it to a cell may be used for construction and delivering the vectors of the present invention. Such techniques include but are not limited to the techniques detailed in the examples.

Another object of the present invention is a transgenic animal which includes within a plurality of its cells a cloned recombinant KCNQ2-15b polynucleotide. The terms "transgenic animals" or "host animals" are used herein to designate animals that have their genome genetically and artificially manipulated so as to include one of the nucleic acids according to the invention. The cells affected may be somatic, germ cells, or both. Preferred animals are non-human mammals and include those belonging to a genus selected from *Mus* (e.g. mice), *Rattus* (e.g. rats) and *Oryctogalus* (e.g. rabbits) which have their genome artificially and genetically altered by the insertion of a nucle ic acid according to the invention. In one embodiment, the invention encompasses non-human host mammals and animals comprising a recombinant vector of the invention or a KCNQ2-15b polynucleotide disrupted by homologous recombination with a knock out vector.

In a preferred embodiment, these transgenic animals may be good experimental models in order to study diverse pathologies related to KCNQ2-15b function. In particular, a transgenic animal wherein (i) an antisense mRNA binding to naturally occurring KC NQ2-15b mRNAs is transcribed; or (ii) an mRNA expressing a KCNQ2-15b polypeptide; may be a good animal model for bipolar disorders and/or other mood-disorders.

### 5. Methods of making the polypeptides of the present invention

The present invention also relates to methods of making a KCNQ2-15b polypeptide.

In one embodiment, the KCNQ2-15b polypeptides of the present invention are isolated from natural sources, including tissues and cells, whether directly isolated or cultured cells, of humans or non-human animals. Soluble forms of KCNQ2-15b may be isolated from body fluids. Methods for extracting and purifying natural membrane spanning proteins are known in the art, and include the use of detergents or chaotropic agents to disrupt particles followed by, e.g., differential extraction and separation of the polypeptides by ion exchange chromatography, affinity chromatography, sedimentation according to density, and gel electrophoresis. The method described in Example 4 may for example be used. Polypeptides of the invention also can be purified from natural sources using antibodies directed against the polypeptides of the invention, such as those described herein, in methods which are well known in the art of protein purification.

In a preferred embodiment, the KCNQ2-15b polypeptides of the invention are recombinantly produced using routine expression methods known in the art. The polynucleotide encoding the desired polypeptide is operably linked to a promoter into an expression vector suitable for any convenient host. Both eukaryotic and prokaryotic host systems may be used in forming recombinant polypeptides. The polypeptide is then isolated from lysed cells or, if a soluble form is produced, from the culture medium and purified to the extent needed for its intended use.

Consequently, a further embodiment of the present invention is a method of making a polypeptide of the present invention, said method comprising the steps of:
a) obtaining a polynucleotide encoding a KCNQ2-15b polypeptide;
b) inserting said polynucleotide in an expression vector such that the polynucleotide is operably linked to a promoter; and
c) introducing said expression vector into a host cell whereby said host cell produces said polypeptide.

In a preferred embodiment, the method further co mprises the step of isolating the polypeptide. The skilled person will appreciate that any step of this method may be carried out separately. The product of each step may be transferred to another step in order to carry out the subsequent step.

In further embodiments, said polynucleotide consists of a CDS. In another aspect of this embodiment, said polynucleotide is a polynucleotide consisting of SEQ ID NO: 1, SEQ ID NO: 3, SEQ ID NO: 5 or a fragment thereof.

A further aspect of the invention relates to a method of making a polypeptide, said method comprising the steps of culturing a host cell comprising an expression vector comprising a KCNQ2-15b polynucleotide under conditions suitable for the production of a KCNQ2-15b polypeptide within said host cell. In a preferred embodiment, the method further comprises the step of purifying said polypeptide from the culture.

In another embodiment, it is often advantageous to add to the recombinant polynucleotide encoding a KCNQ2-15b polypeptide additional nucleotide sequence which codes for secretory or leader sequences, pro-sequences, sequences which aid in purification, such as multiple histidine residues or GST tags, or an additional sequence for stability during recombinant production. Soluble portions of the KCNQ2-15b polypeptide may be, e.g., linked to an Ig-Fc part in order to generate stable soluble variants.

A polypeptide of this invention can be recovered and purified from recombinant cell cultures by well-known methods including but not limited to differential extraction, ammonium sulfate or ethanol precipitation, acid extraction, anion or cation exchange chromatography, high performance liquid chromatography, phosphocellulose chromatography, hydrophobic interaction chromatography, affinity chromatography, hydroxylapatite chromatography, immunochromatography and lectin chromatography.

The expressed KCNQ2-15b polypeptide may be purified using any standard immunochromatography techniques. In such procedures, a solution containing the polypeptide of interest, such as the culture medium or a cell extract, is applied to a column having antibodies against the polypeptide attached to the chromatography matrix. The recombinant protein is allowed to bind the immunochromatography column. Thereafter, the column is washed to remove non-specifically bound proteins. The specifically bound secreted protein is then released from the column and recovered using standard techniques.

### 6. Antibodies of the present invention

The present invention further relates to antibodies that specifically bind to the polypeptides of the present invention. More specifically, said antibodies bind to the epitopes of the polypeptides of the present invention. The antibodies of the present invention include IgG (including IgG1, IgG2, IgG3, and IgG4), IgA (including IgA1 and IgA2), IgD, IgE, or IgM, and IgY. The term "antibody" (Ab) refers to a polypeptide or group of polypeptides which are comprised of at least one binding domain, where a binding domain is formed from the folding of variable domains of an antibody compound to form three-dimensional binding spaces with an internal surface shape and charge distribution complementary to the features of an antigenic determinant of an antigen, which allows an immunological reaction with the antigen. As used herein, the term "antibody" is meant to include whole antibodies, including single-chain whole antibodies, and antigen binding fragments thereof. In a preferred embodiment the antibodies are human antigen binding antibody fragments of the present invention include, but are not limited to, Fab, Fab' F(ab)2 and F(ab')2, Fd, single -chain Fvs (scFv), single-chain antibodies, disulfide-linked Fvs (sdFv) and fragments comprising either a V_{L} or V_{H} domain. The antibodies may be from any animal origin including birds and mammals. Preferably, the antibodies are from human, mouse, rabbit, goat, guinea pig, camel, horse or chicken. The present invention further includes chimeric, humanized, and human monoclonal and polyclonal antibodies, which specifically bi nd the polypeptides of the present invention.

Preferred antibodies of the present invention recognize an epitope within amino acids 589 to 643 of SEQ ID NO: 2, wherein said one or more amino-acids are required for binding of the antibody to a KCNQ2-15b polypeptide. Other preferred antibodies of the present invention recognize one or more of the amino acids at positions 545 to 643 of SEQ ID NO: 2,
wherein said one or more amino-acids are required for binding of the antibody to a KCNQ2-15b polypeptide. Most preferably, the antibodies of the present invention bind to a KCNQ2 polypeptide comprising exon 15b but not to a KCNQ2 polypeptide lacking exon 15b.

A preferred embodiment of the invention is a method of specifically binding an antibody of the present invention to a KCNQ2-15b polypeptide. This method comprises the step of contacting the antibody of the present invention with a KCNQ2-15b polypeptide under conditions in which said antibody can specifically bind to said polypeptide. Such conditions are well known to those skilled in the art. This method may be used to, e.g., detect, purify, or activate or inhibit the activity of KCNQ2-15b polypeptides.

The invention further relates to antibodies that act as modulators of the polypeptides of the present invention. Preferred antibodies are modulators that enhance the binding activity or the biological activity of the KCNQ2-15b polypeptide to which they bind. These antibodies may act as modulators for either all or less than all of the biological properties of the KCNQ2-15b polypeptide.

### 7. Uses of the polypeptides of the present invention

The present invention is also directed to the use of a KCNQ2 polypeptide as a target for screening candidate modulators. As used herein, the term "KCNQ2 polypeptide" refers to any polypeptide encoded by the KCNQ2 gene. Thus the term "KCNQ2 polypeptide" encompasses all alternative splice variants encoded by the KCNQ2 gene, such as, e.g., KCNQ2-15b polypeptides and all previously described isoforms (see, e.g., SwissProt Accession No. 043526). As further used herein, the term "KCNQ2-fl" refers to a polypeptide of SEQ ID NO: 7.

As used herein, the term "modulator" refers to a compound that increases or decreases any of the properties of a KCNQ2 polypeptide. As used herein, a "KCNQ2 modulator" refers to a compound that increases or decreases the activity of a KCNQ2 polypeptide and/or to a compound that increases or decreases the trasncription level of the KCNQ2 mRNA encoding said polypeptide. The term "modulator" encompasses both ago nists and antagonists.

As used herein, a "KCNQ2 antagonist" refers to a compound that decreases the activity of a KCNQ2 polypeptide and/or to a compound that decreases the expression level of the KCNQ2 mRNA encoding said polypeptide. The terms "antagonist" and "inhibitor" are considered to be synonymous and can be used interchangeably throughout the disclosure.

As used herein, a "KCNQ2 agonist" refers to a compound that increases the activity of a KCNQ2 polypeptide and/or to a compound that increases the expression level of the KCNQ2 mRNA encoding said polypeptide. The terms "agonist" and "activator" are considered to be synonymous and can be used interchangeably throughout the disclosure.

Methods that can be used for testing modulators for their ability to increase or decrease the activity of a KCNQ2 polypeptide or to increase or decrease the expression of a KCNQ2 mRNA are well known in the art and further detailed below. Preferred modulators of the present invention are modulators of KCNQ2-15bx, KCNQ2-15by, KCNQ2-15bz or KCNQ2-fl. The assays described herein and known in the art for measuring KCNQ2 activity can be performed either *in vitro* or *in vivo.*

Candidate compounds according to the present invention include naturally occurring and synthetic compounds. Such compounds include, e.g., natural ligands, small molecules, antisense mRNAs, antibodies, aptamers and short interfering RNAs. As used herein, the term "natural ligand" refers to any signaling molecule that binds to a phosphatase comprising PP2A/Bγ *in vivo* and includes molecules such as, e.g., lipids, nucleotides, polynucleotides, amino acids, peptides, polypeptides, proteins, carbohydrates and inorganic molecules. As used herein, the term "small molecule" refers to an organic compound. As used herein, the term "antibody" refers to a protein produced by cells of the immune system or to a fragment thereof that binds to an antigen. As used herein, the term "antisense mRNA" refers an RNA molecule complementary to the strand normally processed into mRNA a nd translated, or complemantary to a region thereof. As used herein, the term "aptamer" refers to an artificial nucleic acid ligand (see, e.g., Ellington and Szostak (1990) Nature 346:818-822). As used herein, the term "short interfering RNA" refers to a double-stranded RNA inducing sequence-specific posttranscriptional gene silencing (see, e.g., Elbashir et al. (2001) Genes Dev. 15:188-200).

Such candidate compounds can be obtained using any of the numerous approaches in combinatorial library methods known in the art, including, e.g., biological libraries, spatially addressable parallel solid phase or solution phase libraries, and synthetic library methods using affinity chromatography selection. The biological library approach is generally used with peptide libraries, while the other four approaches are applicable to peptide, non-peptide oligomers, aptamers or small molecule libraries of compounds.

One example of a method that may be used for screening candidate compounds for a modulator is a method comprising the steps of:
a) contacting a KCNQ2 polypeptide with the candidate compound; and
b) testing the activity of said KCNQ2 polypeptide in the presence of said candidate compound,
wherein a difference in the activity of said KCNQ2 polypeptide in the presence of said compound in comparison to the activity in the absence of said compound indicates that the compound is a modulator of said KCNQ2 polypeptide.

Alternatively, the assay may be a cell-based assay comprising the steps of:
a) contacting a cell expressing a KCNQ2 polypeptide with the candidate compound; and
b) testing the activity of said KCNQ2 polypeptide in the presence of said candidate compound,
wherein a difference in the activity of said KCNQ2 polypeptide in the presence of said compound in comparison to the activity in the absence of said compound indicates that the compound is a modulator of said KCNQ2 polypeptide.

The modulator may modulate any activity of said KCNQ2 polypeptide. The modulator may for example modulate KCNQ2 mRNA expression within a cell, or modulate the M-current generated by a potassium channel comprising the KCNQ2 polypeptide. Further activities that may be measured include KCNQ2 binding to PP2A/Bγ, and to KCNQ2 binding to other potassium channel subunits. The phosphorylation st ate of a KCNQ2 polypeptide is a further activity of KCNQ2 that may be assessed in order to screen compounds. Most preferably, the activity of the KCNQ2 polypeptide is assessed by measuring the M-current. Methods for testing the above mentioned activities a re well known to those of skill in the art, and may for example be performed as further detailed below.

Preferred modulators of the invention are modulators that increase or decrease:
- KCNQ2 mRNA expression within a cell;
- the M-current generated by a potassium channel comprising a KCNQ2 polypeptide;
- binding of the KCNQ2 polypeptide to PP2A/Bγ, and/or
- binding of the KCNQ2 polypeptide to other potassium channel subunits.

In a preferred embodiment, the activity of a KCNQ2 polypeptide is assessed by measuring the M-current generated by a potassium channel comprising the KCNQ2 polypeptide. Assays for measuring the M-current generated by a potassium channel are known by those of skill the art. An electrophysiologic assay for measuring the activity of the M-current generated by a potassium channel is for example described by Pan et al. and by Schwake et al. (Pan et al. (2001), J. Physiol., 531:347-358; Schwake et al. (2000), J. Biol. Chem., 275:13343-13348). High-throughput fluorescence assays using membrane potential I sensitive dyes has also been described to screen compounds on potassium channels. For example, EVOTEC has developed assays for testing the activity of ion channels (see, e.g., the world wide website evotecoai.com). In such assays, the activity both of ho momeric KCNQ2 channels and of heteromeric channels may be tested. Homomeric channels that may be tested include, e.g., homomeric KCNQ2-fl and homomeric KCNQ2-15b channels. Heteromeric channels that may be tested include, e.g., heteromeric KCNQ2-15b/KCNQ2-fl, heteromeric KCNQ2-fl/KCNQ3 and heteromeric KCNQ2-15b/KCNQ3 channels.

In another preferred embodiment, the activity of a KCNQ2 polypeptide is assessed by measuring the binding of the KCNQ2 polypeptide to PP2A/Bγ. The binding of a KCNQ2 polypeptide to PP2A/Bγ can for example be measured by the yeast mating test as described in example 3 or by the solid phase overlay assay as described in example 6.

In another preferred embodiment, the activity of a KCNQ2 polypeptide is assessed by measuring the binding of the KCNQ2 polypeptide to other potassium channels subunits. This assay may also be performed using the yeast mating test or the solid phase overlay assay described in examples 3 and 6.

In a further preferred embodiment, the activity of a KCNQ2 polypeptide is assessed by measuring the KCNQ2 mRNA levels within a cell. In this embodiment, the activity can for example be measured using Northern blots, RT-PCR, quantitative RT-PCR with primers and probes specific for KCNQ2 mRNAs. The term "KCNQ2 mRNA" as used herein encompasses all alternative splice variants translated from the KCNQ2 gene such as, e.g., SEQ ID Nos 1, 3, 5 and EMBL Accession Nos. NM_172107, NM_172106, NM_004518, NM_172108 and NM_172109. The primers and probes may detect one specific KCNQ2 splice variant or detect all alternative splice variants translated from the KCNQ2 gene. Alternatively, the expression of the KCNQ2 mRNA is measured at the polypeptide level, by using labeled antibodies that specifically bind to the KCNQ2 polypeptide in immunoassa ys such as ELISA assays, or RIA assays, Western blots or immunohistochemical assays. The KCNQ2 antibody may detect one specific KCNQ2 splice variant or detect all alternative splice variants translated from the KCNQ2 gene.

In another embodiment, the activity of a KCNQ2 polypeptide is measured by determining the phosphorylation state of the KCNQ2 polypeptide as described in example 7. In the frame of the present invention, it has been found that (i) KCNQ2 -15b polypeptides are dephosphorylated by a PP2A phosphatase comprising a PP2A/Bγ subunit, the gene encoding the PP2A/Bγ subunit being associated with bipolar disorder; and (ii) phosphorylated by GSK3β, a kinase that is inhibited by mood stabilizing agents. Thus the phosphorylation state of a KCNQ2 polypeptide is believed to be correlated with the biological activity of the KCNQ2 polypeptide. The phosphorylation state of a KCNQ2 polypeptide may for example be measured in an assay as described in example 7.

One preferred embodiment is directed to the use of a KCNQ2-15b polypeptide as a target for screening candidate modulators. Another preferred embodiment is directed to the use of a KCNQ2-fl polypeptide as a target for screening candidate modulators.

Modulators of KCNQ2 polypeptides, which may be found, e.g., by any of the above screenings, are candidate drugs for the treatment of a mental disorder. Thus a preferred embodiment of the present invention is the use of a KCNQ2 polypeptide as a target for screening candidate compounds for candidate drugs for the treatment of a mental disorder.

As used herein, the term "Mental disorder" includes bipolar disorder, schizophrenia, depression as well as other mood disorders and psychotic disorders. Preferably, said mental disorder is bipolar disorder, schizophrenia or depression. Most preferably, said mental disorder is bipolar disorder.

The present invention further discloses the use of a modulator of a KCNQ2 polypeptide for screening for drugs for the treatment of a mental disorder. One example of a method that can be used for screening for drugs for the treatment of a mental disorder and/or for assessing the efficiency of an modulator of a KCNQ2 polypeptide for the treatment of a mental disorder is a method comprising the step of administering said modulator to an animal model for said mental disorder, wherein a determination that said modulator ameliorates a representative characteristic of said mental disorder in said animal model indicates that said modulator is a drug for the treatment of said mental disorder.

Animal models for mental disorders and assays for determining whether a compound ameliorates a representative characteristic of said mental disorder in said animal model are described and used. For example, animal models that may be used in the above method include but are not limited to the conditioned avoidance behaviour model in rats, which is a standard behavioural test predictive of antipsychotic activity, the behavioral activity assessment of mice and rats in the Omnitech Digiscan animal activity monito rs, the purpose of which is to evaluate compounds for antipsychotic-like CNS effects and a variety of other behavioral effects generally associated with CNS activity, the blockade of amphetamine-stimulated locomotion in rat, the protocol for the prepulse inhibition of acoustic startle model in rats, the inhibition of apomorphine-induced climbing behaviour and the inhibition of DOI-induced head twitches and scratches. A preferred animal model is the STOP-/- mice with synaptic defects and severe behavioral disorders described by Andrieux et al. (2002, Genes Dev., 16:2350-2364).

In all the above embodiments, preferred modulators are modulators of KCNQ2-15b polypeptides or of KCNQ2-fl. Preferred modulators of KCNQ2-15b polypeptides are modulators that specifically modulate a polypeptide comprising exon 15b shown at position 545 to 643 of SEQ ID NO: 2. Preferred KCNQ2-fl modulators are modulators that specifically modulate a polypeptide comprising exons 16 and 17 shown at position 588 to 872 of SEQ ID NO: 7.

The present invention further relates to methods for screening for natural binding partners of a KCNQ2 polypeptide. Such methods include the yeast two-hybrid screening that is described in example 1. Identifying natural biding partners of a KCNQ2 polypeptide may be preformed by replacing the CDS encoding PP2A/Bγ with a polynucleotides encoding a KCNQ2 polypeptide. Using a KCNQ2 polypeptide as a target has a great utility for the identification of proteins involved in bipolar disorder and for providing new intervention points in the treatment of bipolar disorder and other mood disorders.

### 8. Biallelic markers of the present invention

The present invention is directed to the use of at least one KCNQ2-related biallelic marker selected from the group consisting of 30-2/62 and 30-7/30 for diagnosing whether an individual suffers from or is at risk of suffering from a mental disorder. As used herein, the term "KCNQ2-retated biallelic marker" refers to a biallelic marker located in an exon of the KCNQ2 gene, in an intron of the KCNQ2 gene, or in the regulatory regions of the KCNQ2 gene. KCNQ2-related biallelic markers encompass the biallelic markers shown in table 3B in Example 12. In one embodiment, a single biallelic marker is used for diagnosing whether an individual suffers from or is at risk of suffering from a mental disorder by determining the genotype of an individual. In another embodiment, a combination of several biallelic markers may be used for diagnosing whether an individual suffers from or is at risk of suffering from a mental disorder by determining the haplotype of an individual. For example, a two-markers haplotype, a three-markers haplotype or a four-markers haplotype may be determined.

As used herein, the term "biallelic marker" refers to a polymorphism having two alleles at a fairly high frequency in the population, preferably a single nucleotide polymorphism. Typically the frequency of the less common allele of the biallelic markers of the present invention has been validated to be greater than 1%, preferably the frequency is greater than 10%, more preferably the frequency is at least 20% (i.e. heterozygosity rate of at least 0.32), even more preferably the frequency is at least 30% (i.e. heterozygosity rate of at least 0.42). In the present specification, the term "biallelic marker" is used to refer both to the polymorphism and to the locus carrying the polymorphism.

As used herein, the term "genotype" refers to the identity of the alleles present in an individual or a sample. The term "genotype" preferably refers to the description of both copies of a single biallelic marker that are present in the genome of an individual. The individual is homozygous if the two alleles of the biallelic marker present in the genome are identical. The individual is heterozygous if the two alleles of the biallelic marker present in the genome are different.

The term "genotyping" a sample or an individual for a biallelic marker involves determining the specific alleles or the specific nucleotides carried by an individual at a biallelic marker.

As used herein, the term "haplotype" refers to a set of alleles of closely linked biallelic markers present on one chromosome and which tend to be inherited together.

Methods for determining the alleles, genotypes or haplotypes carried by an individual are well known by those of skill in the art and further detailed below.

In all embodiments, preferred "mental disorders" include bipolar disorder, schizophrenia and depression. Most preferred mental disorder is bipolar disorder.

In the context of the present invention, the individual is generally understood to be human.

As shown in Example 15, biallelic markers 30-2/62 and 30-7/30 are bipolar disorder-associated markers. Preferred embodiments of the present invention are thus directed to the use of biallelic markers 30-2/62 and 30-7/30. The alternative alleles of biallelic markers 30-2/62 and 30-7/30 are indicated in table 3B in example 12. Positions of biallelic markers 30-2/62 and 30-7/30 on SEQ ID NO: 43 and SEQ ID NO: 45 respectively are also indicated in table 3B. Other preferred embodiments are directed to the use of biallelic markers complementary to 30-2/62 and 30-7/30, i.e., the corresponding alternative alleles that are located on the complementary strand of DNA.

Accordingly, a preferred embodiment of the present invention is directed to the use of biallelic markers 30-2/62 and 30-7/30 and the complements thereof for diagnosing whether an individual suffers from or is at risk of suffering from a mental disorder. Preferably, the individual is a Caucasian individual. Most preferably, the individual is a Caucasian individual of British Isles origin.

The risk genotypes for biallelic markers 30-2/62 and 30-7/30 are indicated in table 6B. "Risk genotype" means that the probability of having bipolar disorder is higher for an individual carrying the risk genotype than for an individual carrying another genotype. The risk genotype for biallelic marker 30-2/62 is "AG". Thus a preferred embodiment of the present invention is the use of biallelic markers 30-2/62 or the complement thereof for diagnosing whether an individual suffers from or is at risk of suffering from a mental disorder, wherein the presence of a genotype "AG" at biallelic marker 30-2/62 is indicative of said individual suffering from or being at risk of suffering from said mental disorder. The risk genotype for biallelic marker 30-7/30 is "CC". Thus a preferred embodiment of the present invention is the use of biallelic markers 30-2/62 or the complement thereof for diagnosing whether an individual suffers from or is at risk of suffering from a mental disorder, wherein the presence of a genotype "CC" at biallelic marker 30-7/30 is indicative of said individual suffering from or being at risk of suffering from said mental disorder.

The present invention is further directed to the use of at least one KCNQ2-related biallelic marker selected from the group consisting of 30-4/58, 30-2/62, 30-17/37, 30-7/30, 30-15/54 and 30-84/37 for determining whether there is a significant association between said marker and a mental disorder. Such determination can for example be performed using methods described in examples 10 to 15 below but using populations that are different from the UCL and the Labimo populations, such as populations having different ethnic origins. The KCNQ2-related biallelic marker may be selected from the group consisting of 30-2/62 and 30-7/30 and the complements thereof. Alternatively, The KCNQ2-related biallelic marker may be selected from the group consisting of 30-4/58, 30-17/37, 30-84/37 and 30-15/54 and the complements thereof. Preferably, the mental disorder is selected from the group consisting of bipolar disorder, schizophrenia and depression. Most preferably, the mental disorder is bipolar disorder.

The present invention is further directed to a method of genotyping comprising the step of determining the identity of a nucleotide at a KCNQ2 related biallelic marker or the complement thereof in a biological sample wherein said biallelic marker is selected from the group consisting of 30-4/58, 30-2/62, 30-17/37, 30-7/30, 30-15/54 and 30-84/37. Preferably, said biological sample is derived from a single subject. It is preferred that the identity of the nucleotides at said biallelic marker is determined for both copies of said biallelic marker present in said individual's genome. In a preferred embodiment, the identity of the nucleotide at said biallelic marker is determined by a microsequencing assay. Preferably, a portion of a sequence comprising the biallelic marker is amplified prior to the determination of the identity of the nucleotide. The amplification may preferably be performed by PCR. Such a method of genotyping may for example be performed using any of the protocols described in examples 10 to 14 of the present specification. Further methods of genotyping are well known by those of skill in the art and any other known protocol may be used.

Methods well-known to those skilled in the art that may be used for genotyping in order to detect biallelic polymorphisms include methods such as, conventional dot blot analyzes, single strand conformational polymorphism analysis (SSCP) (Orita et al. (1989) Proc Natl Acad Sci USA 86:2766-2770), denaturing gradient gel electrophoresis (DGGE) (Borresen et al. (1988) Mutat Res. 202:77-83.), heteroduplex analysis (Lessa et al. (1993) Mol Ecol. 2:119-129), mismatch cleavage detection (Grompe et al. (1989) Proc Natl Acad Sci USA. 86:5888-5892). Another method for determining the identity of the nucleotide present at a particular polymorphic site employs a specialized exonuclease-resistant nucleotide derivative as described in US patent No. 4,656,127. Oligonucleotide microarrays or solid-phase capturable dideoxynucleotides and mass spectrometry may also be used (Wen et al. (2003) World J Gastroenterol. 9:1342-1346; Kim et al. (2003) Anal Biochem. 316:251-258). Preferred methods involve directly determining the identity of the nucleotide present at a biallelic marker site by sequencing assay, microsequencing assay, enzyme-based mismatch detection assay, or hybridization assay.

As used herein, the term "biological sample" refers to a sample comprising nucleic acids. Any source of nucleic acids, in purified or non-purified form, can be utilized as the starting nucleic acid, provided it contains or is suspected of containing the specific nucleic acid sequence desired. DNA or RNA may be extracted from cells, tissues, body fluids and the like.

Methods of genotyping find use in, e.g., in genotyping case-control populations in association studies as well as in genotyping individuals in the context of detection of alleles of biallelic markers which are known to be associated with a given trait. In the context of the present invention, a preferred trait is a mental disorder selected from the group of bipolar disorder, schizophrenia and depression, and most preferably bipolar disorder.

Accordingly, a preferred embodiment is directed to a method of diagnosing a mental disorder in an individual comprising the step of genotyping at least one KCNQ2 -related biallelic marker selected from the group consisting of 30-2/62 and 30-7/30 using a method of genotyping comprising the step of determining the identity of a nucleotide at a KCNQ2-related biallelic marker or the complement thereof in a biological sample derived from said individual. Such a diagnosing method may further comprise the step of correlating the result of the genotyping step with a risk of suffering from said mental disorder. Typically, the presence of the risk allele, risk genotype or risk haplotype of the genotyped KCNQ2-related biallelic marker(s) is correlated with a risk of suffering from the mental disorder. Preferably, said KCNQ2-related biallelic marker is selected from the group consisting of 30-2/62 and 30-7/30 and the complements thereof. In one embodiment, the presence of a genotype "AG" at biallelic marker 30-2/62 is indicative of a risk of suffering from said mental disorder. In another embodiment, the presence of a genotype "CC" at biallelic marker 30-7/30 is indicative of a risk of suffering from said mental disorder. Preferably, the mental disorder is selected from the group consisting of bipolar disorder, schizophrenia and depression. Most preferably, the mental disorder is bipolar disorder.

The present invention further discloses to the use of at least one KCNQ2-2-related biallelic marker for determining the haplotype of an individual. When determining the haplotype of an individual, each single chromosome should be studied independently. Methods of determining the haplotype of an individual are well known in the art and include, e.g., asymmetric PCR amplification (Newton et al. (1989) Nucleic Acids Res. 17:2503-2516; Wu et al. (1989) Proc. Natl. Acad. Sci. USA. 86:2757-2760), isolation of single chromosome by limit dilution followed by PCR amplification (Ruano et al. (1990) Proc. Natl. Acad. Sci. USA. 87:6296-6300) and, for sufficiently close biallelic markers, double PCR amplification of specific alleles (Sarkar and Sommer, (1991) Biotechniques. 10:436- 440).

Thus the present invention further discloses to the use of at least one KCNQ2-related biallelic marker for determining the haplotype of an in dividual. For example, a method for determining a haplotype for a set of biallelic markers in an individual may comprise the steps of: a) genotyping said individual for at least one KCNQ2 related biallelic marker, b) genotyping said individual for a second biallelic marker by determining the identity of the nucleotides at said second biallelic marker. Preferably, both markers are KCNQ2-related biallelic markers. Methods of determining a haplotype for a combination of more than two biallelic markers comprisi ng at least one KCNQ2-related biallelic marker in an individual are also encompassed by the present invention. In such methods, step (b) is repeated for each of the additional markers of the combination. Such a combination may comprise, e.g., 3, 4 or 5 biallelic markers. These biallelic markers may all be KCNQ2-related biallelic markers.

When estimating haplotype frequencies in a population, one may use methods without assigning haplotypes to each individual. Such methods use a statistical method of haplotype determination. Thus another aspect of the present invention encompasses methods of estimating the frequency of a haplotype for a set of biallelic markers in a population, comprising the steps of: a) genotyping each individual in said population for at least one KCNQ2-related biallelic marker, b) genotyping each individual in said population for a second biallelic marker by determining the identity of the nucleotides at said second biallelic marker; and c) applying a haplotype determination method to the identities of the nucleotides determined in steps a) and b) to obtain an estimate of said frequency. Such a method may also be performed for a combination of more than 2 biallelic markers. Step (c) may be performed using any method known in the art to determine or to estimate the frequency of a haplotype in a population. Preferably, a method based on an expectation - maximization (EM) algorithm (Dempster et al. (1977) JRSSB, 39:1-38; Excoffier and Slatkin, (1995) Mol Biol Evol. 12:921-7) leading to maximum-likelihood estimates of haplotype frequencies under the assumption of Hardy-Weinberg proportions (random mating) is used for performing step (c).

### EXAMPLES

### EXAMPLE 1 : Yeast two-hybrid screening

### 1. Construction of pGBKT7-PPP2R2C

The full-length coding region of the *PPP2R2C* gene, which encodes the PP2A/Bγ subunit, was first amplified from a Human foetal brain cDNA library (Marathon -Ready cDNA, Clontech) with the two gene-specific primers of SEQ ID NO: 8 and of SEQ ID NO: 9. This first PCR product was then amp lified with a new combination of primers of SEQ ID NO: 10 and of SEQ ID NO: 11. The amplified fragment encompassed nucleotides 52-1540 of the full-length cDNA, genbank accession number AF086924 extended, respectively, with *Eco*Rl and *Bam*Hl cloning sites. The resulting 1503-bp fragment was digested with *Eco*Rl and *Bam*Hl, purified and inserted into *Eco*Rl and *Bam*Hl cloning sites of the pGBKT7 vector (Clontech).

### 2. The Yeast Two-Hybrid Screening

A yeast two-hybrid screening was performed to find polypeptides interacting with the PP2A/Bγ subunit. The *Saccharomyces cerevisiae* strain AH109 *(MATa, trp1-901, leu2-3, 112, ura3-52, his3-200,* gal4Δ, gal80Δ, *LYS2:: GAL1_{UAS}-GAL1_{TATA}-HIS3, GAL2_{UAS}-GAL2_{TATA}-ADE2, URA3* :: *MEL1_{UAS}-MEL1_{TATA}-lacZ)* was transformed with the pGBKT7-PPP2R2C construction. A lithium acetate transformation procedure was done according to the manufacturer's instructions (Matchmaker Two-Hybrid system, Clontech). The *MATa* transformed cells expressing the bait were then mixed with a pretransformed Matchmake r Human brain cDNA library in the Y187 strain (MATα, *ura3-52, his3-200, ade2-101, trp1-901, leu2-3, 112, gal4Δ, met⁻, gal80Δ, URA3* :: *GAL1_{UAS}-GAL1_{TATA}-lacZ).* Three independent mating were performed with respectively 5.10⁶, 5.10⁶ and 2.10⁵ clones of the Human brain cDNA library. The resulting diploid cells able to grow on SD/-Leu/-Trp medium containing plates were further selected onto the medium-stringency SD/-Leu/-Trp/-His selective medium for the identification of bait-prey interactions. Positive colonies were then picked up and plated onto the high-stringency SD/-Leu/-Trp/-His/-Ade selective medium. Only cDNA of colonies able to grow at the same time on SD/-Leu/-Trp and SD/-Leu/-Trp/-His/-Ade media was retained for sequencing and further studies.

### 3. Results of the he Yeast Two-Hybrid Screening

494 clones were obtained, sequenced and analyzed. Among these clones, the 2E11 and 1D3 clones comprised partial cDNAs encoding a novel splice variant of the KCNQ2 potassium channel. 2E11 comprised a cDNA encoding amino acids 433 to 643 of SEQ ID NO: 2, and 1D3 comprised a cDNA encoding amino acids 454 to 643 of SEQ ID NO: 2. The full-length splice variants were cloned and sequenced as described in Example 2.

### EXAMPLE 2: Cloning of the full-length KCNQ2 splice variants

### 1. Cloning and sequencing

Poly(A)+ mRNA from Hu man brain, thalamus (Clontech) were reversed transcribed (RT) using the murine Moloney leukemia virus reverse transcriptase (RT-PCR Advantage kit, Clontech) with a primer of SEQ ID NO: 12 hybridizing specifically with the novel splice variant cloned in 2E11. After a phenol-chloroform extraction and precipitation steps, the products obtained by the previous RT-PCR were directly PCR-amplified using the following gene-specific primers of SEQ ID NO: 13 and of SEQ ID NO: 14. The amplified fragment encompassed nucleotides 127-148 of the KCNQ2 full-length cDNA, genbank accession number AF033348. These primers were respectively extended with *Eco*RI and *Bg*/II cloning sites. The PCR products were digested with *Eco*Rl and *Bg*/II restriction enzymes (New England Biolabs), purified and then ligated into the *Eco*Rl and *Bgl*II cloning sites of the pCMV-Myc vector (Clontech). The two pCMV-Myc-3H9 and pCMV-Myc-3H2 clones were fully sequenced. The sequence of the insert in pCMV-Myc-3H2 comprises SEQ ID NO: 1, and the sequence of the insert in pCMV-Myc-3H9 comprises SEQ ID NO: 3.

Similarly, a cDNA was cloned from a poly(A)+ mRNA library from human foetal brain. One clone was obtained and fully sequenced. Its insert comprised SEQ ID NO: 5.

### 2. Description of the novel splice variants

SEQ ID NO: 1 encodes the polypeptide of SEQ ID NO: 2 (KCNQ2-15bx). SEQ ID NO: 3 encodes the polypeptide of SEQ ID NO: 4 (KCNQ2-15by). SEQ ID NO: 5 encodes the polypeptide of SEQ ID NO: 6 (KCNQ2-15bz). SEQ ID NO: 7 corresponds to the full-length KCNQ2 polypeptide (KCNQ2-fl).

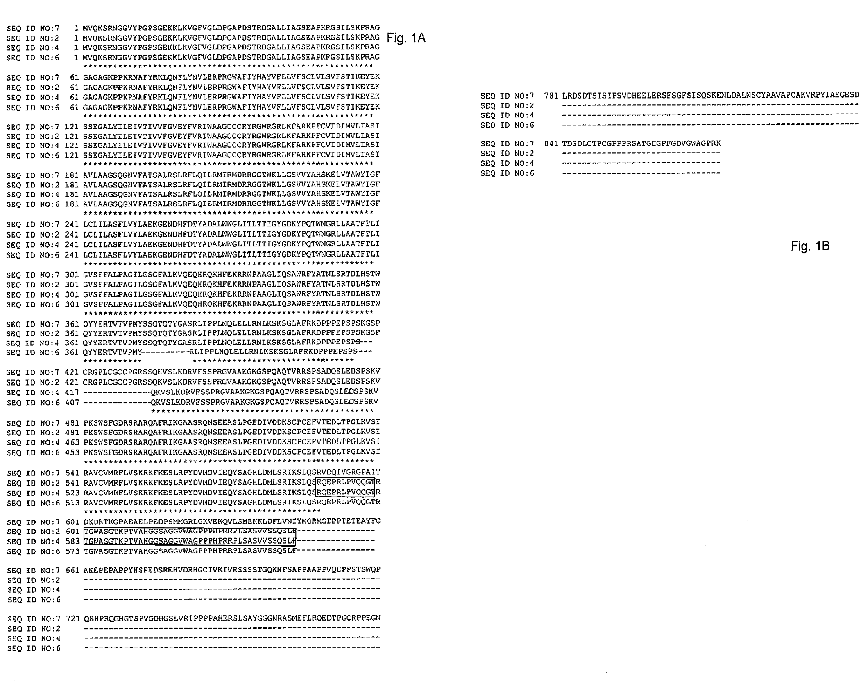

As shown on the alignment between SEQ ID NO: 7, SEQ ID NO: 2, SEQ ID NO: 4 and SEQ ID NO: 6 (Figure 1), the three splice variants display a novel carboxyl -terminal extremity compared to KCNQ2. The 55 carboxyl-terminal amino acids of SEQ ID NO: 2, SEQ ID NO: 4 and SEQ ID NO: 6 are unique to these three splice variants. These 55 amino acids correspond to the amino acids at position 589 to 643 of SEQ ID NO: 2.

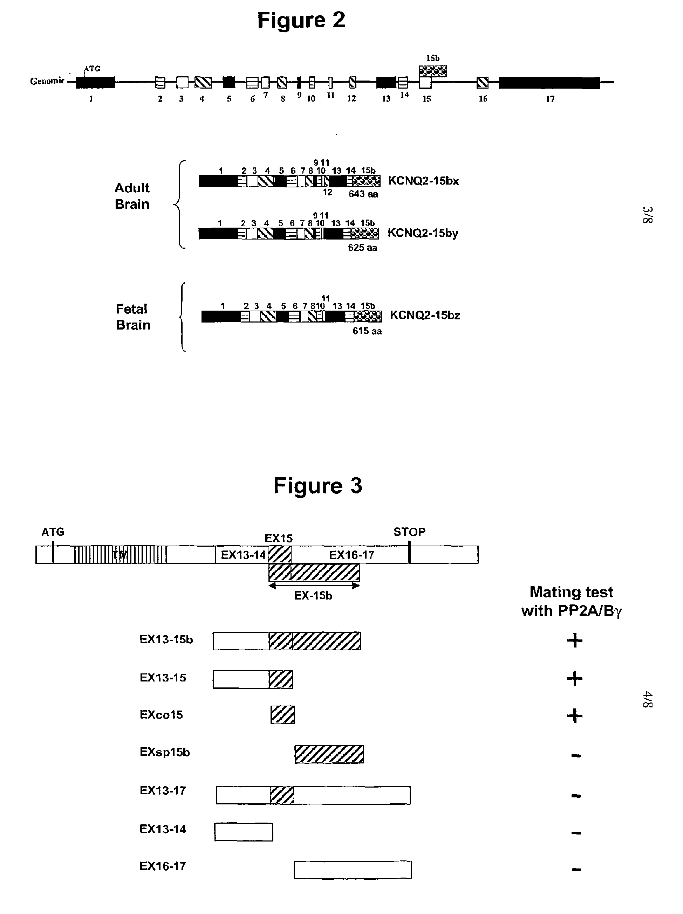

The genomic structure of the KCNQ2 gene is shown on figure 3 and in table 1. The KCNQ2 gene is comprised of 17 exons. None of the novel splice variants displays the exons corresponding to exons 15, 16 and 17 of the KCNQ2 gene. They all display a novel exon, exon 15b, which encodes the amino acids at position 545 to 643 of SEQ lD NO: 2. The 44 first amino acids encoded by exons 15 and 15b are identical (amino acids at position 545 to 588 of SEQ ID NO: 2). The 55 last amino acids encoded by exon 15b are unique to exon 15b (amino acids at position 589 to 643 of SEQ ID NO: 2). Furthermore, the novel splice variants do not display exons 16 and 17 of KCNQ2 -fl. The most carboxyl-terminal exon of these splice variants is exon 15b. SEQ ID NO: 2 further comprises exon 1 to exon 14 of KCNQ2. Exon 12 of KCNQ2 is lacking in SEQ ID NO: 4. Exons 9 and 12 of KCNQ2 are lacking in SEQ ID NO: 6.

The insert of the 2E11 clone, which corresponds to a partial cDNA, comprises exons 13, 14 and 15b.

**Table 1**

| Exon No. | SEQ ID NO:1 | Encodes AA of SEQ ID NO:2 | SEQ ID NO:3 | Encodes AA of SEQ lD NO:4 | SEQ ID NO:5 | Encodes AA of SEQ lD NO:6 | Encodes AA of SEQ ID NO: 7 |
|---|---|---|---|---|---|---|---|
| 1 | 1-296 | 1-98 | 1-296 | 1-98 | 1-296 | 1-98 | 1-98 |
| 2 | 297-387 | 100-129 | 297-387 | 100-129 | 297-387 | 100-129 | 100-129 |
| 3 | 388-514 | 130-171 | 388-514 | 130-171 | 388-514 | 130-171 | 130-171 |
| 4 | 515-690 | 173-230 | 515-690 | 173-230 | 515-690 | 173-230 | 173-230 |
| 5 | 691-816 | 231-272 | 691-816 | 231-272 | 691-816 | 231-272 | 231-272 |
| 6 | 817-927 | 273-309 | 817-927 | 273-309 | 817-927 | 273-309 | 273-309 |
| 7 | 928-1023 | 310-341 | 928-1023 | 310-341 | 928-1023 | 310-341 | 310-341 |
| 8 | 1024-1118 | 342-372 | 1024-1118 | 342-372 | 1024-1118 | 342-372 | 342-372 |
| 9 | 1119-1148 | 374-382 | 1119-1148 | 374-382 | / | / | 374-382 |
| 10 | 1149-1217 | 384-405 | 1149-1217 | 384-405 | 1119-1187 | 374-395 | 384-405 |
| 11 | 1218-1247 | 407-415 | 1218-1247 | 407-415 | 1188-1217 | 397-405 | 407-415 |
| 12 | 1248-1301 | 417-433 | / | / | / | / | 417-433 |
| 13 | 1302-1525 | 435-508 | 1248-1471 | 417-490 | 1218-1441 | 407-480 | 435-508 |
| 14 | 1526-1631 | 510-543 | 1472-1577 | 492-525 | 1442-1547 | 482-515 | 510-543 |
| 15 | / | / | / | / | / | / | 545-587 |
| 15b | 1632-1929 | 545-643 | 1578-1875 | 527-625 | 1548-1845 | 517-615 | / |
| 16 | / | / | / | / | / | / | 588-629 |
| 17 | / | / | / | / | / | / | 630-872 |

### EXAMPLE 3: Yeast mating test

### 1. Construction of vectors

### 1.1. EX13-17, which comprises exons 13, 14, 15, 16 and 17.

The pGADT7-EX13-17 plasmid was constructed as follows: a 1414-bp fragment was first PCR-amplified from a Human total brain cDNA library (Marathon-Ready cDNA, Clontech) with two gene-specific primers of SEQ ID NO: 15 and of SEQ ID NO: 16. This first PCR product was then amplified with a second set of gene-specific primers of SEQ ID NO: 17 and 5'of SEQ ID NO: 18. These primers are extended, respectively, with *Eco*RI and *Bam*Hl cloning sites. After digestion with *Eco*Rl and *Bam*Hl restriction enzymes, the 1338-bp purified fragment was ligated to the same cloning sites of pGADT7 (Clontech).

### 1.2. EX13-15, which comprises exons 13, 14 and 15.

The pGADT7-EX13-15 plasmid was obtained as follows: a 484-bp fragment was PCR-amplified with primers of SEQ ID NO: 19 and of SEQ ID NO: 20, which are respectively extended with *Eco*Rl and *Bam*Hl cloning sites, from the first PCR product of the pGADT7 - EX13-17 construction. The resulting fragment was then digested with *Eco*Rl and *Bam*Hl, purified, and ligated to the same cloning sites of pGADT7 (Clontech).

### 1.3. EX16-17, which comprises exons 16 and 17.

The pGADT7-EX16,17 plasmid was obtained as follows: a 883-bp fragment was PCR-amplified with primers of SEQ ID NO: 21 and of SEQ ID NO: 22, which are respectively extended with *Eco*RI and *Bam*HI cloning sites, from the first PCR product of the pGADT7 - EX13-17 construction. The resulting fragment was then digested with *Eco*RI and *Bam*HI, purified, and ligated to the same cloning sites of pGADT7 (Clontech).

### 1.4. EXsp 15b, which comprises the region unique to exon 15b.

The pGADT7-EXsp15b plasmid was constructed as follows : a 400-bp fragment was PCR-amplified with a primer of SEQ ID NO: 23 extended with *Eco*RI cloning site, and with a primer of SEQ ID NO: 24 from the pACT2-2E11 plasmid (see example 1). The resulting fragment was then digested with *Eco*Rl and *Xho*I, purified, and ligated to the same cloning sites of pGADT7 (Clontech).

### 1.5. EXco15, which comprises the region common to exon 15 and exon 15b.

The pGADT7-EXco15 domain plasmid was constructed as follows: a 146-bp fragment was PCR-amplified with primers of SEQ ID NO: 25 and of SEQ ID NO: 26, which are respectively extended with *Eco*RI and *Bam*HI cloning sites, from the pACT2-2E11 plasmid. The resulting fragment was then digested with *Eco*RI and *Bam*HI, purified, and ligated to the same cloning sites of pGADT7 (Clontech).

### 1.6. EX13-14, which comprises exons 13 and 14.

The pGADT7-EX13-14 plasmid was constructed as follows: a 300-bp fragment was PCR-amplified with primers of SEQ ID NO: 27 and of SEQ ID NO: 28, which are respectively extended with *E*coRI and *Bam*HI cloning sites, from the pACT2-2E11 plasmid. The resulting fragment was then digested with *E*coRI and *Bam*HI, purified, and ligated to the same cloning sites of pGADT7 (Clontech).

### 2. Protocol of the yeast mating test

Yeast mating tests were performed to map the interaction domains between the different partners. The chosen *Saccharomyces cerevisiae* mating partner strains (AH109 and Y184) were transformed separately with the plasmids to be tested in combination with the plasmid of interest. The lithium acetate transformation procedure was done according to the manufacturer's instructions (Matchmaker Two-Hybrid system, Clontech). Transformants were selected on the appropriate SD dropout medium (Clontech). One fresh colony of each type to use was picked from the working stock plates and both placed in one 1.5 ml microcentrifuge tube containing 0.5 ml of YPD medium (C lontech). Cells were then incubated for 24 hr at 30°C with shaking at 200 rpm. 100 µl of a 1:100 dilution of the mating culture were then spread on the appropriate SD medium: SD/-Leu/-Trp, and SD/-Leu/-Trp/-His/-Ade. After 7 to 15 days of growth on selective medium positive colonies were counted.

### 3. Results of the direct mating tests between KCNQ2 polypeptides and PP2A/Bγ

Mating tests between each of the above constructions and the pGBKT7 *-PPP2R2C* construction described in example 1 were performed. The results are shown on Figure 2. The sign "+" indicates that colonies grew, thus indicating that the tested polypeptide is capable of interacting with PP2A/Bγ. The sign "-" indicates that no colony grew, thus indicating that the tested polypeptide does not interact with PP2A/Bγ.

EX13-17, EX16-17, EX13-14 and EXsp15b do not interact with PP2A/Bγ. EX13-15b, EX13-15 and EXco15 interact with PP2A/Bγ. EX13-15b interacts with PP2A/Bγ, showing that KCNQ2-15b polypeptides are capable of interacting with PP2A/Bγ. Since EX13-15b, EX13-15 and EXco15 but *not* EXsp15b interact with PP2A/Bγ, the common region between exon 15 and exon 15b plays a role in this interaction. Furthermore, since EX13-17 does not interact with PP2A/Bγ, the fact that exon 15 or that exon 15b is located at the most carboxyl extremity of the KCNQ2 polypeptide is of importance for efficient interaction with PP2A/B γ.

### 4. Results of the direct mating tests between different KCNQ2 polypeptides

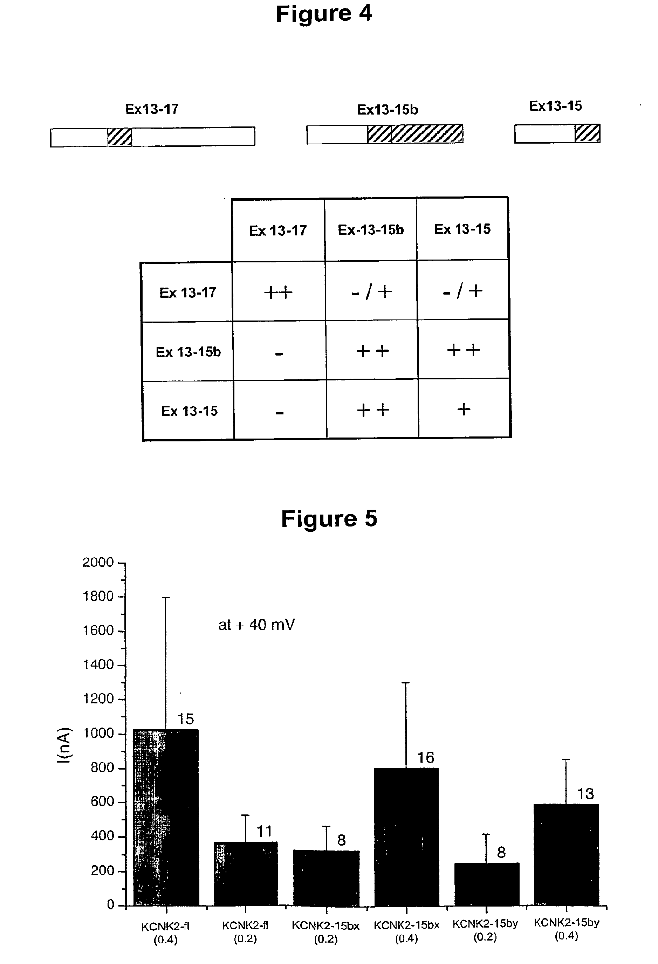

Mating tests between the different above constructions were performed, and the results are shown on Figure 4. 4 mating tests were performed for each pair of constructs and the results are shown on Figure 3. The sign "++" indicates that all 4 colonies grew. The sign "+" indicates that 3 colonies out of 4 grew. The sign "-/+" indicates that 1 colony out of 4 grew. The sign "-" indicates that no colony grew.

This experiment shows that KCNQ2-15b polypeptides can associate and form homodimers. KCNQ2-15b polypeptides can also associate and form heterodimers with KCNQ2 polypeptides comprising exon 15 at their carboxyl -terminal extremity. KCNQ2-15b polypeptides associate with KCNQ2-fl polypeptides at a lesser extent.

### EXAMPLE 4: Expression and Purification of

### Glutathione S-Transferase Fusion Proteins

### 1. Construction of plasmids

### 1.1.pGBKT7-2E11

The pACT2-2E11 plasmid rescued from yeast two-hybrid screening was digested with *Eco*RI and *Bg*/II and the resulting 687-bp fragment inserted after purification into *Eco*RI and *Bam*HI cloning sites of the pGBKT7 vector (Clontech).

### 2.2. pGEX-2TK-2E11

A partial cDNA of the KCNQ2 splice variants was PCR-amplified from the pACT2-2E11 plasmid rescued from yeast two-hybrid screening using a gene-specific primer of SEQ ID NO: 29 and a primer in the pACT2 vector of SEQ ID NO: 30. These primers were respectively extended with *Bam*HI and *Eco*RI cloning sites. The 892-bp PCR product was digested with *Bam*HI and *Eco*RI, purified and inserted into *Bam*HI and *Eco*RI sites of pGEX-2TK vector (Amersham Pharmacia Biotech). The pACT2 plasmid used for this construction was recovered from diploid cells as follows: a fresh colony of diploid cells was inoculated into 5 ml of SD/-Leu/-Trp (Clontech) and let to grow overnight at 30°C with shacking at 200-250 rpm. Cells corresponding to 2 ml of the overnight culture were spun down by centrifuging at 4300 rpm for 10 min. The pellet was resuspended in 100 µl of zymolyase (1U/µl) (Seikagaku Corporation) and incubated 1 hr at 30°C. Then 100 µl of a proteinase K mix (100 mM NaCl, 10 mM Tris-HCl pH [pH 8.0], 25 mM EDTA, 0.5 % SDS, 0.1 mg/ml proteinase K) were added for 2.5 hr at 40°C. DNA was extracted by two successive phenol:chloroform steps and precipitated with 0.3 M sodium acetate and 2.5 volumes of ethanol. DH10B ElectroMAX competent cells (Invitrogen) were transformed with DNA and selected on agar plates supplemented with 120 µg/ml Ampicillin. The protein encoded by pGEX-2TK-2E11 was named GST-2E11.

### 1.3. pGEX-2TK-PPP2R2C

A 1485-bp fragment of *PPP2R2C* encompassing nucleotides 55-1540 of the full-length cDNA of PP2A/Bγ (genbank accession number AF086924) was PCR-amplified from the pGBKT7-PPP2R2C plasmid using gene-specific primers of SEQ ID NO: 31 and of SEQ ID NO: 32, which are respectively extended with *Bam*Hl and *Eco*Rl cloning sites. The fragment was digested by *Bam*Hl and *Eco*Rl, purified and ligated to the same cloning sites of pGEX-2TK vector (Amersham Pharmacia Biotech). The protein encoded by pGEX-2TK-2E11 is named GST-PPP2R2C.

### 1.4. pGEX-2TK-KCNQ2-Cter

A 1393-bp fragment of a KCNQ2-fl encompassing nucleotides 1544-2924 of the full-length cDNA (genbank accession number AF033348) was PCR-amplified from the pCMV-HA-KCNQ2-iso1 construction using gene-specific primers: of SEQ ID NO: 33 and of SEQ ID NO: 34, which are respectively extended with Xhol and *Eco*RI cloning sites. This PCR product was digested with *Xho*l and *Eco*RI, purified and substituted at the same sites for a 767-bp *Xho*I-*Eco*RI fragment of the pGEX-2TK-2E11 plasmid. The pCMV-HA-KCNQ2-iso1 plasmid used for the construction of pGEX-2TK-KCNQ2-Cter was obtained as follows: the full-length coding region for KCNQ2-fl (encompassing nucleotides 126-2924 of the full-length cDNA, genbank accession number AF033348) was first amplified from a Human brain cDNA library (Marathon-Ready cDNA, Clontech) using gene specific primers of SEQ ID NO: 35 and of SEQ ID NO: 36, which are respectively extended with *Eco*RI and Bg/ll cloning sites. The PCR product was digested with *Eco*RI and Bg/ll, purified and ligated to the same cloning sites of the pCMV-HA vector (Clontech). The protein encoded by pGEX-2TK-2E11 is named GST-KCNQ2-Cter.

### 2. Expression and purification

Glutathione S-transferase fusion protein expression and purification by adapting the method described by Kaelin et al. (1991, Cell, 64:521-532). Overnight cultures of MAX Efficiency DHSαF'IQ competents cells (Invitrogen) transformed with either the pGEX2TK plasmid or the pGEX2TK-2E11, pGEX2TK-KCNQ2-Cter, and pGEX2TK-PPP2R2C recombinants were diluted 1:10 in LB medium containing ampicillin (100 µg/ml) and incubated for 1 hr at 37°C. Isopropyl-β-D-thiogalactopyranoside (IPTG, Promega) was then added to a final concentration of 0.1 mM and bacteria let to grow for 3 additional hours at 37°C. For fusion proteins recovery using the glutathione-Sepharose 4B beads (Amersham Biosciences), bacterial cultures were pellet ed by centrifugation at 5000 x g for 15 min at 4°C and resuspended in 1/10 vol NETN (20mM Tris-HCl [pH 8.0], 120mM NaCl, 1mM EDTA, 0.5% Nonidet P-40) supplemented with 1mM phenylmethylsulfonyl fluoride (PMSF, Sigma) and one tablet of protease inhibitors cocktail (Complete^{™} mini, Roche) for 7 ml of buffer. The bacteria were then lysed on ice by mild sonication and centrifuged at 10,000 x g for 10 min at 4°C. Aliquots (1 ml) of bacterial clear lysates were then rocked for 1 hr at 4°C with 50 µl of glutathione-Sepharose 4B beads, which had been previously washed four times in NETN containing 1% Albumin Bovine (BSA fraction V, Sigma) and resuspended (final concentration 1:1 [v/v]) in NETN. The glutathione-Sepharose 4B beads were then washed three times with NETN. For recovery of the bound recombinants proteins, beads were washed two more times with 100mM Tris-HCl [pH 8.0], 120 mM NaCl and elution was performed in the same buffer containing 20 mM glutathione (Sigma). Quantification of the eluted fusion proteins w as performed by the standard Bradford's method (Biorad Protein Assay).

### EXAMPLE 5: In vitro Labeling of the GST Fusion Proteins

Beads with bound GST fusion proteins corresponding to 1 ml of bacterial clear lysate were washed three times in NETN and one time with HMK buffer without DTT (20 mM Tris-HCl [pH 7.5], 120 mM NaCl, 12 mM MgCl₂). Beads were then resuspended in 30 µl of reaction mix (3 µl of 10X HMK Buffer with 20 mM DTT, 10 units of Protein Kinase A Catalytic Subunit [PKA from bovine heart, 250 units/vial, Sigma] in 40mM DTT, 2 µl of [³²P]-γATP 6000 Ci/mMole and 24µl of distilled water) and incubated at 4°C for 30 min. During incubation beads were resuspended time to time by flicking. Reaction was stopped by adding 1 ml of HMK stop buffer (10 mM Sodium Phosphate [pH 8.0], 10 mM Sodium Pyrophosphate, 10 mM EDTA, 1 mg/ml BSA) and beads washed five times with NETN buffer. Elution of radiolabeled fusion proteins was carried out with 1 ml of freshly prepared 20 mM glutathione in 100 mM Tris-HCl [pH 8.0], 120 mM NaCl as previously described.

### EXAMPLE 6: Solid Phase Overlay assay

### 1. Protocol of the solid phase overlay assay

Solid phase overlay assays were performed by adapting the method described by Kaelin and collaborators (Kaelin et al., 1992, Cell, 70:351-364). 100 ng, 10 ng and 0.1 ng of GST and GST-2E11 recombinant proteins were resolved by 9% SDS-PAGE and were transferred by electroblotting onto nitrocellulose membrane (nitrocellulose transfer membrane Protran BA 83, Schleicher and Schuell). The membrane were then blocked in HBB buffer (25 mM Hepes-KOH [pH 7.7], 25 mM NaCl, 5 mM MgCl₂) with 5% (w/v) non-fat dry milk, 1 mM DTT, 0.05% Nonidet P-40 for 1 hr at room temperature. The binding reaction was carried out at room temperature in Hyb75 buffer (20 mM Hepes [pH 7.7], 75 mM KCI, 2.5 mM MgCl₂, 0.1 mM EDTA, 0.05% Nonidet P-40) with 1 % (w/v) non-fat dry milk, 1 mM DTT, 1 mM PMSF and 3.5 10⁵ dpm of a [³²P]-γATP GST-PPP2R2C radiolabeled recombinant protein used as a probe. After 4.5 hr of incubation, the memb rane was washed with Hyb75 buffer, 1 mM DTT, 1% (w/v) non-fat dry milk three times for 15 min at room temperature. The blots were analyzed by autoradiography.

### 2. Results

This experiment was performed to validate the interaction between KCNQ2-15b polypeptides and PP2A/Bγ. In this experiment, the PP2A/Bγ subunit was radiolabeled but not the proteins present on the nitrocellulose membrane. Thus, a signal appears when visualized by autoradiography only if the loaded protein interacts with PP2A/Bγ. GST-2E11 corresponds to a fusion protein between a KCNQ2-15b polypeptide comprising exons 13, 14 and 15b and GST. GST corresponds to the negative control. In the three lines loaded with the GST-2E11 recombinant protein, bands located at a position corresponding to a pr otein of a size of about 45 kD appeared. This corresponds to the protein size expected for the GST -2E11 protein. Furthermore, the intensity of the bands was proportional to the quantity of loaded GST -2E11. Thus GST-2E11 interacts with PP2A/Bγ. In the three lines loaded with the GST protein, no band appeared, showing that PP2A/Bγ does not interact with the GST protein. Thus the interaction between PP2A/Bγ and the GST-2E11 fusion protein is due to the part of the protein encoding 2E11 and not to the part of the protein encoding GST. This experiment indicates that KCNQ2-15b polypeptides can interact with PP2A/Bγ *in vitro.* Furthermore, this shows that KCNQ2-15b polypeptides can interact with PP2A/Bγ without a third binding partner, a hypothesis that can not be excluded by a yeast-two hybrid assay.

### EXAMPLE 7: In vitro Phosphorylation Assay With Recombinant GSK-3β Kinase and In vitro dephosphorylation with HTB-14 Whole Cell Extracts.

### 1. Phosphorylation assays

Phosphorylation assays were performed to determine wh ether the phsophorylation state of KCNQ2-15b is modulated by GSK3β, a kinase that plays an important role in the central nervous system by regulating various cytoskeletal processes through its effects on MAP1B, tau and synapsin 1. GSK3β is known to be inhibited by two mood stabilizing agents used in treatment of bipolar disorder, lithium and valporate.

### 1.1. Protocol

Expression and purification of the GST-2E11 fusion protein were performed as described above. Beads with bound fusion protein corresponding to 1 ml of bacterial clear lysate were washed three times in NETN and one time with HMK buffer without DTT (20 mM Tris-HCl [pH 7.5], 120 mM NaCl, 12 mM MgCl₂). Beads were resuspended in 240 µl of reaction mix (24 µl of 10X HMK Buffer with 20 mM DTT, 40 units of Protein Kinase A Catalytic Subunit [PKA from bovine heart, 250 units/vial, Sigma] in 40mM DTT, 5 µl of 24 mM ATP and 207 µl of distilled water) and incubated for 30 min at room temperature. Beads were then washed three times in NETN buffer and one time in GSK-3β reaction buffer (20 mM Tris-HCl [pH 7.5], 10 mM MgCl₂, 5 mM DTT) (New England Biolabs). Beads were then resuspended in 50 µl of reaction mix (5 µl of 10X GSK-3β reaction buffer, 1µl of [³²P]γATP 10mCi/ml, 50 U of recombinant GSK-3β[New England Biolabs], and distilled water for a final volume of 50 µl) and incubated at room temperature for 30 min. After three washes in NETN buffer, phosphorylated proteins were boiled in 2X Sample Buffer (125 mM Tris-HCl [pH 6.8], 4% SDS, 20% glycerol, 1.4 M β-Mercapto ethanol), resolved by 10% SDS-PAGE, and visualized by autoradiography.

### 1.2. Results

In this phosphorylation assay, non-radiolabeled polypeptides to be tested are incubated in the presence of GSK-3β, PKA and radioactive ATP. The proteins are then resolved by a 10% SDS-PAGE migration and visualized by autoradiography. A signal is visualized by autography only if the protein to be tested is phosphorylated by GSK -3β and PKA during incubation. In the line loaded with the GST-2E11 protein, which corresponds to the fusion protein between a KCNQ2-15b polypeptide comprising exons 13, 14 and 15b and the GST polypeptide, a band located at a position corresponding to a protein of a size of about 45 kD did appear. This is the size expected for the GST-2E11 protein. Thus the GST-2E11 protein is phosphorylated by GSK-3β and PKA *in vitro.* Three experiments corresponding to negative controls were performed in parallel. One experiment was performed without adding the GSK-3β kinase during incubation, one was performed without adding the PKA kinase during incubation, and one was performed with a GST protein instead of a GST-2E11 protein. No bands appeared in the three lines corresponding to the negative controls.

Accordingly, this experiment shows that KCNQ2-15b polypeptides are synergistically phosphorylated by the GSK-3β and PKA kinases *in vitro.*

This result was confirmed by a competition experiment in which CREB phosphopeptides, which are known to be phosphorylated by GSK-3β and PKA, were added during incubation. In this competition experiment, 5 µg of CREB phosphopeptides (New England Biolabs) was added to the kination mix. A band did still appear at a position corresponding to the size of GST-2E11, but the intensity of the band was very significantly lower.

The influence of LiCl on the phosphorylation state of GST-2E11 was further studied by adding LiCl to the kination mix at a final concentration of 0, 8.3, 25, 75 and 225 mM respectively. The intensity of the band appearing at a position of about 45 kD decreased in the presence of LiCl, and the intensity of the signal was negatively correlated with the concentration of LiCl added to the kination mix. In the presence of about 50 mM LiCl, the phosphorylation state of GST-2E11 was reduced by 50%.

This shows that LiCl, a well-known mood-stabilizing agent used in the treatment of bipolar disorder, inhibits phosphorylation of KCNQ2-15b polypeptides *in vitro.*

### 2. Dephosphorylation assays

Dephosphorylation assays were performed to determine whether the phophorylation state of KCNQ2-15b polypeptides is modulated by PP2A.

### 2.1. Protocol

*In vitro* phosphorylated GST-2E11 fusion protein was incubated at room temperature for 30 min with 500 µg of whole cell extracts of Human glioblastoma, astrocytoma cell line (ATCC number: HTB-14) with or without 400 µM of the PP2A phosphatase inhibitor okadaic acid (Sigma). HTB-14 whole cell extracts were prepared as follow: cells were washed three times with ice-cold TBS buffer (10 mM Tris-HCl [pH 8.0], 120 mM NaCl) and lysed at 4°C for 30 min in EBC buffer (50 mM Tris-HCl [pH 8.0], 120 mM NaCl, 0.5 % Nonidet P-40). Then the lysate was centrifugated for 10 min at 13.000 x g at 4°C to pellet cell debris. Proteins present in the supernatant were quantified by the standard Bradford's method (Bio-Rad Protein Assay). The proteins were then resolved by 10% SDS-PAGE, and visualized by autoradiography.

### 2.2. Results

The phosphorylated radiolabeled GST-2E11 proteins obtained from the previous assay were incubated in the presence of HTB-14 cell extracts containing the PP2A phosphatase to determine whether PP2A is capable of dephosphorylating GST-2E11 proteins. In this experiment, a protein that is dephosphorylated by PP2A is not radioactive after incubation in the presence of HTB-14 cell extracts any more. Thus dephosphorylation of the GST-2E11 protein is monitored by disappearance of the signal visualized by autoradiography. One line of the 10% SDS -PAGE gel was loaded with phosphorylated GST-2E11 fusion proteins incubated in the absence of HTB-14 cell extracts, as reference for the intensity of the band appearing for phosphorylated GST-2E11 proteins. In the line loaded with GST-2E11 fusion proteins incubated in the presence of HTB-14 cell extracts, the band had an extremely weaker intensity. Thus GST-2E11 fusion proteins are dephosphorylated when incubated in the presence of HTB-14 cell extracts. When the GST-2E11 fusion protein was incubated in the presence of HTB-14 cell extracts and okadaic acid, a known PP2A phosphatase inhibitor, the intensity of the band was only slightly weaker than the intensity of the band corresponding to phosphorylated GST-2E11.

Thus the PP2A phosphatase is responsible of the dephosphorylation observed for GST-2E11 fusion proteins incubated in the presence of HTB-14 cell extracts. Accordingly, this experiment shows that KCNQ2-15b polypeptides are dephosphorylated by the PP2A phosphatase *in vitro.*

### EXAMPLE 8: Cell Culture, Transfection,

### Immunoprecipitation and Western Blot Analysis

### 1. Cell cultures

HEK293-H cells (Gibco Invitrogen Corporation) were grown in DMEM medium (Gibco Invitrogen Corporation) supplemented with 0.1 mM Non-Essential Amino Acids and 10% Fetal Bovine Serum (Gibco Invitrogen Corporation), and transiently transfected with 20 µg of the pCMV-Myc-3H9 or pCMV-Myc-3H2 plasmids per 60 mm dish using the Invitrogen calcium phosphate transfection kit and protocols. 48 hr after transfection cells were washed three times with ice-cold phosphate buffer (PBS, Gibco Invitrogen Corporation), scraped and solubilized for 2 hr at 4°C in solubilization buffer containing 150 mM NaCl, 5 mM EDTA, 1% Triton X-100, 0.1% sodium deoxycholate, 10 mM Tris-HCl [pH 8.0] and supplemented with protease inhibitors (1 mM phenylmethylsulfonyl fluoride, one tablet of Complete^{™} mini protease inhibitors cocktail [Roche]) and phosphatase inhibitors (1 mM Na ₃VO₄ and 1 mM NaF). The lysate was then centrifugated for 10 min at 13.000 x g at 4°C to pellet cell debris. Proteins present in the supernatant were quantified by the standard Bradford's method (Bio - Rad Protein Assay).

### 2. Immunoprecipitation

500 µg (final volume: 500 µl) of the clear cell lysate were incubated for 2 hr at 4°C with 1 µl of rabbit preimmune serum and 50 µl of protein A Sepharose CL-4B beads (Amersham Pharmacia Biotech) saturated with 1 % Albumin Bovine (BSA fraction V, Sigma). Depleted supernatants were then incubated overnight at 4°C with 1 µg of anti-Myc monoclonal antibody (Myc-Tag 9B11 monoclonal antibody, Cell Signaling). Protein A Sepharose CL-4B beads saturated with 1% Albumin Bovine were then added and the mixture incubated at 4°C for 2 addtional hours. After five was hes with ice-cold solubilization buffer immuno-complexes were boiled in 2X Sample Buffer (125 mM Tris-HCl [pH 6.8], 4% SDS, 20% glycerol, 1.4 M β-Mercapto ethanol), resolved by 8% SDS-PAGE and subjected to 3. Western blot

Proteins were transferred onto nitrocellulose membrane (nitrocellulose transfer membrane Protran BA 83, Schleicher and Schuell) using Towbin buffer (Towbin et al., 1979, PNAS, 76:4350-4354) and an electrotransfer device. After transfer, membranes were blocked, in 5% non-fat dried milk in TBST (10 mM Tris-HCl [pH 8.0], 150 mM NaCl, 0.05% Tween 20) supplemented with sodium azide (0.1%) for 2 hr, and then incubated for 16 hr at room temperature with the anti-Myc monoclonal antibody (Myc-Tag 9B11 monoclonal antibody, Cell Signaling) diluted 1:1000 in the same buffer. After several washes with TBST, the blot was incubated with a horseradish peroxidase-conjugated secondary antibody (Antimouse IgG, Fab specific, peroxidase conjugate, Sigma) diluted 1:5000 and developed using ECL Western blotting detection reagents (Amersham Biosciences).

### EXAMPLE 9: Electrophysiological Analysis

### 1. Protocols

### 1.1. cDNA injection in Xenopus laevis oocytes

The animal was anesthetized and pieces of the ovary were surgically removed and individual oocytes were dissected away in a saline solution (ND96) containinng 96 mM NaCl, 2 mM KCI, 2 mM CaCl2, 2 mM MgCl2 and 5 mM HEPES at pH 7.4. Stage V and VI oocytes were treated at room temperanre for 2h with collagenase type 1A (1mg/ml) in the presence of 0.2 mg/ml trypsin inhibitor in saline solution to discard follicular cells. The concentrations were determined by measuring the absorbance at 260 nm. DNA corresponding to KCNQ2, 3H2 and 3H9 K+ channels were subcloned in PEKO vector in order to generate the respective cRNAS. cRNA concentrations were measured by absorbance at 260nM. cRNA solutions were injected (about 50 nL/oocyte) using a pressure microinjector (Inject+matic, Genève). Oocytes were then kept for 2-6 days in ND96 solution supplemented wirn 50U/mL penicillin and 50 U/mL streptomycin.

### 1.2. Electrophysiological measurements

In a 0.3 rmL perfusion chamber, a single oocyte was impaled with two standard glass microelectrode (0.5-2 Mohm resistance) filled with 3M KCl and maintained under voltage clamp using a Dagan TEV2OO amplifier system, USA. Electrical stimulations, data acquisition and analyses were performed using pClamp software (Axon Instruments, USA). Current to voltage relationships were obtained applying incremental depolarizing voltage steps (10 mV increment) from a holding potential of -80 mV (equilibrium potential for K+ ions) Repolarizations to -60mV allowed K+ channel deactivation measurements from the "tail currents".

### 2. Results

The activity of KCNQ2-15bx and of KCNQ2-15by homotetrameric potassium channels was tested and compared to the activity of KCNQ2-fl homotetrameric potassium channels. 0.2 ng or 0.4 ng of DNA were injected to the oocytes. The results are shown on Figures 5, on which the intensity of the M-current generated by the potassium channels is indicated. An intensity of about 1 pA is found for the current generated by a of KCNQ2 -fl homotetrameric potassium channel when 0.4 ng of DNA is injected. This value is similar to the value reported by scientific literature. A KCNQ2-15bx homotetrameric potassium channel yields a current of about 800 nA when 0.4 ng of DNA is injected, and a KCNQ2-15by homotetrameric potassium channel yields a courant of about 700 nA when 0.4 ng of DNA is injected. Thus the KCNQ2-15bx and KCNQ2-15by splice variants can associate as functional homomeric potassium channels *in vivo.*

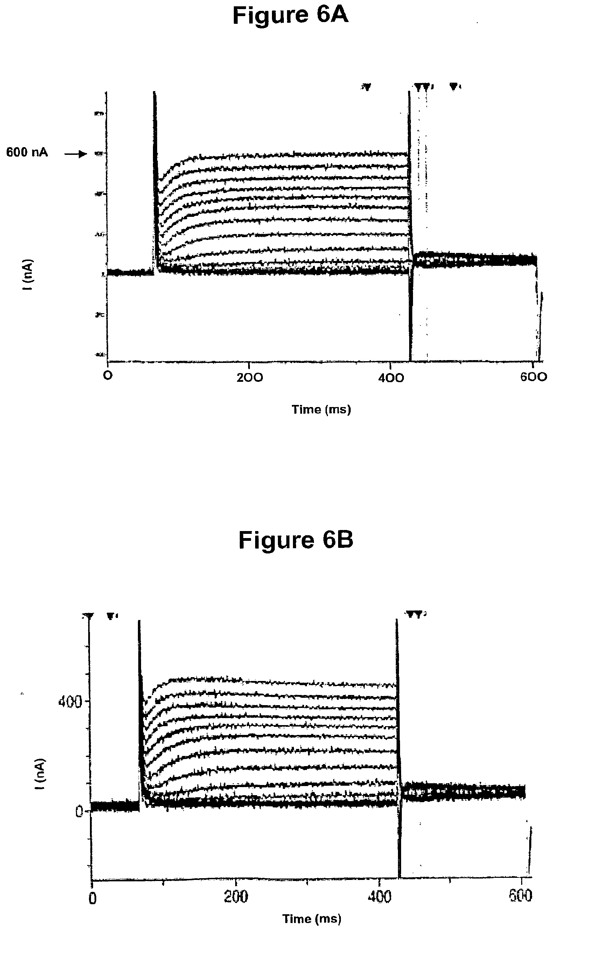

Figure 6A and Figure 6B show the voltage clamp traces corresponding to the currents generated at different voltages by KCNQ2-15bx (Figure 6A) and by KCNQ2-15by (Figure 6B) homotetrameric potassium channels. The slow activation that is observed on the traces is a characteristic feature of members of the KCNQ potassium channel family.

### EXAMPLE 10: Collection Of DNA Samples From Affected And Non -Affected Individuals.

Donors were unrelated and healthy. The DNA f rom 100 individuals was extracted and tested for the detection of the biallelic markers.
30 ml of peripheral venous blood were taken from each donor in the presence of EDTA. Cells (pellet) were collected after centrifugation for 10 minutes at 2000 rpm. Red cells were lysed by a lysis solution (50 ml final volume: 10 mM Tris pH7.6; 5 mM MgCl₂; 10 mM NaCl). The solution was centrifuged (10 minutes, 2000 rpm) as many times as necessary to eliminate the residual red cells present in the supernatant, after resuspension of the pellet in the lysis solution.
The pellet of white cells was lysed overnight at 42°C with 3.7 ml of lysis solution composed of:
- 3 ml TE 10-2 (Tris-HCl 10 mM, EDTA 2 mM) / NaCl 0 4 M
- 200 µl SDS 10%
- 500 µl K-proteinase (2 mg K-proteinase in TE 10-2 / NaCl 0.4 M).
For the extraction of proteins, 1 ml saturated NaCl (6M) (1/3.5 v/v) was added. After vigorous agitation, the solution was centrifuged for 20 minutes at 10000 rpm.
For the precipitation of DNA, 2 to 3 volumes of 100% ethanol were added to the previous supernatant, and the solution was centrifuged for 30 minutes at 2000 rpm. The DNA solution was rinsed three times with 70% ethanol to eliminate salts, and centrifuged for 20 minutes at 2000 rpm. The pellet was dried at 37°C, and resuspended in 1 ml TE 10-1 or 1 ml water. The DNA concentration was evaluated by measuring the OD at 260 nm (1 unit OD = 50 µg/ml DNA). To determine the presence of proteins in the DNA solution, the OD 260 / OD 280 ratio was determined. Only DNA preparations having a OD 260 / OD 280 ratio between 1.8 and 2 were used in the subsequent examples described below.
The pool was constituted by mixing equivalent quantities of DNA from each individual.

### EXAMPLE 11: Amplification Of Genomic DNA By PC R

The amplification of specific genomic sequences of the DNA samples of Example 10 was carried out on the pool of DNA obtained previously. In addition, 50 individual samples were similarly amplified.
PCR assays were performed using the following protocol:

| | |
|---|---|
| Final volume | 25 µl |
| DNA | 2 ng/µl |
| MgCl₂ | 2 mM |
| dNTP (each) | 200 µM |
| primer (each) | 2.9 ng/µl |
| Ampli Taq Gold DNA polymerase | 0.05 unit/µl |
| PCR buffer (10x = 0.1 M TrisHCl pH8.3 0.5M KCl) | 1x |

Each pair of first primers was designed using the sequence information of genomic DNA sequences and the OSP software (Hillier & Green, 1991).

### Primers Biallelic markers located in PPP2R2C

The genomic sequence of PPP2R2C that is shown as SEQ ID NO: 37 was constructed upon bioinformatic analysis based on (i) BAC clones constructed at Genset S.A.; (ii) BAC clones corresponding to EMBL Accesion Nos. AC114815.5, AC004599.6, AC122939.3 and AC004689.5; and (iii) RefseqN Accession No. NT_006051. The polymorphisms were identified as described in examples 12 and 13, and validated as described in example 14.

### Biallelic markers located in the KCNQ2 gene

The biallelic markers located in the KCNQ2 gene were found using data provided by Celera. Each of these markers were further validated as described in example 14.

Table 2A indicates the position on SEQ ID NO: 37 of pairs of primers that were used to amplify specific regions of PPP2R2C. Table 2B indicates the position of the primers on SEQ ID Nos 42 to 47, which were used to amplify specific regions of KCNQ2. The orientation of the primer is indicated in the third column. The sign (+1) indicates that the sequence of the primer is identical to the corresponding region of SEQ ID Nos. 37 and 42 to 47. The sign (-1) indicates that the sequence of the primer is co mplementary to the corresponding region of SEQ ID Nos. 37 and 42 to 47.

**Table 2A: Primer location in PPP2R2C**

| **Name of the amplified region** | **Position on SEQ ID NO: 37** | **Orientation** |
|---|---|---|
| 24-257 | 109495 to 109512 | (+1) |
| | 109963 to 109982 | (-1) |
| 99-24169 | 83709 to 83729 | (+1) |
| | 84146 to 84164 | (-1) |
| 99-24175 | 117228 to 117248 | (+1) |
| | 117659 to 117677 | (-1) |
| 24-247 | 99290 to 99309 | (+1) |
| | 99719 to 99738 | (-1) |

**Table 2B: Primer location in the KCNQ2 gene**

| ***Name of the amplified region*** | ***SEQ ID No.*** | ***Position*** | ***Orientation*** |
|---|---|---|---|
| 30-4 | SEQ ID NO: 42 | 244 to 263 | (+1) |
| | | 324 to 343 | (-1) |
| 30-2 | SEQ ID NO: 43 | 240 to 258 | (+1) |
| | | 319 to 338 | (-1) |
| 30-17 | SEQ ID NO: 44 | 265 to 284 | (+1) |
| | | 345 to 364 | (-1) |
| 30-7 | SEQ ID NO: 45 | 272 to 291 | (+1) |
| | | 315 to 333 | (-1) |
| 30-84 | SEQ ID NO: 46 | 265 to 284 | (+1) |
| | | 334 to 353 | (-1) |
| 30-15 | SEQ ID NO: 47 | 248 to 267 | (+1) |
| | | 312 to 331 | (-1) |

Preferably, the primers contained a common oligonucleotide tail upstream of the specific bases targeted for amplification which was useful for sequencing.

The synthesis of these primers was performed following the phosphoramidite method, on a GENSET UFPS 24.1 synthesizer.

DNA amplification was performed on a Genius II thermocycler. After heating at 95°C for 10 min, 40 cycles were performed. Each cycle comprised: 30 sec at 95°C, 54°C for 1 min, and 30 sec at 72°C. For final elongation, 10 min at 72°C ended the amplification. The quantities of the amplification products obtained were determined on 96-well microtiter plates, using a fluorometer and Picogreen as intercalant agent (Molecular Probes).

### EXAMPLE 12: Identification of Biallelic Markers from Amplified Genomic DNA

The sequencing of the amplified DNA obtained in Example 11 was carried out on ABI 377 sequencers. The sequences of the amplification products were determined using automated dideoxy terminator sequencing reactions with a dye terminator cycle sequencing protocol. The products of the sequencing reactions were run on sequencing gels and the sequences were determined using gel image analysis (ABI Prism DNA Sequencing Analysis software (2.1.2 version)).

The sequence data were further evaluated to detect the presence of biallelic markers within the amplified fragments. The polymorphism search was based on the presence of superimposed peaks in the electrophoresis pattern resulting from different bases occurring at the same position as described previously.

The locations of the biallelic markers detected in the fragments of amplification are as shown below in Tables 3A and 3B.

**Table 3A : Biallelic Markers in the PPP2R2C gene**

| **amplified region** | **BM name** | **Strand** | **polymorphis m** | | **BM position on SEQ lD NO: 37** |
|---|---|---|---|---|---|
| | | | All 1 | All 2 | |
| 24-257 | 24-257/320 | (-) | A | G | 109663 |
| 99-24169 | 99-24169/139 | (-) | A | G | 84026 |
| 99-24175 | 99-24175/218 | (-) | A | G | 117460 |
| 24-247 | 24-247/216 | (+) | A | G | 99505 |

**Table 3B: Biallelic Markers in the KCNQ2 gene**

| **amplified region** | **BM name** | **Strand** | **polymorphism** | | **SEQ ID No.** | **BM position on indicated SEQ ID No.** |
|---|---|---|---|---|---|---|
| | | | **All 1** | **All 2** | | |
| 30-4 | 30-4/58 | (+) | A | G | SEQ ID NO: 42 | 301 |
| 30-2 | 30-2/62 | (+) | A | G | SEQ ID NO: 43 | 301 |
| 30-17 | 30-17/37 | (+) | A | G | SEQ ID NO: 44 | 301 |
| 30-7 | 30-7/30 | (+) | C | T | SEQ ID NO: 45 | 301 |
| 30-84 | 30-84/37 | (+) | A | G | SEQ ID NO: 46 | 301 |
| 30-15 | 30-15/54 | (+) | A | C | SEQ ID NO: 47 | 301 |

BM refers to "biallelic marker". All 1 and All 2 refer respectively to allele 1 and allele 2 of the biallelic marker. The (+) or (-) sign in the column "strand of BM" indicates the strand on which the indicated alternative alleles are found. SEQ ID Nos. 37 and 42 to 47 correspond to strands (+). As a matter of example, the biallel ic marker 24-257/320 corresponds to a polymorphism "a or g" at position 109663 on strand (-). Thus the nucleotide at position 109663 of SEQ ID NO: 37 will be "y", which corresponds to "t or c" according to the standard PCT nomenclature. The biallelic marker 24-247/216 corresponds to a polymorphism "a or g" at position 99505 on strand (+). Thus the nucleotide at position 99505 of SEQ ID NO: 37 will be "r", which corresponds to "a or g" according to the standard PCT nomenclature.

### EXAMPLE 13: Identification of Polymorphisms

### by Comparison of Genomic DNA from Overlapping BACs

Genomic DNA from multiple BAC clones derived from the same DNA donor sample and overlapping in regions of genomic DNA of SEQ ID NO: 37 was sequenced. Sequencing was carried out on ABI 377 sequencers. The sequences of the amplification products were determined using automated dideoxy terminator sequencing reactions with a dye terminator cycle sequencing protocol. The products of the sequencing reactions were run on sequencing gels and the sequences were determined using gel image analysis (ABI Prism DNA Sequencing Analysis software (2.1.2 version)).

### EXAMPLE 14: Validation Of The Polymorphisms Through Microsequencing

The biallelic markers identified in Examples 12 and 13 were further confirmed and their respective frequencies were determined through microsequencing. Microsequencing was carried out for each individual DNA sample described in Example 11.

Amplification from genomic DNA of individuals was performed by PCR as described above for the detection of the biallelic markers with the same set of PCR primers described in tables 1A and 1 B.
The preferred primers used in microsequencing were about 19 nucleotides in length and hybridized just upstream of the considered polymorphic base. According to the invention, the primers used for microsequencing are detailed in tables 4A and 4B.

**Table 4A: Primers in the PPP2R2C gene**

| **amplified region** | **Marker name** | **Orientation of the primer** | **Position of the primer on SEQ ID NO: 37** | **SEQ ID No. of the primer** |
|---|---|---|---|---|
| 24-257 | 24-257/320 | (+1) | 109644 to 109662 | SEQ ID NO: 40 |
| 99-24169 | 99-24169/139 | (+1) | 84007 to 84025 | SEQ ID NO: 39 |
| 99-24175 | 99-24175/218 | (+1) | 117441 to 117459 | SEQ ID NO: 41 |
| 24-247 | 24-247/216 | (+1) | 99486 to 99504 | |

**Table 4B: Primers in the KCNQ2 gene**

| **amplified region** | **Marker name** | **Orientation of the primer** | **SEQ ID No.** | **Position of the primer on indicated SEQ** ID **No.** |
|---|---|---|---|---|
| 30-4 | 30-4/58 | (-1) | SEQ ID NO: 42 | 302 to 319 (primer B18) |
| 30-4 | 30-4/58 | (+1) | SEQ ID NO: 42 | 282 to 300 (primer A19) |
| 30-2 | 30-2/62 | (-1) | SEQ ID NO: 43 | 302 to 320 |
| 30-17 | 30-17/37 | (-1) | SEQ ID NO: 44 | 302 to 324 |
| 30-7 | 30-7/30 | (+1) | SEQ ID NO: 45 | 280 to 300 |
| 30-84 | 30-84/37 | (-1) | SEQ ID NO: 46 | 302 to 318 |
| 30-15 | 30-15/54 | (-1) | SEQ ID NO: 47 | 302 to 323 |

As for the primers in tables 2A and 2B, the sign (+1) in the column "orientation" indicates that the sequence of the primer is identical to the corresponding region of SEQ ID Nos. 37 and 42 to 47, and the sign (-1) indicates that the sequence of the primer is complementary to the corresponding region of SEQ ID Nos. 37 and 42 to 47.
The microsequencing reaction performed as follows. After purification of the amplification products, the microsequencing reaction mixture was prepared by adding, in a 20µl final volume: 10 pmol microsequencing oligonucleotide, 1 U Thermosequenase (Amersham E79000G), 1.25 µl Thermosequenase buffer (260 mM Tris HCl pH 9.5, 65 mM MgCl₂), and the two appropriate fluorescent ddNTPs (Perkin Elmer, Dye Terminator Set 401095) complementary to the nucleotides at the polymorphic site of each biallelic marker tested, following the manufacturer's recommendations. After 4 minutes at 94°C, 20 PCR cycles of 15 sec at 55°C, 5 sec at 72°C, and 10 sec at 94°C were carried out in a Tetrad PTC -225 thermocycler (MJ Research). The unincorporated dye terminators were then removed by ethanol precipitation. Samples were finally resuspended in formamide-EDTA loading buffer and heated for 2 min at 95°C before being loaded on a polyacrylamide sequencing gel. The data were collected by an ABI PRISM 377 DNA sequencer and processed using the GENESCAN software (Perkin Elmer). Following gel analysis, data were automatically processed with software that allows the determination of the alleles of biallelic markers present in each amplified fragment. The software evaluates such factors as whether the intensities of the signals resulting from the above microsequencing procedures are weak, normal, or saturated, or whether the signals are ambiguous. In addition, the software identifies significant peaks (according to shape and height criteria). Among the significant peaks, peaks corresponding to the targeted site are identified based on their position. When two significant peaks are detected for the same position, each sample is categorized classification as homozygous or heterozygous type based on the height ratio.

### EXAMPLE 15: Association Study Between Bipolar Disorder And The Biallelic Markers Of The Invention

### 5.1. Collection of DNA Samples From Affected And Non-Affected Individuals

The association studies were performed on two different populations. One collection of samples was provided by Hospital Pinero, Buenos-Aires, Argentina (the "Labimo" collection). The other collection of samples was provided by the University College of London (the "UCL" collection). Both collections are constituted by individuals that are affected or not by bipolar disorder.

### A) The Labimo collection

### a) Affected population

206 DNA samples from patients suffering from bipolar disorder (cases) were collected for genotyping analysis.
All patients fulfilled DSM-IV and ICD-10 criteria for bipolar type I (ICD-10: F30.x, F31.x) or bipolar type II (ICD-10: F31.8). All patients were of Caucasian ethnic origin up to the 2^{nd} generation.
All potential patients suffering from a medical disorder or from a drug abuse were excluded.

According to DSM-lV criteria, 115 cases were classified as bipolar type I, 69 were bipolar type II, 22 were unclassified, and information concerning the type of bipolar disorder was lacking in 20 cases (8.5%)
The main phenotypic data of the cases were as follows:
- Mean age at first symptoms: 25.6 years (SD, 11; range, 8-58)
- Mean age at inclusion: 43.3 years (SD, 13.8; range, 17-76)
- Gender: 142 females and 84 males (ratio, 1.7)
- Ethnic origin: 213 were European Caucasian, 7 were non-European Caucasians, and information was lacking in 6 cases (2.5%)
- Family history of bipolar disorder was found in 18.5%, whereas schizophrenia was found in 0.9%.

### b) Unaffected population

201 DNA samples from individuals not suffering from bipolar disorder (controls) were collected for genotyping analysis.
All controls were individuals lacking personal or familial history of psychiatric disease.
The main phenotypic data of the controls were as follows:
- Mean age: 43.8 years (SD, 12; range, 21-72)
- Gender: 118 females and 83 males (ratio, 1.4)
180 controls were European Caucasian, and 21 had mixed ethnic origin

### c) Cases and Control Populations Selected for the Association Study

The case control populations were matched for ethnicity and sex which resul ted in 159 cases and 159 control individuals. Among the cases, 96 cases suffered from type I bipolar disorder, 56 cases suffered from type II bipolar disorder, and 7 cases suffered from an undetermined type of bipolar disorder. 33.8% of the cases were males. The mean age of the cases was of 43 and the median age was of 44. 41.4% of the controls were males. The mean age of the controls was of 44 and the median age was of 46.
The presence of population structure can result in spurious association, which is an association between phenotypes and markers that is not linked to any causative loci but due to a different ethnic origin. The Fst test is a general statistical tool for analyzing variances and that can be used to verify that a collection is homogeneous, i.e., that found associations are not linked to the structure of the population. The Fst value is calculated using random markers that are (i) unlinked and (ii) not associated with the trait to be studied. An Fst value close to 0 indicates that the collection is homogeneous and that any significant associations that are found are due to the trait under investigation (see, e.g., Bruce S.Weir, Genetic Data Analysis II, Edition Sinauer, San Francisco and Hartl and Clark, Populations genetics, Edition Sinauer, San Francisco). 66 random markers that were (i) unlinked and (ii) not associated with bipolar disorder were used to calculate the Fst value. An Fst value of 1.68e-01 was found for the found in the Labimo collection, indicating that this collection is homogeneous.

### B) The UCL collection

### a) Affected population

All patients fulfilled DSM-IV criteria for bipolar type I (ICD-10: F30.x, F31.x) or bipolar type II (ICD-10: F31.8). All patients were unrelated individuals of Caucasian origins from the British Isles (including English, Welsh, Scottish and Irish) up to the 2^{nd} generation.

### b) Unaffected population

300 samples fromunaffected control individuals (not suffering from bipolar disorder) were collected for genotyping analysis.
All control individuals showed (i) absence of personal history of psychiatric disease; and (ii) absence of familial history of psychiatric disease in first-degree relatives. All controls individuals of Caucasian origins from the British Isles (including English, Welsh, Scottish and Irish) up to the 2^{nd} generation.

### c) Cases and Control Populations Selected for the Association Study

The population retained for the study was composed of 315 cases and 295 controls. Among the cases, 256 cases suffered from type I bipolar disorder, 26 cases suffered from type II bipolar disorder, and 33 cases suffered from an undetermined type of bipolar disorder. About 36% of the cases were males. The mean age of the cases was of 46 and the median age was of 46. 48% of the controls were males. The mean age of the controls was of 37 and the median age was of 32.
59 random markers that were (i) unlinked; and (ii) not associated with bipolar disorder were used to calculate the Fst value. A Fst value of 3.41e-01 was found for the UCL collection, indicating that this collection is homogeneous.

### 5.2. Association studies

### A) Genotyping of affected and control individuals

The general strategy to perform the association studies was to individually scan the DNA samples from all individuals in each of the populations described above in order to establish the allele frequencies of biallelic markers, and among them the biallelic markers of the invention, in the diploid genome of the tested individuals belonging to each of these populations.

Frequencies of every biallelic marker in each population (cases and controls) were determined by performing microsequencing reactions on amplified fragments obtained by genomic PCR performed on the DNA samples from each individual. Genomic PCR and microsequencing were performed as detailed above in Examples 11 to 13 using the described PCR primers and microsequencing primers.

### B) Single biallelic marker frequency analysis

The difference between the allelic frequencies in the unaffected population and in the population affected by bipolar disorder was calculated for all five markers located in the KCNQ2 gene, and for all four markers located in the PPP2R2C gene. The allelic frequency of markers between cases and controls were investigated using the Pearson Chi squared test for allelic frequency and genotypic frequency distributions. A significant difference between observed and expected alleles/genotypes of a specific marker between case and control populations implies an association between the gene harboring this particular biallelic marker and bipolar disease. Both allelic and genotypic p-values were calculated for all markers. The p-values in tables 5A and 5B indicate the probability of no association between a biallelic marker and bipolar disorder considering the frequency. A p-value under 5e-02 indicates a significant association between the biallelic marker and bipolar disorder.

Odds ratio determination is a way of comparing the probability of having the disease when carrying a given allele versus when not carrying the said allele. An odds ratio higher than 1 indicates that the probability of having bipolar disorder is higher when carrying one of the alternative alleles, haplotypes or genotypes than when carrying the other ones. The genotypic odds ratio allows the identification of the "risk" allele, haplotype or genotype for an associated biallelic marker. The genotypic odds ratio was calculated for one biallelic marker located in PPP2R2C and for two markers located in the KCNQ2 gene (tables 6A and 6B).

**Table 5A: p-values for biallelic markers located in PPP2R2C**

| Marker Name | Location in PPP2R2 C | Collection | Chosen allele | All. Freq Diff. | All. Odds Ratio | Allelic p-value | Genotypic p-value |
|---|---|---|---|---|---|---|---|
| **99-24169/139** | Intron 1d | UCL | A | 0.095 | 1.733 | ***2.19e-04*** | ***3.61e-04*** |
| | | Labimo | A | 0.002 | 1.012 | 9.46e-01 | 5.98e-01 |
| **24-247/216** | Intron 4 | UCL | G | 0.047 | 1.275 | 7.75e-02 | ***2.29e-02*** |
| | | Labimo | G | 0.024 | 1.125 | 4.86e-01 | 7.65e-01 |
| **24-257/320** | Intron 5 | UCL | A | 0.018 | 1.079 | 5.52e-01 | 8.22e-01 |
| | | Labimo | A | 0.102 | 1.557 | ***4.04e-03*** | 1.19e-02 |
| **99-24175/218** | Intron 5 Intron 5 | UCL | G | 0.035 | 1.162 | 2.62e-01 | ***3.99e-03*** |
| | | Labimo | A | 0.096 | 1.546 | ***6.69e-03*** | 2.34e-02 |

**Table 5B: p-values for biallelic markers in the KCNQ2 gene**

| Marker Name | Location in the KCNQ2 gene | Collection | Chosen allele | All. Freq Diff. | All. Odds | Allelic p-value | Genotypic p-value |
|---|---|---|---|---|---|---|---|
| 30-4/58 | 5' of the gene | UCL | - | - | - | - | - |
| | | Labimo | G | 0.03 | 1.24 | 3.03e-01 | 5.85e-01 |
| **30-2/62** | intron 1 | UCL | A | 0.05 | 1.23 | ***7.76e-02*** | ***5.20e-03*** |
| | | Labimo | A | 0.03 | 1.13 | 4.42e-01 | 1.15e-01 |
| 30-17/37 | intron 4 | UCL | A | 0.01 | 1.03 | 7.77e-01 | 9.12e-01 |
| | | Labimo | G | 0.03 | 1.13 | 4.70e-01 | 7.10e-01 |
| **30-7/30** | intron 12 | UCL | C | 0.05 | 1.21 | 1.05e-01 | ***3.02e-02*** |
| | | Labimo | C | 0.02 | 1.06 | 7.03e-01 | 5.32e-01 |
| 30-84/37 | 3' of gene | UCL | A | 0.02 | 1.20 | 3.06e-01 | 3.69e-01 |
| | | Labimo | - | - | - | - | - |
| 30-15/54 | 3' of gene | UCL | A | 0.01 | 1.06 | 6.92e-01 | 7.68e-01 |
| | | Labimo | - | - | - | - | - |

**Table 6A: genotypic odds ratios for a biallelic marker located in PPP2R2C**

| Biallelic marker | collection | genotype | odds ratio | p-value |
|---|---|---|---|---|
| 99-24169/139 | UCL | AA vs GG | 1.9 | 8.50e-02 |
| | | AA vs AG | ***2.06*** | ***7.20e-05*** |
| | | AA vs (AG + GG) | ***2.04*** | ***4.60e-05*** |

**Table 6B: genotypic odds ratios for biallelic markers located in the KCNQ2 gene**

| Biallelic marker | collection | genotype | odds ratio | p-value |
|---|---|---|---|---|
| 30-2/62 | UCL | (AG+GG) vs AA | 1.05 | 4.60E-01 |
| | | AG vs AA | 1.28 | 1.70E-01 |
| | | AA vs GG | 1.51 | 8.00E-02 |
| | | AG vs (GG+AA) | ***1.62*** | ***300e-03*** |
| | | (AG+AA) vs GG | ***1.82*** | ***1.50e-03*** |
| 30-7/30 | UCL | (CC+CT) vs TT | 1.04 | 4.40E-01 |
| | | TT vs CT | 1.14 | 2.90E-01 |
| | | (CC+TT) vs CT | 1.37 | 3.80e-02 |
| | | CC vs TT | ***1.58*** | ***3.80e-02*** |
| | | CC vs (TT+CT) | ***1.71*** | ***7.00e-03*** |

### Biallelic markers in PPP2R2C

Thus the four biallelic markers located in the PPP2R2C gene are found to be associated with bipolar disorder. More specifically, 99-24169/139 is found to be highly associated with bipolar disorder in the UCL collection (significant allelic and genotypic p-values). 24-257/320 and 99-24175/218 are highly associated with bipolar disorder in the Labimo collection (significant allelic p-values). In addition, 99-24175/218 is also associated with bipolar disorder in the UCL collection (significant genotypic p-value). 24-247/216 is associated with bipolar disorder in the UCL collection (significant genotypic p-value).
The risk allele for the 99-24169/139 biallelic marker is "A". The risk alleles for the 24-257/320 biallelic marker and for the 99-24175/218 biallelic marker are also "A". The risk genotype for the 99-24169/139 biallelic marker is "AA". Thus an individual carrying the genotype "AA" at biallelic marker 99-24169/13 is at risk of developing bipolar disorder.

### Biallelic markers in the KCNQ2 gene

Two biallelic markers located in the KCNQ2 gene, 30-2/62 and 30-7/30, are associated with bipolar disorder. More specifically, 30-2/62 is found to be highly associated with bipolar disorder in the UCL collection (significant allelic and genotypic p-values). 30-7/30 is associated with bipolar disorder in the UCL collection (significant genotypic p-value). The risk genotype for 30-2/62 is "AG". The risk genotype for 30-7/30 is "CC". Thus individuals carrying the genotype "AG" at biallelic marker 30-2/62 and individuals carrying the genotype "CC" at biallelic marker 30-7/30 are at risk of developing bipolar disorder.

The association results of the single biallelic marker frequency analysis show that both the PPP2R2C gene and the KCNQ2 gene are associated with bipolar disorder. Accordingly, deregulation and/or dysfunction of KCNQ2 polypeptides and PP2A phosphatases comprising the PP2A/Bγ regulatory subunit contribute to the onset and to the development of bipolar disorder.

### C) Haplotype frequency analysis

The analysis of haplotype frequencies cannot readily be derived from observed genotypic data. The EM (Expectation-Maximization) algorithm (Excoffier L & Slatkin M, 1995) allows the estimation of haplotypes for the population under investigation. Haplotype frequency estimations were performed by applying the OMNIBUS likelihood ratio test (PCT publication WO 01/091026)

The haplotype analysis was performed for two sets of markers located in PPP2R2C. The haplotype analysis for 24-257/320 and 99-24175/218 was performed in the Labimo collection. The haplotype analysis for 99-24169/139 and 24-247/216 was performed in the UCL collection. The results are shown in tables 7 (p-values) and 7B (odds ratios).

**Table 7A**

| markers | Sam -ples | Haplo -type | Chi-S | Ave Chi-S | SD Chi-S | Max Chi-S | p-value |
|---|---|---|---|---|---|---|---|
| 24-257/320 and 99-24175/218 | La bi mo | AA | 7.78 | 0.96 | 1.34 | 14.02 | 3.9e-03 |
| | | AG | 0.02 | 1.02 | 1.40 | 11.19 | 8.79e-01 |
| | | GA | 0.14 | 0.96 | 1.35 | 11.62 | 6.77e-01 |
| | | GG | 7.35 | 0.98 | 1.35 | 14.31 | 5.5e-03 |
| 99-24169/139 and 24-247/216 | UCL | AA | 1,49641 | 1,0350 1 | 1,4668 7 | 14,67815 | 2.28e-01 |
| | | AG | 5,19606 | 1,0854 | 1,5233 6 | 14,42852 | 2.73e-02 |
| | | GA | 13,91081 | 1,2985 9 | 1,8118 2 | 16,01507 | 5e-04 |
| | | GG | 0,42926 | 1,5748 2 | 2,1956 2 | 23,4845 | 6.03e-01 |

**Table 7B**

| markers | haplotype | overall | cases | controls | odds ratio |
|---|---|---|---|---|---|
| **24-257/320 and 99-24175/218** | **AA** | 60.9% | 65.9% | 55.5% | ***1.55*** |
| | AG | 2.8% | 2.7% | 2.9% | 0.93 |
| | GA | 5.9% | 5.5% | 6.2% | 0.88 |
| | GG | 30.4% | 25.8% | 35.4% | 0.64 |
| **99-24169/139 and 24-247/216** | AA | 60,0% | 62,0% | 58,2% | 1,17 |
| | **AG** | 17,4% | 20,0% | 14,5% | ***1,47*** |
| | GA | 13,6% | 9,5% | 17,6% | 0,49 |
| | GG | 8,9% | 8,5% | 9,7% | 0,86 |

The risk haplotype for 24-257/320 and 99-24175/218 is "AA". The risk haplotype for 99-24169/139 and 24-247/216 is "AG". Thus an individual carrying the haplotype "AA" at biallelic markers 24-257/320 and 99-24175/218 is at risk of developing bipolar disorder, and an individual carrying the haplotype "AG" at biallelic markers 99-24169/139 and 24-247/216 is also at risk of developing bipolar disorder.

### REFERENCES

1. Altschul et al. (1990) J Mol Biol, 215:403-410
2. Altschul et al., (1997) Nucleic Acids Res., 25:389-402
3. Andrieux et al. (2002) Genes Dev., 16 :2350-2364
4. Biervert et al. (1998) Science, 279:403-406
5. Biervert et al. (1999) Genet., 104:234-240
6. Borresen et al. (1988) Mutat Res. 202:77-83
7. Dempster et al. (1977) JRSSB, 39:1-38
8. Detera-Wadleigh et al. (1999) Proc Natl Acad Sci USA, A96(10):5604-5609
9. Devereux et al. (1984) Nucleic Acids Res., 12:387-395
10. Elbashir et al. (2001) Genes Dev. 15:188-200
11. Ellington and Szostak (1990) Nature 346:818-822.
12. Excoffier and Slatkin, (1995) Mol Biol Evol. 12:921-7
13. Gamper et al. J Neurosci. (2003) 23:84-95
14. Grantham (1974) Science, 185:862-864
15. Grompe et al. (1989) Proc Natl Acad Sci USA. 86:5888-5892
16. Hu et al. (2000) Genomics., 67:83-86
17. Kaelin et al. (1991) Cell, 64:521-532
18. Kaelin et al. (1992) Cell, 70:351-364
19. Kim et al. (2003) Anal Biochem. 316:251-258
20. Lessa et al. (1993) Mol Ecol. 2:119-129
21. Main et al. (2000) Mol Pharmacol, 58:253-262
22. Newton et al. (1989) Nucleic Acids Res. 17:2503-2516
23. Orita et al. (1989) Proc Natl Acad Sci USA 86:2766-2770
24. Pan et al. (2001) J. Physiol., 531:347-358
25. Pearson (1990) Methods in Enzymology, 183:63-99
26. Pearson and Lipman (1988) Proc Nat Acad Sci USA, 85:2444-2448
27. Ruano et al. (1990) Proc. Natl. Acad. Sci. USA. 87:6296-6300
28. Sarkar and Sommer, (1991) Biotechniques. 10:436- 440
29. Schroeder et al., Epilepsia (2000) 41:1068-1069
30. Schwake et al. (2000) J. Biol. Chem., 275:13343-13348
31. Singh et al. (1998) Nat Genet, 18:25-29
32. Smith and Waterman (1981) Advances in Applied Mathematics, 2:482-489
33. Towbin et al. (1979) Proc Nat Acad Sci USA, 76:4350-4354
34. Wang et al. (1998) Science, 282:1890-1893
35. Wen et al. (2003) World J Gastroenterol. 9:1342-1346
36. Wu et al. (1989) Proc.Natl. Acad. Sci. USA. 86:2757-2760

## Claims

1. An isolated polypeptide selected from the group consisting of:
a) a polypeptide comprising a span of at least twenty amino acids of amino acids 589 to 643 of SEQ ID NO: 2;
b) a polypeptide comprising amino acids 589 to 643 of SEQ ID NO: 2;
c) a polypeptide comprising amino acids 545 to 643 of SEQ ID NO: 2;
d) a polypeptide comprising SEQ ID NO: 2;
e) a polypeptide comprising SEQ ID NO: 4;
f) a polypeptide comprising SEQ ID NO: 6;
g) a polypeptide comprising a mutein of any of SEQ ID NO: 2, SEQ ID NO: 4 or SEQ ID NO: 6, wherein said mutein has at least 95%, 96%, 97%, 98% or 99% identity to at least one of SEQ ID NO: 2, SEQ ID NO: 4 or SEQ ID NO: 6; and
h) a mutein of any of (b) to (f) wherein any changes in the amino acid sequence are conservative amino acid substitutions to the amino acid sequences in (b) to (f).

2. The polypeptide of claim 1, wherein said polypeptide is capable of binding to the Bγ subunit of the PP2A phosphatase.

3. A potassium channel comprising at least one polypeptide of claims 1 or 2.

4. The potassium channel of claim 3, wherein said potassium channel is a homomeric channel comprised of polypeptides of claims 1 or 2.

5. A purified polynucleotide encoding the polypeptide of claims 1 or 2, or a polynucleotide complementary thereto.

6. The polynucleotide of claim 5, wherein said polynucleotide is selected from the group consisting of:
a) a polynucleotide comprising nucleotides 1776 to 1929 of SEQ ID NO: 1.
b) a polynucleotide comprising nucleotides 1632 to 1929 of SEQ ID NO: 1.
c) a polynucleotide comprising SEQ ID NO: 1 ,
d) a polynucleotide comprising SEQ ID NO: 3 ,
e) a polynucleotide comprising SEQ ID NO: 5 ,
f) a polynucleotide complementary to the polynucleotides of (a) to (e).

7. An expression vector comprising the polynucleotide of claims 5 or 6.

8. The expression vector of claim 7, wherein said vector is a gene therapy vector.

9. A host cell comprising the expression vector of claims 7 or 8.

10. A method of making a polypeptide, said method comprising the steps of culturing a host cell according to claim 9 under conditions suitable for the production of a polypeptide of claim 1 or 2 within said host cell.

11. The method of claim 10, further comprising the step of purifying said polypeptide from the culture.

12. An antibody that specifically binds to a polypeptide of claim 1 or 2.

13. Use of a KCNQ2 polypeptide in accordance with claim 1 or 2 as a target for screening candidate modulators.

14. The use of claim 13, wherein said candidate modulator is selected from the group consisting of a natural ligand, a small molecule, an aptamer, an antisense mRNA a small interference RNA and an antibody.

15. The use of claims 13 or 14, wherein said modulator is a candidate drug for the treatment of a mental disorder.

16. The use of any of claims 13 to 15, wherein the activity of said KCNQ2 polypeptide is assessed by measuring the M-current generated by a potassium channel comprising said KCNQ2 polypeptide.

17. Use of at least one KCNQ2-related biallelic marker for diagnosing whether an individual suffers from or is at risk of suffering from a mental disorder, wherein at least one of said KCNQ2-related biallelic markers is selected from the group consisting of 30-2/62 and 30-7/30 as depicted in table 3B and the complements thereof.

18. The use of claim 17, wherein the presence of a genotype "AG" at biallelic marker 30-2/62 is indicative of said individual suffering from or being at risk of suffering from said mental disorder

19. The use of claim 17, wherein the presence of a genotype "CC" at biallelic marker 30-7/30 is indicative of said individual suffering from or being at risk of suffering from said mental disorder.

20. Use of at least one KCNQ2-related biallelic marker for determining whether there is a significant association between said marker and a mental disorder, wherein said at least one KCNQ2-related biallelic marker is selected from the group consisting of 30-4/58, 30-2/62, 30-17/37, 30-7/30, 30-84/37 and 30-15/54 as depicted in table 3B and the complements thereof.

21. The use of claim 20, wherein said at least one KCNQ2-related biallelic marker is selected from the group consisting of 30-2/62 and 30-7/30 as depicted in table 3B and the complements thereof.

22. The use of any of claims 17 to 21, wherein said mental disorder is selected from the group consisting of bipolar disorder, schizophrenia and depression.

23. The use of any of claims 17 to 22, wherein said mental disorder is bipolar disorder.

24. A method of genotyping comprising the step of determining the identity of a nucleotide at a KCNQ2-related biallelic marker or the complement thereof in a biological sample, wherein said at least one KCNQ2-related biallelic marker is selected from the group consisting of 30-4/58, 30-2/62, 30-17/37, 30-7/30, 30-84/37 and 30-15/54 as depicted in table 3B and the complements thereof.

25. The method of claim 24, wherein said at least one KCNQ2-related biallelic marker is selected from the group consisting of 30-2/62 and 30-7/30 as depicted in table 3B and the complements thereof.

26. The method of claim 24 or 25, wherein said biological sample is derived from a single individual.

27. The method of claim 26, wherein the identity of the nucleotides at said biallelic marker is determined for both copies of said biallelic marker present in said individual's genome.

28. The method of any of claims 24 to 27, wherein said determining is performed by a microsequencing assay.

29. The method of any of claims 24 to 28, further comprising amplifying a portion of a sequence comprising the biallelic marker prior to said determining step.

30. The method of claim 29, wherein said amplifying is performed by PCR.

31. A method of diagnosing a mental disorder in an individual comprising the step of genotyping at least one KCNQ2-related biallelic marker according to the method of any of claims 24 to 30, wherein said at least one KCNQ2-related biallelic marker is selected from the group consisting of 30-2/62 and 30-7/30 as depicted in table 3B and the complements thereof.

32. The method of claim 31 further comprising the step of correlating the result of the genotyping step with a risk of suffering from said mental disorder.

33. The method of claim 32, wherein the presence of a genotype "AG" at biallelic marker 30-2/62 is indicative of a risk of suffering from said mental disorder.

34. The method of claim 32, wherein the presence of a genotype "CC" at biallelic marker 30-7/30 is indicative of a risk of suffering from said mental disorder.

35. The method of any of claims 31 to 34, wherein said mental disorder is selected from the group consisting of bipolar disorder, schizophrenia and depression.

36. The method of any of claims 31 to 34, wherein said mental disorder is bipolar disorder.

## Patentansprüche

1. Isoliertes Polypeptid, ausgewählt aus der Gruppe bestehend aus:
a) einem Polypeptid, umfassend einen Bereich von wenigstens zwanzig Aminosäuren der Aminosäuren 589 bis 643 gemäß SEQ ID NO: 2;
b) einem Polypeptid, umfassend die Aminosäuren 589 bis 643 gemäß SEQ ID NO: 2;
c) einem Polypeptid, umfassend die Aminosäuren 545 bis 643 gemäß SEQ lD NO: 2;
d) einem Polypeptid, umfassend SEQ lD NO: 2;
e) einem Polypeptid, umfassend SEQ lD NO: 4;
f) einem Polypeptid, umfassend SEQ lD NO: 6;
g) einem Polypeptid, umfassend ein Mutein einer beliebigen Sequenz von SEQ lD NO: 2, SEQ lD NO: 4 oder SEQ ID NO: 6, wobei das Mutein wenigstens 95 %, 96 %, 97 %, 98 % oder 99 % Identität mit wenigstens einer Sequenz von SEQ lD NO: 2, SEQ lD NO: 4 oder SEQ lD NO: 6 aufweist; und
h) einem Mutein gemäß einer beliebigen Sequenz von (b) bis (f), wobei jegliche Veränderungen hinsichtlich der Aminosäuresequenz konservative Aminosäuresubstitutionen an den Aminosäuresequenzen in (b) bis (f) sind.

2. Polypeptid nach Anspruch 1, wobei das Polypeptid an die By-Untereinheit der PP2A-Phosphatase binden kann.

3. Kaliumkanal, umfassend wenigstens ein Polypeptid nach Anspruch 1 oder 2.

4. Kaliumkanal nach Anspruch 3, wobei der Kaliumkanal ein homomerer Kanal ist, welcher aus Polypeptiden gemäß der Ansprüche 1 oder 2 besteht.

5. Gereinigtes Polynukleotid, welches das Polypeptid nach den Ansprüchen 1 oder 2 kodiert, oder ein Polynukleotid, welches dazu komplementär ist.

6. Polynukleotid nach Anspruch 5, wobei das Polynukleotid ausgewählt ist aus der Gruppe bestehend aus:
a) einem Polynukleotid, umfassend die Nukleotide 1776 bis 1929 gemäß SEQ ID NO: 1.
b) einem Polynukleotid, umfassend die Nukleotide 1632 bis 1929 gemäß SEQ ID NO:1.
c) einem Polynukleotid, umfassend SEQ ID NO: 1,
d) einem Polynukleotid, umfassend SEQ ID NO:3,
e) einem Polynukleotid, umfassend SEQ ID NO: 5,
f) einem Polynukleotid, komplementär zu den Polynukleotiden von (a) bis (e).

7. Expressionsvektor, umfassend das Polynukleotid nach den Ansprüchen 5 oder 6.

8. Expressionsvektor nach Anspruch 7, wobei der Vektor ein Gentherapievektor ist.

9. Wirtszelle, umfassend den Expressionsvektor nach den Ansprüchen 7 oder 8.

10. Verfahren zur Herstellung eines Polypeptids, wobei das Verfahren die Schritte umfasst Kultivieren einer Wirtszelle nach Anspruch 9 unter Bedingungen, welche für die Erzeugung eines Polypeptids nach Anspruch 1 oder Anspruch 2 innerhalb der Wirtszelle geeignet sind.

11. Verfahren nach Anspruch 10, ferner umfassend den Schritt Reinigen des Polypeptids aus der Kultur.

12. Antikörper, welcher spezifisch an ein Polypeptid nach Anspruch 1 oder 2 bindet.

13. Verwendung eines KCNQ2-Polypeptids nach Anspruch 1 oder 2 als Ziel zum Screenen von Kandidatenmodulatoren.

14. Verwendung nach Anspruch 13, wobei der Kandidatenmodulator ausgewählt aus der Gruppe bestehend aus einem natürlichen Liganden, einem kleinen Molekül, einem Aptamer, einer Antisense-mRNA, einer kleinen Interferenz-RNA und einem Antikörper.

15. Verwendung nach den Ansprüchen 13 oder 14, wobei der Modulator ein Kandidatenarzneimittel für die Behandlung einer Geisteskrankheit ist.

16. Verwendung nach einem der Ansprüche 13 bis 15, wobei die Aktivität des KCNQ2-Polypeptids bewertet wird durch Messen des M-Stroms, welcher durch einen Kaliumkanal umfassend das KCNQ2-Polypeptid erzeugt wird.

17. Verwendung von wenigstens einem KCNQ2-bezogenen biallelischen Marker zur Diagnose, ob ein Individuum unter einer Geisteskrankheit leidet oder gefährdet ist, an einer Geisteskrankheit zu leiden, wobei wenigstens einer der KCNQ2-bezogenen biallelischen Marker ausgewählt ist aus der Gruppe bestehend aus 30-2/62 und 30-7/30, wie in Tabelle 3 beschrieben und den Komplementen davon.

18. Verwendung nach Anspruch 17, wobei das Vorliegen eines Genotyps "AG" am biallelischen Marker 30-2/62 dafür indikativ ist, dass das Individuum unter Geisteskrankheit leidet oder gefährdet ist, unter einer Geisteskrankheit zu leiden.

19. Verwendung nach Anspruch 17, wobei das Vorliegen eines Genotyps "CC" am biallelischen Marker 30-7/30 dafür indikativ ist, dass das Individuum unter einer Geisteskrankheit leidet oder gefährdet ist, unter einer Geisteskrankheit zu leiden.

20. Verwendung von wenigstens einem KCNQ2-bezogenen biallelischen Marker zur Bestimmung, ob eine signifikante Assoziierung zwischen dem Marker und einer Geisteskrankheit vorliegt, wobei der wenigstens eine KCNQ2-bezogene biallelische Marker ausgewählt ist aus der Gruppe bestehend aus 30-4/58, 30-2/62, 30-17/37, 30-7/30, 30-84/37 und 30-15/54, wie in Tabelle 3B beschrieben und den Komplementen davon.

21. Verwendung nach Anspruch 20, wobei der wenigstens eine KCNQ2-bezogene biallelische Marker ausgewählt ist aus der Gruppe bestehend aus 30-2/62 und 30-7/30, wie in Tabelle 3B beschrieben und den Komplementen davon.

22. Verwendung nach einem der Ansprüche 17 bis 21, wobei die Geisteskrankheit ausgewählt ist aus der Gruppe bestehend aus bipolarer Störung, Schizophrenie und Depression.

23. Die Verwendung nach einem der Ansprüche 17 bis 22, wobei die Geisteskrankheit bipolare Störung ist.

24. Genotypisierungsverfahren, umfassend den Schritt Bestimmen der Identität eines Nukleotids an einem KCNQ2-bezogenen biallelischen Marker oder dem Komplement davon in einer biologischen Probe, wobei der wenigstens eine KCNQ2-bezogene biallelische Marker ausgewählt ist aus der Gruppe bestehend aus 30-4/58, 30-2/62, 30-17/37, 30-7/30, 30-84/37 und 30-15/54 wie in Tabelle 3B beschrieben und den Komplementen davon.

25. Verfahren nach Anspruch 24, wobei der wenigstens eine KCNQ2-bezogene biallelische Marker ausgewählt ist aus der Gruppe bestehend aus 30-2/62 und 30-7/30, wie in Tabelle 3B beschrieben und den Komplementen davon.

26. Verfahren nach Anspruch 24 oder 25, wobei die biologische Probe von einem einzelnen Individuum stammt.

27. Verfahren nach Anspruch 26, wobei die Identität der Nukleotide am biallelischen Marker für beide Kopien des biallelischen Marker, welche im Genom des Individuums vorliegen, bestimmt wird.

28. Verfahren nach einem der Ansprüche 24 bis 27, wobei die Bestimmung mittels eines Mikrosequenzierungsassays durchgeführt wird.

29. Verfahren nach einem der Ansprüche 24 bis 28, ferner umfassend Amplifizieren eines Teils einer Sequenz, umfassend den biallelischen Marker vor dem Bestimmungsschritt.

30. Verfahren nach Anspruch 29, wobei die Amplifizierung mittels PCR durchgeführt wird.

31. Verfahren zur Diagnose einer Geisteskrankheit in einem Individuum, umfassend den Schritt Genotypisieren von wenigstens einem KCNQ2-bezogenen biallelischen Marker gemäß dem Verfahren nach einem der Ansprüche 24 bis 30, wobei der wenigstens eine KCNQ2-bezogene biallelische Marker ausgewählt ist aus der Gruppe bestehend aus 30-2/62 und 30-7/30, wie in Tabelle 3B beschrieben und den Komplementen davon.

32. Verfahren nach Anspruch 31, ferner umfassend den Schritt Korrelieren des Ergebnisses des Genotypisierungsschritts mit einem Risiko, unter einer Geisteskrankheit zu leiden.

33. Verfahren nach Anspruch 32, wobei das Vorliegen eines Genotyps "AG" am biallelischen Marker 30-2/62 für das Risiko unter der Geisteskrankheit zu leiden, indikativ ist.

34. Verfahren nach Anspruch 32, wobei das Vorliegen eines Genotyps "CC" am biallelischen Marker 30-7/30 für das Risiko unter der Geisteskrankheit zu leiden, indikativ ist.

35. Verfahren nach einem der Ansprüche 31 bis 34, wobei die Geisteskrankheit ausgewählt ist aus der Gruppe bestehend aus bipolarer Störung, Schizophrenie und Depression.

36. Verfahren nach einem der Ansprüche 31 bis 34, wobei die Geisteskrankheit bipolare Störung ist.

## Revendications

1. Polypeptide isolé, choisi dans l'ensemble formé par les suivants :
a) un polypeptide comprenant une suite d'au moins vingt résidus d'acides aminés de l'ensemble des résidus d'acides aminés n° 589 à 643 de la Séquence n° 2 ;
b) un polypeptide comprenant les résidus d'acides aminés n° 589 à 643 de la Séquence n° 2 ;
c) un polypeptide comprenant les résidus d'acides aminés n° 545 à 643 de la Séquence n° 2 ;
d) un polypeptide comprenant la Séquence n° 2 ;
e) un polypeptide comprenant la Séquence n° 4 ;
f) un polypeptide comprenant la Séquence n° 6 ;
g) un polypeptide comprenant une mutéine dérivée de l'une des Séquences n° 2, n° 4 et n° 6, laquelle mutéine est identique, pour au moins 95 %, 96 %, 97 %, 98 % ou 99 %, à au moins l'une des Séquences n° 2, n° 4 et n° 6 ;
h) et une mutéine de l'un des polypeptides (b) à (f), dans laquelle tous les changements intervenus dans la séquence d'acides aminés sont des substitutions d'acides aminés conservatrices pour les séquences d'acides aminés des polypeptides (b) à (f).

2. Polypeptide conforme à la revendication 1, lequel polypeptide est capable de se lier à la sous-unité Bγ de la phosphatase PP2A.

3. Canal potassique comprenant au moins un polypeptide conforme à la revendication 1 ou 2.

4. Canal potassique conforme à la revendication 3, lequel canal potassique est un canal homomère constitué de polypeptides conformes à la revendication 1 ou 2.

5. Polynucléotide purifié, codant un polypeptide conforme à la revendication 1 ou 2, ou son polynucléotide complémentaire.

6. Polynucléotide conforme à la revendication 5, lequel polynucléotide est choisi dans l'ensemble formé par les suivants :
a) un polynucléotide comprenant les nucléotides n° 1776 à 1929 de la Séquence n° 1 ;
b) un polynucléotide comprenant les nucléotides n° 1632 à 1929 de la Séquence n° 1 ;
c) un polynucléotide comprenant la Séquence n° 1 ;
d) un polynucléotide comprenant la Séquence n° 3 ;
e) un polynucléotide comprenant la Séquence n° 5 ;
f) et un polynucléotide complémentaire de l'un des polynucléotides (a) à (e).

7. Vecteur d'expression comprenant un polynucléotide conforme à la revendication 5 ou 6.

8. Vecteur d'expression conforme à la revendication 7, lequel vecteur est un vecteur pour thérapie génique.

9. Cellule hôte comprenant un vecteur d'expression conforme à la revendication 7 ou 8.

10. Procédé de préparation d'un polypeptide, lequel procédé comporte une étape où l'on cultive une cellule hôte, conforme à la revendication 9, dans des conditions appropriées pour la production, au sein de cette cellule hôte, d'un polypeptide conforme à la revendication 1 ou 2.

11. Procédé conforme à la revendication 10, qui comporte en outre une étape où l'on sépare de la culture et purifie ledit polypeptide.

12. Anticorps qui se lie spécifiquement à un polypeptide conforme à la revendication 1 ou 2.

13. Emploi d'un polypeptide KCNQ2, conforme à la revendication 1 ou 2, en tant que cible pour le criblage de candidats modulateurs.

14. Emploi conforme à la revendication 13, ledit candidat modulateur étant choisi dans l'ensemble constitué par un ligand naturel, une petite molécule, un aptamère, un ARNm antisens, un petit ARN interférant et un anticorps.

15. Emploi conforme à la revendication 13 ou 14, ledit modulateur étant un candidat médicament pour le traitement d'un trouble mental.

16. Emploi conforme à l'une des revendications 13 à 15, dans lequel on vérifie l'activité dudit polypeptide KCNQ2 en mesurant le courant M produit par un canal potassique comprenant ce polypeptide KCNQ2.

17. Emploi d'au moins un marqueur biallélique associé à KCNQ2 pour diagnostiquer si un individu souffre ou risque de souffrir d'un trouble mental, au moins l'un de ces marqueurs bialléliques associés à KCNQ2 étant choisi dans l'ensemble constitué par les marqueurs 30-2/62 et 30-7/30 figurant dans le tableau 3B et leurs complémentaires.

18. Emploi conforme à la revendication 17, dans lequel la présence d'un génotype "AG" au niveau du marqueur biallélique 30-2/62 indique que ledit individu souffre ou risque de souffrir dudit trouble mental.

19. Emploi conforme à la revendication 17, dans lequel la présence d'un génotype "CC" au niveau du marqueur biallélique 30-7/30 indique que ledit individu souffre ou risque de souffrir dudit trouble mental.

20. Emploi d'au moins un marqueur biallélique associé à KCNQ2 pour déterminer si ledit marqueur est significativement associé à un trouble mental, ce marqueur biallélique associé à KCNQ2, au nombre d'au moins un, étant choisi dans l'ensemble constitué par les marqueurs 30-4/68, 30-2/62, 30-17/37, 30-7/30, 30-84/37 et 30-15/54 figurant dans le tableau 3B et leurs complémentaires.

21. Emploi conforme à la revendication 20, ledit marqueur biallélique associé à KCNQ2, au nombre d'au moins un, étant choisi dans l'ensemble constitué par les marqueurs 30-2/62 et 30-7/30 figurant dans le tableau 3B et leurs complémentaires.

22. Emploi conforme à l'une des revendications 17 à 21, ledit trouble. mental étant choisi dans l'ensemble formé par le trouble bipolaire, la schizophrénie et la dépression.

23. Emploi conforme à l'une des revendications 17 à 22, ledit trouble mental étant le trouble bipolaire.

24. Procédé de génotypage, comprenant une étape où l'on détermine l'identité d'un nucléotide au niveau d'un marqueur biallélique associé à KCNQ2 ou d'un complémentaire d'un tel marqueur dans un échantillon biologique, ce marqueur biallélique associé à KCNQ2, au nombre d'au moins un, étant choisi dans l'ensemble formé par les marqueurs 30-4/68, 30-2/62, 30-17/37, 30-7/30, 30-84/37 et 30-15/54 figurant dans le tableau 3B et leurs complémentaires.

25. Procédé conforme à la revendication 24, ledit marqueur biallélique associé à KCNQ2, au nombre d'au moins un, étant choisi dans l'ensemble formé par les marqueurs 30-2/62 et 30-7/30 figurant dans le tableau 3B et leurs complémentaires.

26. Procédé conforme à la revendication 24 ou 25, dans lequel ledit échantillon biologique provient d'un individu unique.

27. Procédé conforme à la revendication 26, dans lequel on détermine, au niveau dudit marqueur biallélique, l'identité des nucléotides dans les deux copies dudit marqueur biallélique présentes dans le génome dudit individu.

28. Procédé conforme à l'une des revendications 24 à 27, dans lequel on réalise ladite détermination au moyen d'un test de microséquençage.

29. Procédé conforme à l'une des revendications 24 à 28, qui comporte en outre, avant ladite étape de détermination, le fait d'amplifier une partie d'une séquence comprenant le marqueur biallélique.

30. Procédé conforme à la revendication 29, dans lequel ladite amplification est effectuée par PCR.

31. Procédé permettant de diagnostiquer un trouble mental chez un individu, lequel procédé comporte une étape où l'on effectue le génotypage d'au moins un marqueur biallélique associé à KCNQ2, en suivant un procédé conforme à l'une des revendications 24 à 30, ce marqueur biallélique associé à KCNQ2, au nombre d'au moins un, étant choisi dans l'ensemble formé par les marqueurs 30-2/62 et 30-7/30 figurant dans le tableau 3B et leurs complémentaires.

32. Procédé conforme à la revendication 31, qui comporte en outre une étape où l'on corrèle le résultat de l'opération de génotypage avec un risque de souffrir dudit trouble mental.

33. Procédé conforme à la revendication 32, dans lequel la présence d'un génotype "AG" au niveau du marqueur biallélique 30-2/62 indique un risque de souffrir dudit trouble mental.

34. Procédé conforme à la revendication 32, dans lequel la présence d'un génotype "CC" au niveau du marqueur biallélique 30-7/30 indique un risque de souffrir dudit trouble mental.

35. Procédé conforme à l'une des revendications 31 à 34, ledit trouble mental étant choisi dans l'ensemble formé par le trouble bipolaire, la schizophrénie et la dépression.

36. Procédé conforme à l'une des revendications 31 à 34, ledit trouble mental étant le trouble bipolaire.


### SEQUENCE LISTING

<110> GENSET S.A.
<120> NOVEL KCNQ POLYPEPTIDES, MODULATORS THEREOF, AND THEIR USES IN THE TREATMENT OF MENTAL DISORDERS
<130> 794 WO
<150> US 60/391,359
   <151> 2002-06-25
<160> 47
<170> PatentIn version 3.1
<210> 1
   <211> 1932
   <212> DNA
   <213> Homo sapiens
<220>
   <221> CDS
   <222> (1)..(1932)
   <223>
<400> 1
   atg gtg cag aag tcg cgc aac ggc ggc gta tac ccc ggc ccg agc ggg      48
   Met Val Gln Lys Ser Arg Asn Gly Gly Val Tyr Pro Gly Pro Ser Gly
   1               5                   10                  15
   gag aag aag ctg aag gtg ggc ttc gtg ggg ctg gac ccc ggc gcg ccc      96
   Glu Lys Lys Leu Lys Val Gly Phe Val Gly Leu Asp Pro Gly Ala Pro
               20                  25                  30
   gac tcc acc cgg gac ggg gcg ctg ctg atc gcc ggc tcc gag gcc ccc      144
   Asp Ser Thr Arg Asp Gly Ala Leu Leu Ile Ala Gly Ser Glu Ala Pro
           35                  40                  45
   aag cgc ggc agc atc ctc agc aaa cct cgc gcg ggc ggc gcg ggc gcc      192
   Lys Arg Gly Ser Ile Leu Ser Lys Pro Arg Ala Gly Gly Ala Gly Ala
       50                  55                  60
   ggg aag ccc ccc aag cgc aac gcc ttc tac cgc aag ctg cag aat ttc      240
   Gly Lys Pro Pro Lys Arg Asn Ala Phe Tyr Arg Lys Leu Gln Asn Phe
   65                  70                  75                  80
   ctc tac aac gtg ctg gag cgg ccg cgc ggc tgg gcg ttc atc tac cac      288
   Leu Tyr Asn Val Leu Glu Arg Pro Arg Gly Trp Ala Phe Ile Tyr His
                   85                  90                  95
   gcc tac gtg ttc ctc ctg gtt ttc tcc tgc ctc gtg ctg tct gtg ttt      336
   Ala Tyr Val Phe Leu Leu Val Phe Ser Cys Leu Val Leu Ser Val Phe
               100                 105                 110
   tcc acc atc aag gag tat gag aag agc tcg gag ggg gcc ctc tac atc      384
   Ser Thr Ile Lys Glu Tyr Glu Lys Ser Ser Glu Gly Ala Leu Tyr Ile
           115                 120                 125
   ctg gaa atc gtg act atc gtg gtg ttt ggc gtg gag tac ttc gtg cgg      432
   Leu Glu Ile Val Thr Ile Val Val Phe Gly Val Glu Tyr Phe Val Arg
       130                 135                 140
   atc tgg gcc gca ggc tgc tgc tgc cgg tac cgt ggc tgg agg ggg cgg      480
   Ile Trp Ala Ala Gly Cys Cys Cys Arg Tyr Arg Gly Trp Arg Gly Arg
   145                 150                 155                 160
   ctc aag ttt gcc cgg aaa ccg ttc tgt gtg att gac atc atg gtg ctc      528
   Leu Lys Phe Ala Arg Lys Pro Phe Cys Val Ile Asp Ile Met Val Leu
   165 170 175
   atc gcc tcc att gcg gtg ctg gcc gcc ggc tcc cag ggc aac gtc ttt 576
   Ile Ala Ser Ile Ala Val Leu Ala Ala Gly Ser Gln Gly Asn Val Phe
   180 185 190
   gcc aca tct gcg ctc cgg agc ctg cgc ttc ctg cag att ctg cgg atg 624
   Ala Thr Ser Ala Leu Arg Ser Leu Arg Phe Leu Gln Ile Leu Arg Met
   195 200 205
   atc cgc atg gac cgg cgg gga ggc acc tgg aag ctg ctg ggc tct gtg 672
   Ile Arg Met Asp Arg Arg Gly Gly Thr Trp Lys Leu Leu Gly Ser Val
   210 215 220
   gtc tat gcc cac agc aag gag ctg gtc act gcc tgg tac atc ggc ttc 720
   Val Tyr Ala His Ser Lys Glu Leu Val Thr Ala Trp Tyr Ile Gly Phe
   225 230 235 240
   ctt tgt ctc atc ctg gcc tcg ttc ctg gtg tac ttg gca gag aag ggg 768
   Leu Cys Leu Ile Leu Ala Ser Phe Leu Val Tyr Leu Ala Glu Lys Gly
   245 250 255
   gag aac gac cac ttt gac acc tac gcg gat gca ctc tgg tgg ggc ctg 816
   Glu Asn Asp His Phe Asp Thr Tyr Ala Asp Ala Leu Trp Trp Gly Leu
   260 265 270
   atc acg ctg acc acc att ggc tac ggg gac aag tac ccc cag acc tgg 864
   Ile Thr Leu Thr Thr Ile Gly Tyr Gly Asp Lys Tyr Pro Gln Thr Trp
   275 280 285
   aac ggc agg ctc ctt gcg gca acc ttc acc ctc atc ggt gtc tcc ttc 912
   Asn Gly Arg Leu Leu Ala Ala Thr Phe Thr Leu Ile Gly Val Ser Phe
   290 295 300
   ttc gcg ctg cct gca ggc atc ttg ggg tct ggg ttt gcc ctg aag gtt 960
   Phe Ala Leu Pro Ala Gly Ile Leu Gly Ser Gly Phe Ala Leu Lys Val
   305 310 315 320
   cag gag cag cac agg cag aag cac ttt gag aag agg cgg aac ccg gca 1008
   Gln Glu Gln His Arg Gln Lys His Phe Glu Lys Arg Arg Asn Pro Ala
   325 330 335
   gca ggc ctg atc cag tcg gcc tgg aga ttc tac gcc acc aac ctc tcg 1056
   Ala Gly Leu Ile Gln Ser Ala Trp Arg Phe Tyr Ala Thr Asn Leu Ser
   340 345 350
   cgc aca gac ctg cac tcc acg tgg cag tac tac gag cga acg gtc acc 1104
   Arg Thr Asp Leu His Ser Thr Trp Gln Tyr Tyr Glu Arg Thr Val Thr
   355 360 365
   gtg ccc atg tac agt tcg caa act caa acc tac ggg gcc tcc aga ctt 1152
   Val Pro Met Tyr Ser Ser Gln Thr Gln Thr Tyr Gly Ala Ser Arg Leu
   370 375 380
   atc ccc ccg ctg aac cag ctg gag ctg ctg agg aac ctc aag agt aaa 1200
   Ile Pro Pro Leu Asn Gln Leu Glu Leu Leu Arg Asn Leu Lys Ser Lys
   385 390 395 400
   tct gga ctc gct ttc agg aag gac ccc ccg ccg gag ccg tct cca agt 1248
   Ser Gly Leu Ala Phe Arg Lys Asp Pro Pro Pro Glu Pro Ser Pro Ser
   405 410 415
   aaa ggc agc ccg tgc aga ggg ccc ctg tgt gga tgc tgc ccc gga cgc 1296
   Lys Gly Ser Pro Cys Arg Gly Pro Leu Cys Gly Cys Cys Pro Gly Arg
   420 425 430
   tct agc cag aag gtc agt ttg aaa gat cgt gtc ttc tcc agc ccc cga 1344
   Ser Ser Gln Lys Val Ser Leu Lys Asp Arg Val Phe Ser Ser Pro Arg
           435                 440                 445
   ggc gtg gct gcc aag ggg aag ggg tcc ccg cag gcc cag act gtg agg      1392
   Gly Val Ala Ala Lys Gly Lys Gly Ser Pro Gln Ala Gln Thr Val Arg
       450                 455                 460
   cgg tca ccc agc gcc gac cag agc ctc gag gac agc ccc agc aag gtg      1440
   Arg Ser Pro Ser Ala Asp Gln Ser Leu Glu Asp Ser Pro Ser Lys Val
   465                 470                 475                 480
   ccc aag agc tgg agc ttc ggg gac cgc agc cgg gca cgc cag gct ttc      1488
   Pro Lys Ser Trp Ser Phe Gly Asp Arg Ser Arg Ala Arg Gln Ala Phe
                   485                 490                 495
   cgc atc aag ggt gcc gcg tca cgg cag aac tca gaa gaa gca agc ctc      1536
   Arg Ile Lys Gly Ala Ala Ser Arg Gln Asn Ser Glu Glu Ala Ser Leu
               500                 505                 510
   ccc gga gag gac att gtg gat gac aag agc tgc ccc tgc gag ttt gtg      1584
   Pro Gly Glu Asp Ile Val Asp Asp Lys Ser Cys Pro Cys Glu Phe Val
           515                 520                 525
   acc gag gac ctg acc ccg ggc ctc aaa gtc agc atc aga gcc gtg tgt      1632
   Thr Glu Asp Leu Thr Pro Gly Leu Lys Val Ser Ile Arg Ala Val Cys
       530                 535                 540
   gtc atg cgg ttc ctg gtg tcc aag cgg aag ttc aag gag agc ctg cgg      1680
   Val Met Arg Phe Leu Val Ser Lys Arg Lys Phe Lys Glu Ser Leu Arg
   545                 550                 555                 560
   ccc tac gac gtg atg gac gtc atc gag cag tac tca gcc ggc cac ctg      1728
   Pro Tyr Asp Val Met Asp Val Ile Glu Gln Tyr Ser Ala Gly His Leu
                   565                 570                 575
   gac atg ctg tcc cga att aag agc ctg cag tcc agg caa gag ccc cgc      1776
   Asp Met Leu Ser Arg Ile Lys Ser Leu Gln Ser Arg Gln Glu Pro Arg
               580                 585                 590
   ctg cct gtc cag cag ggg aca aga acg ggg tgg gct tct ggg aca aag      1824
   Leu Pro Val Gln Gln Gly Thr Arg Thr Gly Trp Ala Ser Gly Thr Lys
           595                 600                 605
   ccc act gtg gcc cat ggt ggg agt gca ggg ggt gtg tgg gcg ggg cct      1872
   Pro Thr Val Ala His Gly Gly Ser Ala Gly Gly Val Trp Ala Gly Pro
       610                 615                 620
   cct ccc cac cca cgt cgg cct ctg tca gct tct gtt gtg tct tca caa      1920
   Pro Pro His Pro Arg Arg Pro Leu Ser Ala Ser Val Val Ser Ser Gln
   625                 630                 635                 640
   agt ctg ttt taa                                                      1932
   Ser Leu Phe
<210> 2
   <211> 643
   <212> PRT
   <213> Homo sapiens
<400> 2
   Met Val Gln Lys Ser Arg Asn Gly Gly Val Tyr Pro Gly Pro Ser Gly
   1               5                   10                  15
   Glu Lys Lys Leu Lys Val Gly Phe Val Gly Leu Asp Pro Gly Ala Pro
   20 25 30
   Asp Ser Thr Arg Asp Gly Ala Leu Leu Ile Ala Gly Ser Glu Ala Pro
   35 40 45
   Lys Arg Gly Ser Ile Leu Ser Lys Pro Arg Ala Gly Gly Ala Gly Ala
   50 55 60
   Gly Lys Pro Pro Lys Arg Asn Ala Phe Tyr Arg Lys Leu Gln Asn Phe
   65 70 75 80
   Leu Tyr Asn Val Leu Glu Arg Pro Arg Gly Trp Ala Phe Ile Tyr His
   85 90 95
   Ala Tyr Val Phe Leu Leu Val Phe Ser Cys Leu Val Leu Ser Val Phe
   100 105 110
   Ser Thr Ile Lys Glu Tyr Glu Lys Ser Ser Glu Gly Ala Leu Tyr Ile
   115 120 125
   Leu Glu Ile Val Thr Ile Val Val Phe Gly Val Glu Tyr Phe Val Arg
   130 135 140
   Ile Trp Ala Ala Gly Cys Cys Cys Arg Tyr Arg Gly Trp Arg Gly Arg
   145 150 155 160
   Leu Lys Phe Ala Arg Lys Pro Phe Cys Val Ile Asp Ile Met Val Leu
   165 170 175
   Ile Ala Ser Ile Ala Val Leu Ala Ala Gly Ser Gln Gly Asn Val Phe
   180 185 190
   Ala Thr Ser Ala Leu Arg Ser Leu Arg Phe Leu Gln Ile Leu Arg Met
   195 200 205
   Ile Arg Met Asp Arg Arg Gly Gly Thr Trp Lys Leu Leu Gly Ser Val
   210 215 220
   Val Tyr Ala His Ser Lys Glu Leu Val Thr Ala Trp Tyr Ile Gly Phe
   225 230 235 240
   Leu Cys Leu Ile Leu Ala Ser Phe Leu Val Tyr Leu Ala Glu Lys Gly
   245 250 255
   Glu Asn Asp His Phe Asp Thr Tyr Ala Asp Ala Leu Trp Trp Gly Leu
   260 265 270
   Ile Thr Leu Thr Thr Ile Gly Tyr Gly Asp Lys Tyr Pro Gln Thr Trp
   275 280 285
   Asn Gly Arg Leu Leu Ala Ala Thr Phe Thr Leu Ile Gly Val Ser Phe
   290 295 300
   Phe Ala Leu Pro Ala Gly Ile Leu Gly Ser Gly Phe Ala Leu Lys Val
   305 310 315 320
   Gln Glu Gln His Arg Gln Lys His Phe Glu Lys Arg Arg Asn Pro Ala
   325 330 335
   Ala Gly Leu Ile Gln Ser Ala Trp Arg Phe Tyr Ala Thr Asn Leu Ser
   340 345 350
   Arg Thr Asp Leu His Ser Thr Trp Gln Tyr Tyr Glu Arg Thr Val Thr
   355 360 365
   Val Pro Met Tyr Ser Ser Gln Thr Gln Thr Tyr Gly Ala Ser Arg Leu
   370 375 380
   Ile Pro Pro Leu Asn Gln Leu Glu Leu Leu Arg Asn Leu Lys Ser Lys
   385 390 395 400
   Ser Gly Leu Ala Phe Arg Lys Asp Pro Pro Pro Glu Pro Ser Pro Ser
   405 410 415
   Lys Gly Ser Pro Cys Arg Gly Pro Leu Cys Gly Cys Cys Pro Gly Arg
   420 425 430
   Ser Ser Gln Lys Val Ser Leu Lys Asp Arg Val Phe Ser Ser Pro Arg
   435 440 445
   Gly Val Ala Ala Lys Gly Lys Gly Ser Pro Gln Ala Gln Thr Val Arg
   450 455 460
   Arg Ser Pro Ser Ala Asp Gln Ser Leu Glu Asp Ser Pro Ser Lys Val
   465 470 475 480
   Pro Lys Ser Trp Ser Phe Gly Asp Arg Ser Arg Ala Arg Gln Ala Phe
   485 490 495
   Arg Ile Lys Gly Ala Ala Ser Arg Gln Asn Ser Glu Glu Ala Ser Leu
   500 505 510
   Pro Gly Glu Asp Ile Val Asp Asp Lys Ser Cys Pro Cys Glu Phe Val
   515 520 525
   Thr Glu Asp Leu Thr Pro Gly Leu Lys Val Ser Ile Arg Ala Val Cys
   530 535 540
   Val Met Arg Phe Leu Val Ser Lys Arg Lys Phe Lys Glu Ser Leu Arg
   545 550 555 560
   Pro Tyr Asp Val Met Asp Val Ile Glu Gln Tyr Ser Ala Gly His Leu
   565 570 575
   Asp Met Leu Ser Arg Ile Lys Ser Leu Gln Ser Arg Gln Glu Pro Arg
   580 585 590
   Leu Pro Val Gln Gln Gly Thr Arg Thr Gly Trp Ala Ser Gly Thr Lys
   595 600 605
   Pro Thr Val Ala His Gly Gly Ser Ala Gly Gly Val Trp Ala Gly Pro
   610 615 620
   Pro Pro His Pro Arg Arg Pro Leu Ser Ala Ser Val Val Ser Ser Gln
   625 630 635 640
   Ser Leu Phe
<210> 3
   <211> 1878
   <212> DNA
   <213> Homo sapiens
<220>
   <221> CDS
   <222> (1)..(1878)
   <223>
<400> 3
   atg gtg cag aag tcg cgc aac ggc ggc gta tac ccc ggc ccg agc ggg 48
   Met Val Gln Lys Ser Arg Asn Gly Gly Val Tyr Pro Gly Pro Ser Gly
   1 5 10 15
   gag aag aag ctg aag gtg ggc ttc gtg ggg ctg gac ccc ggc gcg ccc 96
   Glu Lys Lys Leu Lys Val Gly Phe Val Gly Leu Asp Pro Gly Ala Pro
   20 25 30
   gac tcc acc cgg gac ggg gcg ctg ctg atc gcc ggc tcc gag gcc ccc 144
   Asp Ser Thr Arg Asp Gly Ala Leu Leu Ile Ala Gly Ser Glu Ala Pro
   35 40 45
   aag cgc ggc agc atc ctc agc aaa cct cgc gcg ggc ggc gcg ggc gcc 192
   Lys Arg Gly Ser Ile Leu Ser Lys Pro Arg Ala Gly Gly Ala Gly Ala
   50 55 60
   ggg aag ccc ccc aag cgc aac gcc ttc tac cgc aag ctg cag aat ttc 240
   Gly Lys Pro Pro Lys Arg Asn Ala Phe Tyr Arg Lys Leu Gln Asn Phe
   65 70 75 80
   ctc tac aac gtg ctg gag cgg ccg cgc ggc tgg gcg ttc atc tac cac 288
   Leu Tyr Asn Val Leu Glu Arg Pro Arg Gly Trp Ala Phe Ile Tyr His
   85 90 95
   gcc tac gtg ttc ctc ctg gtt ttc tcc tgc ctc gtg ctg tct gtg ttt 336
   Ala Tyr Val Phe Leu Leu Val Phe Ser Cys Leu Val Leu Ser Val Phe
   100 105 110
   tcc acc atc aag gag tat gag aag agc tcg gag ggg gcc ctc tac atc 384
   Ser Thr Ile Lys Glu Tyr Glu Lys Ser Ser Glu Gly Ala Leu Tyr Ile
   115 120 125
   ctg gaa atc gtg act atc gtg gtg ttt ggc gtg gag tac ttc gtg cgg 432
   Leu Glu Ile Val Thr Ile Val Val Phe Gly Val Glu Tyr Phe Val Arg
   130                        135                      140
   atc tgg gcc gca ggc tgc tgc tgc cgg tac cgt ggc tgg agg ggg cgg     480
   Ile Trp Ala Ala Gly Cys Cys Cys Arg Tyr Arg Gly Trp Arg Gly Arg
   145                 150                     155                 160
   ctc aag ttt gcc cgg aaa ccg ttc tgt gtg att gac atc atg gtg ctc     528
   Leu Lys Phe Ala Arg Lys Pro Phe Cys Val Ile Asp Ile Met Val Leu
                   165                     170                 175
   atc gcc tcc att gcg gtg ctg gcc gcc ggc tcc cag ggc aac gtc ttt     576
   Ile Ala Ser Ile Ala Val Leu Ala Ala Gly Ser Gln Gly Asn Val Phe
               180                     185                 190
   gcc aca tct gcg ctc cgg agc ctg cgc ttc ctg cag att ctg cgg atg     624
   Ala Thr Ser Ala Leu Arg Ser Leu Arg Phe Leu Gln Ile Leu Arg Met
           195                     200                 205
   atc cgc atg gac cgg cgg gga ggc acc tgg aag ctg ctg ggc tct gtg     672
   Ile Arg Met Asp Arg Arg Gly Gly Thr Trp Lys Leu Leu Gly Ser Val
       210                     215                 220
   gtc tat gcc cac agc aag gag ctg gtc act gcc tgg tac atc ggc ttc     720
   Val Tyr Ala His Ser Lys Glu Leu Val Thr Ala Trp Tyr Ile Gly Phe
   225                 230                     235                 240
   ctt tgt ctc atc ctg gcc tcg ttc ctg gtg tac ttg gca gag aag ggg     768
   Leu Cys Leu Ile Leu Ala Ser Phe Leu Val Tyr Leu Ala Glu Lys Gly
                   245                     250                 255
   gag aac gac cac ttt gac acc tac gcg gat gca ctc tgg tgg ggc ctg     816
   Glu Asn Asp His Phe Asp Thr Tyr Ala Asp Ala Leu Trp Trp Gly Leu
               260                     265                 270
   atc acg ctg acc acc att ggc tac ggg gac aag tac ccc cag acc tgg     864
   Ile Thr Leu Thr Thr Ile Gly Tyr Gly Asp Lys Tyr Pro Gln Thr Trp
           275                     280                 285
   aac ggc agg ctc ctt gcg gca acc ttc acc ctc atc ggt gtc tcc ttc     912
   Asn Gly Arg Leu Leu Ala Ala Thr Phe Thr Leu Ile Gly Val Ser Phe
       290                     295                 300
   ttc gcg ctg cct gca ggc atc ttg ggg tct ggg ttt gcc ctg aag gtt     960
   Phe Ala Leu Pro Ala Gly Ile Leu Gly Ser Gly Phe Ala Leu Lys Val
   305                 310                     315                 320
   cag gag cag cac agg cag aag cac ttt gag aag agg cgg aac ccg gca     1008
   Gln Glu Gln His Arg Gln Lys His Phe Glu Lys Arg Arg Asn Pro Ala
                   325                     330                 335
   gca ggc ctg atc cag tcg gcc tgg aga ttc tac gcc acc aac ctc tcg     1056
   Ala Gly Leu Ile Gln Ser Ala Trp Arg Phe Tyr Ala Thr Asn Leu Ser
               340                     345                 350
   cgc aca gac ctg cac tcc acg tgg cag tac tac gag cga acg gtc acc     1104
   Arg Thr Asp Leu His Ser Thr Trp Gln Tyr Tyr Glu Arg Thr Val Thr
           355                     360                 365
   gtg ccc atg tac agt tcg caa act caa acc tac ggg gcc tcc aga ctt     1152
   Val Pro Met Tyr Ser Ser Gln Thr Gln Thr Tyr Gly Ala Ser Arg Leu
       370                     375                 380
   atc ccc ccg ctg aac cag ctg gag ctg ctg agg aac ctc aag agt aaa     1200
   Ile Pro Pro Leu Asn Gln Leu Glu Leu Leu Arg Asn Leu Lys Ser Lys
   385                 390                     395                 400
   tct gga ctc gct ttc agg aag gac ccc ccg ccg gag ccg tct cca agc     1248
   Ser Gly Leu Ala Phe Arg Lys Asp Pro Pro Pro Glu Pro Ser Pro Ser
                       405                 410                 415
   cag aag gtc agt ttg aaa gat cgt gtc ttc tcc agc ccc cga ggc gtg      1296
   Gln Lys Val Ser Leu Lys Asp Arg Val Phe Ser Ser Pro Arg Gly Val
               420                 425                 430
   gct gcc aag ggg aag ggg tcc ccg cag gcc cag act gtg agg cgg tca      1344
   Ala Ala Lys Gly Lys Gly Ser Pro Gln Ala Gln Thr Val Arg Arg Ser
           435                 440                 445
   ccc agc gcc gac cag agc ctc gag gac agc ccc agc aag gtg ccc aag      1392
   Pro Ser Ala Asp Gln Ser Leu Glu Asp Ser Pro Ser Lys Val Pro Lys
       450                 455                 460
   agc tgg agc ttc ggg gac cgc agc cgg gca cgc cag gct ttc cgc atc      1440
   Ser Trp Ser Phe Gly Asp Arg Ser Arg Ala Arg Gln Ala Phe Arg Ile
   465                 470                 475                 480
   aag ggt gcc gcg tca cgg cag aac tca gaa gaa gca agc ctc ccc gga      1488
   Lys Gly Ala Ala Ser Arg Gln Asn Ser Glu Glu Ala Ser Leu Pro Gly
                   485                 490                 495
   gag gac att gtg gat gac aag agc tgc ccc tgc gag ttt gtg acc gag      1536
   Glu Asp Ile Val Asp Asp Lys Ser Cys Pro Cys Glu Phe Val Thr Glu
               500                 505                 510
   gac ctg acc ccg ggc ctc aaa gtc agc atc aga gcc gtg tgt gtc atg      1584
   Asp Leu Thr Pro Gly Leu Lys Val Ser Ile Arg Ala Val Cys Val Met
           515                 520                 525
   cgg ttc ctg gtg tcc aag cgg aag ttc aag gag agc ctg cgg ccc tac      1632
   Arg Phe Leu Val Ser Lys Arg Lys Phe Lys Glu Ser Leu Arg Pro Tyr
       530                 535                 540
   gac gtg atg gac gtc atc gag cag tac tca gcc ggc cac ctg gac atg      1680
   Asp Val Met Asp Val Ile Glu Gln Tyr Ser Ala Gly His Leu Asp Met
   545                 550                 555                 560
   ctg tcc cga att aag agc ctg cag tcc agg caa gag ccc cgc ctg cct      1728
   Leu Ser Arg Ile Lys Ser Leu Gln Ser Arg Gln Glu Pro Arg Leu Pro
                   565                 570                 575
   gtc cag cag ggg aca aga acg ggg tgg gct tct ggg aca aag ccc act      1776
   Val Gln Gln Gly Thr Arg Thr Gly Trp Ala Ser Gly Thr Lys Pro Thr
               580                 585                 590
   gtg gcc cat ggt ggg agt gca ggg ggt gtg tgg gcg ggg cct cct ccc      1824
   Val Ala His Gly Gly Ser Ala Gly Gly Val Trp Ala Gly Pro Pro Pro
           595                 600                 605
   cac cca cgt cgg cct ctg tca gct tct gtt gtg tct tca caa agt ctg      1872
   His Pro Arg Arg Pro Leu Ser Ala Ser Val Val Ser Ser Gln Ser Leu
       610                 615                 620
   ttt taa                                                              1878
   Phe
   625
<210> 4
   <211> 625
   <212> PRT
   <213> Homo sapiens
<400> 4
   Met Val Gln Lys Ser Arg Asn Gly Gly Val Tyr Pro Gly Pro Ser Gly
   1               5                   10                  15
   Glu Lys Lys Leu Lys Val Gly Phe Val Gly Leu Asp Pro Gly Ala Pro
   20 25 30
   Asp Ser Thr Arg Asp Gly Ala Leu Leu Ile Ala Gly Ser Glu Ala Pro
   35 40 45
   Lys Arg Gly Ser Ile Leu Ser Lys Pro Arg Ala Gly Gly Ala Gly Ala
   50 55 60
   Gly Lys Pro Pro Lys Arg Asn Ala Phe Tyr Arg Lys Leu Gln Asn Phe
   65 70 75 80
   Leu Tyr Asn Val Leu Glu Arg Pro Arg Gly Trp Ala Phe Ile Tyr His
   85 90 95
   Ala Tyr Val Phe Leu Leu Val Phe Ser Cys Leu Val Leu Ser Val Phe
   100 105 110
   Ser Thr Ile Lys Glu Tyr Glu Lys Ser Ser Glu Gly Ala Leu Tyr Ile
   115 120 125
   Leu Glu Ile Val Thr Ile Val Val Phe Gly Val Glu Tyr Phe Val Arg
   130 135 140
   Ile Trp Ala Ala Gly Cys Cys Cys Arg Tyr Arg Gly Trp Arg Gly Arg
   145 150 155 160
   Leu Lys Phe Ala Arg Lys Pro Phe Cys Val Ile Asp Ile Met Val Leu
   165 170 175
   Ile Ala Ser Ile Ala Val Leu Ala Ala Gly Ser Gln Gly Asn Val Phe
   180 185 190
   Ala Thr Ser Ala Leu Arg Ser Leu Arg Phe Leu Gln Ile Leu Arg Met
   195 200 205
   Ile Arg Met Asp Arg Arg Gly Gly Thr Trp Lys Leu Leu Gly Ser Val
   210 215 220
   Val Tyr Ala His Ser Lys Glu Leu Val Thr Ala Trp Tyr Ile Gly Phe
   225 230 235 240
   Leu Cys Leu Ile Leu Ala Ser Phe Leu Val Tyr Leu Ala Glu Lys Gly
   245 250 255
   Glu Asn Asp His Phe Asp Thr Tyr Ala Asp Ala Leu Trp Trp Gly Leu
   260 265 270
   Ile Thr Leu Thr Thr Ile Gly Tyr Gly Asp Lys Tyr Pro Gln Thr Trp
   275 280 285
   Asn Gly Arg Leu Leu Ala Ala Thr Phe Thr Leu Ile Gly Val Ser Phe
   290 295 300
   Phe Ala Leu Pro Ala Gly Ile Leu Gly Ser Gly Phe Ala Leu Lys Val
   305 310 315 320
   Gln Glu Gln His Arg Gln Lys His Phe Glu Lys Arg Arg Asn Pro Ala
   325 330 335
   Ala Gly Leu Ile Gln Ser Ala Trp Arg Phe Tyr Ala Thr Asn Leu Ser
   340 345 350
   Arg Thr Asp Leu His Ser Thr Trp Gln Tyr Tyr Glu Arg Thr Val Thr
   355 360 365
   Val Pro Met Tyr Ser Ser Gln Thr Gln Thr Tyr Gly Ala Ser Arg Leu
   370 375 380
   Ile Pro Pro Leu Asn Gln Leu Glu Leu Leu Arg Asn Leu Lys Ser Lys
   385 390 395 400
   Ser Gly Leu Ala Phe Arg Lys Asp Pro Pro Pro Glu Pro Ser Pro Ser
   405 410 415
   Gln Lys Val Ser Leu Lys Asp Arg Val Phe Ser Ser Pro Arg Gly Val
   420 425 430
   Ala Ala Lys Gly Lys Gly Ser Pro Gln Ala Gln Thr Val Arg Arg Ser
   435 440 445
   Pro Ser Ala Asp Gln Ser Leu Glu Asp Ser Pro Ser Lys Val Pro Lys
   450 455 460
   Ser Trp Ser Phe Gly Asp Arg Ser Arg Ala Arg Gln Ala Phe Arg Ile
   465 470 475 480
   Lys Gly Ala Ala Ser Arg Gln Asn Ser Glu Glu Ala Ser Leu Pro Gly
   485 490 495
   Glu Asp Ile Val Asp Asp Lys Ser Cys Pro Cys Glu Phe Val Thr Glu
   500 505 510
   Asp Leu Thr Pro Gly Leu Lys Val Ser Ile Arg Ala Val Cys Val Met
   515 520 525
   Arg Phe Leu Val Ser Lys Arg Lys Phe Lys Glu Ser Leu Arg Pro Tyr
   530 535 540
   Asp Val Met Asp Val Ile Glu Gln Tyr Ser Ala Gly His Leu Asp Met
   545 550 555 560
   Leu Ser Arg Ile Lys Ser Leu Gln Ser Arg Gln Glu Pro Arg Leu Pro
                   565                 570                 575
   Val Gln Gln Gly Thr Arg Thr Gly Trp Ala Ser Gly Thr Lys Pro Thr
               580                 585                 590
   Val Ala His Gly Gly Ser Ala Gly Gly Val Trp Ala Gly Pro Pro Pro
           595                 600                 605
   His Pro Arg Arg Pro Leu Ser Ala Ser Val Val Ser Ser Gln Ser Leu
       610                 615                 620
   Phe
   625
<210> 5
   <211> 1848
   <212> DNA
   <213> Homo sapiens
   <220>
   <221> CDS
   <222> (1)..(1848)
   <223>
<400>
   atg gtg cag aag tcg cgc aac ggc ggc gta tac ccc ggc ccg agc ggg      48
   Met Val Gln Lys Ser Arg Asn Gly Gly Val Tyr Pro Gly Pro Ser Gly
   1               5                   10                  15
   gag aag aag ctg aag gtg ggc ttc gtg ggg ctg gac ccc ggc gcg ccc      96
   Glu Lys Lys Leu Lys Val Gly Phe Val Gly Leu Asp Pro Gly Ala Pro
               20                  25                  30
   gac tcc acc cgg gac ggg gcg ctg ctg atc gcc ggc tcc gag gcc ccc     144
   Asp Ser Thr Arg Asp Gly Ala Leu Leu Ile Ala Gly Ser Glu Ala Pro
           35                  40                  45
   aag cgc ggc agc atc ctc agc aaa cct cgc gcg ggc ggc gcg ggc gcc     192
   Lys Arg Gly Ser Ile Leu Ser Lys Pro Arg Ala Gly Gly Ala Gly Ala
       50                  55                  60
   ggg aag ccc ccc aag cgc aac gcc ttc tac cgc aag ctg cag aat ttc     240
   Gly Lys Pro Pro Lys Arg Asn Ala Phe Tyr Arg Lys Leu Gln Asn Phe
   65                  70                  75                  80
   ctc tac aac gtg ctg gag cgg ccg cgc ggc tgg gcg ttc atc tac cac     288
   Leu Tyr Asn Val Leu Glu Arg Pro Arg Gly Trp Ala Phe Ile Tyr His
                   85                  90                  95
   gcc tac gtg ttc ctc ctg gtt ttc tcc tgc ctc gtg ctg tct gtg ttt     336
   Ala Tyr Val Phe Leu Leu Val Phe Ser Cys Leu Val Leu Ser Val Phe
               100                 105                 110
   tcc acc atc aag gag tat gag aag agc tcg gag ggg gcc ctc tac atc     384
   Ser Thr Ile Lys Glu Tyr Glu Lys Ser Ser Glu Gly Ala Leu Tyr Ile
           115                 120                 125
   ctg gaa atc gtg act atc gtg gtg ttt ggc gtg gag tac ttc gtg cgg     432
   Leu Glu Ile Val Thr Ile Val Val Phe Gly Val Glu Tyr Phe Val Arg
       130                 135                 140
   atc tgg gcc gca ggc tgc tgc tgc cgg tac cgt ggc tgg agg ggg cgg      480
   Ile Trp Ala Ala Gly Cys Cys Cys Arg Tyr Arg Gly Trp Arg Gly Arg
   145                 150                 155                 160
   ctc aag ttt gcc cgg aaa ccg ttc tgt gtg att gac atc atg gtg ctc      528
   Leu Lys Phe Ala Arg Lys Pro Phe Cys Val Ile Asp Ile Met Val Leu
                   165                 170                 175
   atc gcc tcc att gcg gtg ctg gcc gcc ggc tcc cag ggc aac gtc ttt      576
   Ile Ala Ser Ile Ala Val Leu Ala Ala Gly Ser Gln Gly Asn Val Phe
               180                 185                 190
   gcc aca tct gcg ctc cgg agc ctg cgc ttc ctg cag att ctg cgg atg      624
   Ala Thr Ser Ala Leu Arg Ser Leu Arg Phe Leu Gln Ile Leu Arg Met
           195                 200                 205
   atc cgc atg gac cgg cgg gga ggc acc tgg aag ctg ctg ggc tct gtg      672
   Ile Arg Met Asp Arg Arg Gly Gly Thr Trp Lys Leu Leu Gly Ser Val
       210                 215                 220
   gtc tat gcc cac agc aag gag ctg gtc act gcc tgg tac atc ggc ttc      720
   Val Tyr Ala His Ser Lys Glu Leu Val Thr Ala Trp Tyr Ile Gly Phe
   225                 230                 235                 240
   ctt tgt ctc atc ctg gcc tcg ttc ctg gtg tac ttg gca gag aag ggg      768
   Leu Cys Leu Ile Leu Ala Ser Phe Leu Val Tyr Leu Ala Glu Lys Gly
                   245                 250                 255
   gag aac gac cac ttt gac acc tac gcg gat gca ctc tgg tgg ggc ctg      816
   Glu Asn Asp His Phe Asp Thr Tyr Ala Asp Ala Leu Trp Trp Gly Leu
               260                 265                 270
   atc acg ctg acc acc att ggc tac ggg gac aag tac ccc cag acc tgg      864
   Ile Thr Leu Thr Thr Ile Gly Tyr Gly Asp Lys Tyr Pro Gln Thr Trp
           275                 280                 285
   aac ggc agg ctc ctt gcg gca acc ttc acc ctc atc ggt gtc tcc ttc      912
   Asn Gly Arg Leu Leu Ala Ala Thr Phe Thr Leu Ile Gly Val Ser Phe
       290                 295                 300
   ttc gcg ctg cct gca ggc atc ttg ggg tct ggg ttt gcc ctg aag gtt      960
   Phe Ala Leu Pro Ala Gly Ile Leu Gly Ser Gly Phe Ala Leu Lys Val
   305                 310                 315                 320
   cag gag cag cac agg cag aag cac ttt gag aag agg cgg aac ccg gca     1008
   Gln Glu Gln His Arg Gln Lys His Phe Glu Lys Arg Arg Asn Pro Ala
                   325                 330                 335
   gca ggc ctg atc cag tcg gcc tgg aga ttc tac gcc acc aac ctc tcg     1056
   Ala Gly Leu Ile Gln Ser Ala Trp Arg Phe Tyr Ala Thr Asn Leu Ser
               340                 345                 350
   cgc aca gac ctg cac tcc acg tgg cag tac tac gag cga acg gtc acc     1104
   Arg Thr Asp Leu His Ser Thr Trp Gln Tyr Tyr Glu Arg Thr Val Thr
           355                 360                 365
   gtg ccc atg tac aga ctt atc ccc ccg ctg aac cag ctg gag ctg ctg     1152
   Val Pro Met Tyr Arg Leu Ile Pro Pro Leu Asn Gln Leu Glu Leu Leu
       370                 375                 380
   agg aac ctc aag agt aaa tct gga ctc gct ttc agg aag gac ccc ccg     1200
   Arg Asn Leu Lys Ser Lys Ser Gly Leu Ala Phe Arg Lys Asp Pro Pro
   385                 390                 395                 400
   ccg gag ccg tct cca agc cag aag gtc agt ttg aaa gat cgt gtc ttc     1248
   Pro Glu Pro Ser Pro Ser Gln Lys Val Ser Leu Lys Asp Arg Val Phe
                   405                 410                 415
   tcc agc ccc cga ggc gtg gct gcc aag ggg aag ggg tcc ccg cag gcc      1296
   Ser Ser Pro Arg Gly Val Ala Ala Lys Gly Lys Gly Ser Pro Gln Ala
               420                 425                 430
   cag act gtg agg cgg tca ccc agc gcc gac cag agc ctc gag gac agc      1344
   Gln Thr Val Arg Arg Ser Pro Ser Ala Asp Gln Ser Leu Glu Asp Ser
           435                 440                 445
   ccc agc aag gtg ccc aag agc tgg agc ttc ggg gac cgc agc cgg gca      1392
   Pro Ser Lys Val Pro Lys Ser Trp Ser Phe Gly Asp Arg Ser Arg Ala
       450                 455                 460
   cgc cag gct ttc cgc atc aag ggt gcc gcg tca cgg cag aac tca gaa      1440
   Arg Gln Ala Phe Arg Ile Lys Gly Ala Ala Ser Arg Gln Asn Ser Glu
   465                 470                 475                 480
   gaa gca agc ctc ccc gga gag gac att gtg gat gac aag agc tgc ccc      1488
   Glu Ala Ser Leu Pro Gly Glu Asp Ile Val Asp Asp Lys Ser Cys Pro
                   485                 490                 495
   tgc gag ttt gtg acc gag gac ctg acc ccg ggc ctc aaa gtc agc atc      1536
   Cys Glu Phe Val Thr Glu Asp Leu Thr Pro Gly Leu Lys Val Ser Ile
               500                 505                 510
   aga gcc gtg tgt gtc atg cgg ttc ctg gtg tcc aag cgg aag ttc aag      1584
   Arg Ala Val Cys Val Met Arg Phe Leu Val Ser Lys Arg Lys Phe Lys
           515                 520                 525
   gag agc ctg cgg ccc tac gac gtg atg gac gtc atc gag cag tac tca      1632
   Glu Ser Leu Arg Pro Tyr Asp Val Met Asp Val Ile Glu Gln Tyr Ser
       530                 535                 540
   gcc ggc cac ctg gac atg ctg tcc cga att aag agc ctg cag tcc agg      1680
   Ala Gly His Leu Asp Met Leu Ser Arg Ile Lys Ser Leu Gln Ser Arg
   545                 550                 555                 560
   caa gag ccc cgc ctg cct gtc cag cag ggg aca aga acg ggg tgg gct      1728
   Gln Glu Pro Arg Leu Pro Val Gln Gln Gly Thr Arg Thr Gly Trp Ala
                   565                 570                 575
   tct ggg aca aag ccc act gtg gcc cat ggt ggg agt gca ggg ggt gtg      1776
   Ser Gly Thr Lys Pro Thr Val Ala His Gly Gly Ser Ala Gly Gly Val
               580                 585                 590
   tgg gcg ggg cct cct ccc cac cca cgt cgg cct ctg tca gct tct gtt      1824
   Trp Ala Gly Pro Pro Pro His Pro Arg Arg Pro Leu Ser Ala Ser Val
           595                 600                 605
   gtg tct tca caa agt ctg ttt taa                                      1848
   Val Ser Ser Gln Ser Leu Phe
       610                 615
<210> 6
   <211> 615
   <212> PRT
   <213> Homo sapiens
<400> 6
   Met Val Gln Lys Ser Arg Asn Gly Gly Val Tyr Pro Gly Pro Ser Gly
   1               5                   10                  15
   Glu Lys Lys Leu Lys Val Gly Phe Val Gly Leu Asp Pro Gly Ala Pro
               20                  25                  30
   Asp Ser Thr Arg Asp Gly Ala Leu Leu Ile Ala Gly Ser Glu Ala Pro
   35 40 45
   Lys Arg Gly Ser Ile Leu Ser Lys Pro Arg Ala Gly Gly Ala Gly Ala
   50 55 60
   Gly Lys Pro Pro Lys Arg Asn Ala Phe Tyr Arg Lys Leu Gln Asn Phe
   65 70 75 80
   Leu Tyr Asn Val Leu Glu Arg Pro Arg Gly Trp Ala Phe Ile Tyr His
   85 90 95
   Ala Tyr Val Phe Leu Leu Val Phe Ser Cys Leu Val Leu Ser Val Phe
   100 105 110
   Ser Thr Ile Lys Glu Tyr Glu Lys Ser Ser Glu Gly Ala Leu Tyr Ile
   115 120 125
   Leu Glu Ile Val Thr Ile Val Val Phe Gly Val Glu Tyr Phe Val Arg
   130 135 140
   Ile Trp Ala Ala Gly Cys Cys Cys Arg Tyr Arg Gly Trp Arg Gly Arg
   145 150 155 160
   Leu Lys Phe Ala Arg Lys Pro Phe Cys Val Ile Asp Ile Met Val Leu
   165 170 175
   Ile Ala Ser Ile Ala Val Leu Ala Ala Gly Ser Gln Gly Asn Val Phe
   180 185 190
   Ala Thr Ser Ala Leu Arg Ser Leu Arg Phe Leu Gln Ile Leu Arg Met
   195 200 205
   Ile Arg Met Asp Arg Arg Gly Gly Thr Trp Lys Leu Leu Gly Ser Val
   210 215 220
   Val Tyr Ala His Ser Lys Glu Leu Val Thr Ala Trp Tyr Ile Gly Phe
   225 230 235 240
   Leu Cys Leu Ile Leu Ala Ser Phe Leu Val Tyr Leu Ala Glu Lys Gly
   245 250 255
   Glu Asn Asp His Phe Asp Thr Tyr Ala Asp Ala Leu Trp Trp Gly Leu
   260 265 270
   Ile Thr Leu Thr Thr Ile Gly Tyr Gly Asp Lys Tyr Pro Gln Thr Trp
   275 280 285
   Asn Gly Arg Leu Leu Ala Ala Thr Phe Thr Leu Ile Gly Val Ser Phe
   290 295 300
   Phe Ala Leu Pro Ala Gly Ile Leu Gly Ser Gly Phe Ala Leu Lys Val
   305 310 315 320
   Gln Glu Gln His Arg Gln Lys His Phe Glu Lys Arg Arg Asn Pro Ala
   325 330 335
   Ala Gly Leu Ile Gln Ser Ala Trp Arg Phe Tyr Ala Thr Asn Leu Ser
   340 345 350
   Arg Thr Asp Leu His Ser Thr Trp Gln Tyr Tyr Glu Arg Thr Val Thr
   355 360 365
   Val Pro Met Tyr Arg Leu Ile Pro Pro Leu Asn Gln Leu Glu Leu Leu
   370 375 380
   Arg Asn Leu Lys Ser Lys Ser Gly Leu Ala Phe Arg Lys Asp Pro Pro
   385 390 395 400
   Pro Glu Pro Ser Pro Ser Gln Lys Val Ser Leu Lys Asp Arg Val Phe
   405 410 415
   Ser Ser Pro Arg Gly Val Ala Ala Lys Gly Lys Gly Ser Pro Gln Ala
   420 425 430
   Gln Thr Val Arg Arg Ser Pro Ser Ala Asp Gln Ser Leu Glu Asp Ser
   435 440 445
   Pro Ser Lys Val Pro Lys Ser Trp Ser Phe Gly Asp Arg Ser Arg Ala
   450 455 460
   Arg Gln Ala Phe Arg Ile Lys Gly Ala Ala Ser Arg Gln Asn Ser Glu
   465 470 475 480
   Glu Ala Ser Leu Pro Gly Glu Asp Ile Val Asp Asp Lys Ser Cys Pro
   485 490 495
   Cys Glu Phe Val Thr Glu Asp Leu Thr Pro Gly Leu Lys Val Ser Ile
   500 505 510
   Arg Ala Val Cys Val Met Arg Phe Leu Val Ser Lys Arg Lys Phe Lys
   515 520 525
   Glu Ser Leu Arg Pro Tyr Asp Val Met Asp Val Ile Glu Gln Tyr Ser
   530 535 540
   Ala Gly His Leu Asp Met Leu Ser Arg Ile Lys Ser Leu Gln Ser Arg
   545 550 555 560
   Gln Glu Pro Arg Leu Pro Val Gln Gln Gly Thr Arg Thr Gly Trp Ala
   565 570 575
   Ser Gly Thr Lys Pro Thr Val Ala His Gly Gly Ser Ala Gly Gly Val
   580 585 590
   Trp Ala Gly Pro Pro Pro His Pro Arg Arg Pro Leu Ser Ala Ser Val
   595 600 605
   Val Ser Ser Gln Ser Leu Phe
   610 615
<210> 7
   <211> 872
   <212> PRT
   <213> Homo sapiens
<400> 7
<210> 8
   <211> 27
   <212> DNA
   <213> Artificial
<220>
   <223> oligonucleotide
<400> 8
   acctctgcgg attgcatcgg tgtgtgg 27
<210> 9
   <211> 25
   <212> DNA
   <213> Artificial
<220>
   <223> oligonucleotide
<400> 9
   ggatgacttg catgaggctg ggtgg 25
<210> 10
   <211> 35
   <212> DNA
   <213> Artificial
<220>
   <223> oligonucleotide
<400> 10
   agcgaattct caatgggcga ggacacggac acgcg 35
<210> 11
   <211> 34
   <212> DNA
   <213> Artificial
<220>
   <223> oligonucleotide
<400> 11
   tccggatcct cctgtgtcca cacactgcca cctc 34
<210> 12
   <211> 35
   <212> DNA
   <213> Artificial
<220>
   <223> oligonucleotide
<400> 12
   aatattaaaa cagactttgt gaagacacaa cagaa 35
<210> 13
   <211> 33
   <212> DNA
   <213> Artificial
<220>
   <223> oligonucleotide
<400> 13
   atcagaattc acatggtgca gaagtcgcgc aac 33
<210> 14
   <211> 42
   <212> DNA
   <213> Artificial
<220>
   <223> oligonucleotide
<400> 14
   tgacagatct taaaacagac tttgtgaaga cacaacagaa gc 42
<210> 15
   <211> 17
   <212> DNA
   <213> Artificial
<220>
   <223> oligonucleotide
<400> 15
   gtgtggatgc tgccccg 17
<210> 16
   <211> 18
   <212> DNA
   <213> Artificial
<220>
   <223> oligonucleotide
<400> 16
   tcccgcctca aaacctcg 18
<210> 17
   <211> 35
   <212> DNA
   <213> Artificial
<220>
   <223> oligonucleotide
<400> 17
   actagaattc agccagaagg tcagtttgaa agatc 35
<210> 18
   <211> 27
   <212> DNA
   <213> Artificial
<220>
   <223> oligonucleotide
<400> 18
   atcaggatcc gcgccgcctc acttcct 27
<210> 19
   <211> 35
   <212> DNA
   <213> Artificial
<220>
   <223> oligonucleotide
<400> 19
   actagaattc agccagaagg tcagtttgaa agatc 35
<210> 20
   <211> 36
   <212> DNA
   <213> Artificial
<220>
   <223> oligonucleotide
<400> 20
   actaggatcc ctactggact gcaggctctt aattcg 36
<210> 21
   <211> 31
   <212> DNA
   <213> Artificial
<220>
   <223> oligonucleotide
<400> 21
   aactagaatt cgtggaccag atcgtggggc g 31
<210> 22
   <211> 27
   <212> DNA
   <213> Artificial
<220>
   <223> oligonucleotide
<400> 22
   atcaggatcc gcgccgcctc acttcct 27
<210> 23
   <211> 28
   <212> DNA
   <213> Artificial
<220>
   <223> oligonucleotide
<400> 23
   aatcagaatt ccaagagccc cgcctgcc 28
<210> 24
   <211> 22
   <212> DNA
   <213> Artificial
<220>
   <223> oligonucleotide
<400> 24
   gcacgatgca cagttgaagt ga 22
<210> 25
   <211> 29
   <212> DNA
   <213> Artificial
<220>
   <223> oligonucleotide
<400> 25
   tactgaattc ttcctggtgt ccaagcgga 29
<210> 26
   <211> 37
   <212> DNA
   <213> Artificial
<220>
   <223> oligonucleotide
<400> 26
   acatggatcc tcacctggac tgcaggctct taattcg 37
<210> 27
   <211> 37
   <212> DNA
   <213> Artificial
<220>
   <223> oligonucleotide
<400> 27
   acatgaattc cagaaggtca gtttgaaaga tcgtgtc 37
<210> 28
   <211> 31
   <212> DNA
   <213> Artificial
<220>
   <223> oligonucleotide
<400> 28
   tgatggatcc tcaccgcatg acacacacgg c 31
<210> 29
   <211> 30
   <212> DNA
   <213> Artificial
<220>
   <223> oligonucleotide
<400> 29
   cacggatcca gcagccagaa ggtcagtttg 30
<210> 30
   <211> 31
   <212> DNA
   <213> Artificial
<220>
   <223> oligonucleotide
<400> 30
   cacgaattct ggacggacca aactgcgtat a 31
<210> 31
   <211> 32
   <212> DNA
   <213> Artificial
<220>
   <223> oligonucleotide
<400> 31
   agcggatcca tgggcgagga cacggacacg cg 32
<210> 32
   <211> 34
   <212> DNA
   <213> Artificial
<220>
   <223> oligonucleotide
<400> 32
   tccgaattct cctgtgtcca cacactgcca cctc 34
<210> 33
   <211> 25
   <212> DNA
   <213> Artificial
<220>
   <223> oligonucleotide
<400> 33
   gagcctcgag gacagcccca gcaag 25
<210> 34
   <211> 35
   <212> DNA
   <213> Artificial
<220>
   <223> oligonucleotide
<400> 34
   aagaattctg taaaaggtca ctgccaggag ccccc 35
<210> 35
   <211> 31
   <212> DNA
   <213> Artificial
<220>
   <223> oligonucleotide
<400> 35
   cggaattccc atggtgcaga agtcgcgcaa c 31
<210> 36
   <211> 32
   <212> DNA
   <213> Artificial
<220>
   <223> oligonucleotide
<400> 36
   ccagatcttg taaaaggtca ctgccaggag cc 32
<210> 37
   <211> 151830
   <212> DNA
   <213> Homo sapiens
<220>
   <221> misc_feature
   <222> (10)..(10)
   <223> n = a or c or g or t
<220>
   <221> misc_feature
   <222> (60402)..(60402)
   <223> n = a or c or g or t
<220>
   <221> misc_feature
   <222> (61110)..(61110)
   <223> n = a or c or g or t
<220>
   <221> misc_feature
   <222> (98207)..(98207)
   <223> n = a or c or g or t
<220>
   <221> misc_feature
   <222> (98208)..(98208)
   <223> n = a or c or g or t
<220>
   <221> misc_feature
   <222> (98209)..(98209)
   <223> n = a or c or g or t
<220>
   <221> misc_feature
   <222> (98210)..(98210)
   <223> n = a or c or g or t
<220>
   <221> misc_feature
   <222> (98211)..(98211)
   <223> n = a or c or g or t
<220>
   <221> misc_feature
   <222> (99743)..(99743)
   <223> n = a or c or g or t
<220>
   <221> misc_feature
   <222> (108055)..(108055)
   <223> n = a or c or g or t
<220>
   <221> misc_feature
   <222> (109094)..(109094)
   <223> n = a or c or g or t
<220>
   <221> misc_feature
   <222> (109125)..(109125)
   <223> n = a or c or g or t
<220>
   <221> misc_feature
   <222> (118900)..(118900)
   <223> n = a or c or g or t
<220>
   <221> misc_feature
   <222> (119024)..(119052)
   <223> n = a or c or g or t
<220>
   <221> misc_feature
   <222> (119053)..(119112)
   <223> n = a or c or g or t
<220>
   <221> misc_feature
   <222> (119115)..(119121)
   <223> n = a or c or g or t
<220>
   <221> misc_feature
   <222> (119123)..(119123)
   <223> n = a or c or g or t
<220>
   <221> misc_feature
   <222> (141674)..(141674)
   <223> n = a or c or g or t
<220>
   <221> misc_feature
   <222> (142063)..(142063)
   <223> n = a or c or g or t
<220>
   <221> misc_feature
   <222> (142137)..(142137)
   <223> n = a or c or g or t
<220>
   <221> misc_feature
   <222> (142967)..(142967)
   <223> n = a or c or g or t
<220>
   <221> misc_feature
   <222> (143077)..(143077)
   <223> n = a or c or g or t
<220>
   <221> misc_feature
   <222> (143506)..(143506)
   <223> n = a or c or g or t
<220>
   <221> misc_feature
   <222> (143587)..(143587)
   <223> n = a or c or g or t
<220>
   <221> misc_feature
   <222> (143629)..(143629)
   <223> n = a or c or g or t
<220>
   <221> misc_feature
   <222> (149079)..(149079)
   <223> n = a or c or g or t
<220>
   <221> misc_feature
   <222> (5363)..(5363)
   <223> n = a or c or g or t
<220>
   <221> misc_feature
   <222> (8080)..(8080)
   <223> n = a or c or g or t
<220>
   <221> misc_feature
   <222> (10296)..(10296)
   <223> n = a or c or g or t
<220>
   <221> misc_feature
   <222> (14528)..(14528)
   <223> n = a or c or g or t
<220>
   <221> misc_feature
   <222> (15336)..(15336)
   <223> n = a or c or g or t
<220>
   <221> misc_feature
   <222> (15457)..(15457)
   <223> n = a or c or g or t
<220>
   <221> misc_feature
   <222> (16288)..(16288)
   <223> n = a or c or g or t
<220>
   <221> misc_feature
   <222> (16306)..(16307)
   <223> n = a or c or g or t
<220>
   <221> misc_feature
   <222> (16316)..(16316)
   <223> n = a or c or g or t
<220>
   <221> misc_feature
   <222> (16397)..(16397)
   <223> n = a or c or g or t
<220>
   <221> misc_feature
   <222> (56012)..(56012)
   <223> n = a or c or g or t
<220>
   <221> misc_feature
   <222> (57662)..(57662)
   <223> n = a or c or g or t
<220>
   <221> 5'UTR
   <222> (1)..(54)
   <223> exon 1
<220>
   <221> exon
   <222> (55)..(124)
   <223> exon 1
<220>
   <221> exon
   <222> (91147)..(91244)
   <223> exon 2
<220>
   <221> exon
   <222> (93669)..(93834)
   <223> exon 3
<220>
   <221> exon
   <222> (96310)..(96422)
   <223> exon 4
<220>
   <221> exon
   <222> (99546)..(99723)
   <223> exon 5
<220>
   <221> exon
   <222> (125441)..(125605)
   <223> exon 6
<220>
   <221> exon
   <222> (141176)..(141345)
   <223> exon 7
<220>
   <221> exon
   <222> (145556)..(145647)
   <223> exon 8
<220>
   <221> exon
   <222> (151316)..(151608)
   <223> exon 9
<220>
   <221> 3'UTR
   <222> (151609)..(151829)
   <223> exon 9
<220>
   <221> allele
   <222> (84026)..(84026)
   <223> complement of biallelic marker 99-24169/139
   <220>
   <221> allele
   <222> (109663)..(109663)
   <223> complement of biallelic marker 24-257/320
<220>
   <221> allele
   <222> (117460)..(117460)
   <223> complement of biallelic marker 99-24175/218
   <220>
   <221> allele
   <222> (99505)..(99505)
   <223> biallelic marker 24-247/216
<400> 37
   attgcatcgn tgtgtggcgg cggggcatgc ccagagcacc gggcacggcc ttca atg       57
                                                               Met
                                                               1
   ggc gag gac acg gac acg cgg aaa att aac cac agc ttc ctg cgg gac       105
   Gly Glu Asp Thr Asp Thr Arg Lys Ile Asn His Ser Phe Leu Arg Asp
               5                   10                  15
   cac agc tat gtg act gaa g gtaacgtacg tgttgtctga gacccctccg            154
   His Ser Tyr Val Thr Glu
           20
   gccggccgcg gcgtggggat gccgtcgcac cgaatgccct ccgaaggttt ggaccgcgcg    214
   atgtgtgtcg tgtccccccg ccccacccca ccccacccca tcccacccca ccccacccca    274
   tcccatccca ccctgcccgg ggcccaggag ggagggagcc cgagggtacc ggcctccgct    334
   gcccagcgcc ggcacagggc agcgccctcc tccgcgccgc cctccgggag gcagctttcc    394
   tctcccaagc caggtggcat cctgattcgg ccctgaccat aattttttaa aaggccacgg     454
   ctgtggctaa tctggtgaag aaatctcggg gaaatttaat ggtttaaatc ctggatttgc    514
   catttcagcc ctgcccaaag cccgcagaat tttctaggct gccctctccc tggagaagaa    574
   gagggacccg gggggaaaaa aacataatcc attgccagat cctcctggga ggcccgcctg    634
   cccgggcccc tccctgtcct ccagaggcag ggtccctgag tgggagggag aaggcggctg    694
   gtttggggct ggccttttta ttcctggtga gttatattga gacaggagca gctgggctaa     754
   ctgtcgggat tttccaaaaa agtgggacat gccatcccaa acaggccctg tttaaaatcc    814
   cctaagttgg ccctacaagc ccaaccccca cccccaccct acccccgagg ctggtgggtc    874
   agcgcccctc tcttacaggc ctggaacttc cgggggcccc ctggtctgcc tcgctagggg    934
   aacagtgggg acagcttccg tgcgcaggca gggcccgcgg agtgaccccg atggaggatg    994
   gggaccggga ggtctgggct cggggccgcc tgtgctggag ccctgcccga gtgcggggac 1054
   tgtcagccgc taacccacgg gctggcggcc cggccgcaga agtgtgcgcg gattccccgg 1114
   gtgggtgccc cagtgggagg ggccctgggc acgggtcccg cggggcaggt gcgcgtgggt 1174
   gcctgtgtca gggagaccga gtgggccagg cggcggagac cgggctcctg cggctgggtc 1234
   cgcctcctcg aagcctggct ctgccggaaa tgaggccgag cggagccgga gccccgcgga 1294
   ggcccgggga gcgcagcgcg agcgcgggcg cgggcgggct cggggcgccc tggcccggga 1354
   ggcagaggct gggcgtggga cctgggcgga gggaggctgc gggggccgcg cccgctcccg 1414
   gcgggagaac cgctgagtca cgcacgcttc gccgccgggt gtgtgcgaga gaggggcagg 1474
   gctgcccggg cctccgggcc ggtggggctc tgacccggcc gcggctttgg gaggcccggg 1534
   gagctgagag cggtcctttg tcgcctgctt cggcgaggct gagtcgggat cagcgtgggt 1594
   ccgggatgtg gtttctgctc gcagcctgca gcgacagagg gttggaggaa gccgccgggt 1654
   tgctggcccg tgcctcggtg gcctggctcg ggccgagagc ggatcttggc aggttgcccc 1714
   gctgctccca ggctcgctgt ggtctggctt cctggagcaa gcctctgttt gctcatctct 1774
   acgttgggga tgacggtggg atggggcgca gggctcgttg tgggaccagg cgtaaaaagc 1834
   gccatggtgg acattttcaa acattaattc cctctgcacc ctgcccgtcc ccttttcttt 1894
   cataaattca ccagccctca gcagggcaca ggaacgggga cggcacagtt ctggaggtca 1954
   gagcagtgct atccaatcct tcatccagac acgagatatt taccgagcac ctgctgcatg 2014
   ccgggcactg ttttatccct ggggactgga caaagactcc ctgcccttgt gcggcctgaa 2074
   ttctatcagg gagctatatg accttgaggt ctgtcttctc accagggctc agtctcccca 2134
   tctgtaaaat gggagtgaat cctgcctaca gggtctggag gtttctgtga ggagcaggtg 2194
   caacggtgtt gaaggagccg ttcagagcta tgcattgcta acgtgcagcc aaaggatgct 2254
   gagcacctgc tgtgtgctgg gacctcatac gaaggctcac ctacgcctta tccccacctt 2314
   gagatgaaca aaccaaggcc tggccttgtg cctggtccag accgagtgcc agtgtgtgca 2374
   gggcataggg ctcaggtgct gcggatgcag agagcaggat cggaacaaga aacaataata 2434
   atcattataa taggtagaat tctggcctcc ctaatgaaaa acacactttc acactgttgc 2494
   atcctgacta tatttcatgc tcagagcaca gggaaggtca cagatttgaa tgtcagaatg 2554
   tcagacttga atcatgttaa agtcctgcct ctgactcctg actgctgtgc agccttggac 2614
   aagttactac accttcctga gcttcacttt ccctttgtaa agggagaaat aataacgacc 2674
   tttcatacag ggttgctggg atgatcagtg attttgctaa tatcaaaagt gcccagcaca 2734
   gtgcttgggt tgttggaggc attgaacaca cggcattgtt attatttata tgccttgtaa 2794
   ctggaagagc ctgtgggcaa acagtggatg ctaaaattca gtttgtggaa gaaccaggtg 2854
   cacaaactcc tgttctacct gtggttgagt ctacactccc ccaccacacc ccagctgctc 2914
   tgatctacct cctgttcctt gagcaggcca ttttctttct tgcttcaggg catttgcaat 2974
   ggctgttccc tggaataccc acctcctgcc tttctcacca ctgactcttt ctcatccttt 3034
   gggccccatg tccaatgtca tctttgcccg tgggagccct gcttgggttc ctgagtctct 3094
   tgtaaaatct caaacatctt aggaagagtt taggttttgt tagtcattca catcttggtg 3154
   tgaattcgtc agagcaggtt ggattttttt tttttttttt tttttttttg gtggcagggc 3214
   tgggtggctt actcttggct gggctcaact gcattgaggg ttatggtgat gattaggtgt 3274
   acctgcaggc cacctggggg cacagagaac tttgcatgaa tggggtcagg gtgtggggag 3334
   agcagtgtag ctgatagggc actgggccct ggaccctgga ccctggaggg tgctaggaag 3394
   tctccatcta gttagacatt tctcaagagc tggatatggt tccaggaagg actcttcctg 3454
   gctctgttct ttctcttttt gcggcaggcc tcagtactta gctttagagt gagttgaata 3514
   agcacttcca gactagactg aacttacaaa acccatccat ccttcctttg cttagcacca 3574
   tttgccaagc acatcctgcc ctgggcagca gtgatgacca agacacagga gctcagccaa 3634
   ggggaggtag ttcaggctga ccaggtagat cctggcagtg actgggtctt tctggggagc 3694
   aacagtcact ctcatgtgcc tgacaggtaa ggcacttccg gagcacactt ttcagctgag 3754
   gccagaggca cagcttcccc ttgtttgagc atctcagcca tcacacactg ttggacactc 3814
   atgctttttg catctgcata aaggatgtac ccatgttttc ctaactccca tccctggatg 3874
   tcctgtgtga tttcagagta gtctgtttat tcccacagag ttgtcctgat tttatagttg 3934
   aggaagtgaa gggtgcttgg gaattgctaa ggtcatcatg gggtcctgag gctgtctgaa 3994
   cgcagcaggc aggcaggttt tcctgcatgg aattgtctgc aggttttgaa ctggttgtgc 4054
   cctggatacc atctactttc tgcccaggaa accacactga agaggggatg ctgcttgtgg 4114
   gagactcagc attcagtgaa atctgcaccc ttatggtgga cgttgcagct gatctccaca 4174
   acagttagtc tctcttcttc caaggagaac acatatggtt cctagtaaga gatctcaggc 4234
   catagctgga tgtgatcagt gcaaggatga tctcattccc cttgcagtga ctggttccag 4294
   aatggatgtg ggagtcagtt ctggaggttg agtggaaagt agggagaacc acaggtagtt 4354
   ggtccctctc ttgtgatggg aacagggaaa ccttgtggtg gctggcagcc atctgacacc 4414
   atgaggggag caagtcttag gccaccaccg ttgatggcag ggcagagagt gagtgagatc 4474
   caggacatag aggcctgatg tccaggccgc ctctgaactt ccagttacat gagctgttac 4534
   ttttcctttt cgttgtgcac tttcaagtct ccgttacttg cagcccatag caccccaact 4594
   cgtacagccc tgactgctta tcaagaaaac caggaggctg atctgtaacc cacagctaag 4654
   actcgagata cataaaaccc agagggattt ttgtttgctt tctgttttga gaaccctctt 4714
   tcatttcact caagtcaagg ttgccaattt ctggttttcc ctcctcagtc ctattgagga 4774
   ggtatttttg gtcagataag agagcctgag atctggcatc gggagatctg ggttcctggt 4834
   ggggtctgac cctgggtctg taatattggg caggtcacac ggtctctctg agcctcagtt 4894
   tattcttaat agatcagtaa gtgatgatgt ctattcttta ggggccagag ttagactagc 4954
   ccttagaact gtcatcagac tggctagcct aacttccacc ccaacctatg gggaaactga 5014
   ggctaagagg ggggacatta gtggtggcac agagggagat tagggagagt cagcctttga 5074
   cttttgaaaa tcaagatgtg gactgttcag attctgtgtt cctttcgctt ctgtttggaa 5134
   acgccactta cataagcttc ccttgggctt cacagcgagt gtgggctcca tttcagtgct 5194
   ggggtgccct ccatcaaatc acatcacttt ctgagcctca gtttccctgc actgcctggt 5254
   gccctgggtt gtcatgaggc tgtaaggagc caagggcatg tggactctga agttctacac 5314
   gtgtaaaagg cgactgctgt caatttcccc aaattgtagt gtaggccanc tcccacgccc 5374
   ctgctgtaag caatgtgctc ctttcttttt aatcaacccg aacttaaagc ttggcgcagt 5434
   cacagagcac ggtttttgtc attttctttc tggaagatga aaatcaaatt ctaataaaat 5494
   ttttccactc catctcctga ctgttgtcat ttgctacact ttttagtgtt tatccttaaa 5554
   gcttgcagag ctaagggatg tttggtaaga agtgtttagg ccttgagact ggaagagccg 5614
   ctgtatgaag cgctaggctc catggagagt gtgatgggga gtaagagaag gagagtgaac 5674
   tcctgtgagt ccaccaagca gagggacata ctctcagcaa ctggggtatt tccttccagt 5734
   ctttttttaa tgcccatgtc tgtttttaat gaaaactgta atctgtctgt atcaacaatt 5794
   ttgaaggcta cttttctagt ttggcatgag attataggaa ttttccaggg ctttgctcca 5854
   gggctggctt catggatatg caacctgtgt ggtcatctag gtccccacac tcagaaggac 5914
   ctgtgcttgg gttcatgttc tgctgttact atcttgaaat ttttaagaat ttcactttgg 5974
   atcctgtgtt ttgtaagcga aatctgatgg gacgggggag cctgggaatg agcagagggg 6034
   tacgtgcggc aggcgagtct gtggttacac acattggctc ccacagcacg ctaccctgtg 6094
   ttcacttgag cgtctgagcc ccacgcacag tggacagctc atgcaccttc tcagtgcgtc 6154
   tgtagtttca caggcagggg ccatgctttc aactgatgct ccaaggcatt gatgttctca 6214
   tgcagtgact tctaagaaac atgaatgacg caggaaccct atggtgtcct ttcttaacgt 6274
   gtgttacgtg cccatatttg ccaatcgctg acactgaaaa tgattacaca gaagggaagg 6334
   gaaaaagagg gcacctatag ttccttttcc tcctagtcct tccttgttta ttattgaacc 6394
   aagggtagag ggtgttggaa gaatgtacac atatccagaa atgaaataag aacagttaag 6454
   ttagtttcct ctgctctggt aagaacaaaa tccatatgcg aaatataact tgtgcatttt 6514
   ggtgagtcta cattccagtt acgtgctctt atgtttgcat ttaaaattgg aggtgcacac 6574
   tagaatggtg agtgataaaa tgcacgctga gagtttaagc tttttttttt ttaaatttga 6634
   attgacatta aatagcaaat aacaccatga caaaatatgg aaggcatgaa aaggctttgt 6694
   atcttagcac ctttaatgat gcttttcctt tgctttttga ataagaggct ctgcattttc 6754
   atttttccct gtgccccacg aattataggg ttggccctgc tttgctggaa ctctgggtag 6814
   ggtgaactcc ttggccctgc cttctacctc cttggctttc gtgattggtg gaaatggtga 6874
   gagcctgcca tccattgggc agctcttccc tatggggagg ggttacaata ttaaatgtcc 6934
   gtgctcctct ctcacccatt gggtccatgg gcctctgact tccaggttgc tctacagggg 6994
   agaggccaac agttatctta ttttacaagc tagaagttag acctacctct ccccgcagga 7054
   ccaaggggtg gggaggtgaa gtaggggtcc atagctcttt tttttcctac aggtttacca 7114
   ttaataaagc agattttttt attcctgtct tgcggccagt cttttctgtg taatcccaag 7174
   tcccagcaag gaagaggggt gactggttac cttggccccg cccccaagtc acacagtaca 7234
   cgtattgaca accatcattt cattggctgc atactattcc tgtgatacat gtgtcatcat 7294
   tcagtagttc aattaatcat ttccttatgg ctggatattt agttccctcc cttccctccc 7354
   tccctccctt cctcccttcc ttccttctct ctctctctgt ctctccccca cacactttct 7414
   tattttcata actaacactg caggaatgaa caccatttgt gtgtgtgaag accccctact 7474
   cccatcgttc ccccttaacc ttggaatgat gctcttcaga aggaattcca cacgtgctat 7534
   ttcatataaa gatcactagc atttttaatg ttgttgacac acattggcag attattctcc 7594
   agaaagtttt tctctttaac cacatgctga tcaacattag gtagtctaat ttgtttgacc 7654
   tttgcaaata tcaaagatga aaaatatttg ttgtaattca cgtctgcata ccaatgaggt 7714
   tgaacatttt ttccttttgt tcagcgggta ttggctgatc cctgttatgt gccagggtga 7774
   cattagattc tagacataca aagttgaaga gcaaggctgt gtctgccttg aaatgtggac 7834
   tctcctctcc cagtgtcttg ggtgccactg cgggacctag ctacttctca ggagagagtc 7894
   tgaagctgct ggaggagtct gtacatccct cagcaccctg gggagtcctg ttagttacac 7954
   agggccttgc aaagacaggc atctcaccat taagcccttg agcagaatga tccaagtgga 8014
   ctttaggatg aaataattac aaaacaaaca caatagccat tattatgatt caccatttat 8074
   tggctntgta ctataatact atatgctgga catgaggctg cgtgcatctc attggtgatg 8134
   ccccagacct gatgttgtgt atattatttc cccattttac agaggagaaa actgaggctg 8194
   aggtgcttgt tcaatgtcac atggtttgta agggacagag ctgagttttg aatgcagcct 8254
   gccaaaccca cgctcctgac tgctacatta attggctggg acccacaggg cagggttagg 8314
   tggcctgttc agttctgatt atgcaccaca cttaggaggt tttgggattt gaactagact 8374
   acctggcttg gtgctcttgc tatctgtgtt gcgatgaaag gcaatcgaga ggggtcagca 8434
   ggaggagaga cccccttcac ggcacaggta gatggcagtg gcagaggcca caagtggaca 8494
   cgtggtccag ggactgaagg gcaggtggca tttccaggtt ggggggtgat gatgtgctgg 8554
   gacatggtat tctagcgcat tctgggcagg aggaacggtg tgtgcaaagt ggcataacac 8614
   ttgtcccctg acagtgtccg gctcctctgc ttgagaccag gaggcagtta gttagttgcc 8674
   agccggcccg ggagcaggac acacctgggg gcccctgtgc tgcccaggac aggcactgcc 8734
   ctccttgcac agtgggggcc attgtcctcc agaacccagg gctgaatgtc ccattgaggc 8794
   agaacaaagg ctgctcagag gttcccagct ggggtgtggg ctgccccaag cagggatggg 8854
   atctccaact gcaggccaag ggcctcctcc aggctggcct ggcctcctgc agccccaccc 8914
   ccacccctcc tcaggaccag gactgttgct gctggaggct ggacctgggc ttgggctccc 8974
   aggcctgtgc tctgggtctg gcacagctgc cgtgcccttg gcagcttctt catcccctgg 9034
   gcaatttccc catctatgaa gcagagagag agcgagctcc caaccagcaa ggctttcagg 9094
   cagaattgaa tgaaatagtg cacactctgt aggttaatct caataaaagg gagctctttc 9154
   atgatcatga ttagcttctg aatgtttttt tacaatttca aaaaagtttt gatgcaaact 9214
   ttcaaatttg tgccacttct gggccaaagt gtttaagaag ggagtgcacc ttcccccctc 9274
   cctctgtccc agagaaggga gagatgccgc tccctggagc ccctcatcac tctgtggaag 9334
   ggactgctgg ccaactgtta caagaggaga aacttctttg catttgtgag aaaatagtct 9394
   attgaactgc ttccaatcta tcaagatctt gctgtacttc cttcatttac tctcccctgc 9454
   ttttggctga agaattttta ggcaaatcca agactcctgt cgtttccccg ttccatctgc 9514
   aggcatctct gagtgttgag ggcatttctt gtagcccagt gctgttatcc cacctcacaa 9574
   aacatatcct gattctttgt tacctaaatt ctggtccatt ttatgaacgt ccccagttgt 9634
   ctgagaaatg tctcttatgg ttgggtggtt tggcccagga tccaaagtcc tgcacctgcg 9694
   tttggctgtt ctgtctcttg tcttttctga cctagactca gcctccatcc ccttttcagg 9754
   ctaccaactt gttgaagaca tttgttatta ttttatttta ttttttttaa gacggagttt 9814
   cactctgtcg cccaggctgg agtgcaatgg cgtgatcttg gctcactgaa acccccgcct 9874
   cccgggttca agtgattctc ctgcctcagc atcctgagca gctgggacta catacaggtg 9934
   cccaccacca cgcccgacta atttttgtat ttttagtaga gacggggttt caccatgttg 9994
   gtcaggctgg tcttgaactc ctgacctcag ataatctgcc ttcattatta ttttagagat 10054
   tacagtgtca ctcccactgt ccctaatttg tgcctggatt ccatttgccc tgtgggtctg 10114
   gaaggctgag aggtggttgc tgggacctgg gcatcggcct tggggctgcc cctctctcct 10174
   ccaggacccc tttctgcaga gtggtgccct cgccactccc tggctgagtg atcttgggca 10234
   agttgtccag ccaggccgtg cctgggtgac cacatctgag ctgggggtga gcgtggctgc 10294
   ancatcctct ctgggatgtg gtgggtgttg aatgagatgg tgcatgccac gtgctccgtg 10354
   ggcctggtgc ctgtgggtcc ctgtcttacc cccatgatgg ggatgtggca ggaactgggg 10414
   tagccaccgc ctgcccacac agtgctcact ttctgtaggg gagacacccc tcagctggtc 10474
   actacataca gcaggaccag cactttctga gggaagaggg atgttctctt gggaagtctg 10534
   gatgctgaag acagtttgtt actctgatta ataccagtta caaagaaatc cccacattcc 10594
   aggggttgat gtcatagaag tttatccctt tgtaacagtt cattgtggat gatcccagtt 10654
   ggcccaggag tctcttccac agagtgatgc ggggctccag acccttctca tctgtcagct 10714
   cccatgtcct ctccattctc cggggaagag ggtatgggga aggtgcactc cctccttaaa 10774
   cactttggcc cagacatggc acttgtgact tccctcacat tccattggcc agagctagtc 10834
   acatggcccc acctaatgca aggggctcta ggaaatgtag tccctggctg ggcagcccag 10894
   tggctactct gcagtgggaa gaacctgcat cttggtggat gtcttgccat ctttgccaca 10954
   tgaccccaca aaacaaacct ttacattctc agtccaaaaa accctactaa gaatcctgtg 11014
   ctggagacac cctcactcaa cccctgaccc tcccctctcc ctgcttcagt gtccacacgt 11074
   gcacggtgct gtgagatgca gagtccagag tcatgcggtg gctaggaggt cagggacgcc 11134
   ttcctgggag aggcgatgtc tgagctgagt ctgcaaagcc aaataggtgg tgcccaggtg 11194
   gatcaggtag gagagggatt ccgggcttcg gctgcagcag gggtaaaggc tggtgtcttg 11254
   ggagagggca tcctgtgtag agaggggtct gtgggccact gagatttaga ggatgtgtgt 11314
   ggggtggggt ggagtgggag aggagctgga gcgggatggg aagtgggagg caggactgtt 11374
   tgtgaaaggc ttcaaatgcc gagataagga gtttggattg tatcctattg acattgtgga 11434
   accagatgga gatggggcat ttccctttgt ttgaaagtat tttgatttct attggctgtc 11494
   ttactacaaa aaacatatgt agtcatagca aaaagttcag aaaatttaga aagagaaaag 11554
   gaggaaaaga aaatcctacc actgaaaata ttttggtata tgtgtttttg cctatgggta 11614
   tacatactat ctaggtatat atatattcct acattttttt attcactgaa agatggtttt 11674
   tgagcatcta ctgtgtgcgt atcctatttt gtaatcttta aaattttctc ttaatgatat 11734
   gggagctttc tagcttagaa aataacacag cccattttct tagcttgctg ggacttctat 11794
   aacacagtgc cacataccgg gcagttgaaa caacagagac ttaccgcctc acagtgctag 11854
   agcttgggag tccaagatca aggtgttggc agggttgatt ccttctgagg cccctctcct 11914
   tggcttatag atgaacatct ccctactgtg tcttcacatg gtctttcctg tgtgtgtctg 11974
   tgtccttaga gggacaccag gcatattgag ttagggctca tccatatgac tgcattttac 12034
   cttcgtcacc ttgttaaagg tcctttttgt caaacacagt cacattttga ggttctaggg 12094
   gttaggactt caacaaagga atcttgggga gggggcacat ttcagcccaa agcacccgtc 12154
   atcattggta atatcagtca catgcttctt ctatgtattt taaagagttc catgctggtg 12214
   ggtatcagaa gttatggttg gtttaggatc atgagcagtg ttgtgagggc atctttgcac 12274
   ctttttgcgt gcatatttgg ttatttccct agggtgtgga atggctgctt ctgagagttt 12334
   ggtctttctg aagtatcttc atctaatgtc aacttttcct gatgaccaaa cttggtacta 12394
   acaactccct ttccccagag cattgctagt ggcgactaga tccaacatat ttaatatttg 12454
   ccaatattat ggtaaaaatt gctgtttttt tttgtgtgtg tgacagaggc ttgctctgtt 12514
   gctcaggctg gagtgcagtg tcgtgatctt ggctcactgc aacctctgcc tcccaggctc 12574
   aagcaattct tgtctctcag tctcccaaga agctggcatt ataggctgtg ttcttttaaa 12634
   gcatttttca gtgagattga acttccttta ttatatttat tgaccatggc atatattcaa 12694
   ttgtaagttt ttttgtgcac cgttcttggc ctgtttctct actgtaattg tcatctctct 12754
   tgcaaacact gacccttggt ttgttgtttc tggtagcatt gattttatgt ggttagatgt 12814
   catgactctt gtcctgctga gcatggatct cccattccac aatcatgaga agaaccaaca 12874
   atatggagga ggtgacaccg tgtagctttg gaggctaagt aaaaaatagg tcatgtgctt 12934
   ctgccttgga gccagccacc atattgtgag gaagcccaag cagtttgtgg agaggcacat 12994
   gcagagagga accaaggtcc tggttgagct gcctgcctaa tatccagcac caccttgcca 13054
   gcaggtgagt gagccatctt acaggtagag cctccagccc tcaggcaagc catccatatg 13114
   gaacagagat gagccattcc caccaagatc tacccaaact gcagattcat gaaccaaata 13174
   aatggtttct gcttgaagcc attaagtttt gaagtgcttt gttacacagc agtgggtaac 13234
   tggaagagtc atggattcct gacattgaat tcctggtcct cctccttctc aggctacttg 13294
   tctagatgtt ctgttctctc catgattctg tggatccctc agagccttcc ggtaacttcc 13354
   ttctttgctt gtgttagcct gggtcaatct ctgttgctta taactgacag acatgggaaa 13414
   ccagccccag caatgagagg tgacccagct cagatcatga gacaggacag gaatccaggc 13474
   ctttctgaaa catagcccag ggtcccatcc cacaacgtgt cagtagacac catgcctgct 13534
   gggtcatgcc tgcttccgct gcaccctgca cccagctcag cacctgctat cttccaaagg 13594
   ccattgctga ttgcttgtac acacctgtta gttcatgcac agacagcaaa gcacgtagtt 13654
   gtgctgcctc cttgccttcc tgctatgatc tgaatgttta agtaccccct ccaaattcac 13714
   aggttgaaat ccagaccccc aatgtgaggg tatttaaaag gtggggactt tgggagtgat 13774
   gaggttgtga gggtggagcc ctcatgggtg ggattagtgc ccttataaaa gaccttagag 13834
   agctcccttg cccttctgtc acgtgaggac gcagcgagaa ggcactgttt gtgagtcagg 13894
   aagttggacc tcagtggaca ccaaatctgc tgtgccttga tcttggactt ccagcctcca 13954
   gaattgtgag gaataaatgc ttgttgttta taagccaccc ggtctatgat attttgttat 14014
   agcagcctga acagactaag ccactcccag tgatgagcct gcatgatgtt ttacacaaac 14074
   agatcactga aagaaggaat tggccagcaa agatgatgct cagcagagat gtgaaagatg 14134
   ttaatgctgg aagtgaaatt taaattggag gtaaatggag tcatagaaga aatccatgat 14194
   cttgggaagc tgaagctacc cttcaagaag ctcttatatg cagccagagg agttgagtga 14254
   aggtgaacac actgatgtaa accaggaaag gagttgtgcc ccaaagcatg gagatgtccc 14314
   agaggaagcg aggctgggaa aaacgttaaa ggaactcttg aagatatttc acagtgttga 14374
   aagtgcaaag gataaaatct tggaagctgg tctggagaaa gataattctg caaagcatag 14434
   aaagggtgct tttttggtat cgtaaggtat acaataacag tagcgagcac tgtgcaaact 14494
   ctctccatat gtcttttaca aagaaataaa gcanttgaca tctcaatgtt tctaatgctt 14554
   taaattacat tgtaccaaat aaatattagt tgtactattt taaaaaaact ttcccggttg 14614
   ggcattgtgg ctcacacctg taatccaagc actttgggag gctgaggtgg gaggatcgct 14674
   tgagcccagg agttcgagac cagcctgggc aatatagtga gaccctgtct cttcaaaaaa 14734
   taaaaaaaaa ttagccaagg atggtggcat gtacctgcag tcccagctac tcaggaggct 14794
   gaggctggag gatcacttga acccaggagg ttgaggctgc agtgagctat gattgcacca 14854
   ctgtactcca gcctgggtga cagagccaga ccctgtctca aaaagaaaaa aaaattccct 14914
   gtgcattccc tatggacatt tgtaactgtc cataaaagac tttttaatgt cttgacaaaa 14974
   aattttaaag gccacagaag aattgtaatt tcctcattga ttattaggat ggctttaaat 15034
   ggttttagct ttcatgctct attttttttt tttttttttt gagatggagt ctcgctctgt 15094
   cgcccaggtc ggggtacagt ggtgtcatgt cagctcactg caacttctgt ctcctggttc 15154
   aagcaattct cctgcctcag cctcctgagt agctgggatt acaggtgcct gccacggcat 15214
   ctggctaatt tttgtatttt tagtagagac ggaatttcaa ccatgttggt caagctgatc 15274
   ttgaactcct gacctcaggt gatctgcccg ccttagcctc ccaaagtgct ggaattatag 15334
   gngcaagcca ccgcacctgg ctcatgctca tttttatgga tccacaccac ccgtacagca 15394
   aggactgcct gcactcattc caagtggtca gagtggtcac cgcatgggcc ctccacgtgg 15454
   ccnggccaca gtgatgtttc aaaccctggc tgggggattg cattcaatat ccccttatta 15514
   aaggcggcag ctcaagaata ttaaatcatg ggaattcctc actgtggaag tgggaaggca 15574
   gcccgtggtt caccgtgagg ggcacccaga gctccccctc cactgcgttc agtgtgcagc 15634
   cctccagcca gcctgtctgc ttcgggagca atccatcatg gaatgaactg accgaaggag 15694
   cgaggggctg aatgatgtgg ttcctacgcc gactttcaat gtgaaaggtg ataaaaacag 15754
   ccctgaatat tttatggccc caaggagagg taaggctctt tattgaagct gtgaaaatat 15814
   aatccatcat gataatgtgt cccatatcgt cagactctgc gagctaagtt gtgtgtatgt 15874
   taaggtgctc tttttgagaa gatcttaatt ttatttctct attttatttc attacagaaa 15934
   cgtttgaacg tgtagacagt agaataaagg ggggggggga aataacatcc ttcatttcac 15994
   tactccagaa acactgctca cattttgttg catttccttc taactacccc ctgcagattg 16054
   tactatgttt tgtgtctttt aaaatattaa atgcactgtc aacattttcc cagatcatcc 16114
   tgcactctga gttaacaatt tttattggct gcataatact tcacgatgta ggtattatta 16174
   ttcattcaat aactatttat ggattcattg atccgattag tgttgattgt ctataacagg 16234
   tgtgtgggtg gggtaggggg attcaggaag aaggaatagg gcgcagctcc cccnccccag 16294
   gatttctgga annagggaga cntaacagat ggctgtgatc cggtgtgagg ggcagggatg 16354
   cagtgggccc ggttgcagcc tgggagcagg tggtcagaga tgnaaggctg tgggcagcag 16414
   tggttggtgg tccacagcag taggcagcag ggggaggggg cgacattcat ggcaggaggg 16474
   acattatggg cacagcacgg cgtggtgtgt tcagggttgg tggagcgttc acttacagtc 16534
   ttgcagatcc tggcaggtgt ggtggtgatc tgtccaggca ggggagggag agggaactga 16594
   gagtcatcaa aagtctctgg gagtttggaa aggagagtag ggggctcaga gggagtgtga 16654
   gcacttccag cagaggtgag aaagccccca gtcagttgcc cagggtgggc agtggaaggg 16714
   aagtggaggt gaacgttgtg gggtggagag ggttttcagg caggctggga gctgcccagt 16774
   gtgctggagg aaggctgggt ctccttgaat ggtgtttggt caatgcaaga ccacaggagt 16834
   gtgactagaa ggctgggggt gcagatggtg gcaggtggag gatggagaga gctgctccca 16894
   ctgctgaaca acgactgccc caactttatg ggatgagcat tcttatgaat gcccattgtc 16954
   ctgtattcca gattattctt gtgtctgtcc aggtagggat gcaatttctt gatgtaaggc 17014
   tataggtgtt tttaagggct tgcataaaga tttgaatatg atgttgtcta gtagagtaaa 17074
   aatcaaattg ggcaaaacat ttttgtttgg gtgatttttg gaagagtaag tccacgaatg 17134
   caacgcagct ctggagtcat ctgtagatta cagcaagccc atcagtctct atgtctcttg 17194
   cttacaacaa aggattgatt tcagctccag cactaggtga cttgtgctgt gttcattatc 17254
   tcttgatagg tgtctgacag gagatggggc ttgggctgtg ccagggagga gccgtgtggt 17314
   gcaccaccta tctccgcagg cataactatt ttgtcttcat ggcaaaataa tagcgatgat 17374
   ggtgatgagg agggaagcta ccatttcttg actgctcctg tgtaatgaca tgttggtgat 17434
   cacattaggg ctttatgtcc actctgggag gtggtgagaa tgacatcgcg tttgcacatc 17494
   aggacgctga gcctcagaga ggttgagtcc caggacgaag gccacacagt gagtgccaca 17554
   ggtaccatta ccacctaaca aatgactctg gagctcagtg atgtgatgag aaccatttta 17614
   ttctgtctcc tggattctgt gagtcaggaa tttgggcaga gcttggctgg gcaaaccttc 17674
   tgctctaaat ggccacccat gagtcttcca agctggtggg tggactgatc tggagggact 17734
   gagatggtct cactcacaca ttttctgcgg ggtggggaga gttggaaggc ggggctcccc 17794
   tccccagcgt gcagtctcag ggcagttgga ctcctttgat ggcagctggc tttcccgaat 17854
   caagtatccc cagataccca ggtggaagct gcttggccct tcctgacccc tgggacatct 17914
   cagtgtcaat tgtgtcatct tcatttagtt caagcaagtc acaggccggc ccaggttgaa 17974
   gggcagttgg actcaacctt tgcatgtggg aagggccagg ttacatggta gaaaagcaga 18034
   tgggatagga gaccgtgctc tggccctccc tgggaaacac ggtgtgccac agacgccctg 18094
   ggcagagcca agacccccac tgggctctgt ctgaccttgg agccactgcc ccgctcctga 18154
   gcaacaccct cttgtccccc tgaacagtca caggaagaac gggtccctct ctccatgcca 18214
   ttttcctgtt aaaaaatgca aaaacatccc atacttttgc tcatttaaac acagaggaaa 18274
   ggaggtgagt gaaagctttc tttaggggta gattagatgt gaggcagacc ggtggccctg 18334
   ggtgtgcacc gggtggaaat tattcttaca aacagggccg ggtgggggtg cagcctgcca 18394
   ccgcccctct ggccgtctgc ctccacagga ggcttgcagg tgcccacatc agccaacgtg 18454
   gccctcggtg gggctgtgct tgccttcttg ccagggccac tgcagtaggg aggagtgcag 18514
   agcagaaaca ggtgagctgg gctgaatttt ctgcttggct aattcagtgt ggcttgactc 18574
   caagaaggac acaccgacct ccccatcatc ttgtttgttc agccttgcag aagcagtttt 18634
   atgagaaacc attacagccc cggtggtctg ggcccagacc cggtgcacac cacgtgcccg 18694
   cactggtgcg gggggaccat tctcggtgaa tatgatggat gcacaggaag ccgccctgcc 18754
   attcagtgag agctcaccat gtgtctgccc ctggggtggg gtggggtggg cagtttccag 18814
   cctttgccca tgggatagag ctgctggaag tctccccgag ctgaggaggc agagctgggg 18874
   tggctggggc tgggggttgc catggatact tcctgcaagt cctgacgccg ctccttcctc 18934
   tttggggatc tgtctcccat actgtccctg ctgcctttac atcttcaggg tggagaggga 18994
   ctctggccat cctggggcca accatcctgt gtcactgctg gtgtttgtga cctgcggtgg 19054
   gccctccctc aactccgtat ctccagctct aagccagaga caagaatatc ctctgtgggg 19114
   gggtcccttc aagggtggat ggagatgagg cgttaggtgt atccgatgct caggaacggc 19174
   cccgcacctg ctcatcttta tgatgagcag tgggactgcg ggcagaggga gccacacgca 19234
   tccatcctgg ctctcagcat cccagggaaa gatgttctgc tctatcctga tcagcctcgc 19294
   cctttaacca accacaggct gcctgcagtg tggccgtggg gagctggagt caggcatggt 19354
   ggcagccctt ctagacagta ggcagtaggt aagcctgctg atcacggagc cgagattctc 19414
   tgtgggacag agctggtccc cagcatccct gtggcctttg ggccaagaac tcagtcggct 19474
   actttgcttc caggcttggg aacactcagg gtaggctggg agtcccctgg tctcaccctg 19534
   tgagccccac ataagcctgt ggatagcacc agctcagcag gtgaccccct catcaaaacc 19594
   ccaaactggg atgcttctct ggctacatag gcatggccac atggggacag tgggaggaca 19654
   tgtgataatt tggggcagcg gctgaaagcc taggggttag ggctactgtg tccttctaag 19714
   gtggtgcagg gcgcacagcc ctctgggcct cagtttcctc ttctgtgaaa tggggactct 19774
   atcttggggc cgcaaatgcc agtgtcttct ctggaagaaa gggcgactgc tgagggaagc 19834
   agcacacagg tgtgagggtc caggccccag acgggatccc acaaagacct aggacagtga 19894
   gccaagagtg gagagagggg acgagggtgg actggggtgg gccccaggag ctggaaagtg 19954
   aggaaaatcc agctgtgtcc tgagggttag actccactgc cagtgttcac aggatctgga 20014
   gctgatgggg acctgcggtg tcaccctgaa gggacagatg gcccccaggc tagcaggagg 20074
   tggcagtgtc cgtttggcag caacatttga caagcagaag gcagttggtc cctcctgctt 20134
   cctgtccagg ctcttggggc tgggacccca ctcccagccc tgtcctcccc aacctcccca 20194
   cacttacaca ggccactctg gggcagagga ggggtgctgt gatttgtggg tttgggagaa 20254
   gttggaagca taatgggtca ggcctgcagc tcggtccaca ctgcctgtgc caggtggagc 20314
   aggtgagggc atccctggct ctggggtggt gtcactgttc acactttgtc ctatagccag 20374
   gcccttcttg ggggtgaggg ttccgtggag ccctccatct gcctggctct gccgatccaa 20434
   ctcttttctc tctcttgggg gtttcaaact tagacaggaa taggggtgtc atttattggg 20494
   ccccagacaa cctgaccagg tccctcagag cactgaggcc gggaggagga gggtggaagg 20554
   agatgggaag agtttccttt gtcctctctc cctggccatc cccaaacctc cacacaaacc 20614
   tggggtggct gagcattcat tatgctttgt ctttgtaaat aggcagctat aaaaacctat 20674
   cagcttgcag caccttctcc ataacacagg ctggatggat ttataaccca ggtcccctcc 20734
   ccgagagaag ctggcaaagc agaccccagc ccgcgctggc tgccatcacc ctccctgccc 20794
   ctgccccacc tcatgcaaga aacagaaggg aaagcacatt gagttgtaat atgttttcga 20854
   tggaatttgt cacaataaga aactggattt tgttggggct catgggatgt ttaggaaaga 20914
   gccagagagt ggtgcaagct gtgggccctg ccgagaagcc tgggctacag gagggcaggg 20974
   gctggagtgt tggcagggtc gcacagtggc tcatctggac agtccacagc ggatccagcc 21034
   cacactgtgt caggcacttt gctgggactg ggggatgtgg ctgtgggtac gattgacaag 21094
   gtctgtgtcc tgaggagccc gcagagcaga tgagatggac atgtggtcag tgatggtacc 21154
   gtgtcgggtg gaagagacaa taggctgagc tgcccagagc atcgcctgac cagcttgggt 21214
   ggtggcacgt ccaggagggc ttcctggaag aagtgaattt attcaacaca tgttcactag 21274
   agccagtgat gcttaggcac tgagagtgtt gccagggata caggagagaa tgggagagtc 21334
   cctgagtcat tccagactgt ggggctgaag tgtccgccga tggaggtgtg ggaagggcac 21394
   agcggcttcc cacgtgagca aggagctatg caatgtggca ggtggcaggg ccaggcggag 21454
   gtgcctccac ctgtctccag accccacccc ctacccaggt atggaattgt tgtctccagt 21514
   tggcagagaa ggaaactgaa atgggggttt cacctctcag gaatgggtag gccaggattt 21574
   taacccaggc ctgcagacac caaatctatc cctcgctcag gcctgcactg acctccgtgc 21634
   acctctgggg ctccaggcag ctgcctgggt gggtgctgtg tctggggtct ctcctggcgt 21694
   tccttaggcc cctcccctat caccgtcctt cattattcac ttggatgcct tgatggtcgg 21754
   ggctggaacc ccccgagctg acccaccatg cggctcatct tccttctcct tccagtgctt 21814
   ggtgatcttg agagtgaggc tgaaccgttg cttgattttt ctgtgaccca gatgaagagc 21874
   tgggtaacca tttgctcaat aaagtgagag accccatgtt ctggttaaag tggaggcact 21934
   gaggaccagc gaggggaagg cagtacttgt atttgtcagc ctggaggaga cgccagatac 21994
   cagccagagc accccagcct gtatctcgac caccacctgc agttggtgct gaacccccca 22054
   ctccacccca tagatgagac aattgaagcc cagagaggcc aggcttcttg ccgagggctg 22114
   cacagccggc agggatgctg gaattgggat ttggccccag ccttgtctga ctccaaagcc 22174
   aatgctattt ccaccatacc cagtgtctcc cagagctaat tttgcggctg gaactgcaac 22234
   ccgcaaagct atctaggaca ggcaactcga tgaaagagaa ttaggaggga atcctagaaa 22294
   aatggggctc ggcagctccc ggggaagcct ggagaggagg tggcgccgaa gcctctgcca 22354
   gcagattggg gtggggctgt tttcagtcct ctctggcgag gtgttttgaa gcctcctctg 22414
   ggaaccgtgt gcctctgtcc aggactggct gtctctctgg aaatcatacc ctggcagcat 22474
   ttggctttgg gtgaaaggag aagagaagat tctggccatt cagagcaggc ccttgtgcgg 22534
   gatggaaccc attttccaga actcttggga cagggaccag ggtggcaggc aggggcccgt 22594
   ggactgcctg ggggacctgg tgcttgggga cttagagatt tgttttcctg ctgaatatat 22654
   tgctttctcg tgcctgcttt gtgcaaccac gtgaggatgt gggggtgagg atggccgaca 22714
   ggacacggga gtccctcccg acaggggcca ggcggcggcg ggggtccgca tgtctcacgt 22774
   cagcatggct ctgtgttttc actcctctcc agcacatatt tagtggaaat gaactcattt 22834
   tattattaaa aattaaagtc atgcattcat agggtaaaca agattgagag catgtggagg 22894
   tgcactgtga aagtgcagtt ctctcggaat gggcacttag agacgcgcct gttctctgca 22954
   gctgccgcag gggtctcatc ttgttgggac agaacacggt tgattcatgc aattggctgt 23014
   tgatcaacat caggttgtgt ctagttttgt ttttttcccg tttcgcacgg tgctgcgaat 23074
   tcacagctgt gccagtgtat ctgaaggtaa atcccacgag tgggccttgg agagtcagag 23134
   gatggggcct tctacgtgga cttggtgtgg ttgggtgtgt gatgcctgca tggggctatg 23194
   tgtttttagc ccttccttct gacaggttct ggaggcctcc tctgtgcctg ccagccatgc 23254
   agccgctgag ccgagcatca cccaaggctt gcctgaacct ggcctgggtc ccaaaggaac 23314
   actgctctgg ggcatggagg ttggctggtt gagaactaaa gccacatcag caggggcact 23374
   gcccccacct gctggggtca gcccccgccc ggagttcagc aggacctccg tgagccttcg 23434
   tgcaggtggc tcattgcagc acgtcccctt ggggtggtgg ccattggctt gtggttcctt 23494
   tgctcactgc ggggaggagg acagccaggc acaggtgaaa ggggcttgcg ggtgacgatt 23554
   ctagtccttg gcccggggaa tgtccctggg cttctgaggc ctcacctccc tgggtagtca 23614
   ggagggttac gagggtgggg cctggccctg gggactccag ggtgtggcag cggtgggagt 23674
   gaggaaacag ccctgagacg gagggagaga agggcgatcc agatggcggt ggcctcctca 23734
   cccctcggcc agtgatgcat ggtagtggtt ttgacgggct gacctcgagg gtctgcctgg 23794
   gagccgcttg gaactctctg gaggtggggc cggccctggt gggggcagga aggtcccaga 23854
   gcagcttgtt aagtgggctg aggacaagtg tcaggagacc tgggtctgga tcccgctcca 23914
   atacccctct ccgtgtgacc tccaagggat ccacctgcct tggcctctca aagtgctggg 23974
   attacaggtg tgagccactg tgcccagcct gtgcccagtg tacccatcag taaagcaggg 24034
   atcaaacagt tcttaaatcc tgcagcggtg gtgagagcca cctgaggaaa cgatgcaaag 24094
   ggctttgacg gagtctggca cagagaacgc acccaataaa tgactgccgt gacgatcttt 24154
   cttctcgccc tcaggtggtc tctggaagct cctctgtgcg gggttttctc atttgccagc 24214
   tgtgcatccc ccggtcgtag tgcggctccc acgggggtgt accaggagcc tctgctcctc 24274
   ctatgcttcc tgaaaaaggg cccagagaat atttccatca ggaataactg agtgaatccc 24334
   agaaacttcc tatcacattt agggtgatta ggcagatgca tacgattctc actgtgggaa 24394
   aggagctggc gacctcgatg ggttgtggtt cccgcaggga tgtgcttgcg ctgctgttac 24454
   tccagccgta gctgaggcac gggagaaaac ggagacccca agaagttcag aggcttgtgc 24514
   aggtcacgct gttcctacga ggtagaccct gactttgacc ccaggctgtg tccctgccaa 24574
   gcttggagcc tctttctcaa aagggcgacg gaaggatgtt gttacagatg tggttgccag 24634
   tttcctcctt ttcattaaat caccagggaa atggtctctt gcaacccccc taaagcaggg 24694
   ggaaggagga ggacaaaggt caggtcacca tctttgctgg catgtgagtg gggtgggggt 24754
   gggggtgggg agctaggaga cctggcgctg ggcccttgaa atatccacat ttccacaaca 24814
   ttctgggtgt cagtgagccc ctgccttcct ccctcacatt tatccggagc tcttcctccg 24874
   cagggaagaa caacagcccg agatggggtt atttcaaggg gatttccatg gaaacgggag 24934
   ggtgggaggt tcctcccagc acttgtataa tgggagttgg ctgaggtggc agcgtgtccc 24994
   cacggaaggg tgcgagggac cttctctgca ccgcaggcct cctcagagtg ggaggcaccg 25054
   acccgagagt ggctggctcc cctttcatgc tcccaccctc tctacccagc tcaagacccc 25114
   ggggctcctt ggtgtgagtg agagccaggc cagctcccca gggaccccca aggcctggtc 25174
   cttcccatgg tctcttttct ctagcaggtc tttgtcttgg gctgctgcca gccacagctt 25234
   cctggcagga cctcctggca ggacctctgt gctttgagcc gctgttgctc tgccaagacc 25294
   ttgccccgca ccgtggtctg aatcagccca gcaccccttc gcctctgttg cagtgctcac 25354
   atttatccct cactcctcca tccagcatgt tttgtttttt ttttttttac aagcagacac 25414
   tttgctttat aaaagaattc tgctgtgagc tgccgtatcc tctctgagcc tcccttttgt 25474
   catctgctga atggtaacag cagcgcctgc catgcctgct tggtgaggat tccatcaagc 25534
   agggagacag tgggccgttg gcggggagtc tgagcaggtg taccagtatt tccagtcagc 25594
   tgatggctga tggacatgtt cttggaggca gggaactcgg aggcctgcag acgtgcccca 25654
   ggatgacaag attcatcagt tcctacaagc cctgcctggg cctcatgctt ttcagtgtgt 25714
   cctgggcttt cccgtgtgaa atcttacctg atttttatgc caccttgaga agagtgatat 25774
   tcatctccgt tgtacagatg aggaaactga ggctcaggga ggcaacgtga tctttgcaag 25834
   gatccgtctg ttccccccgt ggcctggctg cccctcctgg cagtgcaggt ggagttaaaa 25894
   ccatacagga gttaaaatga gcctcgatgg gggtgggaag ctacgactgg aaaacgtccg 25954
   atgctctccc aagtcaaatt gtgcttggtg tctgtgggtg tgtcggtgtg ggggagggaa 26014
   gctcagccct tttgaaaagt ggggggtggt ttgacgacgc tgcaggggca gctccgagtt 26074
   ctagagtctc agaacgtggt tctaggcggt tcatgtggat caagtgctgt tctgagcact 26134
   ttaatatcca gcgtgaccat aaggataagt gccactgtta ctggcttttt cacagatgag 26194
   gaaactgagg cacagaggga ttaggtaaag tgactggagt cactcagcca ggatgtgaat 26254
   cacagcccac acccatgtgc accaggaagc cttggctttc agggtccttg gagggtgtgc 26314
   cgggcagtcg cctaagctgg gaaaccttgg gcttgtctcc aggccatgtg gccatgtgag 26374
   ataggagtcc tcctgtgtta tgttctgcga cactgtgggc agagggctga ggaccccagc 26434
   cctcccttag aacatcatgt tggtgtgaga catttagagc caggcctccc tgcttagaaa 26494
   gcacctcttg ggtcgcttgc attagtgaag ttatacattt gaaactccat ttatttattt 26554
   atttatttag agacagggtc tctccttgtc acccagacta gagtaccgtg gtgccatcat 26614
   agctcactgc agcctcaaac tcctagactc aagtgatccc cctgccgggg cctcccagag 26674
   tgctggcatt acaggcatgg gccacagcac ctggctgaaa ctcccttttc atgaaaagaa 26734
   acagcttcaa ctttgcaatc tcatctgtct gtctatgagg ctgtgccttt gtgtgagatg 26794
   agagcagtca ctgtcacttg ctctttgaat atttgattaa caggtaaaca gcctgaaatc 26854
   catttgacat cttatccttt tgcaaacttg gctaaattct cttaaattgg ttccagttgg 26914
   attaattaaa tgcatggttg cttatacatg tgtgtggaaa tgattctggc aggtcatgtc 26974
   ttagctagat agtgaacata agcgtctaga atattctcag ctgttgcaga gactgccagg 27034
   aatgaccttg aaaaagtttg ggagagggtt tttttttttg tttttatttt gcttttgttg 27094
   agacagggtc tcactctgtc acctaggctg gagtgcagtg atgtgatctc agctcactgt 27154
   aacctctgcc tcccaggctc aagcggtcct cctgcctcag cctcccgagt agctgggact 27214
   acaggcaggc accaacatac ccggctaatt tttgtatttt ttttttgtag agttggggtc 27274
   tcaccatgtt gcccagactg atctggaacc cctaggctga agggatccac ctgccttggc 27334
   ctctcaaagt gctgggatta taggcgtgag ccactgtgcc caggctggag aggtttttgg 27394
   atgcactggg ccatggatgt gaaggtgaac acatggaaac gatccctgcc acctgcttgt 27454
   gtgtccagtg gacatgtctc tgatctatcc agattgttac actgtcaaag tgaaaactgc 27514
   tgagagtaga gccatctgcc tggccaggca tcgcttggaa gcgtgaagac actttgcctt 27574
   tttgtctcat gattctctct ccatgtgcag cttcgttggc ttaaaagaaa ttaagaaact 27634
   gggcccccgc ttaggacctg ctgaagtgca gagttactgt ctttgaagtg gtggggtagg 27694
   gaaaaatagg aaataagggg tctgatcatt ttgagaaacc tcagggagat ttacacctgg 27754
   gctgtgcgag gaccccggag agtggcagag tgtatttgga atttccagta gtcctcattc 27814
   ctcccttaat atccagggga tctggggcct cagtcttctt atctgttaaa tgggacaagt 27874
   aacgactagg ctttggggtt gtcaggaaga ctgaataaga aaatgggtat gaaaacagtg 27934
   gtcacggtgc ctggccctcc atccctgtct ccaccaggcc tacctgtctg gcccaggcct 27994
   ccctgatctc cgcgggagca gacctcctgt aatggtgtca aaggaccctt gttctattta 28054
   tccatctgat ttccattttc ggggccactg cctctagcca tgttaggcac atggtgagtg 28114
   tctgtcccat caatccttgt cgattctgtg gtcctgggtg ggccatagcg tttctaacct 28174
   gtccactctc tcctaatcag gcatttggac ctgtttgggt tcccaaactc tgtcacgggc 28234
   agagggctgc aggaggctac tcacgggcca gggttgtttg gacctggttg ggtttccaag 28294
   ctctctcgca gtcagggggc tgcaagaggc tacacatggg ccagggttgg gctgctgggc 28354
   tgctgggctg ctgctgtgtt ggagctgcct agcacttgct tcgttgctgc acctgagagg 28414
   ctgtgtgggc tgagacagcc agaaaagatg caccgggagc catctgtttg cagcccttgg 28474
   accagatgct ctgcaaggac tccggggggg cggtggggtg gggagggaat acatttgctg 28534
   agcacccagc atctttcaga gcctcagcac agccctacaa gctgggcatt gccatcatgt 28594
   ttatacggac caggaacacg aggctcagag tgactgagtc actggtgaca gtcacccagc 28654
   caggaagtgg cataggtggg gcttgaaccc agggcttcct gagtcccagg ctagtgttct 28714
   ttgcctcagg ctgctgaggt tccagctgaa tgttgcggca gagtgacttc tgagaagtac 28774
   cacggaaggg ggtgactcac gccggggtgg tctggcttct ctgcccagtg cctgaggaca 28834
   cccaggtccc tctgcggcct tggggcttta cccagcgtct gcatggcatc ccgcagcacc 28894
   ctgcctctgg agcgcaccct gtgtgtatcc taaagtgcgc tttgcctaga aaacctttct 28954
   aatgaaactg gtggaagatg gagaagccaa attcagtttt cagagatgac actaatccta 29014
   ttaaggttga tggggccaga gcatgtgtgg aattagtcct gccaggcggc ggccgggcac 29074
   ctgcctggaa ggctggaggg gatcctaaca agagtggtgc cgaggagaga gagggaaggg 29134
   gcctcatctc tcccagaggt ttaaaaaaac tgaggccact gtagagcttg gttctcccag 29194
   gttcctgggg tggaaaacag ggcttcccac agcagacgga aatgggaggt gggcagtagt 29254
   caccgagcag ggaggctgtg aagtagtcat ttaggtcggg aaagtcacgc aagatctcca 29314
   agcttcagtc tccttagctg tgatgggggg aatgggtgcc ccccttgggc gacttgtggg 29374
   acccagaact catcacgcag agcgtcccac tgctgtgcag ccactagctg ctcggtgctg 29434
   gggctgtggt atgagcatgg actctggagt cagaattcct gccttcaaag ccccactcct 29494
   caccttgcta gctgtgtggc ctggggtgag ttgcgtaacc tctccatgcc tcagttcctt 29554
   catctatgaa atggggagac tgaaactgta ccccacaccc taggggtgtt ggaacttaag 29614
   tgagttaatt catataaaac acggagtgag tgcctggtac gcaggaagtg ctcagttcct 29674
   cttggtgcct gtgattattc ccataatcat cactggtgtc accttgtcgc ctctcccagc 29734
   ccttggggcc atgctatttg tggtaggaaa tggggcctga aaccatcaaa ctaaactgga 29794
   ctgaatgatc agtccaccga ccaccacagt cacggcttgg tggccccacg gactgaagca 29854
   cgtggccaag ggcagttttc ccttctctgt gtgggttcca ctgggtccaa gtacattgtt 29914
   cctaagccca ggcctctggc cactcaccca cctcctgctg agagcgggca gagctgatgc 29974
   ccctctctgg gcagaatcag cccacggctg ggaggggagg ccaggcctgc tgctgggggt 30034
   gcagatagtg gggagctgca ggccagccac tggaaacctg gcctgtgtgc tgagacagca 30094
   cattggacac agtctggtgg ccttcccata gatcaggcca cagagtcctc atcctgggtc 30154
   cacccaaggc actggccatt tccagatcaa agagcaggtg gattccaggg tgagacggtc 30214
   ctctctgctg gctgccttcc cccacccacg gacaccgttt ggctttgatg gggctgtgtc 30274
   ctaggcttga cccaggtggt caggagcctt ccactatgca gtgggatatg ctgcaggagt 30334
   agggcggaga aggaagggaa tggccggtgg tgaacagctg ccatgcccca ggcatttaat 30394
   tttgaccgca gtgctggggg gccataccca gtaatacaga tgaaggactg agactcaagg 30454
   gtcagtgctt gctcagagac ccacagtgaa gagtggggcc atcgtgttgc atgtgtgtgg 30514
   ctgggcccca tcccctcctt tctacagcac cacccctcta ttccttatca agttcacctt 30574
   taaggctcct ttgcccctca ctggggcagt catgagggga gcagaggcct cggatctggg 30634
   catggatgag ggtagagccc tggctgtgtc ctgcaggtga cacgtacaga gcagaaggtg 30694
   atgctggaac catccgccag ggaagggctg tccaggaaga ggtcacagcc tgaacagagg 30754
   cgaggtggag agagagtgct cgtgtgtggg gactggtggc agctcaggag ggcaagactg 30814
   tgacttgcag gctgggaacc agggaggctg caggtgatgg aggccccatg ggtctcatgg 30874
   ggcgggctga ggagcgtggc tcctcccctg cagctggagg ggccaggaag ggctttaagc 30934
   catggagtga gggaccagag ttgggtttac aagggtcact gtggtgtcgg tgtggagggg 30994
   cttgaaggga ggagtttgga gggaggggct gtagcaatgt tgagattccc taatcacttt 31054
   agtgttttca tgcatgaggg ccccgaggtg ctttttccca gagcctgcac tgaggttccc 31114
   tgttggcggc ttccctcagg ctgctggaac ccccttttcc tgtgcacctg cagagctgga 31174
   ggttactgga aactcatgtc ctctgccaga gtcagccctc acccagtcac tgacaggtgc 31234
   agtggtataa ataccccagc tccctccctc ctgatcagga cactttgaga tgggacctac 31294
   actgtcccca gagctcccat gggactgagc tcaagtcgca ctccttgaga tttttcctgt 31354
   tatcacaccc cacttggcct ccttcatgtc ctggccccac ttccctacac ccttcctggc 31414
   tttcccagca acacttccta atcacttcca gaaaaatgtt ggcctcaggg tctgcttctg 31474
   gagaacccag cctagcagag ggcagcaaag gtgttgaggc actaggagaa acatggtgag 31534
   atacagactc aggcggcgtc catcagctat gccaggaggc tctgagacgt gccagtcagg 31594
   gagggaggtg ctgaggccac agcaaactgg gggccagaag taagcaccac agacagcacc 31654
   aaggctgcca gggtccatca tcttgggtct cccaattggt tgggtccaga gaccagcctg 31714
   ctggggttta cagagccagc atgttacatc tctctgtgtc cccactgctg aaagcctctg 31774
   gtcagtgcca tggtgctgag ggagcttggg cctcccttag aggttgctaa gagcccccca 31834
   cacctgccct gtgagttgtt ggccccaggg gactctgagt ttccctgttt ctggttttcc 31894
   tgctcatggg attgggagtc tgacctgagc ttgctgagac agataactga tcattcagat 31954
   acaaaactct aaaggttaga actcttttct gataacttaa atagaaaatg aattcatgca 32014
   acatgtactc atctgtccat ctgcgcatgc atccaacatg tatgtatctc agttgatggt 32074
   tgtttccatg ccaggccttg gggatctaga gatggcttgg tccctatggt catacccatg 32134
   tgaagggaga tagaggtgga cagaaagaca accataatgt cattttgaca aattctggga 32194
   aaagatgggc cacccagcct gtgagacctg tatgtagtga tggagggatg gggaacggtg 32254
   gaggtgtcag ggaagctccc caggggagct gttccttgag gtggttttaa gagcttagca 32314
   agagttttct acgcagtgaa gtgaagacgt agggcaggtg gagtggaggg ttggcaccta 32374
   aaggtcctga aaaggaatgg tgttgcaaga ggcagcaaat agcatggggt gggctaagct 32434
   ccagctggtt gagtttgggt cagaagtgtg gacttggttg gtaaggactc ctgagagtgt 32494
   catgtccagg cacttggact tgatgctgaa aatacagcca ttcaacagag aagtatgtat 32554
   gtctgagtgt gatgtggtta gatctttagt ttaggaagat ctctctgttc atggtgggga 32614
   gagtggatga gatgggggtg gactggaggc agaagagcag aggagaagag cagactttct 32674
   aggagacgaa gcgggagggg ctgctcccag ggcctggctg tgagtggtac tgaccagagc 32734
   catccatcag cccagatcat gtctccctaa tttagaagaa aatgagagga aagagccttt 32794
   tcatctctgc ctcttctggg atggaaatga gctattttga atcacgctaa agttagattt 32854
   ctagttttcc ttttaatccg tgtctcattt agagcattag aaactggaaa agcacccccg 32914
   tatgaatgta gtgtcataaa tcagtgcacg tcacaaactg cagaaggagt catttctaca 32974
   agagtgtgca gccttgcatt tatgataaat ctaatgcttt atctttttgg gcttcagact 33034
   agagtaatta gtcatttctg ctgatactgg caaaccattg catgtgcagt gttaatggag 33094
   agcaaatgta tttgaactgc ttttgcaatt gcttgtgagc attgctgtgt atctcttcta 33154
   cctgtttcta gaggggaaac atcctatata tttcattctt gcctattcta ggaataggtg 33214
   tggtaatagt atcaggtaac acttactggg tttcttccac atctctggca ctgttcagag 33274
   attcaataag aaaggatcct ttgtccaaat ggaagctgcc gtgtgtgtgt tgaggtcctt 33334
   gggtcaatct gatcatgctc tgtctcctgc tctgtgcccg cactgtgtca gggaccacct 33394
   ggataccact catgtattat tcacctaata ggtttcttta aatgttggcc ctctaccata 33454
   aatggaaaac cagcaaatca ggccaaaaat agaagtaacc aaagacaagc acaggtgttt 33514
   ctgctctaat gaaatagaga catcctgaga aacttctcat tctgcaaaat cccaaacaaa 33574
   tgacatcaga gctgatggac agaatggggt tgggacagat gactggaagt ctatgcagtg 33634
   tgtcaccaga cacccataaa aatattaagt tttagaaaaa tctggcacaa tgaaagaaaa 33694
   acatattaat tcctgttaaa gaaacattcc aaccattata caatcttatc ttgaaggaaa 33754
   aagtgaaggt catttaatgg aaggggctga aagggaggga gctgaattat ggctgggaca 33814
   tggcctgggt acagagggag acccgtccac caaactcttc caaactgagc taagaggtag 33874
   gtgcgcaggg aatgagtcta ttctcttggg ccacattcct ttgaggcttg atgttcagcc 33934
   attagtgtat tttgcattca gttgcttcct ctggctggca caaaacatta acacactgag 33994
   aaaggctgcg tttgaaccaa cctaactttt acattatact gacatcattc ccctatgtac 34054
   aaattgcata cgacctactt tgcaaaggca atggtcatag taactgtacg atggtgagtg 34114
   gggttgttaa atgcagacca gatactgctg taatagggct gtgactcagg actatcattc 34174
   cagttgcctg atatgaaaaa ggacatgaag gagaaggttt tatgagaact ctggcaaaga 34234
   cccatgaaac cactcttctt tgccctccag aaaacctggt aaaactgaaa gtaagccagc 34294
   agccccacgg gtttgggatt gatccagaag actgaaggaa taaaaacaag ctaatatatt 34354
   tctatcccct cccttctaag gcctgtagtt ggttccatta tgatgagaca gcagccgatg 34414
   cacgtaaaaa cagaggccat gcatctacac aggggtgttg aggattgaat gagattaggt 34474
   ctgaaagaac acacagtgga gacagtgttg tcattgtggg gacaaagatt tcttttcaca 34534
   atttgagtga cacttgtcat ttctataggc agttaagaga caataactgc atactgttgg 34594
   ccgctgtacc tttcctgtgc actgagtccc tgagcatatt cgtgagtctt taaaagtaca 34654
   ttctagtcaa tgtagaaaac agatagtaag ggacatgact ctacctagat taagctcagt 34714
   tttgaagcag ggcctagagg atttagaagt gacagagtcc tggattaggt gacatttgat 34774
   aagagctcta tccctaactt cctcttaatt ctggacaagc cactccccgc tctggctgta 34834
   gttcccccat ctttaaaatg aaacagttga atcagacaac tacaaaagga cttgccaggc 34894
   cttctgtctc agatcctgtg ttgttgaagg gatgtctgag gccagagatg cagtttactt 34954
   ctgagcaatt acagttgtat ttcttgtgtt cagcctagcc ctttaccaag ctttgttgaa 35014
   gaaatggagc aagatccttg tcctttgctt cggtggtttt taaaacttgg atgcctaaag 35074
   aaaccaggaa ggtcatatca gtgagcaaag tacaaaatgt ataagcaaga cacagtgcac 35134
   catgacgctg agagtggcaa agtaacagtc caaccatgca tcagggagtg gtggggactg 35194
   tggcgtgctg gcaagcatag ccttgtggga ttctgcattg gacagctctt acaatttctg 35254
   gagagaaatc agaaattcag gtctacatgt tgaatctttt aatattgaaa tgttcacttg 35314
   gcaatgtgta aacatcattt gggtcaaagc ccatctgtga accaagttag tcaaagacaa 35374
   cttgggcatt tggacctcag ctttatagtc tatataaggg ttggtaaact atggcccaca 35434
   tgctaatctg acccacctcc tgtttttgtg aataaagttt aaatgaagat gcagtcacac 35494
   ctatgtgttt aggtattgcc tgtggctact ttcacactac aacagcaggg ttgagtagtt 35554
   gtgacggtag ttgagacagt gtggccctgt atttattctc tggaccttta tggaaaaagg 35614
   ttgctgaccc ttggttcatg tcaatgactc atatggagca gcaggaaaca ttctgaattt 35674
   ggagctgaat gaccagggtt tgacgcctgg ctcagggaac tctagtaaca gagtagagag 35734
   tgtttctctt ttctcctttt ggaaatgatc ctgaagcaac aaatggaaca aatggaaatg 35794
   caaatgccat ctttgatgga accatagctt ctggaacctc atatgtgggg aagtgcagta 35854
   aaggtcaaaa atgggtgcag gatgtctcca aagggaaata aaatgcccat ggctgtaaag 35914
   ctcagacaga gcaaacaaag cagaattttc tacaccttct gtggtgggct cagatggcaa 35974
   gacctatagt tcctctcatg gagcaataag aacaagtgca ttggctggtg tcttagtctg 36034
   ttcatgctgc tatagcagaa taccatagat tgggtggctt gtaagcagca tacttttatc 36094
   tctcacagtt ctggaggctg ggaagtctga tacccaggtg taacagattt ggtgtctgct 36154
   gaggacctgt tttcttgttc atagatggtg ccttcttgct gtgtgcttgc gtggcagaag 36214
   gggctaggga gctctctgag atctctttta taagggtact aatcccattc atgagcactc 36274
   caccctctaa gacctaatca gttctgaaag acgttacttc ctaagaccat cacattgggg 36334
   gctaagattt ctatatatga atttcggggg gacacacatt cagaccataa caactggtgt 36394
   gggagccttc ccagatctag ttttgcacaa agtgaaatca gggaagtctg gggtgaacaa 36454
   ggtctcacat ggacaagcta tattttcaaa aagcagtcta ctagagaggc aaagtggaga 36514
   aaaggggctg tgttatgtgc cagcctgtgg catctgatct taacaagaaa gaagtaaagg 36574
   ccaaaagaac tgactctcac tcacaagcct gttttctaag aactagatta ctcttttgga 36634
   cactctggtg cacgaatctt gtataaatag atggttcagg aagaactcat cacattcaga 36694
   gatgagtaat aattagtaag gaccagtgca agctctatct caagatcctc caaaaatgaa 36754
   gaaagtaaca gatgaaaaat atgcgcaaga aaacatttgc cataaatcag atgaaaatgt 36814
   caacagtaca taatgccttg gactaaggaa aaaaataatt gagcaatcac acctctaaac 36874
   aagagtataa agttgagata caagaactca ggagaggtgg taacatgaca gaagagagtg 36934
   aaacctgata tgatgagctc cacaaagtag atagattaac aaataaaccc atctaaaaag 36994
   tgaagtatgc aatggaatga ttatgacagg gaacacagaa aacacagtaa gagatatgga 37054
   agatgagatt gggaaaagca agataaatta atgtaaagag agagagagaa atattagaac 37114
   atgatacaga ggacagataa ggggatccaa catacacata attggtgtcc ctggagataa 37174
   gaatgaaaat aattaaataa aaaaaattta aggcacaatt ccagaacttt ctaaaaataa 37234
   gatttgaatt tatggactga aaggaagtac agtgagtggc tgagaaaata ttgatatggt 37294
   gaggtcaaca gaaagacata gagaagtact aatttcctca taaatcaaga acccccaaaa 37354
   aaattcaata gaagaatgga taaaggatgt gatgaggaga tggtttacat aaaaggaaat 37414
   agaaatatct tttaaacaca taaaaactca acctcactca taataagaaa actccaagtt 37474
   aagatactgt tttttatcta tcgtataggc aaggactaaa atagctgata ttaccatgat 37534
   ggtgcaggta tgagggaaac aagaactctc agaaacatgg ctatggattt cacttctaat 37594
   ccttgtctga tggaagtaca caagtaagag gccaggtgtg gtggctcatg cctgtaatcc 37654
   cagcactttg ggaggccgag attggcagat cacttgaggt gaggagtttg acaccagact 37714
   ggccaacatc acgaaacccc atctctacta aaactacaaa aattagccag gcgtggtggt 37774
   gcatgcctgt aattttaggt acttgggagg ctgaggcacg agaatcgctt gaacccagga 37834
   ggtggaagtt gcagtgagcc gagatcacgc cactgcactc tagcctgggt gacagtgtga 37894
   gactctgtct caaaaaaaaa aaaaaaaaaa agaaaagaaa aaagaaatac gcaggtgaga 37954
   aatgacattt atccacattt gtcatagcat tgttggtaat gggaaagaat ggaaatatct 38014
   gtaatggcca tcacagggga ctgtttacat aaattattcc agagcgtgat agtggatagc 38074
   aatgaaaaga atgtgacagc tcagggtgcg ctgatgtaga atgatgttta agattcttta 38134
   ggtgaaatga gcaaggtgca aaatagtgga tgatccatga catgctttat gattaaaaca 38194
   tggtatctta tgactgcttg tgtgtagact ccttctggta ggatgatgca cctgaaagtg 38254
   ctcatagtgg ttgccactag acaggagagc tgagtgagtg ggagaagggc ctaagggaga 38314
   cttattttca ctgttagctt ttttgtgccc tttgcatttt atactcattt cttaaccaaa 38374
   ggctgcacag cttaggttgt gaccataagc tctagagtca gctgttcgag ttcgaaccct 38434
   gactgggctg cctactcgct gtgtgacctt ggacaagtta cttcacctct gtgggtctca 38494
   gagttgtcag atggatgtaa caatggtgcc tatttcataa gttgttgagg atgaactgag 38554
   tcaattcaag ggaaagaatg aggacagaac ctggcacaaa aaaatacagt caaattagct 38614
   attatagtga ctgcatgtat agttgaaccc agggtctgga tttggaaccc gtatgcttat 38674
   tggcccaatg ggggggatga cttggccaca gaacagccac agccaggcag taaagggcac 38734
   agggaggggg ccttagcctg gggcttcagg gaagctgccc aggggaagtg atgctgctgg 38794
   gtcttacaga atgatgggaa aacttggcgg caccgtttca ggcgacgcta cttagatctg 38854
   caaaactgtt gatgtcttac aataccaagt gtagggcggg gctggagaaa tggaaactca 38914
   tgccaccact ggaaatggat atgaaagcaa accatttgca aagtcatttg ccaatatcca 38974
   gatatgtcca agcagcccct ctgttcccct tctaggtaaa tagcagggaa gccttcagct 39034
   gtgcacagag agccattcag caacagagca gaccctcgaa caacacgggg ttgggggagc 39094
   caaccccctg cacagtcaaa aacccacata caacttttga ctccccaaaa acttaactaa 39154
   tagcctcctc ttgattggac ttccaaaaac atgaacagtt gataaacata cattttgtat 39214
   gttatatgta ttatatactg tattctaata aagtaagcta gaggaaagaa aacatcatta 39274
   agaaaatcat aaggaagagg aaatatattt tctgttcatt aagtggaagg ggatcactat 39334
   aatctttatc ctcgttgtct tcatgttgag taggctgagg agaaggaaga ggacgggttg 39394
   gtcttgcagt ctcaggggta gcagaggcga aagaaaatca tgtataagtg accctcacag 39454
   ctcagctcat gtttgagggt caactgtgtt ctaaaatcca agaggctctg aaagcctaaa 39514
   gattttttcc taacttattt ggcagtgaaa cccaacctaa attgatgtga aaccatttat 39574
   agcccttctc tatccctgta gtgtgaagtt tcatatgtta tgtacagaaa tagaaatgcg 39634
   tatgcgtgtt gtcccagaag cccttggggg tgacagatgt cctaggtgac ctgtgcacca 39694
   tattactgta cagtccaaat cctctatcct gaaatactgg gcccaccctt tgggatttgg 39754
   gagatgacag tgaagctggt gtgtgttcag ggaattccag gttgttgaag gtgctggagt 39814
   agaggggaga gggggaaacg aggcagcagg cgctgggtcc cgacggtcct tggacaccta 39874
   ctgaggatgt gggtacaaca cttctttcat ccctggggct cagagatgtc tgctgaatca 39934
   gtcgaggggc cttcagtcca ctgtgagccc cagaaggcag agctgtggcc tcctccgact 39994
   tgtttctcag aaaggtcact tgggcagcag gtgaacatgg ctcaggagag gcaagggagg 40054
   gtggggacag gtaggaagca gaagccactt actgggcaaa gaccatagcc atgaggatgg 40114
   ggagggactg agtagccaga gaggcgctca tcaaagccta ctgctgtcgt ctgaaggtct 40174
   ggtttctccc aaattcgtat attgaaatta agccttaaga tgatgtgtta agaggtgggg 40234
   cctttgggag gtggtcaggt catgaagatg gagccctcat ttatgggatc agtggcttta 40294
   taaagggacc ccacagtgag aagatggcca tctatgaact atgaagaggc cctccccaga 40354
   cactgaagct tctacacctc gatcttggac ttcccagcct ctagaactgt gagatgtaaa 40414
   tggctgttgt tataagcccc ccgagtctgt agtattctgt gggagcagcc ccaaatgact 40474
   aagtccacgg cccatcctcc acccactgag gccataccac tgggaagggt atcagaggca 40534
   gggttggggt cttagaggtc cagggtctct tgtccaggct ccccagaggc aggccccgag 40594
   gtgtggacta gagtataagt ggtttatcaa ggaagtgtac ccaggggagg tccagataca 40654
   agtaggaggg gctcaagcac agaggagcca gggaaggggg ggctctggac cgaaaccatg 40714
   tcccctccac ggctttggac ttagcccctt ggggacacag acgtggctgg ggatgggtca 40774
   caaagtaacc agggtgactg ccagcctgaa ctctgggctc tccagacttc tggggaccgt 40834
   agtctcagcc tgagcagctt tctccaagga gggtcctgga aacctggggc ctgcccctga 40894
   ttttgtcctg ggagcagggc cctccatggg tgttctggag cccctcatat tagcttttga 40954
   ggttgattgc ttaattttca gtttggagag ccagcagaca tcttgttggt agtttgaaat 41014
   tggccagggc aggggtattt ataccatgga agctggcagg ggctgccttc cagaacctct 41074
   ctccctccag gtgcagggaa acacggacct gcacaccctg gtggctttca caccttcccc 41134
   tcctcaggcg gctcgtcccc ttgctcagtt atactgatcc ttgggcttgc aggcagaacc 41194
   atctctgtca tccagaatct ccctagtaac cccaacccca aaagtgggct gccggaagag 41254
   gcagatggca gtgtcccagg gctatggaag ccactgataa acatcttcca acaaacagga 41314
   tcagggatct gcaaagtttt ttttttttgt tgttgttgtt tttttgagac agagtcttgc 41374
   tctgtcgccc aggctggagt gcagtggtgc gatcttggct cactgcaacc tctgcctcct 41434
   gggttcaagt gattctcctg cctcagcctc acaagtagct gggattacag gtgcctacca 41494
   ccacaactgg ctaatttttg tgtttttagt ggaggcggtg tttcaccatg ttggccaggc 41554
   tggtctggaa ctcctgacct catgtgatcc acccgccttg gccccccaaa gtgctaggat 41614
   tacaggtgtg agccaccgtg cccggctgga tctgcaaagc tgttgaaagg aggagtggat 41674
   aggattacaa agttggcctg attctgctaa atgaacattt caacatgttt cccctcgtgc 41734
   accgaggagg catgcactct cctctgaggg tctgaggctt tcttcacgga gactttgtaa 41794
   accacttctc cccagcactc tggagtgggg gagtcacgta gggaagtcat ttctctcctc 41854
   ttggacctct cagtcccctt tggcttctcg ctgataccag gcagtcaggt ggatcagaga 41914
   agacatggac tctggagctg gatgtctggt gcaggcagtg tggcttcagg caacttgctt 41974
   tgcctctctg ggcctcagct tcctcctgga aaatcatgat gctcatacct agggtttcat 42034
   gggggtcagg caggacagtg caggtgcagg tatgagcacc atcccatcaa ggacatagtc 42094
   ctggctgctt tcagtgatct tggtgctggg gaaaatgggg ctgattctca tggaagagga 42154
   aatggattca tgtgcccact tcttaaatgg aaattcaagg ccttgatcgt ggcccactta 42214
   tgaggtgggg actagtgagg cttcaggtgg tgtcctaaga ttagaaaact ggagcattca 42274
   gttgggagtc agacctctgc tgatgtgctg tgtgacgttg ggcaaggtgt gtgacttctc 42334
   tgggccttgt gttcagtaga agcaggtcat ctttcaatag acctccttcc cagaccctct 42394
   cggatgctgt gggcatccag ctggccccca tgccacacct ggcttctcgg ggactggcct 42454
   gcattctcac acccgttctt tctggcaacg atgggcattt gtttctcccc tgccagcaaa 42514
   ctcctttgga ccaagaattg atgccatggt gttagcatac aataaagctt caaggagctg 42574
   gtgaaacact ctgagacggt cataaaattg gcttttcatc cctgtcggag ctttcttatt 42634
   acagacaaca agcagtgtca gggagcagca cggtagatgc tgcttaaccc agcagcaggc 42694
   gagactgagg gggtctcgtg ctgtttgtca ccaccgcctc tctgagatca tccccaaccc 42754
   atactgttga cattcactgg gatacctggc tacctgtttt gggtttcctt acaatttttt 42814
   catttgttta atagttttta tttttcaaag tcttaaaaaa cacaagtcag tgaaagtttt 42874
   ggtctttgtt actgtcttcc taccaggtac gagggcctga aagtcactgt taattgatta 42934
   ttgattatct gattagaacc gtacatttac tttttaagga aatgtgaaaa ataagcctag 42994
   aaatgaaatc aaaagtgcgg tgttgaagtt agaaatggca gcgtcacggt caggaattaa 43054
   agctgctgct tctctgtcac atggtatacc acagggggtg gtagctacag cccatgggca 43114
   aaacttggcc cagtgcctgt ctttgtaaat aaagtttgat tggaacagag tcatgcccat 43174
   gtatctacac attgtctgtg gatgcctttc tgctataatg gcagagttgc agagttgcca 43234
   aagaggacat atgtcccacg aagcctaaaa tattggctat ctggcccttt gcagaaagaa 43294
   tttgctgacc cttggtatag aggaaagtta gtggcacaag tttagatcgc ctatagtgaa 43354
   agaccttgca ggaccaggag tcaggcagac ctgggttcga aacccgctct gccacttcct 43414
   ggttgtgact tcaagctgct tgccttttcc tctcagggtt ctgtttctgt ctctgtcaaa 43474
   tgcagccaaa tactgcccag tttgcacagc tgtttttgga gacagagaca ctcagcaagg 43534
   ggtccagccc ctggtgggtg gtatcccaat gtcactgttg gcatttgcag tgtcagtgat 43594
   gtctggctta ttcatcaggc cctcttgagt ttgccccaaa cgagtcaacc ctccagggct 43654
   gtgaaacttc tgctgccttg atacgatgtg atttccgaac cagaaaaatg gaggaggccc 43714
   tggcccctaa tgcttaagtc acaggtgact ctgtgcaatt ggtttcttcc ctgtctcctg 43774
   tccctctaaa aagaaccacc tgagctcccg gactgtgcta tatccaggcc taaaggctgg 43834
   tggaccagga cacagtcacc agcaatcagc tttcatcgac ttggctggac cttggaaact 43894
   taaggcttgc cctgccctgg cttcttgcgg tgagcaggtc ttatggagtt gctctaacca 43954
   ctgtccatcc aggccggggt tagaggatcc aaacacagcc atgttgatgg gaacctggag 44014
   gcaaatagag ggcatgagac cctcctctcc tgaaaccttt ctggccttct tattggatac 44074
   ccaccaaaca gtcttgatct taaaactttg tgcaaagcaa taggatgaac ttgtaggaca 44134
   tgcataaaga ggaaggtatg gattgtgaca tgaggctctt agggatgctc ctcctacagt 44194
   gctgagtgac cctcctcaga ggcacaggtg tgggcacagc ttgttctttc aaatcctggc 44254
   accccagctg ttctgctgtc tagctcgttc cctcattgac aaaacaggaa taacagtgcc 44314
   cacctccttg ggctgttagg ggatcaactg agatgctaag tcatccagga gggggagcct 44374
   ctttcccaga gtcctgcctg tgcccggtgg ctgtgcagac ctgatacccg cagccatcta 44434
   cctctacacc tcatgtgtgt tagagaacac cactctgatt tcagcctctc agactggggg 44494
   ttcagtcact ggctgctaaa agcttcctaa agccaaggcc tctccttgcc tccaggggct 44554
   ccgtgtctgt tagatgtgta aacagatcca tcaccgagca tactgatgag tcctggccta 44614
   gggacaagca tggccacaaa tgaggtgtct ggaaagggct cacaggggaa ggagccactg 44674
   aggagggcaa gaagaaggct tggccaggaa gatggggtag ggctcattcc tggcaggtcc 44734
   agcctatgtg aagcccagag acccagtgag gggcttgccc atcctttggc cttgctggag 44794
   ccaagggtgt gaaatgggag ctgagagtag gatgttagta gcagtggctt cctgggaagg 44854
   gcccccacat gggagcctct ggcctggttg caaatggcag gaccaaggag gtggttgttg 44914
   agttagttgg aaggcactgt cctgccagga tttcccacgt cctcgtttga gcattgagat 44974
   gctgaacgtt aaactgtcct tcattctcca gctcagttct tttattcatc caacaaacac 45034
   agagtagcta ctgtgtacca gatactattc taggcactgg gcatacagtg gggcccccag 45094
   ctcgtcctgg cctcccagcc cagtgggtgt ggagcagtga gcaggaggac ctcgagtgtc 45154
   acacttgctc cccttggtac acattggcac atggcgttgc caggtcagat attctcttct 45214
   tggggttcac cagcatcccc cttggatgtg ccctccgtgg tatgtggccg cttccatctc 45274
   agtctcaggc cgatggaaga tattttcaaa attaacttag cttttggaat tggttcctcc 45334
   ccatgttggt gctcaaagac tccccagtga gacagctgcc tccttgagct ctgtgtgcaa 45394
   aacttgggga gcaagacttg cttgagccag tgtttggcct ggcccaagct gttgggcctg 45454
   cagagcctca cctgcccctg tggctcaggg cttggtgtct gaagtggatg gaggcagctg 45514
   aactggtttc tccagggccc catccagcct gcgtttccca ggctgctgtc ccagctctgc 45574
   tccacccaag tctactctgc cgtcccctga tactgacact gagtgtgttt ctgaacactg 45634
   cagggggtga ggaataaggt gggcatattg tagcttcagg aggtgatgct tgtgtctgaa 45694
   atataaggac cacaattgcc atgcaggtgt aaatatctcc agtgattaca catttccctg 45754
   caccgaccga gtggctgcac ccagtctggg gctctgtctc tcctggtcag tctgttcttc 45814
   ctgaagccag gaacacagga gggcaagttt ctctccatcc ctagtcccac cctggagcca 45874
   gcacacagta ggctgcagga agtgctcgtg gaacaaacac aggcagcaat ggaagaatcc 45934
   ccaccactgg gattttcagc agctgatcgt agaagggagc tggactgcaa ggccgtctcc 45994
   gaccgcctcc ctctcactgc ccagctggct agggtgatga gtatggacga ggatggaggc 46054
   aggctgtggc tgctgtctca tccaagtagc cttttcctga caccccaagg ctgggcagag 46114
   ggactgagtt ggtcccaggg cggagcctgc tgggaaggaa ggctgttagc cgctgccttg 46174
   caggtgccat agcaatgcca ggcagcccac ctgctccttg gattagatgg gggatcagag 46234
   ccgtgaggga agcaggcctg tacccaggaa caggctcatc ccgcccctgc cctggcccgg 46294
   tactgatgga cctggagcca tagagtgcct ctccccgcaa cacacatgca catgcatgca 46354
   cgtgtgcatg cacacacata caccacatac acaccacaca cacacacccc acacatacac 46414
   acataacaca cacaccacac acacacacac acacacacag ccaagcatcg attctgggcc 46474
   ctgttctgta tgcacaccaa ttttcctgaa cagactctcc ttgtcttcta gcacccggga 46534
   ggattcagaa gtgtcctggc tgccctgtgg tgatggggat ggcagggacc tgcatttttg 46594
   tgagttcctg cacgtgatga aggggaaatt gcaactgccc aggcagcctg tgcaaaaagg 46654
   cagtgggttt caagtgtggt cataaaccaa aagcctcagg ttcacacgtc atgggggagg 46714
   ctgaggggaa cacacagcct ctgagctgtg ctctcgggct gattctgagc tcgtgctggt 46774
   ggggacaccc ccagacttcc agcagcactg gatgtccctg gatcctcaca tgactcatga 46834
   ggaggggctg gcatgttcct gaacttcagg tgttgactct gcagtgaggc gatttggatc 46894
   agagagactg tgcaggtgcc caggacacat agcaggtgag gtgcacagcc aggcttggct 46954
   agcaattggg ctcttaatga tgctcacact ggacgtgtca gagtgcctgc tcctgggcgt 47014
   gcacagcctt gtgggagggg ccgacaggtg gacagggaat tatagggtgg ggggacaggg 47074
   agctgtgctg gctgttgtgg ggacaccaca gggaaggccc cctgactctc atgttccagc 47134
   attgccactt cctgtcatcc tctcggcaac cctatatgaa tgggaatagg agtaagtgct 47194
   ctgtaaagac tcctctggct gccaggcacg tgctaagctc tttgcccaca ccactcaggc 47254
   attcttcatg atggccctaa aagtagagac tcttgtgtcc ccattttaca gagtaggaaa 47314
   gtgaacccta aagaggctgc ctgctgttct gcggccacgc agctgggcag tggcggcatc 47374
   gggatttgaa cccagaaact ctggctgtac ggtcttagcc ttggctcact ctcacctcca 47434
   agaaatagac tctgagcagt gaggtgacgt gtcccaggtc tcatgggggc tgagggatct 47494
   gggaagggtg atttgccccc acccctgcat ctcccacacg gtcccatccc tgaacccttg 47554
   cacacacggc ccctctgcct ccatgccctt cccctcactt tgtcaaaccc cagcagctgt 47614
   tcagcaggca caccacagcc gcctctggag gaggcattgg ctggtctcct gtgtcaatgt 47674
   atcgacactg cctggcgagg tctggctctc cggcctccct gtgtatccac aggtctcccc 47734
   agctctgcaa tccctttagg gcaggctcag gtcagtcatg ctatggtcac ccctcagtgc 47794
   cccgacatgg ctcagcagtg tccggtggct tgaatggagg accacaaact gtcctctctc 47854
   tgcagagggg ccccaggtca tatctgctga cttagcaccg cctataagat ttaaagacca 47914
   ctctgttttc acctggagga accgtcagct cacgcaggat ggcaggtggt tttccagggc 47974
   tccacagtga ggtctccagt tcatcttttg atgagggtga tgatgggaca gtggccctga 48034
   aagcacatgg ctctagagtg cttaggagcc tggtgacctg ctgctgtgac ttcctctcca 48094
   gagctggagc tccaggggct gaggttcccc ttccctgggc agccccttcc tcctaatccg 48154
   gctttccctg tgaggaagag cctcctggcc ttaagccaca tccttagcag cttctgccgt 48214
   ggcccttcct ccagccgggg accctggcag aatgcatggg caggaggagg tgccttggcc 48274
   ccaggacagc caggtgggca gtgaatggac gaaatcagtc tgtgcaggag ggaaacacta 48334
   acactaaccc taaccctaac cctaacccta acacattctc aaaacatttg attctaactt 48394
   aaaacacaca agggcactcc tttccatggc cctactacat gatctggcct gacctgcctt 48454
   tctggcctct gctcatacca ctacctccct gatctttcag ttcctcaaac acaccaagct 48514
   ggttcctacc ctctagctcc ctcccactga ctcctcctca ccctgagatc ctcctcagct 48574
   tatcaaaaaa agattccccc attttctcac ttcaacccct tgttcatttc ctccaaagta 48634
   ctgtattaca gttaatgatt attttttggg tctgtttacc tgtttgggct tcgcctcccc 48694
   agtggtatgt aagatcttta agggcagggt gcatgtgtgt ttcttgcttt ccaaaatatc 48754
   cctagtgggg gccgggtgtg gtggctaatg cctataatcc cagcactttg ggaggctgag 48814
   gtgggtggat cacttgaggt caggggttcg agaccagcct ggccaatgtg gtgaaacctc 48874
   gtctctacta aaaacacaaa aattagctgg gcgtggtggt gtgcacctgt agtcccagct 48934
   acttgggagg ctgaggcagg agaactgctt gaatccggga ggcagaggtt gcagtgagcc 48994
   gagattgcac cactgcactc cagcctgggt gacagagtga gactgtcttg aaacaaacaa 49054
   acaaaaaact aaaacaagaa aacccccaaa atatctccag tgggtagcac agagcctgac 49114
   acacagtagg tctgcatgaa tatttgttga aggaatgaaa cagtgcctgg atgagtgtat 49174
   tgcagcccct tccaggtagg gcacagccat cggggagcca gcgtgacaga tgtgagtgct 49234
   gtggccctgg gatgcgatga caaccccttc tcactggggt gtaccctgga ttggggtggt 49294
   gcatgtagaa tatatggcat gctgctggca cttggcaggt gctctgtcaa ggttagttgt 49354
   tgttacttaa aaactctaat caagtaagca tgacatatac tgaggtccta ctgtgttact 49414
   gttatgggct gaattgtgtc tgctccaaaa ctcacatgtt gaagtcctaa cccccagcac 49474
   ctcagaatgt gactgtgttt tgagacaggg ttttcacaga ggtacctaag gttaaactgg 49534
   atcattaagg tgggccctga tccaatatga ctgtgtcctt ataagaagag gagattagga 49594
   cagagacaca cacagaagga caactgtgtg aagacacagg gagaagacag ccatctgcaa 49654
   gccaggagag aggcttcaga agaaaccatc ctgctgacac cttgatcttg gacttccagc 49714
   tccagaactg tgaggaaata gatttctttt gtttaagtgg cacagtctgg ggcactttgt 49774
   tatggtggca ctagaaatga atacagttac caaatttaat ttttcaagct ctctctagca 49834
   gcaaagtatt tcccccatat aaaaaaatga ggacgctgag gctcagagag gtgaactcgt 49894
   ttgccggagc cacagggctt gctggcgggc gttggcactg atagttgaac cctggtccac 49954
   tagtgtgcct ccagagatgg tgcaggccac gccgtctgcc ttggtctcac gcaagtggcg 50014
   gctaagccca gaggcacttg tctccatttc agtgccctgc tattggctgg ttagaaagtc 50074
   aatattttat ggagcctcat caacctccca agtcttgggt ctcaattccc ccttttggtg 50134
   tgagcctctg aagactggtt gagcatttgt gagaaacaaa gttttgacta ctcaagactc 50194
   tagttaaaaa aaaaaaaaaa ttgcaccaat gtgccatccg gctgcagcgt ctcaggggca 50254
   tgattcggtt tttgcaacag ggagtagctc ttcttgaaac attatgggat ttctttccta 50314
   agttgacttc acttacattc tgctcagcac atgcaaagca tttggttatc gggaggcaga 50374
   gtaagatgac aaccctatta ttgaaggtaa gcagacctgg cctgttcaca aagccctttt 50434
   tacatcttgt ttcattctgt taccaacagg gtaactgtga agtgggtggg caggaactgt 50494
   gacttctttt caagtctcag aagatgtcag tcccccaggg aggctggcct ccgagtgcca 50554
   gtgcaggtgg ctccccctcg gccgtcctat gccacgtgcc ctctcctcgt cattctcagc 50614
   cccctcgcgt ctggagtcac ctcgctggtt tatcgtcttg tggtccatct gcccgtgagc 50674
   attccagcca ggtctgtggg cttcgttttc acggccgcca ttttggctgc ccgcagagtg 50734
   cctggtgcaa agcggatgac actcagtatc tagagcggca cttccgggta atccacaaac 50794
   atttatcaaa cacttaggtg tcacttgggg tgctcggaat tgcaaacatg aggacaaacc 50854
   ctcactccat ttggggcctg cagattcagc aagagtggaa aaaccagctt tttaaaatgg 50914
   cagcctggga atcccagaat taggaagcac aagcttgtct ttggcaggcc aggccagctg 50974
   ctgacccgca cggccttcag gggccagcca gtgcgaccat gattgaatgc acagccctga 51034
   ctcctttgtg gcattggctc ctgggtccag gtgggagcat ctgattggcc cccactggtc 51094
   atgtgctaac caggaagtgg tgtgtgatgg gctgagccta gggctcatac ctgcatgtgt 51154
   tttgggggaa acagagagaa aggtgtggcc tctccacttc tgtggacacc ccaaatagga 51214
   agaagggtgc acacaaagca ccgcttcctc cacgaagccc tcctcaggca catcgaggag 51274
   aaccagctgc ccaagctctt ggctgctgcc cttctgaggc ctgcctggta ggtggcgtag 51334
   gaggaggctt ggtaagcaga tgatgggaca tcatctacca catcccatca gtaacatcca 51394
   acaagtgtgt gtgccagcac agacctgggc actggcatgt cccagccgtc attccatgtc 51454
   aaatacttat ctgtgccgtc atcgtcgagg tctggtttcc aactctatgg ctccattgtc 51514
   tcaggtttaa ccagtcctct tattgggcat cagcttttcc atttttgctc tgtgagaggt 51574
   tgttgagtta gttggaggac caagcccata atttcctgga tgacagttcg tgtcccgcag 51634
   atagagctta accaacagtg gttccctttc cagacctaag caggtgggag tcagagatga 51694
   gcatagagaa agggtgaagg ggtgcctctg gattcccaag gcagggacat agctggggga 51754
   gctgcctccg gactcgcaag gcgggggtgt ggcagacact ttgcagaaaa agtacatctg 51814
   tccttggggc gagaggccgc acatgcagcc ccaagtccct ggacctgtga gatcctggct 51874
   gcctgctgtt gatagaacgg gacaggtggg gggatgcttt tgattcatgt acggtgaatg 51934
   atcttattct cacctttatc actggtggca cattctgatg gaaggagaca ttggcagtgg 51994
   gactgggcaa gaaacaggtc atttgtcatg gctgtgtgcc agcctcctgt cgttgcacag 52054
   cctgtccttg ggaagtcttg ctcacctgtc atgtggagta gttgtatgtg tgtgagtgtg 52114
   tgtgtgactg agagagacat acacatatgc acacacacgc agaagtgtct gtacctctga 52174
   gaatatagtt ctccagagga tgcagaaaaa ttggggattg cagactgtcg tgcgtttaaa 52234
   tgtgtccttc tctcaagaat aatgtccact tggaacctca gaatgtgacc ttatttggaa 52294
   atagggttct tgtgtatgtc actagttaag ttgaggtcat attggattcg agtgggcctc 52354
   aagtccaatg gctggtgtcc ttctaaggag aggacacaca gagacacagg gagaggaagg 52414
   ccgtgtgatg acagaggcag agattggggt gatttatcta cacaccagaa gccaggaggg 52474
   agtcagggag cggctcctcc ctcagagctt ccagagggag ccaggcctgc ccacgctgtg 52534
   atgtcacatt cctggcctcc agaactagga gaggataaag ttcgattgtt ttccagctgt 52594
   ctgtgtgtag taatatgtta cagcagccac aggacactga tacccagaca catcccaggc 52654
   cctaacatcc cagaagagga gtccctgata ctcctctttt gcaaaatgag gtgtgggctc 52714
   agagcaagtt cgattttctg tgaagggcct gatagtagat attttagact ttgcaggctg 52774
   tgtaaatctc tgttgcatat ttttcttttc ttttctcttc ttttcttttc ttttctttct 52834
   ttttttagac agagtctcac tctgtcgcct aggagtgcag tggcagtggc accatctcag 52894
   ctcactgcaa ccactgcctc ccgggttcaa gcgattctcc tgccttagcc tcccgagtag 52954
   ctgggattac aggcacatgc caccacgctt gcctaatttt tgtgtgtgtg tgtttttagt 53014
   agagatgggg tttcaccacg ttggccaggc tggtctcgaa ctccctacct caggtgatcc 53074
   gcctgcctca gcctcccaaa gtgctaggat ctgttgcaca tttttctttg tttgttttgt 53134
   tgtcatttgt ttgttgtttg tttcacaacc ctttgaaaat gcagaaaaca ttcttagctc 53194
   ttgggctggg tgtcactgag tttgccctag gggaaccctc accgcatgcg aagctgagtg 53254
   ggcacggttt cctccgctgt caacgggaat attgatggtt tccctgagtg aggacccagg 53314
   ggaagcactg ggctggtgcc cgcctcctgt ctctgtgccc aggcctactg tctgcttcac 53374
   actttactcc ccagaaccag gcatggcact tagtacaggg caggccgagc tcgggggaga 53434
   tttgcttgaa gaatgaggaa cgtttctgga agcctctctg accacccagc aggcaggggc 53494
   aggtcttcct ctgagaccca agtcctctat gcttccctcc atcctagcag tgacttccat 53554
   gacacaccat tgtccctttg ggagcacagg actctggctg agtcatctct gaagtgaggt 53614
   gaacccagca cagggactga tggcttggga agagcagctc agcctatgtg gggggtgttg 53674
   agggtgccca ggcccagggg cgatttgatc ccaccctacc ctagcgcagc cagaatgctg 53734
   gggccgaggt tcacctggga gatgcaggag tttagagaag aggaggagga tgtttaagcg 53794
   tgcgagctgg tgataggagc gtggagttgg agctagaccc agacttgaat tctggctttg 53854
   ctgcgtggca gcagctgtgt ggcccttgat gagtgtctta accttgctgg gctttggctt 53914
   cctctccagg agggcatagc aagtcctgcc tgttagggcc atcctgagga tggggtgacc 53974
   agagcatgag cagccgggtg cccagggcac ctgggcaaca gccctgtggg taggtgggtc 54034
   tgaatggttg agccccacgt tttagtcaag accactctcc ttgcagagca gttcatgtcc 54094
   acttgcccac atccttcctg tcccagcacc gtctgtaggt tgctatgatc accccatttt 54154
   acagatgagg aagcacatgc ccaaagaagc aagtgacttc ccaaggcggc aaggcaaggt 54214
   gaggggcaga atgaggaccc caacccagtt ctccccaccc cctccttccc ttccagagcc 54274
   cctgcctgct ggaggcattg tcccagcggc agagggtcag gccacatctg ccagagctaa 54334
   ggggagtctc cgtcatctca aatcattggg tcaaatgaca gaaatctgct ggcatgctgc 54394
   cttttgaggc tcggcgactt ctctgaggct ttcctgatgg ctgctctggg tgtctggaat 54454
   gagctgtgga cgagggccgt cctctgagtg accatgcccc ccacgcagcc gaggttcccc 54514
   tctgcctggc tcagtgcggg ttctgagaaa gcctgcctgc cctcactgga ggcacctctg 54574
   actcactgct gcaaggatgt ggccatggac tggggtcccc gggggccccc gaaagcctca 54634
   gcaggagaac atgtttatcc agaagcctgg gcaacaccca gagggtcctg gcatgggtgt 54694
   gccaggcaga ctgggcatgg aaacatgccc cgtgatgtct ttgggatcct gggagcctga 54754
   ggcttgttct cagtgtgtag ggcaggggct gggtgccacc caaggggggc aaactgaggt 54814
   cacagagagg gaggcagtac ccccagaaaa ggtgattctt ctcattggaa gattcccaag 54874
   gcccaaggcc tgggtcagca gacagtggaa caagggtaat ggtcatgtcc ttaattcacg 54934
   taaccccgag gtgggtccct gtgaaaatcc cgtggatggg gcctggtgat ggctacactt 54994
   atattctcag aagcagcatt accacctgga cccttccctt ttctcttact ccctcctcct 55054
   cgtggggtct gcaagggggt ctggtaaatg gcatccccct ccccgcccag gggctggacc 55114
   cgtggagcat aagccaaaag gggcggcaaa gagagacacg gaacctcact gccactgccc 55174
   agctgccggt gctgcaggac ctggcttagg ttcaggggcc cggtgggctt ccccagtgct 55234
   ggaccccagc aggcaggtgt ggagggagca gggggtagtg gagcagagta cagtggcccc 55294
   tggctgttct cccccaggaa gagaggagag gcggggctgg actctttccc atctccctcc 55354
   ccaatactcc agcgtcagtc agtctccccc cgacaggggg aagggagaga ggccaggagc 55414
   cttcccctgg ggcacacaca gagagcccag caggcttgga agaagtgtct ccctcctcct 55474
   ccagccctgc cagatggtcc cagccctcct gcagggttcc cgaagtggaa ggatctggac 55534
   tcagagccca gtgctgctgc ttggcatctg tggtcccggg ctggagggcg tgttggcagc 55594
   tcatgcctgg ccagcgcatc ggggtggctg cagcagagga tgggggaggg gagcagggac 55654
   aagaggagcc tgcaggctgc tgggtgcagc cagggcttct gcttcacccc gggggtcaca 55714
   gctgctttct gttgggaaag ctcccactgg aggtatgggg taggcagtgc tggagaccct 55774
   ggtggcaagt gctgagctgg ggcacaggtg aggatcagga ggagctggga gggacatccg 55834
   caggttgtgc tgatggactg cctctgagta ttttcacggg atggtggaaa gaggctttga 55894
   gtcagacctg ggttcgaatc ctggctccta ctcttggtag cagtgtgact ttatgtgacc 55954
   tttctgaacc tcagttttct ctgttgtaaa atggggaaaa ccacacatcg tgggggcngt 56014
   tgtgtgcacc taaggaagcc accccagggc tgggcacacg ggtagccgat tgtcatatcc 56074
   ttgttatgga aggttgttat ccttgttagg ctggtgggca ggggtggtag aggagtaggt 56134
   tgtttttgga aaatggtgga cttagtctta gacatgttgt gtttaaattg ctcatgagaa 56194
   atccaaatag aaagacccag ggtgacagca gggactgcta ggaacttggg ataaaaaatt 56254
   ggtcctgggg tgctggtcac tgatcctgtg gataaaggag ttggggtgct ctgtttgggt 56314
   ccagctggca gagttggacc agttgagaga aacttctagg aggaaggttt agaagaagaa 56374
   tgctttcacc atcagagcta tcttaatagc tgtgtaatgt ggtagtgagc tccccatcgt 56434
   aggaggtatg tgagtcatgg ctggaggact acttggaggt ctgttgtaga aggattcaga 56494
   caccagagag ggttagactg gatgaccttc agttcccctc ctgggtgacc acagctcttc 56554
   aaagcccctc actgctgccc gcttctgcag gattcagggt tgaatgtcag aagtctgctg 56614
   gaatgctgtc gattgaggca cggtgacttg gccaagcctt tcctggtggc tgctctggta 56674
   tctggaatga gctatgggtg agggccgtcc tctgagtgac cgtgcccccc tcacagctga 56734
   ggttcccctc ggcctggatc agcgtgggtt ctgagaaagc ctgcttgccc tgactggagg 56794
   cacctggagg tggttttcaa agtgtcactg tcaccgtgac ccagacatgc ttgagcccca 56854
   ggctgggaaa gctttttttc tttgctttaa aaaagcagaa acagtgaagc aagcataaat 56914
   ttgcctttga aataagaggc tgaatgtctg ccaaatgcaa atggattgaa atggccggtg 56974
   gtctgacctt gggatgttcc attggtagtt tcagaggggc tctgagcccc caggccggct 57034
   gtaacagtgg acatgctggc gctggccagt ttgatccctt tgaaagcccc ccgatattca 57094
   cctccaagag agaaggccct ggcaggtcct ccagcatgcg gacgaggcgc atgcactgtg 57154
   aggcagcggc tctgtgggag gacggggcct tttgtccact gtcctggcat tccatggccc 57214
   tgtgtcccca gatatccgca cctgttcctg acaccatccc ttcctgtcgt gtgagcgaga 57274
   tgctcttctg tggaacctgg gttcctatgg tcactgggag ggtgaccctg agttcacaga 57334
   gaaaggggag caggctgttc aggaaaacta agcacatgtg gtgagcatcc acaggcggct 57394
   agggatggaa ggcttttcat ccgggctttc agatgaggcc cctgaagctc agaggggtta 57454
   ggcaattggc ctgaggttgc acagccagga aggagtgatg gcgtagtgat tcccattggg 57514
   ctctcccacc ccctgttcct ccctccatcc agcccagggc ctcgtggata cattaggagc 57574
   ccaacaagag ttcccctccc ttctcccttg cttctctgag cctcggtttc cccatccata 57634
   taccaggagg aaccccctgc ccatctcnta gggctctttg acatggtgaa atgagatcca 57694
   gatactggct gtctgtgtca actgtaaagt gctgtcctga gtgaccagtt gttgttggtg 57754
   cctgtagcca tccttgaggc taaaagagac tgctgcttgc ctgatgctcc tcccgccagc 57814
   ctcctgcttt ctgcgtgact tccctcgccc cagcaatgaa ccctggagcc acctgcaaat 57874
   gggaaagctc ttttgttagt ttccacagaa tgcccaccgt gcacatgatc agagagtttg 57934
   cgagcctagc tcaggtcagt ggaactggtt cttttcgtac atgtaggaag taatgcaggg 57994
   tgctgccgaa taaatccact gcctgcctgg cctctgatga acttaccagc tcacgccagt 58054
   tctagctcca gccaggagga ggaaaaaggc atactaggcc gtattttgtt tctttcaatg 58114
   tttatttacc tgtacacaca cacacacaca cacacacaca cacacacaca cacatacaca 58174
   ggcaaaactt atgttactgt cccatgcata attctctaga tttgggccac atagatctgg 58234
   ttttggccat tgactggcaa catggcagta gccaactgca gagccaccag ggagcagcct 58294
   cattctgatt gagtataacg aagacgctga tgggaaagag agacacgggt gagcaggggc 58354
   tgtggggcat catgagagtt gggagggtcc ccatcgtgga gctggccagc cctgagtggg 58414
   agccccggca cagtcctctg ctagctgtgg cctttggcca gtctccactt ctcagagtct 58474
   cagtgacccc agctgccacc accaggattg ttggggtgtc caagaatgaa aataggggtc 58534
   atccctggcc ctcaggggat atgtgggctt tcccctcatc atgaatggaa caggtgagtg 58594
   ctgtggtgca ggctgagagg cggcagggtg ttctctcaca gcagggtcgg gtcttgctct 58654
   gataactgca ccaggacaaa catccccagc ctctgggatg cacacccacc agcatctgcc 58714
   cggcaggttt aattaccggc tgagatgggt tgtgttgggc tcactaagta tgctccataa 58774
   gtgatgaaat tgggcaagtt ctcacctctg gaggctctga ccccgagtag ctttgaccct 58834
   gagtagctga aggaacaggc agttcagcct ctgcttatgg gctgtgtgtg cacacatgca 58894
   cgagtatgag agtgtgtcag tgtgtatcgt gtgaatatgt gtgcaggtaa gtgtccatgc 58954
   atatgtgtaa gtacatgaca atgtgtgtgc gagtacatga ctgtgtgttt gtatgtacaa 59014
   ctgtgtgtgc acatgtatgt ccgagcacat tggcgtgtgt gtgaatgtga gtgtgagagc 59074
   gtgtgatttc atgactgtac atgttcactc tgtttattct cgggggaaat agcaggcaat 59134
   ctgtaagaaa atgactgtgt aagtttcatg ggtggaggct tatgatactg ttagagtcta 59194
   tcttagtctg ttggtgctgc cataacaaag ataccataga ctgggtgatt cataaacaac 59254
   agaaacattt ctaacagttc tagagctggg aagtccaaga tgaaggtgcc agcagtttcg 59314
   gtgtctgttg aaggccctat gcctggttca caggtggcga cttcctgttg tgtcctcaca 59374
   tgggggaagg ggcaagggag cttttgtagg cccttcttct aagggcacta atcccattca 59434
   tgggggctcc accctcatga cctcatgacc tcccaaaggc cccacctcct aacaccatta 59494
   tcttggggtt agggtttcaa catatgaatt tgaggggaca cagccattcc atctgtgacc 59554
   gtcctcaatc agccttctcc ctgcagggct gcaaatgagg gtgctgccgg attcataaga 59614
   gaagatacat tgtaacatcc acagcacaca ggcactaggt tgggagcctt ttccagcact 59674
   cagatcaggg ttcaaaccct aactgtgccc ttcaggagct gtgtgacttg ggcattcctt 59734
   cattttttat tcaagcacac agctggcatc tacatgcctg ctgtgtgtca gggagataga 59794
   aatagagggg tgagcccctg tgatattagg caggcagctt tcccttccag ggtctcattt 59854
   ttctcactgg tgaaatgagg gcaggggctc ctcccctggg gttactttga ggggtcagta 59914
   aggtgaacag ttacagtctc ttgcacagcc cctgaacgtt gtttagaagt caatactctg 59974
   ttccatccca tcagcttcct cttcccttgt tttgactggc aaatagtcat caccttctga 60034
   agtcattaag agaaggtctt tttaaactat tatgatgcca tcaaaactga cttttgatcc 60094
   aaaatatctt ttaaacaagc tgaccattca gtgaggattt gccaggctcc tttgtgatcc 60154
   atccttttgg ggccagggtc tgtggggccc agagctggac atcatggaac tagctctgag 60214
   gagctcatgt tcaggtggga cacagtctcc tggaatcaca gttctaagca ctttccaact 60274
   attaactcat ttaatcgtta attactacca tttcccagat gatgcaccag ggatgttaag 60334
   taaccaagga atatggcagg gccactggga tagtgggctg ttagaaaagt cagggtggag 60394
   gccgggcncg gtggctcatg cctataatcc cagcactttg ggaggccgag gtgggcggat 60454
   cacgaggtca ggagattgag accatcctgg ctaacacagt gaaaccccgt ctctactaaa 60514
   aatacaaaaa attagccagg tgtggtggcg ggcgcctgta gtcccagcta ctcaggaggc 60574
   tgaggcagga gaatggcgtg agcccaggag gcggagcttg cagtgagctg agattgcgcc 60634
   actgcactcc agcctgggcc atagagcaag actccctctc aaaaaaaaaa aaaaaagaaa 60694
   tgaaaagtca gggtggagta cagacacaaa ttagagaact taagaggcac caacaagagt 60754
   atgggcagta tagggagaaa tggaattagc acggagcagg ggcttcctag ggaggaatga 60814
   tgggaggaga cacctccttc ctgctgctcc tgctgtgtgc cagcctgggc tgggagcgtc 60874
   acgccatcag ctccagtatt cattccagcc ctgtgaggct ggcagcaggg tcctcctagt 60934
   ggggctgaaa ggtgcaatgg tgaggaggag gattgcaggg gctggagtcc tggccccacc 60994
   acttcctggc tgtgtgactg tgggcacgtt actcaccctc tctggccctc catttattca 61054
   tcattaaaat gggattaggg gccaggtact ggtggctcac acctgtaatc caagcncttt 61114
   gggaggctga ggtgggtaga tcacttgagc tcaggagttc aagaccagcc tgggcaacat 61174
   ggtgaaactc catctctaca aaaaatacac aaattagcca ggtgtggtgg catgcgcctg 61234
   tagtcccagc tacttggtgg ggctgaggtg ggaggattgc ttgaacctgg gagggttgaa 61294
   gctgcagtga gcagagatcg caccaccgca cttcatcctg ggtgacagag tgagactctg 61354
   tctaataaat aaataaataa ataaataaat aaataaataa ataaaacagg gggaatagta 61414
   gtccctactc catagggctg ttgtgaagat ctagtgagat caatgttcat gaaggacttt 61474
   ggcgctgcac acgtttctga gaaagggtga cctggccagg ctcactgggg tggtaagtgg 61534
   cagggatggg cttctccacg tctcccctgt gatattaggc aggtgtatgc agttgcttca 61594
   cgtccagcca caccccctca cacacctgca gaccagccag catggcacac cagacacatt 61654
   tctgctgcac ctttccccac acagtaccta ccacctggcg tgcctctctg cccatcccca 61714
   tctttttgat attttacctt ccacctaaat ccgaactgaa atcccagacc tcactagctg 61774
   gtgggtgcat ggggtctctg ctctgccact tctggagctg ctcctagtgc atgccccagt 61834
   tactcactcg tggccaccac cccccgagtc aggggtcgag agtccccttt gtccatgatg 61894
   ggtgacgagg gcatacccgg aacagagggc taattaaatc acacgctgag gtttgtagaa 61954
   catgggacgc ttcattaggc tctgtgtgaa caagtgcgcc cagcctgctc ccactactcc 62014
   agcagactcg agcctggtat aatcacagct cacactctgc ttcatcagct cgctgaggct 62074
   gtggggaggg ctcacacctt tgggctctaa agagagtggg cctgaggtgt gggatcctgc 62134
   ttctgagcag gggtgtctgc cccgagccag cttgcccagt ggtgtggagc tggagccccc 62194
   tctgcttaag taaaggcctc tgagggacac atgtcatgca gatgatgaca ccagactggc 62254
   tcctcacttt aaactctact tcctgagagc agaaggcagg gaaggcctca gttggaggtg 62314
   aaaatggaac tttcatccac cagatgttag ataaaagata ggaagggtgt tccaggcaga 62374
   ggggaaagtc tgggcaaagg cttggaggat aaaagggcac cgcatgtttg gggagcgggg 62434
   catgttccct ggggccagag acctgagttc tcctcttggc tctatcccca gctccaccca 62494
   tgacttgggg tgagtcctga accccgacca accttagtgt ctccagggct gggatatggg 62554
   ggggtggcag cctctgatct gtggtcacag ggagtctcag ctgtggtgct cctgtgcctc 62614
   cctcttgcta ctctctggcc agtgacgatg gcactttact ctcctgcaga gacctgtccc 62674
   cagtgcctgc ctcctccagt cctcttcctc atgggctggc tgctctgggc acatctcagg 62734
   gcagccccag aaggtgggct gagctgttat ttggggcgtg gggttctcgg ttcctccgct 62794
   ccatgctggt gggcctggtg cctggcacca ctggcccgtc ttccaggaag ctcactgaca 62854
   ccatcctgga tgggccttgt tttctctctg gcctgggagt cagctgcact ctcgcaggtc 62914
   ctaggccaag gtgacctgct tgtttcattc ttgtctttct cctttgcttt atggtatatt 62974
   tttaagcata agatatattt atttgtaaaa taaaagttca ttataaaatg atgccttaat 63034
   taccttcctg caatataaca attatttggt tgtatagttc ctgttctccg tattttgtgt 63094
   aataaaatgg acccaatggg tgctgcacaa tgctgggtgc cacagggaac acacactgct 63154
   gttttctcct ggatttttaa aattttaatt ccagcatggt ccttttatat gtgctttcac 63214
   tctagctgtt tgcctacatc tccacagttt tgattgtcca gggtggtgaa taaaatgcaa 63274
   cacttggcat cttttaatgt ttaaaaaaat caacaagtat tttatttaaa ataaaatgtg 63334
   aatatctgta atcctaaaaa aaaaaaaaaa aaagccacag gatccttgag aggccctctg 63394
   agacagtgac acccctgacc agctgcagcc ccctgtccac tccccaggct ccattctctc 63454
   tacccactgc ctctgcatcc tccgcccttg gagtttctca gggtgccccg cagatcacct 63514
   gtggcccaag tgctcaggga cttgtttaaa attcagattc ccaggccccg cccaggcttg 63574
   caggcagcca gggccaagga atccaaatac taatcaagcc cactgtaatt gccctgcatc 63634
   tgaggtctga aatcactcgc ctgctctgga ttatctgtgc tccagggtgg cctcagttta 63694
   caccatccta cctccccatt gaaaccttac tgctcacgat ggcccaggtt cccacccatc 63754
   ctgggagaga aagatgattg gctcagttct ctgggaggcc tggtgattgg ctgcctctag 63814
   gagggtgtgg ctacctcttg atccaatcag tggagggcag catggggatg tgatcatgca 63874
   gtgaagaacc tgtaccgggg gtagttaggc gccagggaag gggaggagga gaaaccagtt 63934
   ctactgttag tatcagctca tctaacattt ggcccctggt gggggttttt gcatcctttt 63994
   gggggtctag gtctcttctg ccattttgtt ggcactttaa tttcttgtta acaaacccaa 64054
   gagcacatac aacttaattt ttaaattccc aataatttcc agcatcctct aagttagtgc 64114
   tgagcgatag aaatgtaacg gaagctgtac gtgtagtttt aaaacttctg atagccaggt 64174
   tatgcacgta aaaagaaaca ggtgaaatta attgtaataa tttgtatatc caaaatagta 64234
   taaactgtaa tcaatctaaa gtaattgaga cattttacat tctgtttttc atattaaatc 64294
   tttaaaattc ggtgtgtgtt ttatgcttac catacatccc agctcagact agccacattt 64354
   caagggttca ttagccaccc atggctggtg gtccctgtac tggatagcgc agcactaaat 64414
   gatggcttta ctttgtatcc ttggggaagt atgtattttt gaatccattt tcctcacttt 64474
   tgaatagttt aagaacttcc cagtccattt gtctaagggt atttgacttc taaatgttgg 64534
   gtttcttggc cgggctcagt ggcttacacc tgtaatccca gcctttggga ggccgaggta 64594
   tgcggatcac ttgaggtcag gagtttgaga ccagcctggc caacatggtg aaaccccgtc 64654
   tctactaaaa atacaaaaaa aataattagc cgggtgtggt ggcgggtgcc tgttgtccca 64714
   gctacttggg aggctgaagc tggagaatcg cttgaaccca gcaggcgagg ttgcagtgag 64774
   ctgagatcgt gccaccgcac tccagcctgg gcagcagagt gagactccat ctgaaaaaca 64834
   aacaaacaaa aaaaccagtt gggtttctag caataaatgc tcataaaatc tgctggagtt 64894
   ttggaggatt agtccattca tcctgacttg tgtcctgttt ctgctgggtc attgttgact 64954
   tcccccactt gcctcacact tgtaaggcca tgtggcacac catcaacatt catctctgat 65014
   taaggagctt gtttcttgta gtggctgcca taacatatta tcacacgttt ggttgtttaa 65074
   acagcagaaa cttacactct tcctgttcta cagtccagaa gtccaaaatc atggtgtccg 65134
   taggcctacg ttccctctgt agcctgcagg ggtggatcct tccttctcta gaggctgcca 65194
   gcatctcttg gctttatggc cacattactc taagttctgc ttcggttttt acatcacctc 65254
   ctcctctttc ccccatgtct tctcctctgt gtgtctctta taaagccact tgccactgca 65314
   tttagggctt actcagataa ttcagaatga tcttctcatc tcagaatcct taattatgtc 65374
   ttcaaagacc ctttttccaa ataaggtcac attcacaagt tccagggatt agagtgtgga 65434
   catacctttt gtttttattt ttattttatt taatgtattt atttattttt gagacagagt 65494
   ttttccctgt ttccctggct ggagtgcagt ggtgcgatct cggctcactg cagcctccgc 65554
   ctcccaggtt caatggattc tcctgcctca gcctcccgag tagctgagat tacaggtgcg 65614
   tgccaccata cccagctatt ttttttttgg ctttttcttt agtagagaca gggttttcat 65674
   ccaacagatg ttagataaaa tgttgcccag gctggtctcg aactcctggg ctcaagcgat 65734
   ctacccgcat tgggctccca aagtgctggg attacaggcg tgagccctgc accctgccaa 65794
   gagtgtgtac atatcttttg tggggcacca ttcaacccat tgcagtgagg tgtcagtgtt 65854
   ttgacaggaa gaagcagcaa agaagacaaa tcagcacttt atttttctaa gctttgaaat 65914
   tcttaaaatg atagtgggtt aagaaagacc aacttcccca tggaaggtgc tggtccacac  65974
   tggctgggtt tagatccctt tggctgaggt ttagatccct ttggagggga gtcaatttga  66034
   agaagaagca catcagacac attcaggaag gcaaacccaa agacagcaga aaggcaagaa 66094
   tgtctcaaat tgttcaaaaa taaatacgtt agttcttgta acgtgctccg aatagggtga 66154
   ggctcaacat gagcacttaa taaatgctca gtttaattgt ggtaattgta attctcattg 66214
   gtatatattt atgtcctgat cctatacctc tgttattaat gagactgata ataggtaagt 66274
   gaagggaaaa agagaccccc ctgctgccag gaaacaaaac aaagtatcga gccaaatgcc 66334
   aaatccataa gtcaccaaga tgggttttga tgacaaaacc tttctttctg gactccccct 66394
   gcccctccgc gaagccgttc cccatggcca cgcttgtccg tggctggctt tataggccca 66454
   tccttctttt atatgatcat caagatggca ctaatttggg tttgcaaatg aggttttcgt 66514
   gatgttgtca tgtcagcatc tggattcaag aggggctgct tcccagaccc tgaccctccc 66574
   ctgtggcctg ggagcaggcc cagcccctcc ctccatctaa tcactcccca ggaacctggg 66634
   ggatttgcta atgggcccag gaagccataa agagtaatga aagtgctgtg tgactgctct 66694
   gcacggggtt gcccttgggg tcccttcctc ataattaaat tcggaatcct ccttctgcct 66754
   ttgagctgga agagagggtt ttatcctccc tgtggtctgt tcatcccttc ttccccccat 66814
   tcatttgttc agcacttatt atccagttat ttgtttatca agccacagtt gaatgcctgc 66874
   tgagagccgg tttagggcac tgagcttcct gttctcaaca ggcagaggtg ggagcagcca 66934
   ggctgctgtg ggaagagcag agtggctatg gagccgccct gagccagctc tgctgtgcgg 66994
   gagttctgcg acctggggaa cccgctcgct tggcttcctc atctgtaaaa cggggagact 67054
   aatataatag cagctacctt ccaggggggc tgtgagaatt aaaggggacg atggatataa 67114
   actcccacac actgtcgggc acagggcgtt aatagcagcc ctcagtctca tgtcccactt 67174
   attttgtgcc cgacacactt ctgagcatct tatgtctatt cactcattta atttgcccag 67234
   taactctagg aaggaggtca cgtccatggt tcttattttg ctgatgaggc agttgaggta 67294
   ggcagaggtg cggtgccttg ctcaagatgg cacagtggaa ggacacgggg cagggcggac 67354
   ctgaccgcag ttggccctcc cttgcctgcc tcttagcacg catgcagccc cctcgcctgc 67414
   tctgcccgtt gtgtctcaga atctccactg ccatttgcgg gtgccctgaa tccacctcgc 67474
   ttttccgtgc ctccctgctt ttgtgcttgg tgcagacccc tgcttcctca tgggcacctg 67534
   cctccttaga accccccttg gaatctgtta actacagctt cctgggccca caccagcatc 67594
   cctctgcaac tcagaatttc cagggcccat aatttcctac aggcccctcg ggtgattgct 67654
   ttctcagtag gcaacttagg gccgtccctc caaaccttta gagtgagaag cgtgctacct 67714
   gggtgcttca taactgatgt tgcaaatgtt caaccaagtg taggaagtga acacactttg 67774
   tatctgcaaa gtgctgtgca tacaagcgag gtcaggagca gctggcaccc gtccccaagg 67834
   gtttccatgc ctctggcgag atagggtagg agtatctgca agtagctgcc ttcttggcca 67894
   tggaagaaca agttcaacag ggcgcctctg tgtgcctgcc ccttgctgtg tacaaggacc 67954
   ttgcacttca gggacatcaa ggcaccttca gccgggcttt ggggaacttg tgtatgggag 68014
   ggggggacct ctaaggacct ttcctgtccc aaggagctcc ccagaagcct gttgcagtgc 68074
   ccagcacccc agcctcatgg ttaaggtagg cagtccccag gagtcccctt cctgggacat 68134
   caactctcct ggtgggaaca aatgctgagc acctcagcat gagatgggta cagtcaggga 68194
   ctccacaggc aggtcatgtc acagacaagg tcacacagct aggtggtggc aggttggaga 68254
   gagagggcag tcccctgact cccatcccag tgctcttttc tcccatgaca gtgacgggtg 68314
   aaatggcaca gtgtatccct gtcaggcagg aggggatttg ttgtactttt tttttttttt 68374
   ttctgaggcg gagtttcact ctgtcaccca ggctggagtg cagcggcgca atctctgcac 68434
   actgcaacct ccgcctccca ggttcaagcg attctcctgc ctcagcctcc tgagtagctg 68494
   ggattacagg cacccaccac cacgcccagc taacttttgt atttttagta gaggcggggt 68554
   ttcaccatgt tggccaggct ggtcttgaac tcctgacctc aaatgatccc cctgcctcgt 68614
   cctcccaggt tgtattttta atcctccaca actaaatact catgtcagtg gaaaggttcc 68674
   aagctcttta aagacagcct ctgccagcgt gctcccaggg gaggccaggg aaggctttgg 68734
   gaagcctccg tgggggtggg gacctcctgc aacccctggc gcacagcctc accccgctgt 68794
   aaacagagct gggattgaag tgcgatttcg gtttcttttt ctttttttct aatcaaataa 68854
   aaacacctag ggggcaggaa gcgaggagga agaggccagg gagtaattct tgttgccaaa 68914
   ccagtttcta aggggctccg ctccgctccc agcatttctg tctctgaggc tccgacctct 68974
   gagatgatca atcctcccat ttcagccaga tgagaattgc tgtgggccct gccttttctt 69034
   aatattttgc atgagagcga cagcccggcc agcggcagta atctcccacc cacgtgggga 69094
   ggcacccact gtcctgccat gtgcataatt gaagtcttca gactgctcag tggttctaat 69154
   tagcccaaag tggccccttc tggcctcagt ggaaattact gccctcctgc cgcctggtct 69214
   tactagctga tctttgaagg tgtgagtgag ccgggagacg tgcagagcct agcactccct 69274
   ctgctgcatt ggtctttctt ggcaggcctt catggctttg cagagggcgg gcagttgagg 69334
   cctctgtctg ccgttgaggt cctgcctccc tcctctctgc caccactctc catgggacag 69394
   gtgctccagc tgggcgcatg catgcccctc tcacctgaat gcctcccact agcagtgtct 69454
   ctgctctttc tgacccttca ggttatgcag tgcctcctcc tccaggaagt catccctgat 69514
   ccccaggctg ggccctcaga atccccatgt ctccctccat cgcagccctg gccatgccat 69574
   gtggtgcttg tttacatcca tgactgcccc atgagactgg gagcccctag ggggcagaag 69634
   ccgctccatg tcccacctac tcctggccta gcgcggtccc ataggacatg tctacggaaa 69694
   tggagggcgt gagggtggga ggaaggaagg aaggcaagaa ggaaagaagg aaggagaagg 69754
   aaaaaggaga agagagaaaa gagggagggt gggcaggtag caccatgccc attttgcagg 69814
   tgagaacact gaggagacat aaccgggaag tggcagggcc aagcacaccc ttggcagctc 69874
   attatacttt cccttcagcc tagcaaagct actgttcgat ttggggaaat ggggccgctt 69934
   gtatgaagag ccgcaggcaa agtggaaagt gccgagggct ggaagtcaga tgggtgtggg 69994
   tgcgcagcct tcctgtgtac ctgtgggcac cacttacctg ctctgtgacc ctgagcaggg 70054
   cacctttgcc ttcgaaactt gaagatcaca gtcgtccagg tggattcaag gcgatgaaca 70114
   gcaacagcgc atgctcttcc caaacagtat tcttcttatc ctcactgaag ccccagaagg 70174
   tagttgatcc ttaccacatc attaacctca atgcgcagat gaggatgcca aagcacagag 70234
   aggttgagca actggcccaa agtcacacag tgcgtgagtg acagagccag gatttgcgcc 70294
   tgggttcttg gctctgatat tagtgccatt gactccactc tgttgtgtga ggtatgaagg 70354
   catccagtac agtgtctggc atcggcaggt agcttccatg gcagataccc cccctctccc 70414
   accagattct gccctttgag cgacaggaag ataaatagcg ctgttctcca gtgtcacttg 70474
   tgactttcct ctgtgagttc attgtcaggg tgctcctgag ctgagctgtg gccaccactc 70534
   attggtgtgg tccctggaga cacttggata aaattgtgat gacagagggc tctcaggctc 70594
   atgagatcac acagaggagg gtggagctca aagggctatc tcagaccggg atgctccaga 70654
   ggttaggtct tggtgctgtg tttggccccc agctgctctt tcaggccttg gtcttgccct 70714
   tgcccttggt ctctggaagg ctgaatagct gcatcttgtt catctggaaa ctctctggga 70774
   tgagtgactt tagcatctcg gatgttgcca tcgtagccca tgtgcataac aacttattgt 70834
   gcaagcatgt acagtcatat tgaaaataaa tgtagaagtg aaggaggcag tccaaagacg 70894
   gttcctgaca atactcggaa acagcttcat taggtattgg ccagagcttc tatttgtggg 70954
   ttgctttggc gaaactgctt ccttaccatt aaccatatcc aggagatgga ggggaaagtc 71014
   ttcacctggg ttggtttttc tcagaggggc aggcaagctg aataacggga gtactaacat 71074
   ttccaagaag attaatgatt aaaatgttga atacaatttg agacttgttt tcatgttcat 71134
   attttatagt ccagaagttc tttctataat gtcctcataa atattttatt tggccttagg 71194
   ttaaatgtca gtttccatgt cctgccacgt gccacttgac caacgtgtgt gggaacgtct 71254
   ctactttgga cacactgtct atatgaatgg aggcccttct accccttgaa atgttcctca 71314
   actcccagct cagtttccct tccgcatccc tttgactgca tgagcggaag cgaggcatta 71374
   cagagaggtt tgaggagcca gccagcaagc tagcaacggc ttcagttcat ttctgagatg 71434
   tttgcattca ggggtcccat tggccaacct taacttctcc tttgagctct aggcatcttg 71494
   tgatgaatgg cataagcctt agaacatggg agaggcttgg atgggccagg agctccaaac 71554
   actaaggaat gttgcttaca tcaatagacg ccttagttac ttaggtcaat agatacctta 71614
   gttgcttaga tcaatagatg ccttagtaga tcaaacaccc aaatcaccca atgtactttt 71674
   agggcaatgg ttgccaaact ttttggtctt aggacccctt tttacactct taaaaattat 71734
   tgtggctccc aaagaggttt tgtgtatgtg ggctatatcc ttcaatattt gccaactttg 71794
   aaattaaaac tcagacagtt aaaaagattt atatgaattc attgaaaaat aaaaagtgta 71854
   aacccattac atgttaacgt aaataatatt ttttttggaa aataactgtg ttttgtttta 71914
   aaaagtgaga agagtggcgt gtgtatcaat ctctttaatc tctagcttaa tagatgatgg 71974
   ttggattctc acatcagctc ctgcattcag tctgttgtga tacattgttt ggttgaggta 72034
   ttgtcgaaca tctatcctca aacagatatg tagttggaaa agggagaacc ccacaatccc 72094
   ctgaaaacgt cttggggtcc cctggaggta cttagaccac agtcttagaa ctgctgattt 72154
   aggtgatggg tgaaactcaa gatgaagaca aaagagaaaa catttcaacc ccaagagact 72214
   tcaatgcatt cacatgcatt gactcaggat cattaaagct ctaactgaac ccaacatgga 72274
   gacagagcag gaaacacagc catggctggc ttgacttcag cctcccatca aagaccccca 72334
   tctttggctc aactgccccc agtggtttca atcagccaca gacttccctt gtccagctgg 72394
   actggacgtt ctgcatttga ctgtctgtcg gctgcccact gattttcagg ctgctgaatt 72454
   acccattagc tcacctgaac ccctaatgcc acatctaaac ggtcctcact ctgcctttct 72514
   cagcatccga gaatgtccta taaaaggctt cttccatgga ggtgggcagc cttgggcctc 72574
   tcctagggtc agctcaggct gcccacgctg gctatggctt cctgctggcc cctggaagct 72634
   gtgtacacct gggagagtga ggtgaatgtt aatttattca gcatgcaatt aatgaattct 72694
   accctgtgcc tggcacgact catttctcat gccaaagcta gggtggatca gaccaccttg 72754
   gacaaatctg gatgggtgaa cttgtggatt gatcaatgat cttagttgat gaaaaatgca 72814
   ccaaatgact gtttccttta atgcgattgc ttgggtggtg gactcgatta cgttgagggg 72874
   attaaattag cagcttgctg ggtttcagat gagtgcaggt ggctgctggc acttcctctc 72934
   ccctcttttc tttctttccc ttttactgca aacgaagact tttcaaagct ttactaagta 72994
   caataaatgc catcccctta aagtgtagac ttccatattc ttagaattgg caagctatgt 73054
   atatgtttgt gaaatcaccc ccataattaa gatttggagc atggccttca cccacaaaac 73114
   gtttcctgtt ctctgtgtgg tccattccta tctctgccct gcaccctggg tagccagtga 73174
   tttgcctttg gtcactgtag gatagtctgc actttcaaga atttcatatg aatagagtca 73234
   tagatgcatg cattttttgg tctggcttct ttcactcagt gtgggtattt tgaacttccc 73294
   cctggttgct gtgtgtgtca atgatttgtt cctttttctt tgaggagaat tctgttctgt 73354
   gaatatgcca tgatttattt attccttccc ctgctgatga atatttgagt tttttctaat 73414
   tctgagctac tgtgcagaaa gctgctatga acacgtacgt atgcaccttt gtgagtttgt 73474
   gagctttcgt gactcttggt tacaaggatg gttggttcgc atggtagacc cgtgcttatt 73534
   ttcacgcttt aagaaactgc cttgctgttt cccacggggc tctatcgttt ttcattcccc 73594
   cagcagtgtg tgaggggctg tttgctccac ctcttcacca actccatatt attagtcttt 73654
   ttaatgttcg ccactctgat gggtgtacac tggtatctcg ttgtgatttg catctgcgtc 73714
   ttgtgatgac tgatgatgtt aagccccttg ccatgtgctt cctgaccatt cttgtatttt 73774
   ggtttgggaa gtgtctgccc aaatcttttg cctattttaa atcaggttgc ttgtgtttat 73834
   attattgagc tgtaagaggt ctttatacat ccgggctaca agaggtttac tagatacatg 73894
   tactacgaat attttctctc attccatgcc ttgccatttt cttttcataa cagtttcttt 73954
   cacatagcaa aggtttaagc tttgataaag ttcagtttgt ccattttttg ttgtttttca 74014
   tgctttttgt gtcttgagaa atcgaagttg caaggagttt cttctataat gtctcctaga 74074
   aggctcatgg ttttagcttt tatgtttagg tagatgatgc atttcaatgt cagtttgtca 74134
   gatggtgtaa gagttgatat tcattttgcc cccagtcatt tattggaaag actgtgcttt 74194
   cctcgtggaa tcacctgagt gcctttgatg acaatctgag ttcaccacag acttgtgggt 74254
   ctagttctgg attctctgtt tagttgtatt gatccacatg tctgtccttt taccaggacc 74314
   acactgtctg tatcactgta tctttatatc agctggggta aatcctccaa ctgtgttcct 74374
   ttatttattt atttattgca aagtcatttt ggctattcta ggtcttgttc taacaggaac 74434
   atattttcgc ttatttaatc cttaggaccc tctgaagcag gtgtgaatat taagcccatt 74494
   ttatggataa ggaaactgag gttcggagag gatgagtaac tttcccaggt aactgaggag 74554
   gaagtgagcc ttgcccctgg taggtctggc agctgcactt gcccttagcc tctaggcagg 74614
   ctgcactgcc ctctctgtgg gttccaacca ttcctggtac acacccctag ccaagaactt 74674
   gtgaggttct tggaacctca tgcaagaggc tcttgcatga ggcctctttt ccaacttctg 74734
   tttatcctgt cccaaagagg tcagtgaggg acccccaggg ccgcctctgt tccctcaccc 74794
   tgcaccttgg ccgggagacc cccgcagcgc agggagcatc cctccggatg tttcttccca 74854
   ggcaacaata ttctgtttat caagccaggc cccgaggctg aggcctcccc ctggcctggg 74914
   gtgatttttg gttggtggtg agaagcagca ggggtgcctg ggggcctggc tgtcatcgga 74974
   gccagctccc tgtcctggtc tctgctgtgc gggatggcat gggtaggggc tttgtgcagg 75034
   atgagggtcc tgccgactgg gacgtgggct ttggaagggc cacgcttctc ttacaggggt 75094
   gcccttgtgg ctgtatgagg agtccaagct ctgaggttga acactgggat tgaaggattc 75154
   ctgccagggt gcacttaggt ggctcacttg gcccagagtg agccagagct ggctcgcatc 75214
   agtgcgcggg agtcaatagt tacattttta ggaatttttt gagccagttg ttaaactgtg 75274
   ggtagcatgg aaatcagccc tggcttgagt aggctcgcat cagtgcgcgg gagtcaatag 75334
   ttacattttt aggaatgttt tgagccagtt gttaaactgt gggtagcatg gaaatcagcc 75394
   ctggcttgag tattcatgcc acggacttgg caaatgccgc aaatcagggc cttctctcta 75454
   ccccctccca gccccaaccc gaagacctga ttgttaactg cactggcaaa cccctggctc 75514
   tgcctcctgg agcctcaggt tccttatctg gaagcgggga gaataatgcc cacaccttaa 75574
   ggcatcatca acacccttaa atgagatcat gggtatgaaa tgcttgtgaa tagttttgag 75634
   tgtgatacgc aagtgtgagg ggtgctcacc accctggcca ttgccattgt cctcctcatg 75694
   gcagcagctg gacaatgtgc ccaggctggg agttggtgtc tccaggaaaa actggaggaa 75754
   aggagcccga taacccatgg gcagcagctg ggttagggag ggctccagac ctgcctatct 75814
   ccgtcagccc tgctgaatgt gcctctgttt gctcatcttt ataatgggtg cactgatccc 75874
   agctgagagg ggtgtggtgg gctttggggt acaggccaca aggtacaagg catggcctga 75934
   ctgcagctcc gtcactctca tggctcaggt gcatggctgt cccacagtgg aaggaccagg 75994
   ggctggcctg atctgcactt catggcatca agagcacagg ccctggagtc caccaggcac 76054
   agagaagcag cctgctcctg ggtggagtac aagttcaacc ctcgggagtt gtggtttccc 76114
   catgtggaag ctggcagcat gccacgcacc tgccagggct gccgcgaggg ttcgcacaca 76174
   ctgtgtgtaa tgctcctgtg tgagttcccc ggtgacccag gtcccactgg ggtggccggc 76234
   gtgtaaccag gacaggaaca tctccaaagc cactccttgt ttgctgcctg gtggctcaag 76294
   cagaacatct gtgcctgcag ggggcccata agccttaatg ccctgcagag agggggcctg 76354
   ggggaggctg ggactcacac cagctgggga acaaggcctg cagctggggg gctaccagga 76414
   tgctccttgc cagtaaagag ggtcctcagg agatcagcca cagctcaatc aggagggtct 76474
   gcccatttca caggggagga aagcgagcct ctggaggctg agctgccagc agtcacgggg 76534
   ccagcgcaca gccagagctg accactctgt atgactggcg ggtctgcgcc cttggcatac 76594
   cacagcagct ctcacaccaa cagaggctgg acctgctgat cagcccccgc tgtggggggt 76654
   cccgcctctg cccacaccct accccacacc ttcacagggg tggactgtca ccctctagca 76714
   gactgtcctc cagagaccga gacacggagc ctctccacaa ggagggagga aagggaggtg 76774
   gaaaccagcc ttaccctgga caccgggtgg tccttgctgc atacccaacc ctcattggtc 76834
   agactggtct gggagggcct ggacatgccc gcggggtccg tttctgaatt cccgttgcct 76894
   ggcataagct ctggcactta gtaggtcctc agttaatact tgtcagataa ataaatggcc 76954
   tcctttgggg aaatgaagaa tgtccaggct ggccacgggg aaggggcagt gttaggagtg 77014
   gagagcgaga gggtgagcca ggcagatggg acctttattc aaagggcagt ggggagccat 77074
   ggaaggtttt agggagggcc atcggatgag gttttcattt tagaaaagtc cttctggctg 77134
   ctgggtggat ggggtcaaga tgaagacagg aaatccagag aggaggcggc tgcaggctcg 77194
   cgggtgagag gtgaccatgg ccaggccagg gcggaggcag ggaggtgggc ggaggggggc 77254
   tgctggctct gccaactgtg accccagcac ccagctcagg gaattatggc cagagacaaa 77314
   gcagcttaga gcctgacccc tgccctggag cagataggcc tgggaagggg gctgatggac 77374
   acgctgatgt gatgggacct gagaggtgtt cttgtcacca ggccagtttg gcccccatcg 77434
   cccgcctgcc ggctggggtc taaaaggctg gctgtagccc cagcagagag gacagggcct 77494
   gtgggacccc aggtgctggg agcacagaag ccagccaggc catctggccc ggctgagact 77554
   caagtgatag gtccgcatca gtgtcaggcc tccatgcccc ccatcctggg atgggaacag 77614
   cacatcacag tttgcagagc cctgcggcag gggtgactct tattgccatt ttgcaaatgg 77674
   gaaaactgag acttagagtc tctctgactc acataagcag gacaggagac agtcgaacca 77734
   ggtcccccag ctgcaattta ccacatctca aacccttttc ctctctctgt cattgattaa 77794
   tctttgcttc aatgtccaca tagttctgtg atttgctttg ggtacatagt agcaataatt 77854
   atagcaacaa tttctagcag tttcttggtg ccagacactt ctgtctttgt tgcctcgttg 77914
   aatcttcacc atcaaccttg aatatgaaac ccattttaga gattaggaaa ctgaggctca 77974
   gagactgttt ataacttact gagaatcagg cagccccact gaggtgcttt accagtctcc 78034
   cctttaccag tggttctcaa accttggcac accacaggaa aacctgtcag aatgcagatt 78094
   ctaggcccca gcaattctgg tcctgtccgt ctgggcagag ccttggcaag cttcgtcttt 78154
   aggagagaag cccaggtgac tgggggtggg gctttccgga gcctgtgttg caagaaggaa 78214
   ggcagggagc atgtgatctt ctctcggtct ccctcacctt gaccctgtcc caaggccagc 78274
   tgctggcctc aggctgcctg gttgcctttg aaggtccatg gggatccgat ggagggttgg 78334
   tcgtgctgaa gccaggctgg gtgtcatcct ggctctgagc acagtccagc ctggctagag 78394
   tcaaacccag cagcttgacc ggatcctgct gaccacagac aaatccacaa ctaccccgtc 78454
   cacctccccc aggcccatct gcctctgtgc cagccaggct ggcagacact ccatcgggcg 78514
   ggccccacct ggcttggcca gggagaaact ggcagccaca gcccctggcc tccccgatcc 78574
   ctgtcccttg tgcactgccc atgggagggg cagtggcctt ccacgctggg tctggctcct 78634
   gcagtagcag agctgggtgc agacacgggt tggaggccgg ggccatgggg gcattcgtag 78694
   gctcatgagt cccttctcac tctcttctgg ggactgggtt tctgctggaa gagaaaggga 78754
   ccagccttaa aagacatcgt gattaaaatg gaattattat ttcagcctgg aaaaggaatg 78814
   atgtactgat ctgtgtcaca acatggatga accttgaaaa cattctgcga agtggaagaa 78874
   gccaggcaga ccccaaaggc cacgtgctgc tgtatgattc tatttatgca aaatatccag 78934
   agtaggcaaa atccatagaa acaggaagta gatcagtgat ttccgggggc tggggagagg 78994
   aggggatagg gaatggctgc cgacggatgt ggggtttctt tttggggtgg tgggaatgtt 79054
   ctgagtttct ttttggggcg gtgggaatgt tctggcatta gactgtggtg atggttctag 79114
   aattctgtga atctgttaaa atccaatcaa gtgtacattt aaaagtggtg aatttcatgt 79174
   tatgtatatt gtacctcaac tttagaaatg ctaaaataga aacaacaaaa tcatcatgat 79234
   gcaacgcctt tcctcataga cctggagcga ggcctgagag agaagccata taaggcagat 79294
   gaggggtggg atggagagct tcctttcatt cgctgcctgc ccagtgaggg tctgcttcct 79354
   ctggcactta cttaacaaac ccgagagtca cccatgtgtg aggcatgggc tgggcacaga 79414
   agggtccacg cccacccctg aaagtgctcg tccatgctag aaagacagaa ggatggggtg 79474
   gtcattgaac ctaagtcaga ccgtggcttc ccagctcatg cagaataaaa tgctcaggcc 79534
   tctcccggcc tgctgggtgc aggaccccgg cccagcccac ctctctgagc tctgagctca 79594
   tgtcctgccc catccctctc acacgcacac tggcctcctt gctggcctgc aacagatcaa 79654
   actcacccta cctcagggcc tttgctcgtg ctggtcacct gatatctgtg aggctccctc 79714
   tctcacatcc ttcaggtctc tgctcaaatg cgtctccccc aggaagtctt ccctggcctc 79774
   cccagctaaa acagcccctc ccacccctcc catgccattt accatactat agttttcatc 79834
   ttagccctta tatctccctc caagagctat ttattgacat gaaaatttgc ttattggctg 79894
   tctctcccat tcgagagtgg gctttctctg gggagtgtgc actgctgaat ttccagtgta 79954
   tgcaacagtg ctgaggagag ggtaggtgct cagttaatgc tttttaatta aattttctat 80014
   tgtggtagaa tatacatgac atagaattta ccatctgaac catttttaaa tgtacaatca 80074
   atgtcattaa gtatattcac gttgctgtgc acctgtcacc accatccatt tctgggactt 80134
   tctcatcatc ccagacagag actctgtacc tgttaaacaa gaactcgcca ttcattaata 80194
   ttcttttgaa tgactaattg aatgactaag tgtatttcct gtgtgtcggt cacaagctag 80254
   gtgatgagcc agtaaacctg gcgcctcgga gggtgtgtgt tcccgtatct gtctcggaga 80314
   tggacagatc gactcagaga ggtgtgaagg gctttgccca aggtcacaca gctggtcagg 80374
   gtgggattca aacgcaggtc tttccccctt tccacacagt ggcagttctg ggggatcact 80434
   gcatgcaaag ggcatagcca tggcacctcc aggcctccag ccccacccag ggcttcccct 80494
   gctgccgctc ccccaagggt cactgtgtcg gccacatgtt cctggtgtcc tcggctctgg 80554
   caggctgacc accagtcaca ggcacgtctt gggaccacac ttggaggttt cagcttccca 80614
   tttcacctgg tgctcttggg gtggaggccg ggatcatgga atcctttttc catggccttt 80674
   aacacaaatc aatactgtgc ccagtttaaa tgtttatgta actcatcatc tgccggctct 80734
   gctattacgg tgtgcttttc tttttataaa ccaagctcag gggaggtgat attaagtcca 80794
   ggacttgcct ggcgaggccc agcgcgtgtg cagaggattc aatgtggcaa taagggcgag 80854
   ttccctgcac agcgcctgcc atggctcgtc ccagcctcct ccctcatctg cttgtgtcct 80914
   ctacacggta gggaggaggg agaggtccag agaggggagg tggcttgccc agggtcacag 80974
   ccacaatgag gtggaacagg gattgtttcc aaggtgtgtg tgggggtggg acccaggctc 81034
   ctgccagagg ccagggcggc agagccctga caggcagatc ctgggttcct gggtgtccag 81094
   gttacgtggg tgggcaggtg gcagctggca cccaccccat acagacactc tctgacaccc 81154
   cccaaaaata ctcccaccag ccccttcaca ttttcctcca gccaagtgac caaagcacag 81214
   ctcagggccc atttgccctg gccacactgt ccccactctc cccacccaac ctctgaccac 81274
   aggcctggga gctgccccat ctccaccaag ttcaggctgc aggaggaaac gtctcaccag 81334
   gcccgctgga aggtggggtg gtgatgaaat gtgggggttc ttcagcgaga gtggaattca 81394
   gtaataaggt gatcatcact gccgggcggt gcctggactc atgatgagcc aggactaggt 81454
   gagtgcagag ggctggtgag caggcctgat ctgtgacccc ggtggccctg cggctctcag 81514
   acccctcaca taaaatccca tcagggatat tccgttccct ggcccggaat gaggtcttcg 81574
   tctttctgat cgcccttgga gagcccaacg gccttccgtc actcactcag tgctgatgtc 81634
   cagtcccggc aacagtctga ctccgcccag acttggcgct tcccctccca tgcgcccgcc 81694
   gcccggcgtg ctccctgctg gagggtgaca tggggagggg cctggctgca gcccttttca 81754
   ggcagctctt gttggctggg aagggagcct ggggatgggc cgccttctac ctgccggtgg 81814
   ctgtgcccac tctgagtgtg cttggtgggt gattacagct gctcacagct gtcaggagtc 81874
   cttcacaggg cagtctggaa gtttcctgta gagctgtctc ctgcagagtc cttacctagg 81934
   gagaccccgc tgtatgtcct gtggccagta gcatcctgtg gctttgctca atcccccagg 81994
   cacagtgtgt gcacccccag cctccctctg ctgtgttctc cacgcccctc tgggcagccg 82054
   gcactcagga cctaaggtgg tcctaagcag ctctcccctg tcccctggaa gtacgtggag 82114
   gtaaaagctg catccctggc agaatcagac cccaaactcc agaccctttt gctgtgagtc 82174
   tcaggctagg gtctcccacc tgcaggaatc attttgggcc ttttgagggt ctctttcata 82234
   gacgtgggtg gggcgtctgc tcagcacctg ctttctcctt caccaactcc tcagcctccc 82294
   atctctgacc ccataaacgc tgagaggaga accaggcctg gggggcaagt tcaaggactc 82354
   aggaggcaga ccgctggaat ccaatctctc ccctgccctt actagctgag cggctgttgg 82414
   caagttgtct tctctgtgct tcagtttctt catctgtgca atgggggtga taacagcaca 82474
   caagaacatc ttggagttgt taagaagatt cagtaatagg tgtaaaatgc taaagctggg 82534
   gcctggctcc caggaagtgc ccagaaggtg tactgtgata accacagacg ccacgtcacc 82594
   ccgctggacc tggactccgt gaggaggggg ctccccatct ccttatccca gtgtcagccc 82654
   gctgctgggt aaattcgctc gccaagccca taccaggtgc taatggctgc aggagcctct 82714
   tcctctccct gacatttccc ttcggcagca ggcggacccc tccttcctcg cccacctcca 82774
   tccttagcag ccctgatacc tctgacaatg ctaattaaat gcaacaacct catcagaagg 82834
   ctcgagctgg gcttgcctaa ggccccctct ctgccctgag ccctgatgga tcattaatgg 82894
   tgtcagcagc acttatggtg atggacgagg ggagcaagtc ccccgacgtg ctggggagga 82954
   agtccgtgac gagcatcttc atctcctgtc tgcaggcctc ggccagagtt tccagggaac 83014
   gtcctagtcc acagaatcgt ttgaatttca catttaccct gggagaacca tggccaggca 83074
   gggatgtcaa ttcccattgt atccatgagg cagtcagggc tcagagaggg caagagattt 83134
   gggtgagggc acacagctgg taagtagtgg ggctgggact ggattaccac ggctttgatg 83194
   aaagctgcag gggcatctgt taagatccaa gggcgatgtc tcccccaggt ggcacgtgga 83254
   gaagaaagcc agcaagtgcc accggcttgg gggtagccca gttctcgggg gctggagcct 83314
   gcacactctc atctcagctc tgtgtcagtt tcctagggaa gctgtgacaa agtgctacaa 83374
   actgcgtggc ttaaaacaac cgaaatttgt tctctcacag ttctggaggc cagaagtctg 83434
   caatcacgat gctggcaggg ctgggctccc tctgacggct ccagggagga ctctgttcca 83494
   ggcctcccca ccagcttcta gtggtggctg gcaatccttg gcatttccgg gcatttccag 83554
   gcattgggag gcccggctcg gccccccaaa gtgctgggat tacaggtgtg agccaccatg 83614
   cccgtcctgc ccttcattct tgatgcctcc cttcagcctc tgcctctgtc tccccatggc 83674
   ctcctccctg tatctgtgtg tgctgtgtct tcatatggac ttcttatcag gataccagtc 83734
   cttagatcta gggcccacca taatccagta tgaccgcacc ttaacttgat catctgcaaa 83794
   gactctattt ccaaataagt tcccattcat aggtaccagg ggttaggatt ttaacatctt 83854
   tttgtgggat gcagttcaac ccacgatacc atctttgccc atctctggct ctggcgtgca 83914
   gggacctgtt actgggcggg tcactaggga taccacctct cacctctctg ggcacccatt 83974
   ggtaggcctg tgctgtgccc agcaccaggg ggctggctga ggcctccttg tycagtggct 84034
   cctgtgtgcc tctccacttg ggggttgccc tagagctgcc caggaaggga ggaggcacct 84094
   caggggggga aggacagcat gagtttccat gggcttctcg tcacccacac agagtgactt 84154
   ggaataatgc acccagtggc tgatctgggg ccctgagctc gaggctgcag ccagtggcag 84214
   ctccaggccc tgcagtgtgg ggacagcgat ggccaaaaag agcacatgcc aaaggccctg 84274
   ccacttaggg gactttggga aagtttgacc aagtttcctt gtctctaagt aaggaatgat 84334
   aattggtcga tgggattgtt ctgagatgaa agtgctggtc cttgggaaag cttcagctga 84394
   aatgtctttt tattttgccc ttcactcttt tttttttttc ttcactgttg tcgcccaggc 84454
   tggagtgcaa tggtgcaatc tcggctcatt gcaacctcca ccacctctca ggttcaagca 84514
   attctcctgc ctcagcctcc cagatagctg ggattacagg cgcccgccac catgcccgga 84574
   taattttttt gtatttttag tagagacagg gtttcatcat gttggctagg ctggtctcga 84634
   tctcctgagc tcaggtgatc cacccggctt ggactcccaa agtgctggga ttacaggtgt 84694
   gagccactgt gcccggcctg cccttcattc ttgacgaata tttcgactga attcagaatt 84754
   ctgggtttcc aggttttaac attttctttc ggcacattaa aagagtcttc tgggtgccat 84814
   tatttctgct gacaagtcag cagtctctga gtcctggtgt ctctgaatgt aatatgttgt 84874
   ttttattcta gctgctttaa cattttctca ttatttcagt ttccagtagt ttgcagtttg 84934
   accaggatgt acctgggtat aattttcctt gtgcttatcc tgcttggagt tagctaagct 84994
   tcttaaattt gtaaattgat gtcttttacc aaattgagga atttttcggt cattattttt 85054
   ttatgtgttc tttttagtat atcttctctc tcccctccct ctacaactcc aatcacatgt 85114
   gtgttgaacc ttttgatatg caacaggtct ttgaggatct ttttcttttt aaaaaaattt 85174
   tttttctctc tattcttcag actgggtaat ttctattgaa atcatcttca agttcactaa 85234
   ctcttctatt ttctcagact ttctgttaag caatttctgt taaggtgtga ttttttttct 85294
   gtctcagaca tggtattttt aagttataga attttcattt agttcttttt taaattgttt 85354
   atatttctct gctgagattt attctttcat gaagtatgag ccaattttct ttacgtcttt 85414
   taacacagtt atcatagcaa gtttaaaaat ctctatttgc taattgcagc ggccagcaca 85474
   tttcaggtga gtttccactg actgtctttt atattaagta tggctggttt tcctatttct 85534
   ttgtatatct tcatataatt ttggatgata tcctgggtgt tgtgaatgat agcataagag 85594
   cgactctaga tttttgttac ttctccccaa agagcatttt tgttttgctt tgtatttgca 85654
   agcagttaac ttggctgaaa gcaactgtaa accatctgca gtcgtgagtg gtgggcagtg 85714
   gcaggagcct ctgttcactt cttctagccc tggctgggtt gcagctgctg gaagtctgtc 85774
   ctgtgtgtgt gtggtttggg gtcagcccaa gacatgggca gagtttattt gccccctctg 85834
   gggatctgtc ttggtggctc tcttcattcc agaatttact cccacatttt tccagctgct 85894
   gcgagtgtct cagactctgt ttgctgctct atcaagccag tgagactctg gctttccgtc 85954
   tgagttctgg cagccctatg acacaggctg ggacttgtcc tcaggctgtg aaagccatga 86014
   gaccaggaaa gccaacgccg gctgttcctc ccaattgctg accgcctgca gcatctgctg 86074
   cggcctcacc ctccgatgcc ttcagagtgt cattttgttg ttgttttttc aagggtttat 86134
   agtctatagt tatctgtgag agagttgctg cactaggagc tactaggcta ttaccggaag 86194
   catttagagc agggcctggc agacagtaat cacttaagaa atgtgagtta atgtggtcaa 86254
   ccatagtgcg gcgctgtttc ctctggtcaa ggctagagac aacgtggaca cagacagcac 86314
   catgcaggaa gtgtcagtac ctagtgaaga gcacacaatg gccttaagac tgcacttgta 86374
   ggccgggcgc ggtggctcac acctgtaatc ccagcacttt gggaggtcga ggtgggcaga 86434
   tcacttgagg tcaggagttt accaacctga ccaatatggt gaaaccccat ctctactaaa 86494
   aatacaaaaa ttagctgggt gtggtggcag gtgcctgtaa tcccaactac tcgggaggct 86554
   gaggcacgag aatcacttga acctaggagg tacaggttgc agtgggccaa gattgcaccg 86614
   ctgcactcca tcctgggtga caaagtgaga ctctgtctca aaaaaataaa taaataaaat 86674
   aaaagcctgc agttgtagga gagaccactt aactggggga gggggaaagg ggactatcca 86734
   ggaaggcttc ctggaggagg tggtattgga gctggaaatg tcagataatc caagatgaga 86794
   agatggactt tcttcacaga ggtggctgcc aaaacaacca attttatggc cttactgatg 86854
   agaatttctg gcactcaaga gaggcaactt ttcctaaggc aaatcctgaa gacatttacc 86914
   tataaaaatg agtagacatt aaacatccac tagctccttc atgggccaga ggaagcagtg 86974
   ctcaggcaag aggggatctg aggtctggtg gtggttctgt agatgtttgc agggagcgac 87034
   tggcgggact ggtaggcggg accatccaca gtgaggttgg gcgcccagat ccatcgccac 87094
   attgcagagc ttgtggctgt caattctgtg gcagccagga gcacaggtga ccgggagctt 87154
   tagtttctgt ccagtacatt ttggtgattc attattcctg gagtcctcgt cttctcttcc 87214
   tccctcccag accctctcac ctccttgtct cacttgaacc tctaatcctg ggaaggctcc 87274
   accataagca cactacagat aatgaaacca aggctcagag aagttgtgtg gcttacccaa 87334
   gaccacacag ctgaagcact ggagccaaga ctccaaccag cacttctgtc tgcaaagagc 87394
   tcatggcgta gtctctcatg gagggagcct ccatctcctc tgctgccaaa tggaaatcac 87454
   agtagtgtct cccgcagggg ctggcgtgag gttaaaggag gtagcagata tgaaaatgac 87514
   atataaattg taaattgccc acagacatta gtgtgtgttg cctggtaacc acccagcatt 87574
   tgtggactgc atttacttgg gatagcaata gttatattgg cattttaatt taatagcatt 87634
   gttatgttcc catcataaaa ataatataca ctgtgtgtat gcccaccctc atcaaattat 87694
   atacattaag tatgtgcagc gttttatata ttgattgtac ctcaatacag ctattaaaaa 87754
   atgatgtaac ttcattatag aaaatctaga aaatatggac aagttgaaag aggaggaaat 87814
   attattttgt tccagctacc ctaaagctac ctccgttcac attttggaga attctcttcc 87874
   agcttttttc cccactgccc agtttctttg cttatttcac atggctataa tcactgatgt 87934
   agatcattac aagcatttta tacccaattt tatatcccgt tccttcaccc tcagggggtt 87994
   ttaatatgaa atagcaagaa ccagcgcccc atcggggacc ctagcacagc tgggctgggt 88054
   ggttctggct tctgcccaga tgtgtctggg tgtccctggg ccagggcagg ctgccccacg 88114
   gagggacaat gtcaggacag ctgggacctg accctgttct gactccatgc ccggtgggcg 88174
   tttccgtgtt cccacagagc tgcttttctt atcttgtctt tttaagttca gtgacatgtg 88234
   catctgtctt gatggatgga tggatacgtc cagcccgtca tcccacttct aaccaccctc 88294
   tctcaatctt ttctgttgcc tcaaagctgg cattgactca tggctcagaa ggccagggca 88354
   ggccaggagc tgtcctaaaa ggcccagact ggaaaaagaa aaggcatttc ttggatagat 88414
   atctctggcc tggctactgc atacaggcac catttgaagc cgtgaattaa gcctgaggct 88474
   cctgccctca tgaagtttac gttctggcca ggaagagagc ctacagatga taaaagcctt 88534
   tacagaaaga aaaacatcag attaggttgg ggcagggatg gtcagggtgt cctgtatgag 88594
   ggtgctgggg tgcagacagc agtttcagca aggtgatcca agctgtgctc acgggaaggc 88654
   agcagtgggg cagaattgga agaataggaa ggagccatgc tggtacccag ggaaagccac 88714
   tgcaggccga gagagcaggt cttgggacag caggtggcca ggacttttgg gaaagggcaa 88774
   ggagcctggt gtttggaggg gaataagcag gtggagagca gtagatgagg ctggggaagg 88834
   ggaggaggta ccttttgctg agttaaaaac acgagacccc gtgcatacaa cagtaaatgt 88894
   tttcatcatc ttgcatctgc gattctgtgg tctgggcttg gctgggcttg ccctgctctg 88954
   tgggtcagtg gggccctgct ggaggctggg cttggccggg ggttgggggg gttcttctgc 89014
   acccactctt cctcctcccc ctcaggggcc agtgggccag cctgcgcagg tcctgctcct 89074
   ggtaccgcca gaggcgttga tggcaacccc cagtacctaa gcccctttgc atctctgttt 89134
   gcggcttgtc gaattgccgt tctgcaggcc aaagcaaaac acagggacaa gcccagaatc 89194
   aaggtggggg gcagagaaca ggtcacctgg cccaccatgg gaggggtgaa ggtttggctc 89254
   cagatgcggc ccacctgggc ctcggcaggt gggggctgcc tttagaacac tctgtgcttt 89314
   tctctgagta cacaatggga gccgctggag ggttttgagc aggggagtgc caggatcagt 89374
   cctgggtgtt accagcctct ctgctgccag tctgttgagg ctacgtcata gccagggaga 89434
   aagagctgtg gcagtcatcc agtgagaggt gggagagtgg ctggcatccc gatatgctgt 89494
   gaaggctgag caggcaggat tggatgtggg gtgtgtgtgt gtgtgtgtgt gtgtgtgtgt 89554
   gtgtgtgtgt gagagagaga gagagagaga gagagagaga gagagagaga gagagagaga 89614
   gagaacgaga aactccaagg cttgggcctg agcaagtgaa caatagagct gtcatcacct 89674
   gagactgggg aagctgcaga ggatgaggct gaggagggat cagaattcag ttatggtgac 89734
   caatgaaggg ttttgaatag agggacacag ggatgcactg gctcaggagt ttggtcatca 89794
   cgttggccgc cagagggaga acagaggctg ggacagggaa ccctgtggag tgtcctctct 89854
   gtggctacag ggagcaacgg tcagatctgt ggctggtttg aaagtagatc cattttgcta 89914
   aaggtggtat cacagatttt ggagactaca gaatccaggc caggagagca aagaacgaca 89974
   gaggtcccat tcacagcagc gggccatccc gggctcctac tagcttttgc tgcttgggcc 90034
   tatggggatg gtattctaga actttccatc ttccaagaca tactggaaat gtggattttt 90094
   ataggaaatc ttctactttt caaatattaa gaatgacttc aaattaaaag taataatcat 90154
   tttggggctg cagtaaaact tgttggtgga gcaggcctca ggcttcaggt tggtgagctg 90214
   tcctccagtg ggggtttctc ttccaggcta gggccttgtc ctcccctgct ctcccccact 90274
   cccagggctg gccacccaga atgtgcaggc cactcatctc tgtgactcgt agaacctgag 90334
   aatgttggtg atggaagggg tgttccccta gttcccacat gcaaaaagtg gggcctggag 90394
   atggggctgt cttttttctc caaacacttg cccatccagg aaggccagcc ctgggcattg 90454
   aagactccag gtatgggggt gactccataa ggagaggttc ctgagtgacc tgaagaaaca 90514
   tggttgggat gaagtgcagg gagggtcagg gcaggcaggg ctgagcttcg cttctgcgag 90574
   cagagattag agagcgatcc cagaggcctg cctggaggtg gggccacaga gcaggagtct 90634
   gaaggagagg gggtaattca cctctgtcct actgtgtggg aagccctgag tgggccccta 90694
   gcagagggtt gccttgcgct tatctatgga gatattggga agggcttccc tgacccaggt 90754
   tgctggagca tgggggcctg ggtggcaggg gtaggtgtgg gagattcaag acaggaggat  90814
   gaggccacat ccaggtggct tcagatacca ccgtgagtcc catagagtgt ccagttacta  90874
   taaagtctgc cagccactgt ggtcgcccca ggcctgtgcc aagtcccgag atctctgccc  90934
   tgcagccagt gcagagggaa gaggagggca ggaggggaca ggttggtggc ccctcgccga  90994
   tgctcctgac ccctcaggct ccctgctgcc tcctgcccag ggctgtgggg cctcggggag  91054
   cggggagctc gccattgctg tggcactgtt gagcacccgg gagaggcggg ttctgacagc  91114
   caccccttcc cgtcttgctg ttcccctccc ag ct gac atc atc tct acc gtt     91166
                                    Ala Asp Ile Ile Ser Thr Val
                                        25                  30
   gag ttc aac cac acg gga gag ctg ctg gcc aca ggt gac aag ggc ggc    91214
   Glu Phe Asn His Thr Gly Glu Leu Leu Ala Thr Gly Asp Lys Gly Gly
                   35                  40                  45
   cgg gtc gtc atc ttc cag cgg gaa cca gag gtgcgaagcc ctgggtctgg      91264
   Arg Val Val Ile Phe Gln Arg Glu Pro Glu
               50                  55
   tgggaggtgg gaggggtggg gagcagagtc aggtgggagg cgggcatggt gggaggggat  91324
   aaggataagg gggataaggg gtgatgcggt gagaggtggg aggggactat actccaaccc  91384
   acaagagggc ttttaactga caggaggtgt ggggtgtctt gcctggatca catctcccag  91444
   gggcatggat tccaggggtg ggaatgggat gctttggaat gccgaggtgg gagtggcggg  91504
   cggtggtagg gggagtgcac tgggtgggct ggcaaagcca ggttctgagc cccctgccct  91564
   cagccgatgt ttgcggagag ctgcctgcat cccatgctct gtcctagacc ctggatgaat  91624
   ggtcaggatg tggcacaggc agctgctgcc ccagcttgtg ccttcagtga aaccttcctc  91684
   tgcctgggtg acctgcgttg tcctggccag actggggagc gggaggactc cactcttgcc  91744
   cagctgtcac agccctcaga ctgagctagg tggtttttgc tcaggcctgg gcccttgtcc  91804
   tcacccctgt cctcaccccc acccccgggg gcttgttgtc cagcagaact ctgggtaaga  91864
   cagcagtggc ctggacaacc tgcccaggac cacgtttctg gaacactgaa agagcctcgg  91924
   gacaaaagcc catttactga gaacacttgc taagtattct ggagttgctt ctggtttact  91984
   ctgcactgct cacctccgag ggccagatcc acacccctgt gacagatgcg gaaactcaga  92044
   gaagccaccc agctagtgaa cagcagacct ggctgcccga cgccaggaca gtggccagca  92104
   acgtcacaca ccacccggcc tcagagaggg cgtctccccc cgcaaacacg gactgtgcct  92164
   ggaattagat gcagaagaca tccaggaaac caaggaggag ggaggagggg atggagcagg  92224
   agaggcaggg gaggggaggg gaggaggaga agagaaggtg gagaagggga ggtaggaggg  92284
   aggggaggaa gccgggggtg aagagaggac ctgcggaatc atctttcccc cgattcactc  92344
   ctgagcatgg cccacgaact gaggatcctt gagtctgact ttatcttggt ggctcacata  92404
   accctcctca cggtgaccct gtgggtgtag tgcagtgcgg ctactcccct cattttacag  92464
   aggaggagcc cagagtgcag agggttaaaa tagttgacaa ggggcacatc ctagctgcca  92524
   atcagttcct caaacacagc actcggccag cacctgtggg cttcaccaga tgcacctgtg  92584
   gggtgtccca ccaggcactg cgaagctcag ccttcttttt gccaagctgc agaaagagat  92644
   cggattggtt gatgcgattt tcttcatttt gaatttttct cctttagtgt tttcttcctt  92704
   ctagtcccac tgacctttgt catagcgagt gctcatggga agtgaagtga attagaggca  92764
   aaaatattgg agaacttcta agcttttcaa aagcaagaca gggatagaaa tatagatact  92824
   taaaaaaatt accaggaaat cttgaaattg ttctgggttg aatgtctccg tttctccctg  92884
   gttggggttt cgtgttgtcg tatgagttga actctctcct gggaacgtgg tccctggcgc  92944
   acacagtgga ctcaccacct gactatgctg ggagctccac ggtcaagggt ctttattaga  93004
   aggagctctg ctgcctaaac caggccaagc actgctgagg aaagccgggg acagattcca  93064
   atccaggtct gacattgcca gacattctgt cccaatgtta aagtctgctc ccaaattcat  93124
   ttattcctca agtcaggcct gagttggaca cgtggaatcc aggagcaagt cagggcccaa  93184
   gacacgaggg tggccagagc ctcaagcctt ggcgtcaggc aggcctcggc agaggggtgc  93244
   tcgccggaga gtgacctcac ggctctggga agtcacttcc cctctgagtc tctgtttgct  93304
   ggtctgtgaa atggggagag tcgtggtgct caagcagagg aggcctggag ggtagaatgc  93364
   gatggtgcgg ggaggactta gtggggtgtc cagcttgtag caggcgctgg ggaacattcg  93424
   cattagcaag ttagccggct cgggtgtgag tgtttcatgg gaccgcacgg gagcggggct  93484
   tgtccctggc acactgcaag tcatgggccg gttaagctgc agagagtttc atttgaccct  93544
   cgaattggat ccctggcacg gctcggcact tggtcccacg gccggccctg ctgggtcccc  93604
   ggaggtccta gccgtcgccc tgcaggtcac ggtgctcagg ccccttctcc gggtttccct  93664
   gcag agt aaa aat gcg ccc cac agc cag ggc gaa tac gac gtg tac agc   93713
        Ser Lys Asn Ala Pro His Ser Gln Gly Glu Tyr Asp Val Tyr Ser
                    60                  65                  70
   act ttc cag agc cac gag ccg gag ttt gac tat ctc aag agc ctg gag    93761
   Thr Phe Gln Ser His Glu Pro Glu Phe Asp Tyr Leu Lys Ser Leu Glu
               75                  80                  85
   ata gag gag aag atc aac aag atc aag tgg ctc cca cag cag aac gcc    93809
   Ile Glu Glu Lys Ile Asn Lys Ile Lys Trp Leu Pro Gln Gln Asn Ala
           90                  95                  100
   gcc cac tca ctc ctg tcc acc aac g gtgaggcgct gcccggcctc            93854
   Ala His Ser Leu Leu Ser Thr Asn
       105                 110
   gcttgcatgg gcacaggccg tagatgtttc taccaaatgc tggtttgtat ttcacttata  93914
   ttgtgagcat ttttctatgt cttcagaacg cctagaaaat aatatccttt ttgagcagca  93974
   tataaatccc atgagtgagt atgccagacc ccctcagacg ctccctgtgt ctggacacag  94034
   ggctgccccc ctttttgagc agcatgtaaa tcccatgagt gagtatgcca gaccctctca  94094
   gaagctcccc atggccagac acagggctgc cccctgctgt gaatagagcc atccgagcca  94154
   tctttgtagg cctttgcttt ggggtgaggg agtgtgatgg acaaggatgc aggtcgcaga  94214
   cacgggggat atttgacaca aagtgaaccc ctcctcccca cgccgaatcc agacccctag  94274
   actggggctg tcaccgcggc cttgcggggc agtgggagga gccttggttt aggaccccgg  94334
   ccggcctctt aatcctcttg cctcagggag gagcatgtac ccctcaggca ccggaggctc 94394
   tcggcctgac gcctgcttcc caggctccac tctgagggag tggctggggc tgtcctgctt 94454
   ggctcaccat gggcctgggt cccccactca cgccccctgc ccatcctcct tcacctgggc 94514
   cccctgcgga cccaggcagc ctggggtcgg gtaggacaca cctggattct tttttttctt 94574
   tttttgagag acaagagtct tgctctgtca cccaggctgg ggtagagtgg cgcgatctct 94634
   gctcacggca acctccgcct cccgggttca agcaattttt ctgcctcagc ctcccgagta 94694
   gctggaatta caggcgtcca acaccatgcc cagctaattt ttgtattttt tagtagagac 94754
   ggggtttcac tacatgttgg ccaggttgat ctcgaactcc tgacctcggg tgatccgcct 94814
   gcctcagcct cccaaagtgc tgggattaca ggcatgagcc actgtgccca gccaggacac 94874
   acctggattc tgtccctgcc ctgcctccct tgggcagtga ccttgctttc ctgagcccca 94934
   gcagggtgga cacatctccc ttctggggtc cgtgaaatgg tgcccactca accccctgag 94994
   aacagtaacc gctgcacaag caccagccag gcttcgaggt gctgtcccca cgccttcccc 95054
   gaggacgtcc acgctcctca gcggggcctc caaggctctc ccgaacatgg ctaggcgcag 95114
   cttctgccag cccgcctgtg cctgaaggct atcctcgccc tgtacctggc caactccctc 95174
   ctcatcccat aagccttgct gggtgcccgc cttttctgac ctctccactg agccaggcat 95234
   ccctcctgtg acaccctggc tcctcttaac tccggttcac cccctctctg ggcacctgct 95294
   gttctgatgg tgttctcccg ccatgcacct caaaggcagg atctgtgtcc cctaaggggc 95354
   cctgcccagc aaatctgtca cctgagccag cagagcatga gagatcctcc cgaagggcag 95414
   gccctggggg acaggagcct cgtcctgtcc ccatgcacct gctccttttg aaaatattta 95474
   agaaggacgt gtcagccaca tgcggtggct catgcctgta atctcagcac tttgggaggc 95534
   tgagatggat ggattacttg agcccagaag tttgagacca gcctgggcaa cgtggtgaga 95594
   ctctatctct acaaaaaaat aaaatattag ccaggtgtgg tggtgagagc ctgtagtccc 95654
   agctactcag gaggctgaga ttgaaggatc atctgagacc ggtaaagtca aggctgtagt 95714
   gagctgagat ggtgccactg cactccagcc tgggtgatgt gagtgagacc ctgtctcaaa 95774
   agaaaaaaaa aaaaagacat gtcactttct tcctgcctct ctacagaaaa ggtcatcttt 95834
   cagcactccc tacactccgc tcccctcaaa gcttcccacc tgggtttgaa tcctggcttc 95894
   actctttcct aatggcaggg ccttgggtac ctttcagatg ctccactgac cccagttctc 95954
   agttctcttc ccagttcctt ctcatcaaca tggtgctatt tgttttgact cctgtagtag 96014
   tcagggttct ccagagaaaa agaatcagta gggtatgaga gtgagaaaga gaaaggggga 96074
   tttattttaa ggaagtggct cacctgactg cggaggctgg ccgttccgaa atctgcaggg 96134
   aggccgaagg ctggaggcct cgagacagag ctgcagttct ggtccaaagc cgtctgttgg 96194
   cagaaccccc tccttcccca ggaggtgcct catgatttca ccttctaaag tggatcttct 96254
   cttccatccg gctgcttaat tatgtgactc gctttatttt gttctgtttt taaag at 96311
   Asp
   aaa act atc aaa tta tgg aag att acc gaa cga gat aaa agg ccc gaa      96359
   Lys Thr Ile Lys Leu Trp Lys Ile Thr Glu Arg Asp Lys Arg Pro Glu
           115                 120                 125
   gga tac aac ctg aag gat gaa gag ggg aaa ctt aag gac ctg tcc acg      96407
   Gly Tyr Asn Leu Lys Asp Glu Glu Gly Lys Leu Lys Asp Leu Ser Thr
       130                 135                 140
   gtg acg tca ctg cag gtgagctccg gtgaggggga agcaggcaca cgcctcttta      96462
   Val Thr Ser Leu Gln
   145
   ttacacctga ggattttagg gctggaaaag cctttgagat ttgagccaga gtcaggtgca   96522
   gaccctggtt gggctgctgg ctgctgggcg gccacgggcg tcttcctgcc ccttgcctca   96582
   gtttcctcac cagcagcata cagatgacca cgtttcttct aggcttcttg tgagcatgca   96642
   ggggtggtgt atggctgtaa agtgcttttc acctatagct gagggtggag agcaaggcag   96702
   ataatccctt gttataagaa gggggaaact gaggcccaga gagagacagc aactcaccta   96762
   aaactgctca gctgagtgag taggcagagg cagaaagaga atgggtcagc agagctttgt   96822
   ggctcctgag tcctttactg agggcagaag gaagcctgga ccgtgtgagg ccttcagcat   96882
   ctacaaaggc ctgaaacaga cataccctga aacagacaca cacgtgccca gcatccagtg    96942
   agccgccata aatataaagc agtagtcagt gctcttcctg agaagaagaa ataaatgttt   97002
   aaatacattg gcctaaatga ctggcaaatt gcacaaataa attctgagga agtgggcagc   97062
   cgattagctt cgatgttgag taatgagggt gctggtgccc cctgccccac cccccgtccc   97122
   aagaaagttt atgagggttt catctagagg gaaggataca gcgatcacat cttgatcacc   97182
   ctggtgtttg gggcagagcc agtggccttc ataaaaatcc tgtttatgtc cctcccagcg   97242
   ttggagattt ttctctgtaa gtcacacaca tcccaagaca cttaactgtc actgagaacc   97302
   tgggtgccct ggtctgtcct catcctgcct ctcaccttgt ttgcttccga tttcacccac   97362
   gccacgccaa ccctgcacag cattctaccc accctgcgca gttcacactg gaggcagctg   97422
   tttactgagg agctgctcca cgctaggccc cgggctagcc cttggggccc tagaggtgag   97482
   caaggttcaa ctcagtacct gtccccaaag tgtccccagc ccccaaggaa acagacatgg   97542
   aatcgccatc acactggcgc tcagtgcggt gacagaggaa cacagagagg ccgtgggcac   97602
   cccaggaggc ctttctctac tttgtcaatt tggtgaactc ccatacatcc cataaaaccc   97662
   tactggcctt cacctctgta aagcctttct gcgcaggcca gatgacaatt gtgatctttc   97722
   ctgtgtcccc tcttgccttt tggatccgac actgtctgtc cttagtgtct gtatcttcct   97782
   tgcccctcat cttgactaga ctgtggcttc tgcggaggca gcatgtttta ctcatgaaac   97842
   ccccaaacct tcagggaagg tttcctttct ctcttcttct ctttactaaa aaactggttt   97902
   tgagctctgg tttggtggat ggcagcaaat ggctggagca ctggatgaac tgggtcccaa   97962
   ggccgcctgg agaaaacaga aagcaatgtt gccggaagga agcaggaggt ggactgagat   98022
   gccaagactt tgctgggcca gtgatgctct gcccttctcc agagcagctg ggctccctct   98082
   ctctcctgag agtccaggga tccccagggt gggcagtggc tgcaccccga aggaagggaa   98142
   ggttagcagc atagctaaaa tatctgacac aggaatacgt gcagggagga tgccttggag  98202
   gagtnnnnnc acactaacac caactcccag gcacacaacc tagaagcaaa gacacagagg  98262
   caacaatgca agaaccaccc agagccaggc tgtcactcac accggtgctg cgtgcacaca  98322
   cacacacaca cacacacaca catgcatgca catacacgta tactcagtca tgcatgcaca  98382
   ctcacatgca cactcatatg cacacactca tacatgaact ctcagccatg cacactccca  98442
   cacacacagg ctgggtgccc tggtgtgtgg gaaaatttaa cgtgggctgc agagctgcct  98502
   ctgctccaaa ggagctcagt gcttgtcact ccgactgcag caccgaggct gtcacctccc  98562
   atctcagctg ttccctgaga gctcagggac acagcccatt cattcattct ctcacacatt  98622
   cactcattca ctggagagtc tctctgctag gccttggact gagcgctaga gagatggaga  98682
   gataaacaag aggtgacgcc ttgctcccgt agctgatccc tagtccactg ggaaagctga  98742
   tgggaccagt tgacagcctc agggttggtc agggctgtgc aagggacctt cagggctgtg  98802
   aaagctcagt gtctgatgcc atggcaagga ctcctcgcac caactgtctg agatgaaagt  98862
   tcgtatgagt caggctaact ccaccgctgt aacaaaacaa caccagcagt ccagggccga  98922
   gcacggaaat gcatttctca ctatgggaag gttcgatgtg gatgtgctgg ttgggacaca  98982
   gctctcctgc aggatccttc catccccatc ccatgagtct cagtccctaa ggcttgggtc  99042
   ctcaccttcc aggtggggaa ggtgctcccg ctgcttagct gcatcagggc caggtatcac  99102
   tttgctcaca tcccattggt cagaactagt catgtggcca tgtctgggca caaaggctgc  99162
   tgggaaatgt agtccacatg tagacagctg tttcccagtg acagcactgc attgcaggag  99222
   gaggtgagga ggcctggcag acaattggct ggctctgcca cagtatcttt ttgtttcatt  99282
   ttagagttga ggaaattggg gtataggagg gttcagccac ttattcacag acccacagca  99342
   ggtaagcatc catgcctctc ttgcagggct ggtgggcggc gggcatttgc catctgtcac  99402
   tgcctatttc agaccacgga ggatcaggat ggacgcccac ccctgtgttc cagcctccgg  99462
   ttgggatcac atgctcacgg catgctatgt cctgatgcca ccrcctggcc ttcacctctt  99522
   ccctgcctcc tcatcctctg cag gtg cca gtg ctg aag ccc atg gat ctg atg  99575
                             Val Pro Val Leu Lys Pro Met Asp Leu Met
                             150                 155
   gtg gag gtg agc cct cgg agg atc ttt gcc aat ggc cac acc tac cac  99623
   Val Glu Val Ser Pro Arg Arg Ile Phe Ala Asn Gly His Thr Tyr His
   160                 165                 170                 175
   atc aac tcc atc tcc gtc aac agt gac tgc gag acc tac atg tcg gcg  99671
   Ile Asn Ser Ile Ser Val Asn Ser Asp Cys Glu Thr Tyr Met Ser Ala
                   180                 185                 190
   gat gac ctg cgc atc aac ctc tgg cac ctg gcc atc acc gac agg agc  99719
   Asp Asp Leu Arg Ile Asn Leu Trp His Leu Ala Ile Thr Asp Arg Ser
               195                 200                 205
   ttc a gtatccttca ctgtggcctn ggccagtgcc tcccacgggc agagtagctt  99773
   Phe
   ccgttgggtg gtgggtttgg tttgattggc agacagctgg tttggggatg gctgcattgt  99833
   ttaacttctt cagtgaggca cctctggctc ctagtatgcg tgtgaggccc agatacaaaa 99893
   tcatgtcacg tctgtttctg aaaaccgcaa agtcgtggtt gctgagcatt gcacccatcg 99953
   cctcctccag catggccatg atcccctcat cctagggcct cacaaggggc caggaaggag 100013
   acagagttca gggttcagct gctctggacg agggactgct ggccttgctc agcgtccact 100073
   gaaggcgcct cggggccctc cccactggga cccaaggcag gctgtgttag cataggagga 100133
   ccagcactgg ggccccggcc agggcttcgg gtgacaacca gggtgtcaga agcccagctg 100193
   ggttggggtc ctgagggccc cctgctcggt gttctcctgt gtcagggcaa gctaggggaa 100253
   gcagcagcat tgacgattcc cgccgactgc tgggaagaga gctggtgatg acatgagcac 100313
   cagcctgcag gcagggcgag gcttggcctg ccggggggct ggtgggcggg caggcaggtt 100373
   gctgtgatgt cactattctg catacaacca atgataataa taattataat aacacacagt 100433
   gagactctgt gtgccaggct ctgtgactaa catcaacaag cattcatttt aactcatttc 100493
   accttcacaa caaccctatg aggtaggtac tattctaagt ctcctttaca gatgaggaaa 100553
   ctgagtcaca gagcactcaa gttaacttgc tcaaagctca cagccagcaa gtgtcaaagc 100613
   tgggcctgaa acccaggccc tcaggcccca gagtccccgc ttctaacctc cataccacac 100673
   tgccctttgt ggaggatgcc acccaggtgt gtcttggaag gggtgggaag accctcctga 100733
   gagcctccat gcaactggat ggcctcccga ctccaggcag cctgggcacc cagcagtggt 100793
   caggagtggg ctgtgtccct cggagcagta gaagccggga tagctgtggg gagcagggga 100853
   ggcagggagc attccaggaa gggtgaaaga gagtaacaaa tggcttttgt ggtctgagat 100913
   ccaggcacag ccagagaccc ctgggcaggg agacccttgg aaaacagcgg gaagggaaca 100973
   gagcaaagcg ttggatgtcc cctgagccag ccagagggtg cggggcacat gtgtgggctt 101033
   cggaggcaga aagtctgttt gggtctgact gccaccttca agctttgtga ccctgggtga 101093
   gtacactcac ctccctctcc gagactccgt cttttcaatc atgcctcctc atagacctgt 101153
   ggggtgccgt cagtgagccc ctatgtgtga tgtgccgaac cctaagtcag cacttggtgg 101213
   gtatcaggaa gcatcagcat ctctctccct tctcttgtcc caaaggcatg gagtgcccag 101273
   agctgggagg ctggattggg ccagccagga aggttccagg aaggattggg acctgagctg 101333
   ctttgaaggc ttgaagctca gatttctctg gacatagagg gctgggctgg acatctgtct 101393
   gaccctccac tgaagccgaa aggacatcgg gccccaccag tccacagtgc tcatagcagc 101453
   agttggttag actctttctg ctgcacgagc atgtttgatg tggataactg taagagagct 101513
   gcgggccagg tggcttcagg ggctggagca tcgtgggtga tggctctctg ggctgattgc 101573
   ctctgctgtc ttctgtgttt caatctcagg tgccagtagt tggggcccca ccctcccaga 101633
   ataaagtcca ggagaaacga gaccagttct ctctgaactg tcccactagg accctaaatg 101693
   gcatcttgtg ggctctaatt gggggtgaat gccttgttgt gattggccag gccggactcc 101753
   tgtacctctc cctggtgctg gagggagtca gctccgcccc caacccctgg atgtggggga 101813
   ggggcggtct cctagcagga gctccaggtc tgcaaatggg gagggtgtgg ggctcactga 101873
   gcggcctgga ccgtaccgta cagtttacaa atcagcctgc cgttcgttgt ctcacttggt 101933
   gctcagagac agtggtaatt tgatgatcac cccatttcac agatgaggaa acggaggctg 101993
   ggggagatga gggactggcc cagagtcccc agttggcagg ggcagagcca gagctcatgc 102053
   tcgggcctcc atgcctggtc cagggctttt gccctggcct cggcctgccc cctgcagccc 102113
   tgggacagtg gcagccaccc ttctcgagca cctgcttcct tcctgccgga cttgggtggg 102173
   gatggtaatt gctgctcggt tttcctgtct gggctgctgt gaggattccg tgacactatg 102233
   gatgccgaaa ccctttctaa agaaccataa agccctagag tatggaaggg atgctgcttc 102293
   atttcattta ttcaaccatt cattccccac gcctttctac tcccaggccc acactgagcc 102353
   ctagggacat ggggatgaat gggagaaact caacccttcc ctggagggct caggggctgc 102413
   tgggggacac agccccgcca tggcagtgga agtgagggag tcatccttgc tgtagcccag 102473
   ggtctggaca agctgctgtg gggccgccca aggaagccag agaaggaatt tcggtaggaa 102533
   gctccctggg ctcgaggagg ctgcctgagg gcatgggtgt gcaggaggcg gatgcatgag 102593
   gaggcagaag gtgccggagc tgtggcactg tggatggtgc aggcaggggg ctttctcttg 102653
   gggtaggggc cccgaatgcc agtcattcac aaaacccccc acagctgtgc ctgcctccac 102713
   atgccacgtg tgcttccctt tacttttccc atttaaaaaa aattgttttc tttaagtcaa 102773
   gttgctttat ttttgccgaa gtaattttct tttgaaaggg aattgaattt caccatgata 102833
   agttaaagta tcacatacca taaataggag gtagcctcaa ccataacggc tgtgcacaca 102893
   aaatggtggg aataaaatac aggctcagcc ctgtctgttg gaactttctg tggtagtaga 102953
   ctgcttcgtc tgcactcatg gggcggcagc caccagcccc atgtgcctgc tgagtgccta 103013
   aaacgtggct tggtgactga ggagcgacat cgttaatttg atttagttcc agttaatcat 103073
   acttcaaacg aaatagccac atgtggccag tggctactga acaaggacgc tgcagcctct 103133
   ctccaggttg cccttagacc ctgaagctgg ttctctctga aaagatggag gtaaacatgt 103193
   gcttcaatgt taagggggag agtgcaaaac agagacatcc cccttcattc atcagaagga 103253
   ttgaatcctg tctccccgtc tgcatctata ttacattact ggaggctgtg ccgacttccc 103313
   acctgcctgc ttccagggaa cgggaaacta tgaagctatt gatgtactgt aaacaggggc 103373
   gtgtcactag ccagctttgg aggtgtgtat gtcgtgggtg tgtgtcgtgg gtggattgga 103433
   ttccagcaga caaggctggc ggcttgggaa ccagcaagga gacccgggga gagagagtga 103493
   gaggcagggc cagagcgtgg ccacgggtgg gaggcggtgg aggaatgaga ttgtaaggat 103553
   ggaggtgtca tagatttgga ttgtatcata accagttttg gatcggggtg gccacaagac 103613
   attcagacag gttggctgta aagaagaaaa gggggttggt tcatcccagg actgcaaagt 103673
   cctccctggc cgttaccgtg agccttgcct cctgtcccgt cccgaacaca cagattgtgc 103733
   cctgtccgca tgttgcagag cacaccattt accagcaggt atttattaag cacctactgt 103793
   gtgccagccc tcttgggtga tgctggaact agataaagat tctgcctcca ggagtggaca 103853
   cgtgataaac cattggtaaa atacccggca ttggagcagg tggtccgtgg gtgctacagg 103913
   gagtcggtgg cccagccagc gcagccgtgt acatttctct ccagccacgc ctgctgctgg 103973
   ctgcaggcct caggtcggtg gagggtaagc caggggtgtg gtttttgcca agtgaggatg 104033
   tagacacgga gctgatggtg tgtgcaagag agtgaaaata atgattattg accaaagaat 104093
   ctgagccagg tgagggacag tgacaaggga ggagtgtcag cagatcgggg gcccctgtgg 104153
   atcctgggaa catccctggc cgcacaccct gccgtgtaac tcagtgtcag atctctgtgt 104213
   aacttcccat tctgtgaaaa tgattattaa ttcatcaaaa cataaagcac cctggcctta 104273
   gtgatagagg agtgtgtgtc tatagcatga acaagattct gagctgggct tgaggcagcc 104333
   ctgatagggc aacttaggca tttggcagga gttgagtgga gtctccaggg gcacatggtg 104393
   gccatggaaa cctccccgat tggatctatt cattggggaa cctcagtgca cctggtgctc 104453
   cccatggcct gttgccgctt ggctccccat ggggtgctgc tgcagtgaca agacacagat 104513
   ccagggctca caccacagag agaagcctta cttggcagag aaagtgtgag gactgtgggc 104573
   tgcaggtgct gcgatgagga ctgatagcag ccgagtaatg agaggaggcg tttcatttct 104633
   gacatgggag gaggccagaa aggcagctga gaccccatgc ggagctggga attgctctga 104693
   atgtttgaga taccttctaa gcaagtctca gtggggtgga ttctggctgt cagagggtga 104753
   cagttctgat gagtcctgac aagaaaaacc cagggtcagg aggcctgtgt ccgcgcagag 104813
   ccaggggaag gcttgggctg gacttggggt ttgcatcctt ctgacccctg ctctctgggg 104873
   tcctcactcg gcccgatgac actgttcagt cctccgtgcc cactggaggt tttccaccat 104933
   gtcgcatctc ctaagtccaa tcactaacac ttcccgtctc agcaaaatgg acttttagta 104993
   accatatcaa gagaaaggaa aatgtttctt agtaaaggag aaaaagaggg cattgctttg 105053
   actattggac aatgagaaag gtgactcaga ggacatctct gctggaggga gggagcctgg 105113
   tgatactgag gctctgtgac ctgtctcggt gtagtcacca aggttcaagg aagaggagcc 105173
   ctcccagcct cctccctctc tctcaccctc agaatggaaa gaggtgccca gccctctcta 105233
   ctctccccag acacttgcct gaatcccgcc ctgtcctgcc cagagccttc tgcacagctg 105293
   tgaaattggc tcatcggaac ctctggtctg accatgtcag ccccccttaa aactctcacg 105353
   ggttcccctc tggcttctgg aggaagcctg agcccctcca cagtgccctg tgcctttggt 105413
   gaccagcccc tgcccacctc tcgggtcaca ctggctcact ccttcagctg ctgggtccct 105473
   ggcatggggc agggtggggt ggagcactgc catccgccat cccttctcct gaccatgcca 105533
   tctgcctctt tggcctggct gactcctgtt tgcccacttg cagcatctca cagcccatta 105593
   tctgggacag cctctgacct gtccctgcca cacccctcct aggctggtca gagcccctct 105653
   aagcctgcgg ctccttggct gcccccggtc acactgagtg ttttgttact gtgacagcct 105713
   cctccatttt atggtggaag ccctgagatt aggaacccag ggtgaggcct ctctgcatcc 105773
   cctgctccac ataggacttg gcgataggag gtacttatga acggttgtag gtggagtggg 105833
   ctggaagttc accacttcca tgtggtctta ggatacagac acctcctttt tgatctgggc 105893
   gcctggggaa tcagagaccc cagaccctaa agtgcaggag aaggagcccg ggctttgggc 105953
   cctggcgggc ctgggctgag acccattgct cagcctgctg ggccatcagc ttccctgaat 106013
   ataaaatcag aaaattccct tttataagga tatttgtaag gaataaagaa atctttcaca 106073
   taaagagcct ggtgcctaat tggagcccag caagaggatg gtttattttg tctcttaaac 106133
   aacagccctt caaacattta agattggttc tcataaaatc cctgcatctc tgatggttaa 106193
   aactccgttt tccacggtgc agtttcaacg tctgtttaag caacctctcg cgtcctttaa 106253
   taccaacatt ttgagactgt taacacagca ctgggtgatt tttcttgagt ctgatgcatc 106313
   ttaaaggtaa caaattgcac aggaggtctc agcggatgga cctctgcttt atttttagca 106373
   cccagagaat ttaaaagacg gttcgattga tttttctctc ccttagtgat ttctcacggt 106433
   gggaactggc atcacaccaa gcctcggcct ggagaatttc taattcccat ttacagagca 106493
   ggtccgcacg gccaggtggg ctggaggggg gtgcagccat tcaccgcagg aggctcagaa 106553
   ccttcatcag cccatgcctg cctgaagact cagcttgtct tctgtgcaga gaacaaagga 106613
   gggagcagac acagtccctc tgcatgcaga ctctgtgctg ggccaaggga ccctgagccc 106673
   aggctctacc cctgcctctg ccactgaccc tgatcagtca ctcagtcttt ctgcaccccc 106733
   tttatcccat gggagtgtgt ctcctttacc aggagctgta aaggtggaat ggccgatgag 106793
   tatggggcct ggtgccgtgt agacaagcaa ccctcaccag cacatgaccc actgtcgggt 106853
   accttacagg ccacctcttc ttcctgccct tggtgctaat ttacagagaa ggaaactggg 106913
   atgccctgct cagaaatgct ctgttcacgg catgtcagat acataccact gatggctggg 106973
   agtctggacc cctgggttct tactccagct ctgccactac ctgcccacgg ttctgtgcat 107033
   tcattggttg attcagcagc attggccgag agtcttctct gtgttgagca ttggacttgg 107093
   tgctagggaa gcagacaggg tctctgccag ggcctgcgag tacagttgta tgggttgtct 107153
   gctgcccaaa agaggtgatg atccaagcca agcaccttgg gaagaaggga cttttttgtt 107213
   tttcagttct gcaaggaaga gctgtctttt cataattggc acaaaggcac tttctgagtg 107273
   aatggggatc ctgatgctat ccaggtgaac tttctaggtg agcaaaaaga tagtcttctt 107333
   cttgctcaag cctcgatgag tcctgtgatc ccatcgtcca aatgcaccca gagcccacct 107393
   gcctgtctgc ccccagcatg tgtttaggcc actgtctcac tctgatcatg atggtgacct 107453
   gactggcctc ccttcttagg tctaggagcc agactgccag ggctcagacc ttggccccaa 107513
   gacataggga ctccgcggcc tcgggcagct cacttgggtt ctctgtgcct cagctcctca 107573
   cctgtaaagc gaggaaaagc ccctgctgac accatacggc tgcttgagga ctgggtgcta 107633
   cgatgacatc tgggaagcct ttagggcagt gcctggcaaa tcaccgagct gcagcatcat 107693
   tgcttcatta gctgtgtttc cccaggtgtc tccttacagt ctgttctcct acaacatgag 107753
   aaaaaaaaaa acgacaagat catgtcactc cttgctcaga acccttcagc aacttctccc 107813
   aactggaagt aactccgccc ctttctctgg cctccaaggc cctgcaggag ctggccctgg 107873
   cccctccctg gcccacttcc tcctctctcc ctttgctccc tgggctcagc gttcccggcc 107933
   ccctgctctt caccatattt ccagccatct cctgcagcag agccattttg cactggctgt 107993
   tccctccgcc tgtgaagttt ctccatcctc atccactcag tggacacctt cttaaagagg 108053
   cnttccttga ccagtctgtc ggaactagtc catcccctcc atgactccct cgagcccctt 108113
   ccctagaggc ttggcctcta cctcgttccc tgttcatttc ccgcctctcc ctcctggact 108173
   ggcagcttct tgagagcagg gaccttgttt gatctacctg gaataatgcc tggtccagag 108233
   tagatgctca aataatggaa ctagtgtaat acagtgagat gggtgttagg ataaggtggg 108293
   gaggaccccg gcctccttag aaagtccttg atggaaaggc attgacactg aggcttctgt 108353
   tctggacctg ccacgtggtc ccctggcagt ttgcttcacc ttcctggaca ccagtttccc 108413
   cttctcttgg ctggactggt gtcctgaaac tggctttcat ttagacagtt gggctatcct 108473
   tgccgggtct cccttgatct ggctatgtgt gaccttgtgt cttgccagct gctagcatga 108533
   cacaaggtca cacacagctt gtaacagcca ggtgtgcaca tctcttccca aatccatgtt 108593
   catggacatc tctttggcag cttgaatttg gccatggtgg tagtatttct ttctttttct 108653
   tttctttttt tttttgagat ggagactcac tgtgtcgccc aggctgaagt gcagtggctc 108713
   aatctcggct cactgcaatc tctgcctccc aggttcacgc cattctcctg cctcagcctc 108773
   ctgagtagct ggggctacag gtgcccgcca ccacgcccgg ctaatttttt gtatttttta 108833
   gtagagacgg ggtttcaccg tgttagccag gatggtctcg atctcctgac ctcgtgatcc 108893
   acctgccttg acctcccaaa gtgctgggtt tacaggcgtt agccaccgca cctggcctgg 108953
   tggtagtatt tctacagata tctacaaaca ctacaaaatc aatctctccc ctccccagaa 109013
   gagagggtat tgttcaagtt accagcacac aactgctctg gggccttcac aaggccaggc 109073
   agttggtcag tcagcctcca ncatgactga gggcttcctg cgtgccatgg cnctgtccat 109133
   ggtgctgacc ccgttgtgag ccattgcgtc gaggtcagca tctccacctc ccaccccact 109193
   gtcactactt gctgtgtcac acggtgttct gagcacgctg ccgatgttaa ctggttgaat 109253
   cctcccaaca acactatgtg gtaggcgtta ttaatatccc ccttttacag atgaggaaac 109313
   aaagccacag taagtttcag taatctaccc aaagtcaaag gggaaagggg gtgcagggga 109373
   aagggggtgc cagggctggg attcagattt aagctgtctg gatgcagaac catgcactta 109433
   actgtcatgc tacaccgcct ctcagtcatg atggtaacaa ccccagttgc agcctgcctg 109493
   gtgctggggt gaacaaatgc aagatgccgc tgttgcattt cagtctcatg accactcgcc 109553
   aggtgggggc tgtcactgtc ccaactcagc agaagtggag atgggcactg agaggtgggc 109613
   tcccttgccc aaggcccctc tgagccaagt gtccttctga tggcctgccy agctttgttg 109673
   agacagccgt atttcttggt tcttgcttta ggtcctgtgg aaagacaggc ccaattgcag 109733
   gatagatggg aggttgtttt gctatgagca tggctgtcag tagctgtatg gcagtcctca 109793
   cgtttgccct gcaggtggca tggtagaggt gtgcgaccct ggcctgcctc ctgtcccttc 109853
   ttgacagacc tccctggcat ttctagaact cgctccctgt taaaatcctc ttgaccccag 109913
   gcctgagccc cagacctttg gcctgcttcc ctcttcagga tgggtcctgc cactcccagc 109973
   cttatgtcag gacccctcca ccctcatagc tcatactcca tcaccctgga gcctctccct 110033
   ccctcttaga actcctctcc ttttggacag agcctatgct ccctctgagt tctgctggcg 110093
   ccgcatcctc tccagcctgg ctctcaccct ctctgcctcc ctccctcatg ctcagccacc 110153
   cagcctctca cacctctgag ccttgcactc cccctcttca accccctcac cccctccaac 110213
   acacacacac cccaaacatt ttcccaccca ctagctctgc cgggataatt cctactcacc 110273
   atgggcttcg gagggccctt ccctgactct gcaccatctc agggcctcgt tataaatctc 110333
   tgacatgagc acctgtgtct ctcttaggcc atcgatcgat catggagcta aatctgcttc 110393
   cctgccaggc tgggagcccc agcagtcagg gcggggtctg gtctgctcct tctcagtgct 110453
   gtgggggtac agctgcctgg gtgcacatcc cagctctgtc caaccttgga ttccccctct 110513
   cagttcttgg aaaccaaggc atgccattca ccacagaccc tcctagtcct cccagggggt 110573
   gacaagcact gtaccttttg catcctgctt agctgtatgc ttttttccca gagggagaga 110633
   aagactctgg gagttctaat ttaactaaat tcaatttaac tgtagccatg tgctgggtct 110693
   tttgacatat gtcatctggt gaaatcctca ttacagccct gcaaagcaga agttatcaac 110753
   ccattctaca gataagaaaa gcagagcaca gaaaggctga gttttcaaga gatcacacag 110813
   atgttaggca gtggggctgg actttcaagc tctgggtttt gtgattcaga agtcaggact 110873
   ttcttccttt ttcatagctt ggttgattcc tttctaagca cttatgaatc acctgaggtg 110933
   ggctatggca gagctgtgaa gatggactca ccacacagag agcgtgcagg gctcagtggc 110993
   ttgtgcagtg agcggtgaaa tagaacagca cctttcctac ggttccagca agctgggttc 111053
   tgctccctga gaggaattgc ataatcatta aggtgttccg agtggagaag gaaaggaggc 111113
   tgttacaggc tagattgtgt ctcctccatc cccattcata tgttgaaacc ctaactccca 111173
   gaacctcaga atgagactat atttggagac agggccttaa aggggtaatt aagttaaaat 111233
   aaggctggca gggtagaccc taatccaatc tgactggtgt tcttataaga agaggtgatt 111293
   agaacccaca gagagatgtt aagatgcagg aataagaatg tgaggacaca aggagaaggc 111353
   agtcaccttc aagccgagga gacgggcctc agaaggagcc agccctgcta acaccgtgtt 111413
   attggacttc ggacctccat agctatgaga aacacatact gtttaagcca ctcggtctgt 111473
   agcattttgc tatggcagcc aggaatcctc ctgtaggatc tgacatgcct tggtctgagt 111533
   gcttgcagct catgtttgta gtcaggtcat cttttggaga tttgctgaaa aagcagtttt 111593
   tcaagatgaa ctccatgtgt gggaggcctt ctagggcacc tccgcatccg gcgtccctgg 111653
   tgagctcatg cgtgagtcct tttgtggctc ttggcagggc tgcctccatc cctaactccg 111713
   gtggctgctt ggttgccatg gcgacagctt cctccatcct gagagcaggc tcctgccgag 111773
   acatctggat gagcctcact gtgctcggtg aaatcggtgg agcccaaggc tgcagccctc 111833
   tttctcatgg atccctgctc tgcacccagc atcccccttg ctgaggacac aagccagtcc 111893
   agccagcctg gcatcagggt ccttggggtg cccaccccat ggggaaagct gcagatggtg 111953
   tggaaatgcg tttactccag aacagtggga ggaacgctga ctggaagtta gggagccagg 112013
   atttcagtcc ttactctgcc agggctctgc atgtggccct gggcaaatca ctgcccaccc 112073
   tgggcctccc tctcctcctg ggtggggttg gcacatgggc agttcctgcc tgcaggggtg 112133
   gactatttga ctggtctgtg ctaggcatgg ctctctgggg ttattaccat gtctcaaagt 112193
   tcaattactt gctgtggttt ttagattagc ttgtcgtggg atctccactg gggcccatca 112253
   tgaggtgaac cagtatttgc acactgcttt tggcgtttgt ggagtgcttt gcatacttgc 112313
   ttctctcttg agctccttgc cagaaccaac cagtgatgct tcttcatttc acatacctgg 112373
   ctatatttcc agtcagcaga gatttgccct gtagaggagc gagcagccct tggttcctgt 112433
   gcccacctta caggagagga cgaccggggg gcagactggt gcagcttagc tgccatggct 112493
   cctggaggtg cagccacctc ctccagcctc acgtggggct ggtgtggctg agctcgcgtg 112553
   gctgggctcc aggagagcag gctgtgcctc tggtagcagg agatgaagga gtttcttttt 112613
   ttttttttta caggtgaaat aaattttaat gataaaatta ttttagtaat aaaaatctta 112673
   ataataaaat gtatttaact aaatataccc gaaatattat catttcaaca ttggcactag 112733
   ctacatttca agtgcttttt ttgttattta agttctaggg tacatgtgca caatgtgcat 112793
   gtttgttaca taggtataca tgtgccatgt tggtgtgctg cacccatcaa ctcgtcattt 112853
   acattaggta tttctcctaa tgctatccct cctccattcc cccactccat gacaggtcct 112913
   ggtgtgtgat gttccccgcc ctgtgtccga gtgttctcat tgttcacttc ccacctatga 112973
   gtgagaacat gtggagtttg attttctgtc cttggaatag tttgctcaga attatggttt 113033
   ccagcttcat ccatgtccct acaaaggaca tgaactcatc cttttttatg gctgcatagt 113093
   attcaatggt gtatatgtgc cacattttct taatccagtc tatcattgat ggacatttgg 113153
   gttggttcca agtctttgct attgtgaata gcgccgcaat aaacatacat gtgcatgtgt 113213
   ctttatagta gcatgattta taatcctttg ggtatatacc cagtaatggg attgctgggt 113273
   caaatggtat ttctagttct agatccttga ggaatcacca cactgttttc cacaatggtt 113333
   gaactagttt atagtcccac caacagtgta aaagcgttcc tatttctcca catcctctcc 113393
   agcacctgtt gtttcctgac tttttaatga tcgccattgt agctggtttg agatggtatc 113453
   tcattgtggt tttgatttgc atttctctga tgaccagtga tgatagcatc ttttcatgtg 113513
   tctgttggct gcataaatgt cttcttttga aaagtatctc ttcatatcct ttgcccactt 113573
   tttgatggtg ttgtttgatt ttttttttgt aaatttgttt aagttctttg tagattctgg 113633
   atattagccc tttctcagat gagtagattg caaaaatttt ctcccattcc gtaggttgcc 113693
   tgtttgctct gatggccatt tttttttttt ttttgctgtg cagaagctct ttagtttaat 113753
   tagatcccat ttgtctattt tggcttttgt tgccattgct tttggtgttt tagtcatgaa 113813
   gtccttgccc atgcctatgt cctgaatggt attgtctgga tttttttcta tggtttttct 113873
   ggttttaggt ctaacattta agtctttact ccatcttgaa ttaatttttg tataaggtgt 113933
   aagggaggga tccagtttca gctttctaca tatggctagc tggttttccc agcaccattt 113993
   attaaatagg gaatcctttc cccatttctt gtttttgtca ggtttgtcaa agatcagatg 114053
   gttgtagata tgtggtgtta tttctgaggc ctctgttctg ttccattggt ctatatctct 114113
   gttttggtac cagtaccatg ctgttttggt tactgtagct ttgtagcata gtttgaagtc 114173
   aggtagcgtg atgcctccag ctttgttctt ttgcttagga ttgtcttggg aatgcgggct 114233
   cttttttggt tccatatgaa ttttaaagta gttttttcca gttctgtgaa gaaagtcatt 114293
   agtagcttga tggggagatg aaggagtttc tataaaaacc tctgcatgcc cgaggactat 114353
   acgggaggcc tgtgtggatc acacctcctg tgtcctcgga agggatggct gcagacttca 114413
   ctcttgggtg gaagaaaccc cgctttgctg actcccccag gtgcaggttc tgagctcaca 114473
   ggggtggtct gaacagctgg gggcacccag cacccctacc cccacccacc agggtgagga 114533
   gctccttgta ctgtggatgg gggaccggga taggcccacc tgtccctcca gggctgcact 114593
   tgctccatct gacattgaac ctgggcctgt gtgcagtaaa gaagggaggc tgtgtgaccc 114653
   aagcaagact gcatcgcctc ctgtaggcct ggggctgtgg gcggcagggc aaatccactg 114713
   tgcgtggggc tttctgtgca catagccatc ctttgtttag ctagcacctc tggctggttt 114773
   tctgttacaa cagcagagtt gagtccttgc agtttcgata gaaatcctac gtctggctag 114833
   gcctgaaata ttgactctct gactctttgc agaaaacact tgccaacacc tgtgaatgtt 114893
   ggcactggaa ggaaaagggg gtccatttca agacatgggg ggctgaagcc agacaactgc 114953
   caggtccccg ggcccctcca gggacctgac agcccctcct tgcccagcac ctcgctgtcc 115013
   ctgtctcatg cccatgactg cagctgtgac tttctcctcc tgctccctga gcctcagatg 115073
   acacagagtc cagagaggct gagactgacc cgaggtgcca cagcagatga aagggggatt 115133
   tgaggctggg acccagggtc ccacctgaca gcctttccct gcccagcacc tcgctgtccc 115193
   tgtctcatgc ccatgactgc agctgtgact ttctcctcct gctccctgag cctcagaaga 115253
   cacagagtcc agagaggctg agactggccc gaggtgccac agcagatgaa agggggattt 115313
   gaggctggga cccagggtcc cacctgacag cccctccctg cccagcacct cactgtccct 115373
   gtctcatgcc ggtggctgca gctgtgactt tctcctcctg ctccctgagc ctcagatgac 115433
   gcagagtcca gagaggctga gaggctagcc cgaggctcca cagcagatga aaggtggatt 115493
   tgaggctgga acccagggtc cctggcagcc aggcagaaca ggccgcagac cttctcagca 115553
   gctcacctac agagcgccca ctctgagcct ggtcctgttc tcagcgcttc acgtggacta 115613
   ccttacccca tcctcatctg ggagctgcag agtgcaattg cccttctgaa gttggggaaa 115673
   ccgaggcaca gagaggttca gaaacttgcc caaagacaca tagctagcaa gcggcagagc 115733
   tggagccacc cccagatggt cagggggcaa ggattgcact ctggagccac ggagggcgta 115793
   tggaagactc tggagcccaa ctgagtccca ggcctggtct gacccttctc tccttgtccc 115853
   tgagcaattg gcttctcctc gccgagcctc tgtttccaat gtagaagggg cacacctcac 115913
   tcatggaagg cagaggggat tggatgagct acagatgcga agctagtccc acccagccag 115973
   ccggccgcac gtgttagctg cgacaggtac taggtgcttg gctggggttt gggggatata 116033
   aacagtagaa caactttgcc tagtcaggtg aatgacacaa gcaggtcagc cagtatttgc 116093
   aacctaaaaa gatctcgctc atgggccttg gagcaggtgc agtggagacc agaggaagca 116153
   gtgtaggaac aagttcttcc tgatggactt cactggaccc gcagcacgtg tgacgtgctt 116213
   cccagaccac agtgctcttt tttttttttt tttttttttc caggcagagt ctcactctgt 116273
   cacccaggcc ggaatacgtg gcacgatctc agctcactgc aacctctgcc tcccaggttc 116333
   aagtgattct cctgcctcag cctcctgagt agctgggatt acaggtgccc actaccacgc 116393
   ccagctaatt ttttttgtat ttttagtaga gtcagggttt catcatgttg gctaggctgg 116453
   tctcaaactc ctgacctcaa gtgatcctcc tgcctcagcc tcccaaagtg ctgggattac 116513
   aggcgtgaac caccatgccc ggccagcaca gcactcttgc gtttgtaccg cagcagcaat 116573
   actttcaaca cgctgcaggg ggacggctcc tttgaatggc ctttgtcagt gacgatccac 116633
   tcctaaaaaa cccctcacac cctggggcct ccttgaccat gcctcttttt tttttttttt 116693
   tgtgctttct gtggctttct tttttctttt ttttattata ctttaagttt tagggtacat 116753
   gtgcacattg tacaggttag ttacatatgt atacatgtgc catgctggtg tgctgcaccc 116813
   actaactcgt catctagcat taggtctatc tcccaatgct atccctcccc gggagttgaa 116873
   caatgagatc acatggacac aggaagggga atatcacact ctggggactg ttgtgaccat 116933
   gcctcttatc tcaactttaa cagctgcttc cccttgagat ggtttgcaac ctcctgcccc 116993
   ctgagccctg atcagagggg accacaatgg gctgattcat tcattcagtc agcaactact 117053
   tactaaacac ctactgtgtg ccaggcactg aaggtgcagc agcaatcctg acaggcaggt 117113
   ccctgccctc gctggggaca gatagcaaca aacaggccaa taatgtaata agcagtgatt 117173
   aatgcttgca aaaagagaaa gcagaaaatc tataattaaa caaggtaaaa taaatgttta 117233
   ttttgagggg cagaggagat ttacaggagg ttactttgtt aaggagcatt gaggcaggga 117293
   tctgaagggc atgagtgtga gggccctgca gtgggaatgt gcccaacacg acctgggttg 117353
   attggacacc tcctccgtgc gcacaacctg cagcttcttc taaggggcaa tggggcctgg 117413
   ctgtgggggg ctgtgggcgg aggaacccaa gctggattcg caatcaygca cccgggctca 117473
   ggtatcagct ctgctgctca ctgactgtgg aaccctctga gcctgtctct ggacctaagg 117533
   aaagcaaagt ggagcaccag ctgccttctg caccctgtta gtgtgtgttg gggtgggggt 117593
   ggggggcaaa tgaccgtgaa cagagtttct aaattggaag ggctgttccc agggaaaggt 117653
   tcagagtcgt ctccccatct aatatttgcc cacggatcag tcatgcagcg aacatgtccc 117713
   aggcaccagc tctgggcagg tcgtgtgtga ggggtgtggg ggcacggagg tgaatcagga 117773
   ggtggagctg gccctggagc cgactgcagg gcgggacaga tggtcacata cacagcacaa 117833
   tggtcctgag gatgagcaga cgggtaaggc cttgccttgg tgttcaggca gagactagga 117893
   atgctccggc aacttcgaac tgctggatat ggctggcggg tatggaggga gaagcggcca 117953
   aggtgagact gaggaagagc aggcaggggc agcacgctgg gcaagtccga cctcctcggt 118013
   ggggtgggag aggccaacac gcagggtggt ggggagtccc gcagctgttt gaggaagttc 118073
   actctagctg agcttgggca cggaccccca tggggacaga gatgctgatg gcctgggctg 118133
   aggggcatag cgcaaggggt tcagatggag gaaccccagg acctgaaggc tggctggaca 118193
   agggaagggc ttataatgag gaataagccc cctgatgcct ggcttggtgc cccaaggctg 118253
   gggatgagga aatgggagtt ctagagaagg agcggtgtgg gggctgttgg tgtggggggc 118313
   tgtcggtgtg ggggggctgt cggtgttggg ggctgtcggt gttgggggct gtcggtgtgg 118373
   gggggctgtc ggtgtggggc ggctgtcggt gttgggggct gtcggtgttg ggggctctcg 118433
   gcgtggaggc gctgttgtct taacgccctt tcgttctaac cgcccttctg ttttcaccgc 118493
   cctgggctct aacaccccta tctctccaac cccctctgga tccccccccc cactcttttc 118553
   ctcgcccccc taggcaacgc cttcttaatg tgcccaaagc ccccggcccc cctgcttggt 118613
   tccccacctc tttttatgag tcatatctta ttctcttctc tccaaccctc tctttctttt 118673
   ttttctctgc ctccctccct cctcctccct cctactcctc tctcttcttc tctctccttt 118733
   tctcttttcc tttcttttcc ctcttttcct ttctttctta ctcctctctt tcccctctcc 118793
   ctcctttttt ttcccttccc ctttctccct ctatctcttt tttttccttc tttctttttc 118853
   tcttccctct tcctttcttc ttctttttct cctttttccc ctctttnttt ctatctcttt 118913
   ctctctctct tattatttct ttctctcctt cctcctccct ttctttcttt cctccatctc 118973
   tccttctcct atccctctct ctcttctcca tcttcttctc attcttggct nnnnnnnnnn 119033
   nnnnnnnnnn nnnnnnnnnn nnnnnnnnnn nnnnnnnnnn nnnnnnnnnn nnnnnnnnnn 119093
   nnnnnnnnnn nnnnnnnnna cnnnnnnncn ggcgtaggtg gtagatagtt cttaggtcag 119153
   tcgcatgtac tagcggtggt gtctagctgg tcgtggttgt gtcggaggga ggcgtcgtag 119213
   atagtgttat gtatacgagg tcgaggtcgt cggtgctggt aggtgcgatg ttcgtgctcg 119273
   ttgtgctagg gatcaagatg tattagtggt ctactggtgg gcggtagtac tagaggtgtc 119333
   gcacggatgt gggttcgtgg tggatttgtg agaatgagac tggtgtgtgg cgggagttgg 119393
   ttaagttgtg gggtgtgtaa tggataaggc gtctggctgt aggagtttgt gtgtcggttg 119453
   cgtgggggcg tgtctggttg agggggcggg ctgtgcgtgt gtcaggggta gctgtggttt 119513
   gtgtgggggt cagttctgtg tggggtgggt tgtcttgttt tgtgggtgat ctgtgcggta 119573
   atagggggtg tgttgggttt tggtggactg ttggagtgga ggggcgtggt tggtgtgtgt 119633
   gggggcttga gtgtatgggg gggcgtttgc tgttggggat ctgtcggttt gtgggactgt 119693
   tgatgctgtg gagtgtacgg tgtgttggtg cacttggtgt tgggggggtg cctgtatggg 119753
   ggggttgttg ggtttttggg gtctgatgcg tgttgggggc tgttggtgtg gggggtcggt 119813
   cggtgtgaag ggctgtcagt ttggggggtc tgtcggtgtg gggcggctgt cggtttgggg 119873
   gggccgtcgg tgtggggggc tgtcactgtg ggggggctgt tggtgtgtgg ggctgtcggt 119933
   gtggggggct gtcggtgtgg ggggttgttg gtgtgggggg gctgttgggt gttgggggct 119993
   gtcgggtgtt gggggctgtt ggtgtggggg ggctgtcggt gtggagggct gtcggtgtgg 120053
   gggggctgtc agtgtggggg ctgttggttg gggggctgtc agtgttgggg ggctgtcggt 120113
   gttggggtgc tgtcagtatg ggaaggctgt tgggtgttgg gggctgtcgg tgtgggggga 120173
   ctgtcggtgt tggggggctg tcggtggctg cccatagcac tggcattgcg tctgctctta 120233
   tttcccaact cccaggaagc accttgggtg ggttagtgtc ctgtggctgc tgtggcaaat 120293
   tgccataacc ttcatggctt caaaatacag atatattctc accgtcctgg gggccacaag 120353
   ttctcaatcc aggtgttggc agggccgtgc tctctctgaa ggttctaggg gaggaggctt 120413
   ccttgtctcc tccagctccg gggctccaga tgttcctgta gccatcgctc ccatctctgc 120473
   ctttgtctcc acatggcctt ctcctctggt gtctcctctt ctgtctctta gaatgtcact 120533
   tgtcattgca tttagggccc acctgggtaa tccagggtga tctcatctca agatctttaa 120593
   ctacatctgc aaagaccctt ttcccaaatg agatcacact ccacagtctc tggggatcag 120653
   gatgttatgg acacatgcta tgctgatgta tttgtggggg ccattattca acccactgca 120713
   gtggagaaaa atagtctgtg ctcagttgag gcatatggga ttgaagagcg attttacctt 120773
   ctctcctttt catcctctct gcataacttg ctcctctctc tggaattcct acctgtgtct 120833
   gacaaacttt tcttgcaggg aaaatgactc ttagatgcta gagtgtgatt ggaagggaaa 120893
   caaatcccac cagacagttg gagtggaatt agatgggaaa acagaagatg aataattcaa 120953
   gcagctttgc caggggaaag gggactctta aatgagccta attatgcttg ttcccaaggg 121013
   aggccagtcc tgaaatgaac cttgtgaagc agtttttcaa gcaggattta ggcgggtatc 121073
   atgaagctgg ggtggggcca cctgcggtta gtcctggaaa ggaaagggcc caggccctca 121133
   ttttgcagat ggggaaagca aggcttgcag aatggaaatg atgttccagt aacagccaag 121193
   accagacgcc cagccggggg ccccgagtga cagccaagac cagacgccca gccgggggcc 121253
   ctcagcttct gacctggtgc tggtcctgcc taagttaaag ccaccgagtg tggtcactgg 121313
   gccaaatgct tcaaagtcta cacacggggg agggcatggt gtggggcaga aagctgtgtt 121373
   cacggtagct gagtttgggc ataattctcg tgctccggag gcttctccca cagctacttc 121433
   ccttgtcagg agtccatctt cagggccctg tgttatgatg gattgaggga gcaccaacag 121493
   gggccacccc acttggtaat taattccttt tgtaaagggc tgggctgcag gcccgggtag 121553
   gtcctatggc cacctgagcc aaggtcttgg agaggcggtc gcacggacca ggtgaccatg 121613
   gacccagccc ggtgcccaca ccgtgaaggg tgctgtgctg cccagaggtt ctgggcacgg 121673
   ctctgggccc tgggattcag aggctcggga ctgcttgcct ccccacggag atcttaccgt 121733
   ggggaccctg ttgtggaggg cctgccgcat tggccgtgac agtgattttt ctccttctgc 121793
   agggtgatgt ggatgcaggg cctctgtgtc tcacatggtt gtttcacagc agccaccgta 121853
   tccgaagaca gagagaaaga gcaggagaga ttcccctgtg atggctcccc catgggtcct 121913
   gacccaggtt tgggagactt tatctcccca aagccaggcc ctcgatccct ttgcctttgg 121973
   ggactctgtg tccccagcct atacatgggg tgctcgattg agtgtccgtg cccctcagcc 122033
   ggccctgggt tgccgtggct cgggtgtgtt tgccacaggg ctacagacct aatggagccc 122093
   tgcatacttt tattaattaa ttaattaatt tatttattta tttttgagat ggagtcttgc 122153
   tctgtcaccc agtctggagt gcagtggcgc gatctcagct cactgcaacc tctgcctccc 122213
   cagtcaagcg attctcctgc ctcagcctcc caagtagctg ggatttcagg catgtgccac 122273
   cacgcctggc taattttttt ttatttttta tttttagtag agacggggtt ttggtatgtt 122333
   ggtcaggctg gtctcgaact cctgagctca ggtgacctgc tcgccttgac ctcccaaagt 122393
   gctgggatta caggtgtgag ctctggtgcc tggcctccta catactttga aaagttctga 122453
   aacatcccca ggtgggaaag gaaagagcgt ttgggtggac actgaacctg tcagggggct 122513
   cacgttttgc agtggtagca aacaacctga aggtctccaa ggcctcaaac acccacttca 122573
   cacctcccac tcacgctgca agtgtggccg tcacaggctg gctttggtgc gatctcattg 122633
   cggagggaac agtgtcacag cagaccacgt gctggctctc aaaacttcag cctggaggtg 122693
   acatgcctca cttgtctatt cattggcaga agcaaccagc ctctggccac tctgggttct 122753
   gaaaggccga aggagaggtc tccccttggg agagaacctg gaatgttcag agaaccccct 122813
   gggtgtcagc atttgacttc atcccaccga gtcctcctgc acccactgcg ccccatgagt 122873
   tataacccca ggaaatacaa cgagaatgag tgtcctgatg acgcaaacag aactcctgtg 122933
   ggactcacgt tcctgggcgc ttctgttgcc accaagcctc ctgtggcttt tgtgtgtctc 122993
   agggtgtgca gagagaatgt agcccctctc tgccctgcct ctctactgca cattatccat 123053
   gggccgtcgc actaatgtgc gtgacatttc atcagtggcc gcactttttc tctggatccc 123113
   actttagtga agttcagtaa ggacctgaga gctgctccga gcgggcagcg tgtcctggaa 123173
   agccatggga ggcattcggc ctgggagggc tgggctgcca ggtggacgct ggagaaatca 123233
   gcatggcagg aggagggctc ttttcttgct tggccttcag tatcattttt cattatttaa 123293
   tcgctgcttc ttttcatact ggaaaactgt agtttcctgg gaaaccagcc aggcagtgat 123353
   gccttaactc atttttccct ctctttctat ttttatgtga ttctcagttt gtggttaatg 123413
   acgtgcttcc gggaagcaag atttgagcac gagcacagag acccttttag gtgctttctg 123473
   actgcacaga tcagccattt ttttccctgg cattttataa accctcgggt ttagagaggt 123533
   cagaaaatac tgcttgcttg cttttttttt gacagagtct tgccctgtca ccaggctgga 123593
   atataatggt gcgatctcgg ctaactgcaa catccacctc ccaggttcac gtgattcccc 123653
   tgcctcagcc tcctgattag ctgggactac aggcatacgc cactactaat ttttttttag 123713
   tagagatggg gttttgcaat gttggtcagg cttgtctcca actaatttct ttttgtattt 123773
   tagtagagac ggggttttac tgtgttggcc agaatggtct cgatctcctg acctcgtgat 123833
   ccgcccgcct cggcctccca aagtgctggg attacaaata ctgctttctt actgagagag 123893
   gcagcagctt gggtggagga agagggaggg cagatggatt tcagagtttc agtcagtatt 123953
   ttccagatag aaaataataa aaatggaaac tgacattcat tgagctctgc aacgcatcag 124013
   gcagtgtgtg aagggctttg cctctgatga tcttcacagc gaagtgataa ggctatactg 124073
   ctgactcatt ttacagatga ggaagctgag gcatggatag ctgggaactc actcagggtc 124133
   acacagccag gaggtgacag agctgggatt caaaccccag accttccaac tccagggctc 124193
   acatgcacct gaagagtcag agggaacaga ccgtgcaaag ccccatgcag ggccggggag 124253
   accggagcct gaggtcattg ctgtgagagg ggagagggcc tttctgtcag ggtgatgggt 124313
   gggatctgaa gctggaaagc cggagagaag ctcgggggtg ccagtccttg catgctccaa 124373
   gagattctga tctgctgctg ggcagcaggg aagggatagg aaaagagcag gtgctggggc 124433
   cggggaggtt gtgaggctga atccagcctt accaatcaca cctgggccac cccggacgag 124493
   ttgctcatct tctctgtgct ctagtgtaac agtcagggat actgcctttg caagcagcaa 124553
   agttcttgac tctaaacaaa aaatggacat ttattggctc ccacatttag cagtctgggg 124613
   ggaatggctt caggtgcagc ttgatccagg attccaatgg catcaccaga tctttctctg 124673
   cactctttcc actgtctgcc ccaccctcaa gctccagggt ttctccttgt cctggcagca 124733
   gatagaagct ccttctttct acaccaccca gcacagcagg ggtgaccaag ctgcatccca 124793
   gccttttcag tattccaagt cctggggtct ggagagcaca gatcagagcc gaggtggggc 124853
   cgacccagaa acccagaaga acctttgccc aaaggagaat ctccatactg tggtcagaag 124913
   aaggggagca ggtgccgggg gcaggaaggg gagctcccag atgcccacac actcaattcc 124973
   agacacaatt tgaaaaggga ccgggaagac ttgaaatcat tcaccaatag catccactgc 125033
   ccagcatgca gtcggcacaa tataaatgct ctttttcttc tgcttggcaa ccaacagggg 125093
   aaggaaggca gaggagaggg gctccacagg tcagtgctat gtgtggctgg aactgatgca 125153
   ccctcactgg ggtggggtgc aggtgagggg ggccgcaggt caggagggaa gtggtgggtc 125213
   cagcttcatg ggaacagggg acaggcacct agaaggggta gccagcaggc agttcaccat 125273
   gcaggtctgc agctcagggg gacgcatttt gagggtctca ggaacagaga ggcagctcag 125333
   acgcgagggt ggacggtttt gcctcgggag aggtgtcaga aacctccggg aaggcggagc 125393
   attgagggcg cccttcactg ccccttcctt gaccgcactg cccccag ac  atc gtg    125448
                                                    Asn Ile Val
                                                        210
   gac atc aag ccg gcc aac atg gag gac ctt acg gag gtg atc aca gca    125496
   Asp Ile Lys Pro Ala Asn Met Glu Asp Leu Thr Glu Val Ile Thr Ala
               215                 220                 225
   tct gag ttc cat ccg cac cac tgc aac ctc ttc gtc tac agc agc agc    125544
   Ser Glu Phe His Pro His His Cys Asn Leu Phe Val Tyr Ser Ser Ser
           230                 235                 240
   aag ggc tcc ctg cgg ctc tgc gac atg cgg gca gct gcc ctg tgt gac    125592
   Lys Gly Ser Leu Arg Leu Cys Asp Met Arg Ala Ala Ala Leu Cys Asp
       245                 250                 255
   aag cat tcc aag c gtaagtgccg gtgcctgggg gtggggggct gtgcattggg      125645
   Lys His Ser Lys
   260
   caggcgggcg ggtgggatgt cctgtcctgg tgcagctgct gcaggggtgg tgggtgtggg 125705
   atgagcatgt cctggactgg ccaaggtggg tgggcccatt gctgcttcag cgctggctgg 125765
   tcggggagcc tggtcttgcc acctgctgcc ccccagagtc cctgcacagc caggaacagc 125825
   ccagctttta gggttcaggg ctctagggcg agggtcaggg catccagaag gtaaggaggc 125885
   accaaactta aatgtcacct cctgtaggaa agcttcccgg cctcctactt tagatctgga 125945
   tcctcctctg tggccctgca gcccccaaag cttccctctt ccaaaactag ccacacccag 126005
   caaacttgct gtgggctgcc catcttctcc ggctccttgg agcagggtct gtaacatgtc 126065
   ccctcatcct agaacccacc acaggcctgc tgtgggggtc ccacaggggt atccgtggag 126125
   gtctggggct gaacgagttc caggggacat tttgtgacac agcagccccc agcataacag 126185
   ctcacactga acactcactc tgagcccagc ccttaccaag ggctttacct gtgaactcat 126245
   cgaatcttca cacaagcccc aggagatgct gttcagaaga ggaaacagaa gctccagtta 126305
   ttggcccaag gtcacccaaa ttcaatcgtg gcagagctgg gctgcctggc actgggttgt 126365
   ggccatgggc tgcacactgc cacagccatc ccagaggccc ctgaaggcga gggatcaggc 126425
   cacggcatcc tccttcccag acagccctgc agggctagca aggggccagg caggaggtgg 126485
   ggtgtccccc aggcagaggg cagggacttt gcttatctgg ttcccctcac tgtctgtccc 126545
   cagcacctac cacagtgtct ggaaataaac acttgttgaa taaaccccag aggaccctga 126605
   ctgaagcctg ggtgccggcg gcagagggct gtttgggagc gatggggcca cactgttccc 126665
   ttagggtgtc tggttttgct gggcagtcag agacagggtg cacaccagcc ccaggactca 126725
   ggctcagggc ccaccaaaac ctccagcttc tgcctccttc cttggccaag ttctggcccc 126785
   cacatccacc ccctcatctc ctctgggttt gtccacgctc cgtgtgtcac gctcagccat 126845
   ccaggcattc agtaggcgcc tattcctggc atgggtgtgc agcagaggcc caccgtcggg 126905
   agctggcgtc tgcgggggag acagacagtg aacacacacc tgacgcatgt cagggcatgg 126965
   tgagcaatgg caccaacacc cacaaagtca ggtacaggga cagagggggt cagggagggc 127025
   ctccctgaag agaccacagt ggaacggaca ccctgagggg gtgggtggtg ccgatctctg 127085
   gggaagaatg ttcgagaagg agggcactgc agtggccgtg actccacaca ttgcctgtag 127145
   gtcaagggag ggactgggtt ttgttctggc tgagctgaga atgagaagct gtgggttgtc 127205
   acgagattgg gacttgagga ggaagaggag acgccgcaga gggtccccgg gggagcaggc 127265
   gtcagaggtc gctgcagggt ctgcaggaag aaatggtgac cccactgagg gggttgttgg 127325
   gaggtggggc agggctcacc tggctgcccc ctctggatgt tgcttggagg tcgagctttg 127385
   ccttgtgggg cgtgactctg tggttggttt gagctgtggg tgaaggtggg gccgtggcct 127445
   ggaacaacag ggggtgccag ggaccagcag gcaggtttca gacccgcgag cctgaggccc 127505
   gtattgggtt ccaaggaaat gggtcgagta ggcagccaga tatccaggca gtaatgccac 127565
   ctggagataa gtgtggagtt agtggccgtg gactgatggg aggctggaga ccagctgaga 127625
   ttcccggggt gcagggggct cggggctgcg tcctgtgcgc cacaacaaag gaccacacac 127685
   ttggtggctg aaaacagggc atatgtatag tctcagtccg cagtcccaca gccagctcac 127745
   tggccataat gaaggtgcca cgggctggta ccttcgggag gctctgggga gcaccgtttt 127805
   ccctgcctct tctggcttct agaggcgctg cgttccctgg cacgtggccc ctcccccatc 127865
   ctcccagccc acagcggcca agcctctctc acgtctctga ccctcctgtc tgtctttcgc 127925
   gtataaaggc ccttgtgatc gccttgttcc tgccacccct aatgatccag gatgacttcc 127985
   tcatctcaag gttggctgag ttgcgtcctt gatttcatct ttggccttat tttgactttg 128045
   ccgtgtagca taacatagtc acaggtgcta gggatcagga cgcagccgtc tctgggggcc 128105
   gttccaccgc ctactacagg acactgcccc cccacctacg cctcccgccc attcactcag 128165
   gccacatgtg cctccagctg tgcctgtgta cagccaggtc tgggtccgaa tggaatggga 128225
   gaatattggg gaagagaggg ggttcttcct gctcctggtc cgctgctgac cccgtgtgag 128285
   ccctgtggga aggaggatgg ggcttcattg gcaccctgtt gtttataggg gtggaaatcc 128345
   agaaacatcc tcaatatccc aatcttaaat gctaagatta ttcaactctt ctgaaatgag 128405
   tgaggcagct ttttgtttct ttttctggaa ccgttggtgt aatatatctg ctccttgaag 128465
   tattaggaaa acttgtgtgc aaatctattc ggcgtccttt tgagggagag aaggaaaatt 128525
   ttaagcaacc agtttaaaat gccgagaaaa tgatgtcgac agtgatgaca tccatgtact 128585
   agaatattgt tcggtcatgt cagctattgt tttagaagaa tttttaatgc cacagggaag 128645
   tgattatgtt gaaagattta ggggataaca aaataaggct atgaccacca gaaaagtgga 128705
   aacagagaaa actctggaaa gaaatacact aagttgtccc tgagtggtga gctataagaa 128765
   actgatggaa tattttcttt ttatttttct gtgtattttt actttcatga tggatttgaa 128825
   ctttgaatca gaaaacacat gtgtgtgcag gaagggtgga atttagagag cacagagacg 128885
   ggcataggac agttttgtgc acccctcaat gtttgtgtga catttttcag tgggctagag 128945
   ggggcagtca gacccccttt ggctcccgaa gcctgtgcta tccggtttcc cagggcagaa 129005
   gcagcggtca catatacctt gcacatttcg tgagtccaag cacagacaca tccatttcag 129065
   ccacgtaatt tcatatgcct gatgatgtat gagccttgtg tgaatcagcc aatgaactca 129125
   acagtgcgag aaatatgagt gttttcgatg actcaattca atttcattct tcacccccag 129185
   ctcccagggc tgcagtgaac gttaactttt agggcctcgg cagattccac tcctctccgg 129245
   attgcactga ggttccagaa ggctctggca ggccgaggtg cggagagagg gacatggtgt 129305
   cttccaggcc actgaggact tttgattgtg cctgggcgac cttggggcca ggtccttgct 129365
   gacaaatccc caagggcctg ttggctgtgc tcagtgactc acctgggagg ttggcaacat 129425
   gctgacctct ggcggctctt gcccgcacca gtgatcccaa gtctgtagac agggtggggc 129485
   tgcctgtgat gtactaaggt tttatgtatg ctttttgcac acacagtcat aggtgaagtt 129545
   ggtttataat tttcttttct tatactctct ttgcccaatt ttaagattat tcaactctcc 129605
   taaaatgaac tgggcagctt tttctttctt tttctggagc agttgatgta atatatctgc 129665
   tccttgaaat cttaggaaaa cttgtgtgca aatctatttg gtgtcctttt gagggagaga 129725
   agaatttttt tctttttttt tagacagaat ttggctccat caccctgaca cgatcttggc 129785
   tcactgcagc ctccacctcc tgggttcaag ggattctctc acctcagcct ctggagtagc 129845
   tgggattaca ggtgtgtgcc accatgcctg gctaattttt tttgtatttt tagtagagac 129905
   agggttttgc tgtgttggcc aggctggtct cgaactcctg gcctccagtg ctgggattat 129965
   aggcatgagc caccatacca ggccaagaga agaaaaattt taaacaacca gcttaatttc 130025
   ttttttgatt attgatttat gcaaatttta tttaaaattt ttctagaata tttttcatta 130085
   tcaaactcaa tatcctgtac ggaaactttc tctttcgtgc cagcaaaaag attgcgcagc 130145
   agtttttagc atgcttttac agcttccatt tctcctcatt taatttgccg cacattgggg 130205
   cgagtggctg aagtgtctgt ccttctgcat gccttgtccg ggctgtgttt ttgctctgtg 130265
   gctctcatgg gaaatcaagc acgcagccgc tttccaatta gcaggtggcg gctgttttta 130325
   atctgaacac aaatggctgt tatttgagct aattttttaa aagggatttg gggattagga 130385
   ttagttcttc acctcccact tccatccatg tatccaatta ctgctcaaaa ctcagggagt 130445
   ggctgatagt gacaccccag agatgtgctc acagcatcat tttcctaaca gaatcagacc 130505
   gcgaatgaag agcgtcctgg atgcaggaag gtgagcattt ctgccattgg acagatgagg 130565
   agaccgaggg tccgagaagc tctgtgactt ccctggaccc ctcctctatc agggcagagc 130625
   tgggggcaca caggccactc tgttccctct gcagcttccc acctttctcc gaagcactgg 130685
   aaatccgcgt cttgtgactg cttggcagtg tgacagggta accgtggaaa caaccgtaca 130745
   tgagtcctca aaatagacag ttacttctgc ttatgacagc acagagcctg cggaaagaaa 130805
   gggccgtgcc cggaacactg ggatttctta gaattggggg tgatgatggc tgagaccctg 130865
   atttcttaga atcggggccg atgatggcca agacccttca taggccccca acgacgtgcc 130925
   tggcccggtg ttgggtgaaa taataaccgc agctcattct ttgcacacct agtgcaaaca 130985
   catgctgttt tcagtttata tctcattaaa tactcagcac ctatgatggg tgtgttatag 131045
   tattcatcct acagatgagg aagttgaata cacttgaccc tggaatgatg tgggagttag 131105
   gggttcagac agtgcctgaa tgcagttgaa agtacatgct taacttttga ctccctcaaa 131165
   acttcactcc taatagtcgg ctgttggtgg ggagcattac cagtaacatc aacagctgat 131225
   gaacatttga agagactgat gtctacgtgg attttatgca tttgtgacat acctaacttt 131285
   ttcttttctt atatatatat gtatgtatat atatgtatgt gtgtgtgtgt gtgtgtgtgt 131345
   gtgtgtgtgt gtgtgtgtgt gtgtatcgtg gcagatctcc aaaatgtttt cagtatattt 131405
   actgggaaaa tcgcacatca ggggacctgc aggttcaagc ctgtgttgtg tgagggtcaa 131465
   ggggcaactg tatacgcact tctcattgct gcgggaacaa ttgccatgag cttagtggct 131525
   caaaacaaca caaaagactg ttgtacattc tggaactcgg gctaaaatga aggcgtcagc 131585
   tggcctgtgt tatctctgga ggctgcaggg tggggctgtt ttctgatctt ttccagctac 131645
   tagaggcttc tagaggctgc ctgcactcct cagctcatca ttccaacctc tgcttccatc 131705
   atcccatctc tttcttccat ctttgacctt ccttcttcct cctatacaga cccttgtgat 131765
   gacattgggc ccatccagat tatccagggt cacctccgta cctcaaggtc cttcacttaa 131825
   tgacatctgc aaagcccctt ttgccatgtg agataacagg tgcacagatt ctagggtgga 131885
   ggatgtagat agacgccttt aggggtcatt gttctctgct acattgaagc acagagagat 131945
   taagacattt gcccaaggtc acacagctaa gtagagccaa gatagagcct cagagagtct 132005
   catgccttca acctgcactc tttttccctt tctcatcaca gaagccttga gaactaaaac 132065
   tcttacagga attgtgggtg agctgggttt tttttttttt tttttttgaa atggagtctc 132125
   attctattgc ccaggctgga gtgcaatggc atgatcttgg ctcactgcaa cctccacctc 132185
   ctgggttcaa gcaattctcc tgcctcagcc tcctcagtag atgggattgc aggtgccggc 132245
   caccacaccc gactttttgt atttttagta gagacaaggt tttgccatgt tggccaggct 132305
   ggtctcaaac tcctgacgtc aggtgatctg tccacttcgg cctcccaaag tgctgggatt 132365
   acaggcgtga gccaccgccc cccacctgag tgggttcatt taaaagggct gtgtgggctc 132425
   aactcccagg gctcactcta gtaatggatc tgctgcagaa agaccagcct cagcccagac 132485
   cctgcagctc tccaggtgca ggaccagggc aaagctcccc aacagtggga acagcccatg 132545
   tgagggccct gtggctggat acagaggctg gagagaggag gtgaggtcag agaggtgtgt 132605
   ggctggagga ggcctcacag gccagagcaa gagcttcggg tttcctctga aagggacagg 132665
   aaccacaagg ccgtctgagt agggagtggg ccagcctggc catgtgttca caggatccct 132725
   ctggctgccc agtggggaat aagctgaggg gtgaggtaga agtgggggct ggctgggggc 132785
   tagcgtgttc acgcaggtga gagacgctgg tgcgcagagt gagtggccgg tgcaggtggg 132845
   gaaggtggtg ggccctgccc cccatggcag gtggggcctg ctggatttgc tgctggattg 132905
   gatggagagg gagggagggt cggggatggc ttcaggctct gtacagagac caggctcccg 132965
   tctgtgtgga gctttgtgga gggaccacag gaaggggact agtaaataag taggtgcatt 133025
   ggagtgggaa ggtttccatg aagggcatca ggagccctct gatgcattgc acctcagggg 133085
   tcaggagagg tgtccctgga tgagaaagag cctgggaatg ggggcggggg tgccccaccc 133145
   cacagtagtg tctgagagga acctggctga cgtcaggacc cgtgtggctg gaacaggtgg 133205
   aggggtgggc tcttgtgtgc aaagatgggt taaggggaga gcgtgccggg cctgggggcc 133265
   acagctgggc ttgagttttt tgtttgatgg ggctcgatca catggtgctg ggcagggagt 133325
   gacaactgtc atctcttcat cagtgagcat tcattgtgcc cacctgtgcc attgtaggaa 133385
   ctccctgctc cctccccact gtgtacagcc tcagggatag ggaaagaggc cgaacaggag 133445
   gcccctcccc aaacagtggc atcacctgga acccaggagc aagccagtgt ccaggggtgc 133505
   tcggggatcc ataaccccca tcgtccttct tctgcttctc ggaccctcca agctcctcct 133565
   ggagcgcggc ctgaggtgag gccagctggc ccaggccact ccaccctcgt ggatggcctt 133625
   gtatcccatc agatcacagg agggacccgt ggccagggta gccaggggcg atcactgccc 133685
   aggaccccat aggcccctcc ccctccaggc cacctcctcc catgtttccc acctccaccc 133745
   acctgcaggt ccctcccacc ctcatctgtt tctctggagg cctcactgaa ctgcacttgt 133805
   ccctgaatgc atggctcaag cccatgcatt tctactgtgt gaacccaatc ggcctccacc 133865
   ccagccccag aggagtgtcg tccacacact gacaacgatg ggggtggggc acagcaggtg 133925
   ctaccaagag ggccacagag caagtgccac gggcacacag gggccacccc caggctggct 133985
   gaggcttctc tgccaggcct ggaaactgca gtggaaccac atcaggttgg ggaagaaaga 134045
   tgtcagtgag gggctggagc agaaacggca ggcagctgca caagcttctc tctgctcagg 134105
   gagcagggcg gggccatgag gcagggtctg tgctaggcag gggcgccaag atgatcagtg 134165
   actaatgacc aaggcaggac ccctggccca agggagctta cagccccatg aggggaccct 134225
   gccctcctgc cagtgccaca ggagaaggta cccagcggct tgggggcagc agagggtaag 134285
   cacaggattt ctttagagag catgtgaaca atatgcacag aaagcagggt gtgagcctgg 134345
   ggttctagag gatgtatagg agttggtaag aaagaccagc taaaaatggg aaagggatct 134405
   cagggagaga aaatgatgtg aaaaataact ccaggaatcc ctccgtagtt ttattgagca 134465
   gctactgcaa gccccacact gggccatcgc ctccaggcat gacctctgaa gatcccatcg 134525
   tgctccttct ccagatgagg gaagctgggc tcagagagga ccaggccctt gcctgaggtc 134585
   acacagagca cgatgcattc tgctctctgc ccagcacatg gcggggcagg ggctacggga 134645
   ggcaggggga gatgcctagg gctcaacatt gaggagacac ccacccccag agcctttgag 134705
   tggatgcagt gccctagggg cccagcagga agggggctgg agtgtgcggc agaggggaac 134765
   ttgcgtgtgt gaggcagcag cagcaggctg gttgggaccc attgagaagg agctggtcag 134825
   ctacaggtag gagcctaaac cttgatcctg tgggcactgg ggagccaagg agggcttcaa 134885
   gcagaaggag gtgccgtcgg ctctgtgtga ggctggaaag gaaggtgggc acctgctctc 134945
   aggcttcacc tcctgtttcc tttgattcac aggtccgtca gagccgcccc acaccttggt 135005
   tgggctcccc aggtcccctc caccttggcc tgagccaggg atgggggtgg ggaaggctct 135065
   ctgtggccac aacaccgtgc aacacccagg gaggaagagg atgtggttgt cagggaaccg 135125
   gctcgagcca tgagagccct aattatgcaa aatcattaaa aagaaacagg atggagattt 135185
   cggctgattg agagctctcc aacttttaaa taattagggt gtgggggagt gggtggagat 135245
   gggactgggc ccatcacaag tgatgggggc tttgagacat gtttctttaa acccatggca 135305
   ggaataatta ctgtgaaatg tccttagcga ttcagtcaat tagaggggag accccttatc 135365
   tcttgttagc gctctgaagg tggtgcagcg gggtgtgtgt gtgtgtatgt gtgtgtgttt 135425
   gcgtgcgtac tcgggggcag cgattgatat ggaatagtag attccactat cttttggaaa 135485
   aaaatctatt gtatcaaaaa tttcatagaa acgtctaaaa agaaaaagag gaacttttag 135545
   gaatggtttt tcaggcttat taatatccct tccgatctat cgtttgcatt cctgttcctt 135605
   gtgtccttaa aaaccctcag aaatgcccga gtacagccag cagtggggcg acaagggcca 135665
   gcgccgtttg gctttgtttc cactatcgat tcaattgttc tctcagcctt tgatctttgc 135725
   ggtttttcct gctggaggct ggtaggatac ttggccactc gttctgccct gtgacaccca 135785
   gcaccggtgc cccctgtgat tctggctgaa tccacccgca agtcctgctg ggaacctaga 135845
   ccagccctca aggtctgggg gagtctggtc tgagagaccc ctgagcaggc gagggggtac 135905
   taaggccact ccggaagtca gcctgtgaga ggccgccctg gctcagtttt ggactcacct 135965
   ccgcctcacc tccaagtgac agaaagccta gttcaggcca gacagggttc ccgccactca 136025
   cctgggcagg tgctgggggc caccaggtcc cgctgagatt ctcttccttg tttccatcca 136085
   tttagccacc gggggtccct cttctctcaa gccctctccc tgcttctccc aaatatgagt 136145
   ctctacccac tgctaatgga aaggcagcac aacatcaccc ctttagggaa tcaagcgttc 136205
   gaagtgattt tctttacggt aggcatcaga gagaaaaggg aactgtggtt gatccttctt 136265
   tggcttaaca aagaaaaact acaaatatat atatatatat atatatatat atatatatat 136325
   atatatatat atacacacac acatacagaa acaaagatgc caatttaatt attattatcg 136385
   gggcacaaaa cattatcatg tgagatgacc aattagaact cagaggttca gaagctcaag 136445
   gtgactccca gggagatttt actaccccta ccgtacccat cctcctccac aggcagggag 136505
   gggttggagg gctcacagga gggagcatta gggccagggc ctcctccatt tttatggttc 136565
   ggaaatcaca tcgtatcaag gcagcataag tttgcacagc cctggagggt tgactcgttt 136625
   ggggcaaact caagagggcc tgatgaataa gccagacatc actgactctg tgaatgccaa 136685
   cagtgacatt gggataccac ccaccagggg ctggacccct tgctgagtgt ctctgtctcc 136745
   aaaaacagct gcgcaaactg ggcagtattt ggctgcattt gacagagaca gaaacagaac 136805
   cctgacagga aaaggcaagc atcttgaagt cacaaccagg aagtggcata gcgggtttcg 136865
   aacccaagtc tgcctgactc ctggtcctgc aaggtctttc actataagcg atctaaactt 136925
   gagccactta cttttctata taccaagggt catcaaattc tttctgcaaa gggccagata 136985
   gcccattttt tatggtttcg tgggaacata tgttctcttt gggcaaagtt tgccaacttt 137045
   gccattttta tccaaaaagg gttttcacat aacaatgtgt tggattcctt gggggatgac 137105
   ttcttttccc aatcaatact tttttttaca aaaacctggc tcttgggcca aatttttgtc 137165
   ccatggggcc tgttatctta ccctccccct tgtgggtttt accttgttgg aacaaaaaaa 137225
   accatccatc tttgttattt ttttgccccg ttgacacctt ttctatttat ttaaatttta 137285
   acaaaccgat ccttttggga attcatattt ttggggcttt ttgttttaac gattgtcgct 137345
   ttaaaaattt aaattggtgc cggtggcttc catcctattt atatcaatca tattaatttt 137405
   aagcaagctg atctttcgga ggcccttctg gtcccttggt tgtggaaata actgttttct 137465
   actataccaa agtatctttt acttgggcgg gttggggttt tttaaaatgt tttgggaaaa 137525
   atataaaatg agagagaaag agaaggagag agacgggaag aaagaagaaa ggagagagaa 137585
   tagagaggga agaaggaaga gggggggata gtggagtagg ggggagggga agagagagag 137645
   aagaagggag aggagagaaa agaagagagg agagcgagga agaggagaaa gagaaagaga 137705
   gaggagggaa ggaaggagga gaaagaagag gaaaggggga gagaggagaa gagaaagaag 137765
   agaagagagg aagaggagga gaaagggaaa gggaaaagaa agagagagag aaaagaaaga 137825
   agagaagaag agaagaaaga gaaaggagag agggagggag gggagaaaga gaggagaagg 137885
   aggaggaaaa gaggaggaag gaagaaggga gggggggggg gggaggaaga gagggaggaa 137945
   ggaagaagga aaggggagag agaagatggg aggaggggga aaaaggaaga ggatagaaaa 138005
   aaaaaaaaaa aaggggggga gagaggaagg gagtgtgtga agagggagag gagaagaagg 138065
   gggaagagga aaggaaatga ggaagggaag aagaagagaa aagaggagag gagagagggg 138125
   ggaggaagaa ggaaaagaga ggagggagga gagagagaga gagagggaga ggaggaagag 138185
   ggagagagag gaggagaaaa aagaaaaaaa agggaggaaa ggagggagga gaaaaaagga 138245
   gttcgttaag aagggaggga gggagggaag taagtcagta gggctgcagc actcatcgtg 138305
   cactgggctc actctgtaca gcatctttgc tgcctgcggc cctgcctgta cccagggtgg 138365
   tgccaggcct ggctgcaacc ccctcctcct gccccttcct gaaaagcgcc cttggctcct 138425
   gttgtaggga ggagcaaggc cagggctcag ggaggtgaag gcgttcaagg ccacacgagg 138485
   ggctcgggga ttgagaccct ggcagcctgt tctctcccag ctccctctgc gagcggcctc 138545
   acccttcctt cccatggtgc tgattttctt tctggtcctc ccttcaatgt gcccttgtgg 138605
   aaagaggaaa gctcgggcca ctgagaaggg cgcccctgtg aacgatggag ggagattgag 138665
   gtcgcaggca ggcctgggga ctgggggcgt gtggaggggt gggttgggct cctggaaagg 138725
   gaggcgccta ctgtcagttt tgagtttcgt taaacacctg ccagcctgat agcattctaa 138785
   ttccaaaata tgacaccact tattagtgga taaactaggc tccctccctc cttcctgatc 138845
   ttgacgccag gactggaagg agcgaccacc tgccctcagg atacccggtg cagtgccatg 138905
   gctttgaggg cagaggtgtg gggttgaagg ctgccactgc tcaccagagg ctgtggagag 138965
   gctaaggaca gggatataga tctcctgagc ttcaggggtc tgcttcagag acaggtgttt 139025
   ggaggccttg ctctcaagag aggtgggggg gaccttcctc ctgctgcctg ctctgatttg 139085
   gggggatacc agaggcatga acataaacgt gggagccttt tcctgccctg taagcagctt 139145
   ccctccccct cctctcacct cccctcacct ccctgcatgc agtggcctgg gatcaggatg 139205
   gtgacacctg ggctctgcag ccctggccca ccgggtgctg gaacttcctt cctccgtcgg 139265
   gaggggactc cagcctcctg cctgcccctc cctacctcct tgactcagat ttcatcctcc 139325
   ctgtctctga accctcagtg gctcccgttt ccctaatggt gacttcctca cccctctccg 139385
   tggcccatga gatcctgcgt gggtggcccc ttgcgtccct gttccccctg ctcactcctt 139445
   tctctgcctg cacggccctt ggcgtgtccc tgcctcaggg cccttgcgtg tgccgtccct 139505
   tctgcccagc acacccctct ccaggcatcg ccaccaatct ctccccaact tcactcagac 139565
   cttggtcaaa cagcacctcc cacggaggcc ctgttcacct aaactaccag cccccagcac 139625
   ctatctgcct gctggctctt cctccccagc acttgctgct ccctggcggt ggagggcgcc 139685
   acttagtttt attgtctgta tctgcaagga gaatgttggc tgcacaggaa cagtggcttt 139745
   atgctggtca ctgcatcatc ccagggcctg gcacagagta ggtgctgttt gcattggttg 139805
   actgagtgaa taaatgaatg aatgaatgaa tgagtccatc agggcatcca gtgggccctg 139865
   cagaggggag ctggacagga gttgtatttc tggagaggca gtggccaggt acagtgtcca 139925
   ccttggacag gagggagaat ggggtgctgc catttcccat gaggggataa ggggctggga 139985
   cagacctggg aggcaggaca cgagcccttg tggctgagag gggccagcag ggaggggcct 140045
   ctcgggagcc tcagggtgct gtgatcagct ctgcttccct gtttctgggg tagagaccag 140105
   agcaggccag caggcaaggc tgccactcag ccggtttcca tggggacagc tggacaggtt 140165
   gtcataggtt taggtatttc cagattggct ggtgaatggc tgtcaactcc accaccctgt 140225
   tctcttccca tcgttccctg ggtctctctg tggcccaggt cctagggagt tagtgcctgg 140285
   cccaacaggg gtcctagtcc ccacgctact tcagccccag ggtcactgct accagtgaga 140345
   cagataccag cagaaatgag cttagagacc ttgtcccact ttgggaactt ctgcagctca 140405
   ggaaggccag gttatggggg cagtggggag gggacactgt ctggggagtc ctcattgccc 140465
   actctgtccc agtctataat tgtccaggtg ggcagcaaac cgttgccttt agggaccaga 140525
   taagcaactt cctgtgcaga gcaggtgctc caagaaaaaa ggagatggtc agtggatggc 140585
   atacaggaga ctgtaccaac tctgtggcag tcagatttga ttttgttaca aaccccatgg 140645
   caatgaaaca aaacccacct ataagtaggg ctcagccatg cctgcccagg acaccatgaa 140705
   cagagataac tactggccca aggtcccagg gccagttagt gccagagcca caagcagtgc 140765
   ccagtctggt agaggacatt gtccagcaca tttgagaatg tcaggacacc tttgcaatct 140825
   ggcattcagc atcaccagta gggggcagta gagggcagca catcaagtat agctttggct 140885
   tcaaatcccg actctaccac cttcttccag cactgactcc ccaggcatgg gttttagcca 140945
   gctgctcctc cattttcttg gctatgaatg gggatagtaa tggctatttc tgcacagcac 141005
   agaatcttac caggcttgtt ccctggtaag tgtttagttc taggtttgag gaatgaatga 141065
   atgactgaat aaacagagca tgggcccagg tgcaaaacag agtcatccgt cgtgccaacc 141125
   ccatgggcgg gagcagcgca gtgacggcca ttgcttctct gtctccacag tc  ttt     141180
                                                        Leu Phe
                                                            265
   gaa gag cct gag gac ccc agt aac cgc tca ttc ttc tcg gaa atc atc    141228
   Glu Glu Pro Glu Asp Pro Ser Asn Arg Ser Phe Phe Ser Glu Ile Ile
               270                 275                 280
   tcc tcc gtg tcc gac gtg aag ttc agc cac agc ggc cgc tac atg ctc    141276
   Ser Ser Val Ser Asp Val Lys Phe Ser His Ser Gly Arg Tyr Met Leu
           285                 290                 295
   acc cgg gac tac ctt aca gtc aag gtc tgg gac ctg aac atg gag gca    141324
   Thr Arg Asp Tyr Leu Thr Val Lys Val Trp Asp Leu Asn Met Glu Ala
       300                 305                 310
   aga ccc ata gag acc tac cag gtgggcacca cagcaggaga cccccaatcc       141375
   Arg Pro Ile Glu Thr Tyr Gln
       315                 320
   cgggtctttt ttccctatgc tgagatcccc atggaggggg ccttcctagc caggcgtggc 141435
   tttcatatgc ccggtatgta ggtgaagaca cggaggctga agaaatccag caactcatcc 141495
   ccacacgtag cttggcagag ggcaggagtg ctcagtcttg cctcacaggg agctttggat 141555
   gtccctgagc aaggcctgca gtccagggcc caggggcctc aattccaggg aggaaagaga 141615
   tgtgggaaca gagatgagcg tcaggctggg ccccagtgga gcatgtagac gatggcttnc 141675
   cctccccact ccctgggagc ctgcatcgga gctgtaccat taaagagggt taagcgttgc 141735
   cctcccaggg tgcatccgta agcagtgcac gcagaggtga actgagcaca tgtttccttt 141795
   ccagatcctc agcacaggcc tataagcctg gagtctaggt gaagcctggg cccttcttgg 141855
   acagtattat ttattatctt gctattatca tccattcaga tatgttaggg ggtagacaac 141915
   aaaactcatg tgatgttaaa ataaaatgtg gacttgaaag aaatgtggga tggcttcaaa 141975
   gctggttcca gttaggtggg gagatcaggg aggcttcttg gaggaggtgg catttgtgct 142035
   gggccttgaa ggatgggtaa gatctggncc gtgcaaacat gggtgggaaa acaccccacc 142095
   agaaacccca gccggttcct caccgtgtca ctcctcacac anggggtgcc acgtgcttga 142155
   catctgtcac ctccattctt tactgtccgg tgacaaggag ccatcatcct atttggtgga 142215
   aagggaactc gggcactgag aggtagggag atgagggagg tgtctctcgg ctgggcggtg 142275
   acagattcag agcccaggct cagagcccta cgctttcctt tcctccgtgc gaagacctag 142335
   taggaaagcg tcctgggtgg cgcaggcctc gctgggaact ggtgcagagc tcagagggtg 142395
   ggctgctctg atctgacctg ggccccagag gaacagctca cgctcctgga agccacacac 142455
   ccacaaggac cgctatgggg accgcctgtt tgtcagccac gtgtgtttac ttgagttctt 142515
   gcgactgccc cagctcctcc ctcaggcccc ctcctcacac tgcacacccc aggcaggact 142575
   cagccctcct ctgtccctgt gggaatggca gagaccccag actaggagag ggacaggata 142635
   agcccggctc aagcctgcag gagcaactct ctggctccct ctggaggcag ctcagggaaa 142695
   tgaacatttc caaccccctc cggccccttg tccggtcact tacctccatc cctgaggtca 142755
   gcaaaggccc atgaacccct gaaactacgt ggaatgtgtt ctatgagcat tttcaggggg 142815
   agggggacac agctttctga aattctcatg aaggacccca cgaaggaggg gaatgactgt 142875
   tctggagcgt gaggccgtaa gctgggcact tggcatctgg cccagctgac ctattgatgc 142935
   taaaataata gcaataacta cataaaagca gnacaacaac caacattttt tggtggcttg 142995
   tgatgtgcta gaaattgtgt gtagcaggac tgcgttcggc tggtgtgcgg tgcttaacaa 143055
   atcggggcta tttttctccc anaacaggga gtctggagag ggcagtccag gaaggtgggg 143115
   gggctccatg accccggcag cgccacagtg gttccttctg tgatccagtc caccatcatt 143175
   tgggcgttgg ttttccacct tcacatgtgg tacctcatgg tggcaaagtg gctgccacac 143235
   ctccaagcac tttgcctaca ttccaggcag gaaataggaa atgcactaga tctcacccct 143295
   ggggtctgtt gcgtgtttca ctgtgattgt aaaatcttcc ccagaatgct tctcagaaga 143355
   cttcttacac ctcattgtct agaatgggtc cctggcagcc accatagtgg ctgggaagat 143415
   taatggttta acagcctctg tggttgaaga aggcaagggt gaaggggctg gggacggttg 143475
   ttgaatgagc cacactgagg taagtccata ntatgctttg cctcatctaa gcctcccagc 143535
   tgtcctggga ggtggggccg gtattctccc catcccattt tattggaaga cnattgagtt 143595
   aaatggcaaa tctgtgacag agccagcaag cagnggacct tagattgaca gtctttctcc 143655
   agagctcttc ctcaccctgc cactagaggg caggagatgc ttagtgcacg gcagagagaa 143715
   cagcatgcgc atttggacgc gtgcatcacc aaggctgctg ggagctgagt aaggaggaca 143775
   cacaaaaatg cagagccggg ggtgggacac gaacactaaa acctgctgcc attcatcccc 143835
   cttgatttta gttgttacac atactgagcc agtaccctgt gcccaacaca gtgctagacc 143895
   ctggacccag agagaaggaa gaacctcggc ctcaccctcg agggtcccag acagacagga 143955
   acacagtgac cctcagcttg gtggggctgc agcccagggg cccaagggga aggcaagggt 144015
   caccaggagg acacgggggc tggggaggga tgagcagggc cccaggcggg agtcaggtag 144075
   ccttgtctaa ccatcctgtg tgcagtgaat agcaggccct tcctcccaat cctgcttccc 144135
   ctagtgccac tctgttttcc tgtgtagtcc ttgccaccat attagtcaga tggctcagag 144195
   aaacagaacc aacaggatgg ggatagagac tgagaaagaa gggagatttg aaggaattag 144255
   ctcacgtggc atgttctaaa tctgcagagc aggcgggcag gctggtgacc caggggagag 144315
   tgactatgcg agcccacacg catctgttag cggaatcccc cggtcctcag ggaggtctgt 144375
   ctttgttcta tcagggcctt cgactgattg gacaaggccc cccacatgat ggagggcagc 144435
   ctgctttact cagtatatca gcttaaatgt taacctcccc tacaaaatgt cttcacagaa 144495
   acaccaagta tctgggtacc ttggcccagc caagctgaca cacgaaattg acgttcacac 144555
   cacccttcac attttctgtg ttcgttggag ttgttaattt caactcctgg gagttgaaac 144615
   caggctccat ggcacccagt taccttccct tccgcaccca gagggcagag cccgtgccct 144675
   gttgtcctgg cagctccagc ctcagcagag ggctggcact catgcggccc tccgggtaca 144735
   gggcttgtag gaccggctgc agtcaggtgg atgcaggtcc tggggtgtca ccctctctcc 144795
   tgtggggtac gggaatcctg gggaagggtc ctggtcagcc tcttagaggc tgtgtgaccc 144855
   tgtgagcctc agtgcctcgt gctgtggatg gatgagaaac ctctgtgggt tccctcttcc 144915
   cctttcttga tggccgccac cctgtgttct cggagatcat taccctcaaa aggcctgccc 144975
   tgcacttaat gccagaacca ctgtgaggtt cgccctctta tcactttaag tttgaagaaa 145035
   ctgaggctca gagagatgaa atcacttgtc caagatcaca cagctgggag ggcagagcca 145095
   ggatctggac cccaggtggt cctggcccct gtgctgtgag cgttctgttt gtcacagtgg 145155
   actctgctcc ctggtgctac tcccgtctct ggccacagct cagaggtcag ccgtgtgcct 145215
   ggtcgtgggc ccccgataag atgagcaggg ctgtattggg ctgtgtcacg gtggaggtca 145275
   gccgtgtgcc tggtcgtggg ccccccgata agatgagcag ggctgtattg ggctgtgtca 145335
   cggtcggggt cagccgtgtg cctggtcatg ggcctcccga tgagatgagc agggctgtgt 145395
   cgggctgtgt cagagcattc agaccctcgc tgagatgagc aggtctgcgc tgggccatgt 145455
   cagggcatgc agaccctcgc tgctctttga gacccttctt gtggaagggc caggatggtc 145515
   gggacgcccc gtccactcac ctcatccctt atcccaccag gtc cat gac tac ctt    145570
                                             Val His Asp Tyr Leu
                                                             325
   cgg agc aag ctc tgt tcc ctg tac gag aac gac tgc att ttc gac aag    145618
   Arg Ser Lys Leu Cys Ser Leu Tyr Glu Asn Asp Cys Ile Phe Asp Lys
                   330                 335                 340
   ttt gaa tgt gcc tgg aac ggg agc gac ag gtaagccctg acctcagccc       145667
   Phe Glu Cys Ala Trp Asn Gly Ser Asp Ser
               345                 350
   gcacctcacc tcaccgtagg gagggtttct gccctgcagg ggtctgggct gggattccgg 145727
   tgacccgcag catggggcta ctcagcctca atgggtccag gtgtctgggt gaagcccacg 145787
   ctttccagag caggtccaac tctcagcgct cagattcaag gggcaggaca tgaaattctt 145847
   catcttctgt cactgaacct cacagccacg ttggcgcctg ccctatgggc agtagtggga 145907
   acatgtttag ttaattcagg gtccccggtg atgtgctccc ctctcccagc ttgtcggggg 145967
   cgagggctat agcccagcac ccggtcacca tcatccatcc acacctgtat gtcctgagac 146027
   agccctgcac ccctgtggct ttgaccatcg gtctactcac ccctcctccc atcaccacta 146087
   ctgtctccct ctcttcctgg tgacacccca ctcgggcccg ctgaggctca ggggcacctt 146147
   ggagctccta caccctccag ggcttgtcac aatccacaag tccagccgtc tctcaacccc 146207
   acctgcctgg aaagtggcgc cccagtgcca gaagtgagtt cctgtgtctc cctagcctgg 146267
   gctcagccca gggcgggcat ggacaagggg gctgtggcag gggctcctga cctgacctct 146327
   acccgtgggt ccttacctct gtgtctcttc tctgggattc ttcctccatt ctggaggtgg 146387
   gaaaatccct cttctgccct cccaaatcac atcagctttg tgctcagggt cctgccaggc 146447
   gtaagattct gaaatggaca agcctactct ccatctgtga gtttcgatct cagaagctga 146507
   gaggtggcct ctcagtgtct cctacagctg cttcctcaag gacaggatgc ctctttgtcc 146567
   agccgcccag attcagaagt gggtctccag atgatacaca gtgtggagat aaagactaca 146627
   ctggctgcta gatcagaata ccacctgtca ggagcccatg tactgtcacc tcctccccgc 146687
   agtcctgtcc agtgtggtgg gcaaggaggg tggagtgaga gccagcagcc ctgacttggg 146747
   catcacctgg tggggggctg tccaccctgt ggatggcatc agtcaacatg acaggtctgg 146807
   gctctcccag acctctgagg gtggctggca ctgtggtggt catgtgagag ctgccgcact 146867
   atgactctct gtggctctgg gcatagggct gggaccatca gggttggtgt gtgggatgag 146927
   gggagggctg gacatggcag agacaggacc aggagggagc cccctggaag cagggctgga 146987
   tccaagtggg gggccaagtc attgtgtcca gggaaggaga tttctgagag agttgccaac 147047
   atcctggagc tcccccagcc cgcaggggtc tctcagcaga gcccgagctc aggcaagggg 147107
   ctggcatggt gatcacaggg ggccactaaa ggatgcttag aaaaccagga tggaggcccg 147167
   acccggggct gggctggcac agctgggtca gcaggacaca ggaccttctc tctaggccct 147227
   gcccccagat agatcccaga cacccccagc agacagggct cctcccatgc tgctgagctg 147287
   catttggggt tccctggtgc agtgggtccc aagagggtct atccaaatcg gacgagaggg 147347
   acctgcagct gtaacaagct gattccagct tttatgtgcg ttttgcgggg taggtccccg 147407
   ctggctgcga cccactgccc gtccttcctt caagctgcca ccagggggca cccgcggcca 147467
   ggtgatgcct gctcccagga ctggagaagc cgccaagcat ccccaggctg acagtggtgt 147527
   ctaggcctgg gctctcctgt cctgcctccc acccgccact tcctgcgtgc actttacacg 147587
   ccagccacgc cctgtcctag tggtccccca cccgcctcac tgtctctcgc tccacagctg 147647
   gctctgctct gcccggctgg aacctctgtc cttgtttgcc tcccgcaatg ggtggggtgc 147707
   cctggggggc tactatgact caacctgttc tgagcccttc actggggacc tcaggtgtgt 147767
   ccagtggctg tgggtgtttc tagaaggcat agaggtgtgc cacctcccag ttcactttga 147827
   gcactgttct gagaacaaca tgccccatgg tcaggggtcc caagataaac agaccctggg 147887
   tcctgccttc cagggcctca gaggctcagg agagaagcaa ggattccccc caggttccca 147947
   catccttgca gaccaaagca aactcgaatc ctggcaggct cccagttgct gccctcttat 148007
   tcctggtgac cccctctgac agttggtccg gcccgcagag cgccgctgct gccgcgtggt 148067
   ggctgcccga tggcccctgc cgtggggctt cctagcaccg gcagaacgca gatgggcagc 148127
   gtgctgtgga gaggcagcag ctctcctgct ggactctctc agtctctggt ccacattcta 148187
   ccctcgccac cgtgtgcttg aggaagcccc taagtcagct cggctggaaa gtggttctag 148247
   gaaactggcc ttcgtggccg ctttggggat gagaccatgt gtgatgcctt cagcaagatc 148307
   ccagttcgta tgcgcagggg tgccgcagtg atcctgcgtg gactatccta ttggcaggcc 148367
   tgcccttccc caggttacct acccggagga atcccgcagg ccctcccaca acaggcttca 148427
   acgccccctc ctccatgaag ttctccttga tctgtcctgc ctggggggag agatttgtgc 148487
   cagccgagcg tgctcgggtg cgggagtcaa acacacctac acttgctctg aggagtcctg 148547
   ggcaaggctc ccccaggggc ccatgctgta tccctagggc tgtttttttc tctccggccg 148607
   ttttcttctc tccatgcttc cccatctccg ctcctccctg cttcttctta cacactggcc 148667
   tcatcctctc cctcttcaga gatgaccccc aaatcattcc cctttccatt atcctcagcc 148727
   agccaacccc tcccagggac tgtgtaaaac tctcatggaa ggatctgatt ggctctgtgt 148787
   gggtcacttg cccacttttt gcaccaatca gcatggacag ggatatcaca tgcccaagtt 148847
   ggccaggcca aggggcgagg agagcactgt gattgaccgc tcacagggat agggagtgca 148907
   gtggcgccat ctcggctcgc tgcaacctcc gcctcctgga gtcaaacact tctcctgccg 148967
   cagcctccca agtagctggg attacaggcg ccacgggtgg cgagggcagg agcctttctt 149027
   tccccaaagg aaagaggagc ggagcactgg ggctgaaagc agccggcgtt gnggtcccac 149087
   atgcagatga ggctaggaga ggtgaagcag ctcccctgcc cttttccctg ttaagggaac 149147
   cttctggaat ttaagaaacc tgcctgaatg tgaggaatgg ctctcatggt ggtgcggcct 149207
   ggtgctctag gatgagaggg gcccctccct ctcccccaga gcacgtgtca gctgaattcc 149267
   acacccgaac agggggagga tcaggaactg tgtgggttgc aaatgacttt aattatgtcg 149327
   ctctccttcc actaaatgga tcagaagaac cagcattgtg tgaaatcacc caggttcatt 149387
   ctgtgaactg ttccctgaag aacaaagggg gctgcctccc cactgtgctg gcgcggggag 149447
   ggtgtggcct cccccagtca gcctgtggcg cctgggcagg gcccttctgt gggactcgtt 149507
   cacccggccc ccctcgcatg ctgcttccct tgtcccaggc tctgagtcac atataaagcg 149567
   ggtgcggctt tggtcacatg gacgattagg atcgaggagt acctggtaaa taacaagaag 149627
   caaagtgcct caagcccagc actgtgcggg gtgttcagcc tatgtcccct gattcacgct 149687
   caccaggggc tgcgttccct tgttgatccc agttcttata gaaaactaag agagtgcgca 149747
   tgtcagggta gggcagagct gggatccgga cccaggcgga ctggccagag cccaagcccc 149807
   tgactgcagt gccggagctc acgtgtgagc ttttgtcctc tgtgcataac ccctggagac 149867
   ccagggatcc acctggttct gtcacagagc agtgtcaccc tcaggaagcc cagctcagct 149927
   tccaaaagga aacaggattt cctccctgaa gagccttcag caggacaact tctttatggt 149987
   cgcttttcgt aacatcctct tcctccccag ttaccttgca cctcacagct gctccagggc 150047
   cctgcagagg ccaaacccca aaaccctccc tctgggcgca ggcccacaga actgtgcttt 150107
   ctctcctgcc tcttactatg tggatgaact tacccttcct cccaccggca ggaacccctt 150167
   ctccttagga gcagggagag cagagaaatg gtgtggaatt ctctttagcg ggacggtgag 150227
   ggcagcgagc ccctgcacgt cagcactgct cgccccgacc ctcatggcga tgcatgtttg 150287
   tttattcgcc cgagtcctcg ggacaggcgg gcccctggag ggaagcaggg cctggtggtt 150347
   ttctgtccca gctgcttcca tgtctacctc ctgctgtggt tgcacagtct gtttggaagg 150407
   ccagagcttt ctctcctgga ctaacagaaa ttacaggagg gtcttttctt ttcttttctt 150467
   ttcttttttc gagacggagt tttgctctta ttgcccaggc tggagtgcag tggcgcgatc 150527
   ttggctcact acaaccttgc ctcctggatt caagcaactc tcctgcctca gcctcctgag 150587
   tagctgggat tacaggtgcc caccaccatg tccagctaat ttttgtattt ttagtagaga 150647
   cagggtttca tcatgttggc caggctggtc tcaaactcct aacctcaggt gatctgcccg 150707
   ccttggcttc ccaaagtgct gggattacag gtgtgagcca ccacgcctgg ccaggagggt 150767
   attttcatga tgattggggc agaggataaa aaggatctta gctcctggcc ccaaagtctg 150827
   catgttggtg gcagctgaca tgtgaggggg tataagagac cccaagatac aggggaatag 150887
   aggcaggtgt ggtttccctt tccagggagt gatggctctg taggctctgg gattgctttt 150947
   tcatttgttt gttgttttgg gacagagttt tactctgtca cccaggctag actgcagtgg 151007
   taaatcacag ctcactgcag tctctgcctc cccaggctca ggtgatcctc ctgcctcccc 151067
   aggctcaggt gatcctcctg cctcaacctc ctgaatagct gggactacgg tcatttttaa 151127
   tttttttttg tagaggtgga gtctcgctat gttgtccagg ctggtcttga actcctaggc 151187
   tgaagcaatc ctcccaccgg ggcctcccaa agtcctggga ttatgggcgt gagcccccac 151247
   acctgggctt atttctcgag aaggggcttg tgctcctcct cacctgatgc ctctccttct 151307
   cccaccag c gtc atc atg acc ggg gcc tac aac aac ttc ttc cgc atg    151355
                Val Ile Met Thr Gly Ala Tyr Asn Asn Phe Phe Arg Met
                            355                 360
   ttc gat cgg aac acc aag cgg gac gtg acc ctg gag gcc tcg agg gaa   151403
   Phe Asp Arg Asn Thr Lys Arg Asp Val Thr Leu Glu Ala Ser Arg Glu
   365                 370                 375                 380
   agc agc aag ccc cgg gct gtg ctc aag cca cgg cgc gtg tgc gtg ggg   151451
   Ser Ser Lys Pro Arg Ala Val Leu Lys Pro Arg Arg Val Cys Val Gly
                   385                 390                 395
   ggc aag cgc cgg cgt gat gac atc agt gtg gac agc ttg gac ttc acc   151499
   Gly Lys Arg Arg Arg Asp Asp Ile Ser Val Asp Ser Leu Asp Phe Thr
               400                 405                 410
   aag aag atc ctg cac acg gcc tgg cac ccg gct gag aac atc att gcc   151547
   Lys Lys Ile Leu His Thr Ala Trp His Pro Ala Glu Asn Ile Ile Ala
           415                 420                 425
   atc gcc gcc acc aac aac ctg tac atc ttc cag gac aag gta aac tct   151595
   Ile Ala Ala Thr Asn Asn Leu Tyr Ile Phe Gln Asp Lys Val Asn Ser
   430 435 440
   gac atg cac tag g tatgtgcagt tcccggcccc tgccacccag cctcatgcaa 151648
   Asp Met His
   445
   gtcatccccg acatgacctt cacgaccgca atgcaaggag gggaagaaag tcacagcact 151708
   gatgaggaca gctgcagagg tggcagtgtg tggacacagg aagtttgggc cccctccctg 151768
   ccccagcttt cctaggccag aattgtgttt ggcagtaatt gtctgtttaa aaaaataaaa 151828
   ag 151830
<210> 38
   <211> 447
   <212> PRT
   <213> Homo sapiens
<400> 38
<210> 39
   <211> 19
   <212> DNA
   <213> Artificial
<220>
   <223> primer 99-24169/139
<400> 39
   ctggctgagg cctccttgt 19
<210> 40
   <211> 19
   <212> DNA
   <213> Artificial
<220>
   <223> primer 24-257/320
<400> 40
   gtccttctga tggcctgcc 19
<210> 41
   <211> 19
   <212> DNA
   <213> Artificial
<220>
   <223> primer 99-24175/218
<400> 41
   caagctggat tcgcaatca 19
<210> 42
   <211> 601
   <212> DNA
   <213> Artificial
<220>
   <223> amplicon 30-4
<220>
   <221> allele
   <222> (301)..(301)
   <223> polymorphism 30-4/58
<220>
   <221> allele
   <222> (301)..(301)
   <223> biallelic marker 30-4/58
<400> 42
   tgaagccgac gttgtcgccg ggcagagctt cgctcagagc ctcgtggtgc atctccactg   60
   acttcacctc agtggtgatg ttcactggcg caaaggtcac caccatgccc ggccgcagga  120
   tgccggtctc cacccggccc acgggcaccg tgccaatgcc tgcagagggg agggggtgtg  180
   aggggaaggt ggggcccgag gggatgctgg ggcaggatat tcggggacag agcctggaaa   240
   ccaacaaagc ctgggactgg atccccccga caggcctggg ggttggggcc acatgggcgg   300
   rgtgcagggg aagggaggcc agggacaagg gcagacacag agattccaag ggaagtgggg  360
   gctctcccac ccagctgggg aaataagagg ctgagcagca gagctcccag gaacccacgg  420
   aaaagccaca gggacagaga agcgggagga tgggcagaga ggggctgtct gaacctgggg  480
   tcccatcctt gcccccggag agcactttcc ctcaaaggag gcactatggg acccctcctt  540
   tgtctgagga ctcctccctg tgagtgtggg cggggcgact gactgcttct gcctggggcc  600
   t                                                                  601
<210> 43
   <211> 601
   <212> DNA
   <213> Artificial
<220>
   <223> amplicon 30-2
<220>
   <221> allele
   <222> (301)..(301)
   <223> biallelic marker 30-2/62
<400> 43
   gctaacagga gaaagcacct gcacactagc tccccgacgc tggaacaggg ccatggccct      60
   gcgccccaca ctccagctcc actctccaca ggaaaaggct cccagaatcc agccactcag     120
   tgtgtggggg caggggccct gctgacttag aaacaagtgg cacattgatc cgcattcaaa     180
   cttgccagcc aatcaaccac agccccgcgc acagactctc ccaggtggga ctgagggggt     240
   ctcccctgtc cttggcaggg gcgtctcccc cacgcacccc cagtcccgtc ctctccacag     300
   rctccagatg cccacatccc cagaacactc aatgggacaa ctcagagcag gttacagaga     360
   aagaaaagcc acacaagctc accaagggca cgctatttca gaagtgcctt ctcctcctgg     420
   aaatgtcgac cccaaagctc tcactgggaa acctctggcc tggccccggg aagcgacagg     480
   cgcaggtttg gggctgaggc cgtcccagca gctctgtggc ctgccagacc tcagagcact     540
   cccatcaggg gccacaagag cagagagctc ttcagcccca tgttctcctg gacgaattaa     600
   a                                                                     601
<210> 44
   <211> 601
   <212> DNA
   <213> Artificial
<220>
   <223> amplicon 30-17
<220>
   <221> allele
   <222> (301)..(301)
   <223> biallelic marker 30-17/37
<400> 44
   caccacccgc cacctgcctc caggagcact gcagcctcgc cagtcagccc actttgggct      60
   ctgtctccag ggatataggg gctggatgga cccgtctcct gaggccagca gaggctccac     120
   gccagggtcg gtggcagggc tggcacaggg gaaccaggag gcgccgctgg cttcaccatc     180
   ttagctacgg cagcccattc ccctgagcct cctggcctgg gcaacagtgg ctcgcatggc 240
   cagcccaccg tgccctccag ggtcagtagc gtctattctg gcggccagca gggctggaga 300
   rtcttgggac tgttgagacc ctcccccaac ctccctgagc ctccgggcac agatgtgaaa 360
   agggtgccca ctgcagtcag cactcaaccc ccacagcgtc cagggaggga gaggggccac 420
   cgggggctga cccctgccca ttctgcagac aaagccacca ccctgccagg gctcaagagg 480
   gaagaaaatg gggagggggc catttgagca aatgagccca cccgtgagca aggtggaggg 540
   acagcacagt ctggcaggat ggggtctcgg tcactggggg gcctggggcc cctggaactc 600
   a 601
<210> 45
   <211> 601
   <212> DNA
   <213> Artificial
<220>
   <223> amplicon 30-7
<220>
   <221> allele
   <222> (301)..(301)
   <223> biallelic marker 30-7/30
<400> 45
   gccgagatcc cccgagtccc gaggcgtcgc gtgcttgggg acgtcaggag ccgatggtac 60
   aggctcgctc aggaccccag tcctgagtcc acacccctgc actgcctgag gccaacacac 120
   cgtgcccatg ggggccaggg gtgctcagag tcctggtgct gtgggtgcct ctgtcccaac 180
   ggcctctggt ccccatccca acaacaaaag cacaggtggt cggggagaac cggacggggg 240
   ccaggggagc acatgggcac aggctcagcg ggactcctgg aatgttctct ctttctccac 300
   ygcacgagcc atttcaaagg caagaatagg cccctcctga ccccgctcag gcaggcctca 360
   gggcaagtgg gagtcactgg aagactcaat tcctctctct gcgtttccac ccgaggcagg 420
   tccagtcacc agagagagaa gcagccacct cctttctcac ggcagctggc aaagcaccgg 480
   gtggaggaca gagccggtcg gcccaactgt agcttcgggg ctgcccttgg ctggtctctg 540
   ggcagagccc ggtgctgagg gcttgcagtg ggaaaggcac agcttgagga atgggcatca 600
   g 601
<210> 46
   <211> 601
   <212> DNA
   <213> Artificial
<220>
   <223> amplicon 30-84
<220>
   <221> allele
   <222> (301)..(301)
   <223> biallelic marker 30-84/37
<400> 46
   tgtgctaaaa catagtggct taaaaataat gataaccatt tatcgtctca gtttctgcag      60
   ctcaggagtc ggacggcacg cagccgatct ccactcccaa cgtgcagggc ctctgccata     120
   agccttgaag gcactcattc actcacgcat cgggggctag tacaggctgt gacagaggcc     180
   tgagctggaa ctgttgacca ggacacacac atggccatgt ggcctctggg cttcctcaca     240
   gcatggtgtc tggattccag gagttggcat cctgagaaac aaccatgcag aagcagccct     300
   rtggatcctg gcctggcctt ggagtcaggc agtgtcactc ctgtgccttc taacctgggc     360
   ccccgggccc aaggggagga aatggagacc ccacctccca gtggagggaa ggcaaggtcc     420
   cactgtgggg gtagcacatg ggatacaccc atgtggctgc cgctggagac gttagtttgc     480
   cacacccgtt tcttctacgt gaacatttgc ctgcatctca ccttctaact cctgggtgct     540
   gtttgtccat tttcactaca ccaggggccc ccacagtata tgcagaccag gtttcctggc     600
   c                                                                     601
<210> 47
   <211> 601
   <212> DNA
   <213> Artificial
<220>
   <223> amplicon 30-15
<220>
   <221> allele
   <222> (301)..(301)
   <223> biallelic marker 30-15/54
<400> 47
   gatgctgaat aagccaggaa gaagatccgg ctaaatgttg gcacattcta aagtctacgt      60
   gaggccagtc tgaccctggg aacctccatg aagacatggg cgtggagggt ctgccttttg     120
   cagggcccac caggggctca caggaaaggt cgtggaaaat tacaagaaat cttccctctg     180
   gcactagcgg gtgaggggaa tggaagccac cgccagacag caccatctcc tcaccctcct     240
   gtgaagcaca agactcactt gcagagggaa gagcgcagaa accgtcaccc caggacgctg     300
   mggttgaacg agaggaagcg agaatggaga agccctggcc ctggggaaca ggatggaaaa     360
   cgcttggctc agctccgtgg ctgcgaagga accggcgcgc tcgcggaggc cacaccccga     420
   gacccgagga cacagtgcct gcctgagatg gagccagaaa cattctccac cctttcacgc     480
   aagactaaca agggctccat gaaaataaaa ctggaagagc tgaaagagaa gcattctccc     540
   tgggtgtgaa accaagaaaa gacacaaagc caaggaaaag ccattgagaa aacacctggc     600
   a                                                                     601